# EUROPÄISCHE PATENTANMELDUNG

(11) **EP 1 310 567 A2**
(43) Veröffentlichungstag der Anmeldung: **14.05.2003**
(21) Anmeldenummer: 02090348.0
(22) Anmeldetag: 02.10.2002
(51) Int. Cl.: C12Q 1/68

(54) **Nukleinsäure-Array**

(30) Priorität: 09.11.2001 DE 10155600
(71) Anmelder: oligene GmbH, 10117 Berlin (DE)
(72) Erfinder: Stuhlmüller, Bruno, Dr., 12514 Berlin (DE); Häupl, Thomas, Dr., 15537 Erkner (DE)
(74) Vertreter: Wablat, Wolfgang, Dr.Dr.

(57) **Zusammenfassung**

Um Werkzeuge zur diagnostischen, prognostischen und therapieüberwachenden Analyse sowie zur Durchführung von Screeningverfahren für pharmakologisch wirksame Substanzen und Substanzklassen chronisch entzündlicher Erkrankungen, bakteriell induzierter chronisch entzündlicher Erkrankungen, der Arteriosklerose, der Tumorerkrankungen, der Organ- und Gewebstransplantationen, -und der Sepsis zur Untersuchung von Blut, Gewebe, aufgereinigten oder kultivierten Zellen zu schaffen, wird vorgeschlagen, selektionierte Monozyten-Makrophagen Gene zu verwenden.

## Beschreibung

Die Erfindung betrifft Werkzeuge zur diagnostischen, prognostischen und therapieüberwachenden Analyse chronisch entzündlicher Erkrankungen, bakteriell induzierter chronisch entzündlicher Erkrankungen, der Arteriosklerose, der Tumorerkrankungen, der Organ- und Gewebstransplantationen, und der Sepsis zur Untersuchung von Blut, Gewebe, aufgereinigten oder kultivierten Zellen.

Akute und chronische Entzündungsvorgänge im Blut und Gefäßsystem, sowie im Gewebe können zu pathologischen Ablagerungen, fibrotischen Umbauvorgängen und auch zur direkten Zerstörung von Geweben und Organen führen.

Die Zellen des Monozyten / Makrophagen-Systems sind an der Aktivierung und Aufrechterhaltung von Entzündungskaskaden im Blut und im Gewebe z.B. im Rahmen entzündlichrheumatischer Erkrankungen, bakteriell induzierter entzündlicher Erkrankungen, der Tumorerkrankungen, der Organ- und Gewebstransplantationen, der Arteriosklerose und der Sepsis wesentlich beteiligt. Bei diesen Erkrankungen sind Monozyten und Makrophagen hoch aktiviert, zeigen Veränderungen im Besatz ihrer Oberflächenmoleküle, treten mit anderen Zellen in Kontakt und sezernieren bestimmte Botenstoffe, die dafür sorgen, den Entzündungsvorgang zu unterhalten. Dabei kommt es neben unspezifischen Entzündungsreaktionen auch zur spezifischen Stimulation des Immunsystems.

Es können dabei sowohl durch den Krankheitserreger vermittelte als auch auf dem Boden anderer Ursachen (z.B. äußere, umweltbedingte Faktoren wie Strahlung, Toxine oder Allergene) oder zum Teil auch genetischer Veranlagungen autoaggressive Reaktionen auftreten. Diese können sich sowohl im Rahmen der unspezifischen, als auch der spezifischen Entzündungs- und Abwehrreaktion als überschießende Reaktionen entwickeln und zur Schädigung oder gar Zerstörung von Organsystemen führen. Den Monozyten und Makrophagen wird auch bei diesen autoagressiven Reaktionen eine wesentliche Rolle zugeteilt.

Die molekularen Abläufe in den Monozyten und Makrophagen, die zu einer solchen chronischen Entzündung und / oder Autoaggression beitragen, sind noch weitgehend ungeklärt. Ihre Untersuchung ist dringend erforderlich 1. aus diagnostischen Gründen zur Einteilung und pathophysiologischen Beurteilung der Erkrankung, 2. aus prognostischen Gründen zur optimalen Ausnutzung der therapeutischen Möglichkeiten und 3. aus therapeutischen Gründen, einerseits zur Überwachung einer bestehenden Therapie, andererseits zur Entwicklung neuer therapeutischer Ansätze.

Derzeitige entzündungshemmende Therapien für chronisch entzündliche Erkrankungen aus dem rheumatischen Formenkreis sind unspezifisch und können den Entzündungsprozeß meist nur begrenzt hinsichtlich Intensität und zeitlichem Verlauf aufhalten. In vielen Fällen schreiten diese Erkrankungen dennoch fort mit zunehmender Organschädigung, zum Teil bis hin zur völligen Organzerstörung.

Autoimmunerkrankungen und / oder entzündliche Erkrankungen können prinzipiell jedes Organsystem betreffen. Beispielhaft sind hier aufgeführt Erkrankungen des Skelettund Stützapparates (Rheumatoide Arthritis, reaktive Arthritis, Morbus Bechterew, Osteoarthritis und Abriebsynovitis), des Darms (Colitis Ulcerosa und Morbus Crohn), der Leber (Autoimmunhepatitis, chronische Virushepatitis, primär biliäre Zirrhose), endokriner Organe (Pankreas: Juveniler Typ-I Diabetes; Schilddrüse: Hashimoto Thyreoditis, Morbus Basedow), der Skelettmuskulatur (Polymyositis, bei Hautbeteiligung Dermatomyositis), des Herzmuskel (rheumatisches Fieber, dilatative Kardiomyopathie, virale Myokarditiden), der Haut (Sklerodermie, Psoriasis, Neurodermitis), der Lunge (Lungenfibrose, Goodpasture Syndrom, chronisch obstruktive Lungenerkrankung, Sarkoidose), des Gehirns bzw. des Zentralen Nervensystems (Multiple Sklerose) und des Herz-Kreislauf Systems (Vaskulitis, Arteriosklerose) sowie chronische Multiorganerkrankungen (Systemischer Lupus Erythematosus, Sjögren Syndrom, systemische Sklerose, Sepsis) und Tumoren.

Die rheumatoide Arthritis ist eine inflammatorische, chronisch entzündliche Gelenkerkrankung, welche zur fortschreitenden Zerstörung der befallenen Gelenke führt. Es weisen sowohl die im peripheren Blut zirkulierenden Monozyten eine Zellaktivierung auf als auch die weiterdifferenzierten Zellen des Monozyten / Makrophagensystems, die im Gelenk als synoviale Makrophagen und dentritische Zellen vorliegen. Dies spiegelt sich in vergleichbaren Transkriptions-Mustern für entzündungscharakteristische Proteine (Botenstoffe, Proteasen u.a.) wieder. Bislang konnte die rheumatoide Arthritis nur anhand der klinischen ACR-Kriterien und durch pathohistologische Untersuchungen des betroffenenen Gewebes diagnostiziert werden. Häufig ist die Erkrankung dabei schon relativ weit fortgeschritten und bereits eine irreversible Schädigung des Gelenks eingetreten. Therapeutische Maßnahmen kommen somit nicht selten zu einem zu späten Zeitpunkt zum Einsatz bei dem die autoagressiven Schädigungen bereits stattgefunden haben und irreversibel sind.

Durch Untersuchung der Genexpressionsprofile ist zu erwarten, dass eine neue molekulare Charakterisierung der Erkrankung möglich wird und damit eine Einteilung in Subgruppen nach pathophysiologischen Besonderheiten erfolgt. Ferner steht eine prognostische Vorhersage in Aussicht über die Agressivität im weiteren Verlauf. Dies würde bereits frühzeitig Einfluß auf die Wahl und Intensität der medikamentösen Therapie ausüben.

Hinsichtlich der therapeutischen Maßnahmen, die heute noch weitgehend unspezifisch sind, stellt sich in Aussicht, dass über die Kenntnis der molekularen Mechanismen der chronischen Entzündungen auch spezifische Kandidaten erkannt werden, auf deren Basis neue Therapiekonzepte entwickelt werden können. Dies kann einerseits durch biologische spezifische Substanzen, relevante Antagonisten, oder durch pharmakologische naturstoffbezogene oder auch chemisch wirksame spezifische Substanzen geschehen und, die direkt in den Entzündungskreislauf des Monozyten / Makrophagen Systems eingreifen.

Bei Tumorerkrankungen des blutbildenden Systems oder aber Tumorerkrankungen mit neoplastischen Veränderungen finden sich zahlreiche Areale mit infiltrierten Zellen des Monozyten/Makrophagen-Systems die charakteristische Genexpressionsmuster beinhalten. Diese Genexpressionsmuster beinhalten sowohl entzündungsspezifische Genexpresionen, zum anderen aber auch tumorspezifische Genregulationen.

Bei der Sepsis und der bakteriell induzierten Abriebsynovitis werden Zellen des Monozyten/Makrophagen-Systems durch bakterielle Infektionen vorwiegend über Lipopolysaccharide aktiviert und weisen wieder ein weitgehend gleichartiges entzündungsspezifisches Genexpressionsmuster wie nicht bakterielle entzündliche Erkrankungen, andererseits aber ein spezifiziertes bakteriell induziertes Genmuster auf. Somit sind auch die beiden Verlaufsformen der Abriebsynovitis ohne bakteriellen Auslöser von der rein bakteriell induzierten Abriebsynovitis in ihrem Genmuster zu unterscheiden.

Bei der Arteriosklerose werden Monozyten bereits im peripheren Blut aktiviert und dazu angeregt, an zerstörte, entzündungsspezifische Regionen der Arterien zu binden. Die Kommunikation durch Zell-Zellkontakt mit Endothelzellen trägt dazu bei die Aktivierung und Rekrutierung des Monozyten/Makrophagen-Systems zu unterhalten.

Von führenden Mikroarrayherstellern (Affymetrix, Clontech, Nanogene) werden derzeit kommerzielle Mikroarrays angeboten, die im Einzelset zwischen 4.000 bis 12.000 zufällige Gene beinhalten. Zur Transkriptionsgesamtanalyse aller Geneinheiten sind mehrere Sets, die insgesamt ca. 48.000 Genen abdecken, zur Untersuchung notwendig. Bei der Gesamtanzahl von ca. 40.000 Genen (= ca. 120.000 Einzelgenvarianten) des menschlichen Genoms zeigt sich hierbei eine verschwindend geringe Abdeckung hinsichtlich der Gesamtanzahl verschiedener Transkripte. Es ist offensichtlich, dass es sich hier um ein aufwendiges und teures Verfahren handelt, das mit einem sehr großen biometrischen Analyseaufwand verbunden ist und für den Nachweis krankheitspezifischer, zellspezifischer Gene eine geringe Trefferquote aufweist.

Es ist die Aufgabe der Erfindung, Werkzeuge zu schaffen, die zur diagnostischen, prognostischen und therapieüberwachenden Analyse sowie zur Durchführung von Screeningverfahren für pharmakologisch wirksame Substanzen und Substanzklassen der rheumatoiden Arthritis, anderer chronisch entzündlicher Erkrankungen, infektiös bedingter Entzündungen, Tumorerkrankungen, Arteriosklerose, Organund Gewebstransplantationen und der Sepsis geeignet sind.

Diese Aufgabe wird durch die Merkmale des Anspruchs 1 gelöst.

Dazu sind erfindungsgemäß Werkzeuge vorgesehen, die unter Verwendung der Sequenzen einer Auswahl von nachfolgend genannten Genen oder unter Verwendung der Sequenzen aller nachfolgend genannten Genen ausgebildet sind, auch unter Verwendung weiterer Gene, oder mit genannten Genen komplementärer RNA:

**Tabelle 1**

| **Zytokine und Faktoren und Liganden:** | |
|---|---|
| Interleukin-1α | (Acc.# NM_000575) |
| Interleukin-1β | (Acc.# NM_000576) |
| Interleukin-6 | (Acc.# AF372214) |
| Interleukin-8 | (Acc.# L19591) |
| Interleukin-10 | (Acc.# XM_001409) |
| Interleukin-13 | (Acc.# HSU62858) |
| Interleukin-15 | (Acc.# XM_003529) |
| Interleukin-16 | (Acc.# AF053412) |
| Interleukin-18 | (Acc.# E17135) |
| Angiopoietin-like factor (CTD6) | (Acc.#XM_001529,XM_042319) |
| Inhibin β-B (INHBB) | (Acc.# NM_002193) |
| Tumor-Nekrosefaktor-α | (Acc.# NM_000595) |
| Tumor-Nekrosefaktor-β | (Acc.# D12614) |
| Transforming Growth Factor-β (TGF-β) | (Acc.# XM_008912,NM_00660) |
| Latent TGF-β binding prot. LTBP4 | (Acc.# NM_003573,XM_008868) |
| Melanoma stimulating activity (MGSA) | (Acc.# X54489) |
| Chemokine Gro-α/MGSA | (Acc.# X12510,XM_003504) |
| Chemokine (C-X-C motif) ligand 16 | (Acc.# NM_022059) |
| Chemokine alpha-3 (CKA3) | (Acc.# NM_002993) |
| CC-Chemokine (SLC) | (Acc.# AB002409) |
| EBI-1-Ligand Chemokine | (Acc.# AB000887) |
| Small inducible cytokine subfamily A(SCYA21) | (Acc.# XM_048450) |
| Small inducible cytokine(SCYA21) | (Acc.# NM_002989) |
| Megakaryocyte stimulating factor | (Acc.# U70136) |
| Monocyte colony stimulating factor (M-CSF) | (Acc.# NM_000757) |
| Granulo-/Monocyte colony stimu. factor (GM-CSF) | (Acc.#: E01817) |
| Macrophage inflammatory Protein (MIP-1) | (Acc.# HUMMIP1A) |
| Makrophage inflammatory Protein (MIP-2 | (Acc.# AF106911) |
| Monocyte migration inhibitory factor (MIF) | (Acc.# L19686) |
| Monocyte Tissue factor | (Acc.# M16553) |
| Monocyte Chemoattractant Protein-1 (MCP-1) | (Acc.# S71513) |
| Monocyte Chemoattractant Protein-2 (MCP-2) | (Acc.# NM_005623) |
| Monocyte Chemoattractant Protein-3 (MCP-3) | (Acc.# X72308;S57464) |
| Fraktalin small inducible cytokine | (Acc.# NM_002996) |
| Stromal derived factor-1 (SDF-1) | (Acc.# HSU16752) |
| Insulin-like growth factor-5 bind. Protein | (Acc.# NM_000599) |
| | |

| **Rezeptoren, Ionenkanäle und assoziierte Proteine:** | |
|---|---|
| Angiotensin Rezeptor-II Homolog (ATR-IIh) | (Acc.# L48211) |
| Toll-like Rezeptor-2 | (Acc.# XM_003304) |
| Toll-like Rezeptor-4 | (Acc.# XM_005336) |
| Opoid-Rezeptor Kappa | (Acc.# XM_011716) |
| Interleukin-1 receptor | (Acc.# XM_002686) |
| Interleukin-2 receptor α-Untereinheit | (Acc.# XM_043149) |
| Interleukin-2 Receptor β-Untereinheit | (Acc.# XM_009962,M26062) |
| Interleukin-2 Receptor γ-Untereinheit | (Acc.# XM_047675) |
| Interleukin-7 Receptor | (Acc.# AH007043,NM_008372) |
| Interleukin-8 receptor α (IL8RA) | (Acc.# XM_058007) |
| Interleukin 8 receptor β (IL8RB) | (Acc.# NM_001557 |
| Fc-Rezeptor-I | (Acc.# J03619,AF200220) |
| Fc-Rezeptor-II | (Acc.# M28696,M28697) |
| Fc-Rezeptor-III | (Acc.# Z46223,Z46223) |
| Tumor-Nekrosefaktor-α Rezeptor | (Acc.# S63368) |
| C-Chemokine (C-C motif) Rezeptor-5 (CCR5) | (Acc.# NM_000579,XM_030397) |
| C-Chemokine (C-C motif) Receptor-7 (CCR7) | (Acc.# XM_049959) |
| Chemokin-X-C-Rezeptor-4(CXCR-4) | (Acc.# NM_003467) |
| Progesterone Recept.-assoc. Immunophilin(FKBP54) | (Acc.# U42031) |
| Partial p58 gene for NK receptor | (Acc.# AJ000542) |
| Vascular endothial growth factor | (Acc.# AY047581) |
| Vascular endothial growth factor-β | (Acc.# BC008818) |
| Calcium activated potassium channel (KCNN3) | (Acc.# AF031815,AY049734) |
| G protein-coupled cytokine receptor EBI1 | (Acc.# L31581) |
| G protein-coupled cytokine receptor EBI3 | (Acc.# XM_012857,L08187) |
| EBI3-associated protein | (Acc.# U41806) |
| | |

| **Membranproteine und assoziierte Proteine:** | |
|---|---|
| CD14 | (Acc.# XM_003822) |
| CD68 | (Acc.# XM_008237) |
| CD69 | (Acc.# BC007037) |
| CD11b | (Acc.# J03925) |
| Adhesion receptor CD44 | (Acc.# M31165) |
| Actin binding coronin like protein (HCORO1) | (Acc.# U34690) |
| Integral membrane protein | (Acc.# L32185) |
| Epithelial membrane prot.-3 (EMP-3) / HMPMP-1 | (Acc.# X94771,U87947) |
| Mac-2 binding protein | (Acc.# L13210) |
| Integral membrane protein E16 | (Acc.# M80244) |
| HLA-D II beta chain | (Acc.# X03066) |
| Desmin | (Acc.# HSU59167,XM_002601) |
| Fibronectin precursor | (Acc.# X02761) |
| Adducin 1α | (Acc.# X58141,NM_014190) |
| HLA DRB1 | (Acc.# X88971) |
| Integrin-α 5 subunit | (Acc.# X06256) |
| Integrin cytopl. domain assoc. protein (Icap-1α) | (Acc.# AF012023) |
| Integrin cytopl. domain assoc. protein (Icap-1β) | (Acc.# AF012024) |
| Titin | (Acc.# X69490,NM_003319) |
| Thrombospondin-1 (TSP-1) | (Acc.# XM_007606) |
| Semaphorin-3 | (Acc.# AB000220) |
| Semaphorin-F Homolog | (Acc.# U52840) |
| TSP-2 | (Acc.# NM_003247) |
| TSP-1 / Semaphorin-5a Homolog | (Acc.# NM_003966) |
| VCAM-1 | (Acc.# X53051) |
| Periplakin (PPL) | (Acc.# XM_032727,NM_002705) |
| Envoplakin (EVPL) | (Acc.# XM_008135) |
| Peripheral myelin protein 22 (PMP-22) | (Acc.# XM_052499) |
| | |

| **(Proto)-Onoko-, Tumor-Suppressor-, Differenzierungsgene & assoz.Proteine:** | |
|---|---|
| H19 RNA | (Acc.# M32053) |
| Tumor suppressor Brush-1 | (Acc.# S69790) |
| Pim-2 Protoonkogen | (Acc.# U77735,XM_010208) |
| HOX-B3 | (Acc.# N70814) |
| MEL-18 | (Acc.# D13969) |
| c-fos | (Acc.# V01512) |
| c-jun | (Acc.# NM_002229) |
| c-myc | (Acc.# AH001511) |
| c-myc related oncogen (pHL-1) | (Acc.# X54629) |
| c-Ret tyrosine kinase receptor ligand 2 (RETL2) | (Acc.# U97145) |
| c-Ret tyrosine kinase receptor ligand 1 (RETL1) | (Acc.# U97144) |
| jun-B | (Acc.# XM_009064) |
| c-Jun activation domain binding protein | (Acc.# U65928) |
| Desmoyokin/AHNAK | (Acc.# X74818,M80899) |
| Rad mRNA | (Acc.# L24564) |
| PTEN | (Acc.# AH005966,XM_005867) |
| c-ras homolog gene family, member B (ARHB) | (Acc.# XM_002689,NM_004040) |
| Transforming activity oncogene (TRE-2) | (Acc.# X63596) |
| Transforming activity oncogene (TRE-17 | (Acc.# HSTRE213) |
| Kruppel-like fetal globin gene activator (FKLF) | (Acc.# AF272830) |
| c-fos related antigen (fra-1) | (Acc.# X16707) |
| c-fos related antigen (fra-2) | (Acc.# X16706) |
| | |

| **Akut Phase Protein:** | |
|---|---|
| Large-Ferritin Untereinheit | (Acc.# M11146) |
| Small-Ferritin Untereinheit | (Acc.# NM_000146) |
| | |

| **Enzyme, Enzym-assoziierte Proteine und Inhibitoren:** | |
|---|---|
| Activation-induced cytidine deaminase | (Acc.# AB040431,NM_020661) |
| Phospholipase-C | (Acc.# XM_041310) |
| Prostaglandin G/H Synthase | (Acc.# S36271) |
| Prostaglandin-Endoperoxide Synthase-1 | (Acc.# NM_000962) |
| Cyclooxygenase-1 | (Acc.# HSU63846) |
| Cyclooxygenase-2 | (Acc.# M90100) |
| Endothelin-1 (EDN1) | (Acc.# NM_001955) |
| Endothelin-1 (EDN2) | (Acc.# NM_001956) |
| Clustrin (complement lysis inhibitor, SP-40,40) | (Acc.# XM_027447,X14723) |
| Fettsäure Desaturase 1 (FADS1) | (Acc.# AF084558) |
| Cysteine dioxygenase 1 (CDO-1) | (Acc.# U80055) |
| Histidine biosynthesis protein | (Acc.# NM_007016) |
| Chitinase 1 | (Acc.# NM_003465) |
| Chitinase precursor | (Acc.# AF290004) |
| L-glycerol-3-phosphat: NAD oxidoreductase | (Acc.# L34041) |
| Alcohol dehydrogenase class I gamma subunit | (Acc.# M12272) |
| Procarboxypeptidase B1 | (Acc.# NM_001871) |
| Phosphoenolpyruvate carboxykinase (PCK1) | (Acc.# XM_009672,L05144) |
| Lysozym | (Acc.# BC004147) |
| Transaldolase | (Acc.# NM_006755) |
| Thymosin-β4 | (Acc.# M17733) |
| Metallothionein 1L (MT1L) | (Acc.# NM_002450) |
| Manganese-superoxide dismutase (Mn-SOD) | (Acc.# S77127) |
| Superoxide Dismutase 1 | (Acc.# K00065) |
| Superoxide Dismutase 2 | (Acc.# NM_000636) |
| Superoxide Dismutase 3 | (Acc.# NM_003102) |
| Copper/zinc-superoxide dismutase (Cu/Zn-SOD) | (Acc.# M13267) |
| Catalane | (Acc.# ) |
| Monoamine oxidase-A (MAOA) | (Acc.# M68840,XM_055485) |
| Fatty acid synthetase | (Acc.# U29344) |
| Glutathion peroxidase | (Acc.# X13710) |
| Glutathion peroxidase 3 | (Acc.# NM_002084) |
| Glucocerebrosidase | (Acc.# M16328) |
| Induzierbare Nitric oxide Synthase | (Acc.# AB022318) |
| Transglutaminase 1 (K polypeptide) | (Acc.# XM_007310) |
| Transglutaminase (TGase) | (Acc.# M55153,SEG_HUMETG)* |
| α-1-Antitrypsin | (Acc.# HSATPR1) |
| Protein Tyrosin-Phosphatase | (Acc.# U27193) |
| Carbonic anhydrase precursor(CA 12) | (Acc.# AF037335) |
| Metallothionein-IG gene (MT1G) | (Acc.# J03910) |
| Lymphocyte phosphatase assoc. Protein (LPAP) | (Acc.# X97267,AA011257) |
| Flap Endonuclease 1 DNA repair gene (FEN1) | (Acc.# AC004770) |
| Flap structure-specific endonuclease 1 (FEN1) | (Acc.# L37374, XM_043386) |
| | |

| **Kinasen, Protein Kinasen (PKN) und PKN-Inhibitoren:** | |
|---|---|
| Protein Kinase C-alpha Untereinheit | (Acc.# X52479) |
| Protein Kinase C-beta-1 Untereinheit | (Acc.# XM_047187) |
| Protein Kinase C-beta-2 Untereinheit | (Acc.# M13975) |
| Protein Kinase C-gamma Untereinheit | (Acc.# M34182) |
| Protein Kinase C-delta Untereinheit | (Acc.# D10495) |
| Protein Kinase-C Inhibitor | (Acc.# U51004 |
| Ik-Kinase-κ | (Acc.# AF029684 |
| PI3-Kinase | (Acc.# Y13892) |
| MAP Kinase-11 | (Acc.# XM_035889) |
| p38 MAP Kinase | (Acc.# AF031135) |
| p38 MAP Kinase interacting protein | (Acc.# XM_035930) |
| Serin/Threonin Kinase | (Acc.# AB015982) |
| Thyrosin Kinase-1 | (Acc.# XM_002037) |
| Thyrosin Kinase-2 | (Acc.# XM_005480) |
| Non-receptor protein tyrosine kinase tyk2 | (Acc.# X54637) |
| Mitogen- and stress-activated protein kinase-1 | (Acc.# AF074393) |
| Mitogen- and stress-activated Protein Kinase-2 | (Acc.# AF074715) |
| Casein Kinase 1, alpha 1 (CSNK1A1) | (Acc.# NM_001892,L37042) |
| Thyrosine kinase 1 (TIE-1) | (Acc.# XM_002037) |
| Thyrosine kinase 2 (TIE-2) | (Acc.# XM_005480) |
| | |

| **Differenzierungsgene:** | |
|---|---|
| WNT-6 | (Acc.# AY009401,AB059570) |
| WNT-13 | (Acc.# Z71621) |
| BMP-4 | (Acc.# M22490) |
| | |

| **Proteinasen, Matrixmetalloproteinasen (MMP) und MMP-Inhibitoren:** | |
|---|---|
| Cathepsin-B | (Acc.# XM_035662) |
| Cathepsin-G | (Acc.# M16117) |
| Cathepsin-K | (Acc.# NM000396) |
| Cathepsin-L | (Acc.# NM_001912) |
| Cathepsin-S | (Acc.# M86553) |
| Matricx metalloproteinase-1 (MMP-1) | (Acc.# NM_002421) |
| MMP-3 | (Acc.# X05232) |
| MMP-9 | (Acc.# XM_009491) |
| Disintrigin Protease | (Acc.# Y13323) |
| Tissue inhibtor of MMP type 1 (TIMP-1) | (Acc.# NM_003254) |
| TIMP-2 | (Acc.# NM_003255) |
| TIMP-3 | (Acc.# MM_000362) |
| TIMP-4 | (Acc.# NM_003256) |
| Serin Protease like mRNA | (Acc.# M17016) |
| | |

| **Apoptose- und Zellzyklus Regulatoren:** | |
|---|---|
| Annexin A-2II | (Acc.# BC001388) |
| Growth arrest DNA-damage-induc. prot. (GADD45) | (Acc.# M60974) |
| Growth arrest DNA-damage-induc. prot.α(GADD45A) | (Acc.# XM_056975,XM_040594) |
| Growth arrest DNA-damage-induc. prot.β(GADD45B) | (Acc.# NM_015675,AF087853) |
| Growth arrest DNA-damage-indcu. prot.g(GADD45G) | (Acc.# NM_006705) |
| Lymphocyte G0/G1 switch gene (GOS-3) | (Acc.# L49169) |
| | |

| **Signaltransduktions-Regulatoren:** | |
|---|---|
| STAT-1 | (Acc.# NM_007315) |
| STAT-4 | (Acc.# XM_002711) |
| Adenylate kinase 1 (AK1) | (Acc.# NM_000476) |
| Inositol 1,4,5-trisphosphate 3-kinase (ITPKC) | (Acc.# XM_047369,XM_047368) |
| Phosphatidylinositol-3'-kinase (PI3K) | (Acc.# Y11312) |
| | |

| **Transkriptionsfaktoren, Translationsfaktoren und assoziierte Proteine:** | |
|---|---|
| Transcription factor AREB6 | (Acc.# D15050) |
| Transcription factor 8 (TCF8) | (Acc.# XM_030006) |
| Nuklear factor kappa-B | (Acc.# M58603) |
| AP-1 | (Acc.# AB015319,AB015320) |
| PU.1 | (Acc.# X66079) |
| SPI-B | (Acc.# X66079) |
| v-maf musculoaponeurotic fibrosarcoma (MAFF) | (Acc.# XM_039249,XM_039250) |
| Zinc finger transcription factor (GKLF) | (Acc.# AF105036,AK026253) |
| Zinc finger Protein | (Acc.# M80583) |
| CCAATA enhancer binding Protein-beta | (Acc.# NM_005194) |
| RNA-polymerase II elongationsfactor | (Acc.# L47345) |
| Translation elongation factor-1 α-1 (EEF1A1) | (Acc.# BC009733) |
| Translation elongation factor-1 α-2 (EEF1A2) | (Acc.# XM_028863) |
| Translation elongation factor 2 (EEF2) | (Acc.# NM_001961) |
| L1-Element (L1.20) | (Acc.# U93569) |
| Leukemia Zink Finger PLZF | (Acc.# AF060568) |
| Activating transcription factor 3 (ATF3) | (Acc.# XM_016795,XM_034219) |
| Zinc finger transcriptional regulator (GOS-24) | (Acc.# M92843) |
| TGF-β-inducing early growth response 2 | (Acc.# AA427597) |
| SP1-like zinc finger transcript, factor(TIEG2) | (Acc.# AF028008) |
| snRNA activating protein complex | (Acc.# AF032387) |
| oct-binding factor-1 (OBF-1) | (Acc.# Z49194) |
| Early Growth Response protein 1 (EGR-1) | (Acc.# R75775) |
| | |

| **Ribosomale- / Ribonukeäre Regulatorgene und assoziierte Proteine:** | |
|---|---|
| hnRNP pseudogen(gp43) (Position: 97.026-98.073) | (Acc.# AL034397) |
| Ribosomal protein L19 | (Acc.# XM_002758) |
| Ribosomal protein S13 | (Acc.# XM_039215) |
| Histon-H1 (0) family mRNA | (Acc.# X03473) |
| H4-histone family, member H (H4FH), mRNA | (Acc.# NM_003543) |
| | |

| **Andere:** | |
|---|---|
| IER-3 | (Acc.# NM_003897) |
| Endoplasmatic glykoprotein Gp36 | (Acc.# U10362) |
| Natural resist.-assoc. Macroph.protein (Nramp1) | (Acc.# D50402) |
| Calgranulin - S100A12 protein | (Acc.# XM_001682,NM_005621) |
| 14-3-3 gamma Protein | (Acc.# AF142498) |
| Serum amyloid-A | (Acc.# M81349,M81451) |
| GDF-1 | (Acc.# NM_001492) |
| Solute carrier family 7 mRNA (SLC7A5) | (Acc.# NM_003486) |
| PLAB/MIC-1 | (Acc.# NM_004864) |
| EAP-(HBp15/L22) | (Acc.# NM_006755) |
| Small Proline-rich protein-1 | (Acc.# L05187) |
| NAG-1 | (Acc.# AF173860) |
| BST-1 | (Acc.# D21878) |
| II56KD | (Acc.# M24594) |
| Fibulin-1 D | (Acc.# NM_006486) |
| Nebulin | (Acc.# XM_040435) |
| VDUP1 upregulated by 1,25-dihydroxyvitamin D-3 | (Acc.# XM_002093,XP_002093) |
| Tumor nekrosis factor stimulated gene (TSG-6) | (Acc.# NM_007115) |
| Tumor nekrosis factor stimulated gene (TSG-37) | (Acc.# M31164) |
| Osteopontin | (Acc.# AF052124) |
| Tristetraproline (TTP) | (Acc.# M63625) |
| Nephropontin | (Acc.# M83248) |
| Tonsillar lymphocyte LD78 mRNA | (Acc.# X03754) |
| MB-1 gene (CD79a-B cell) | (Acc.# U05259) |
| Human Glykoprotein (gp39) | (Acc.# M80927,Y08374) |
| Glia derived nexin precursor | (Acc.# AI743134) |
| Heat shock protein 70B (HSP-70B) | (Acc.# X51757) |
| Apolipoprotein D | (Acc.# XM_049984,XM_003067) |
| Dead box, Y isoform (DBY), altern.transcr. 2 | (Acc.# AF000984) |
| Myocilin (GLC1A) | (Acc.# AH006047) |
| DR1-associated corepressor (DRAP1) | (Acc.# U41843) |
| DR1-associated protein 1 (neg. cofactor 2 α) | (Acc.# XM_055156) |
| FK506 bind.- 12-rapamycin assoc.prot.1 (FRAP1) | (Acc.# XM_001528,XM_042283) |
| Microfibril-associated glycoprotein-2 (MAGP-2) | (Acc.# AH007047,NM_003480) |
| Adrenomedullin (ADM) precursor | (Acc.# NM_001124,XM_051743) |
| DNA-damage-inducible transcript 3,clone MGC:4154 | (Acc.# BC003637) |
| Calretinin - calcium binding protein | (Acc.# X56667) |
| Breakpoint cluster region (BCR) mRNA | (Acc.# XM_017097) |
| Adipose most abundant gene transcript 1 (APM1) | (Acc.# NM_004797,XM_003191) |
| Novel adipose specific collagen-like factor | (Acc.# D45371) |
| | |

| **Funktionell unbekannte Gene und EST's:** | |
|---|---|
| IMAGE 745750 | (Acc.# AA420624) |
| KIAA0935 | (Acc.# AB023152) |
| KIAA0618 | (Acc.# AB014518) |
| Homolog zu FLJ23382 fis Klon HEP16349 | (Acc.# AK027035) |
| Hypothetical gene mRNA | (Acc.# XM_005331) |
| HDCMB07P/PCM-1 | (Acc.# AF068293) |
| cDNA clone DKFZp762M2311 | (Acc.# AL512760) |
| cDNA clone PP2684 | (Acc.# AF218004) |
| cDNA clone MGC:1811 (IMAGE:3506276) | (Acc.# BC015961) |
| cDNA clone IMAGE:979127 | (Acc.# AA522530) |
| cDNA clone IMAGE:4279495 5', mRNA sequence | (Acc.# Bf667722) |
| cDNA clone 137308 mRNA, partial cds | (Acc.# U60873) |
| cDNA clone IMAGE:159541 | (Acc.# H15814) |
| cDNA clone MAMMA1001272 | (Acc.# AU147646) |
| cDNA clone IMAGE:2419382 | (Acc.# AI826771) |
| cDNA clone IMAGE: 3941411 | (Acc.# BE797145 |
| cDNA clone IMAGE:3834583 | (Acc.# BE743390) |
| cDNA clone IMAGE:4565371 | (Acc.# BG397372) |
| cDNA clone MGC:2460 IMAGE:2964524 | (Acc.# BC009504) |
| cDNA clone RC3-HT0585-010400-013-all HT0585 | (Acc.# BE176664) |
| cDNA clone similar to CG8974 gene product | (Acc.# XM_018516) |
| cDNA clone BSK-65 | (Acc.# W99251) |
| cDNA clone IMAGE:3844696 | (Acc.# BE730665) |
| FLJ23382 fis, clone HEP16349 | (Acc.# AK027035) |
| FLJ20500 fis, clone KAT09159 | (Acc.# AK000507,BC015236) |
| GABBR1 Region von AL031983 | (Acc.# 12329558) |
| cDNA clone CS0DE006YI10 5' prime end | (Acc.# AL541302) |
| cDNA clone CS0DE006YI10 3' prime end | (Acc.# AL541301) |
| EST371586 IMAGE resequences | (Acc.# Aw959516) |
| MEN1 region clone epsilon/beta | (Acc.# Af001892) |
| | |

| **Kontrollen zum Quantifizierungsabgleich:** | |
|---|---|
| alpha-Aktin | (Acc.# M20543) |
| beta-Aktin | (Acc.# XM_037239) |
| gamma-Aktin | (Acc.# NM_001614) |
| Glyceraldehyd-3-phosphat-Dehydrogenase | (Acc.# XM_033258) |
| Glucose-6-phosphat-Dehydrogenase | (Acc.# XM_013149) |
| 28S rRNA | (Acc.# M27830) |
| 18S rRNA | (Acc.# M10098) |

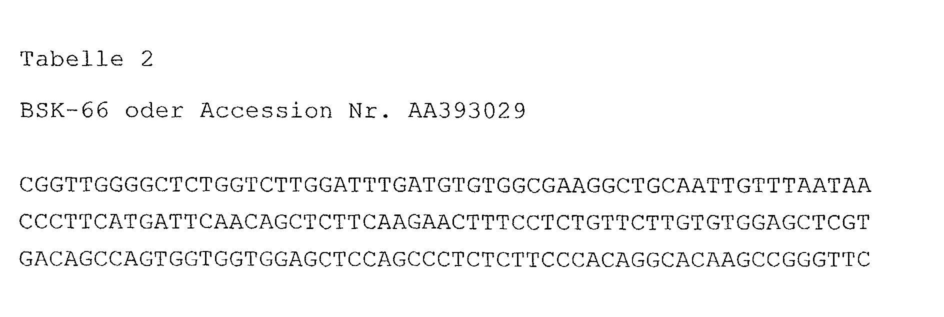

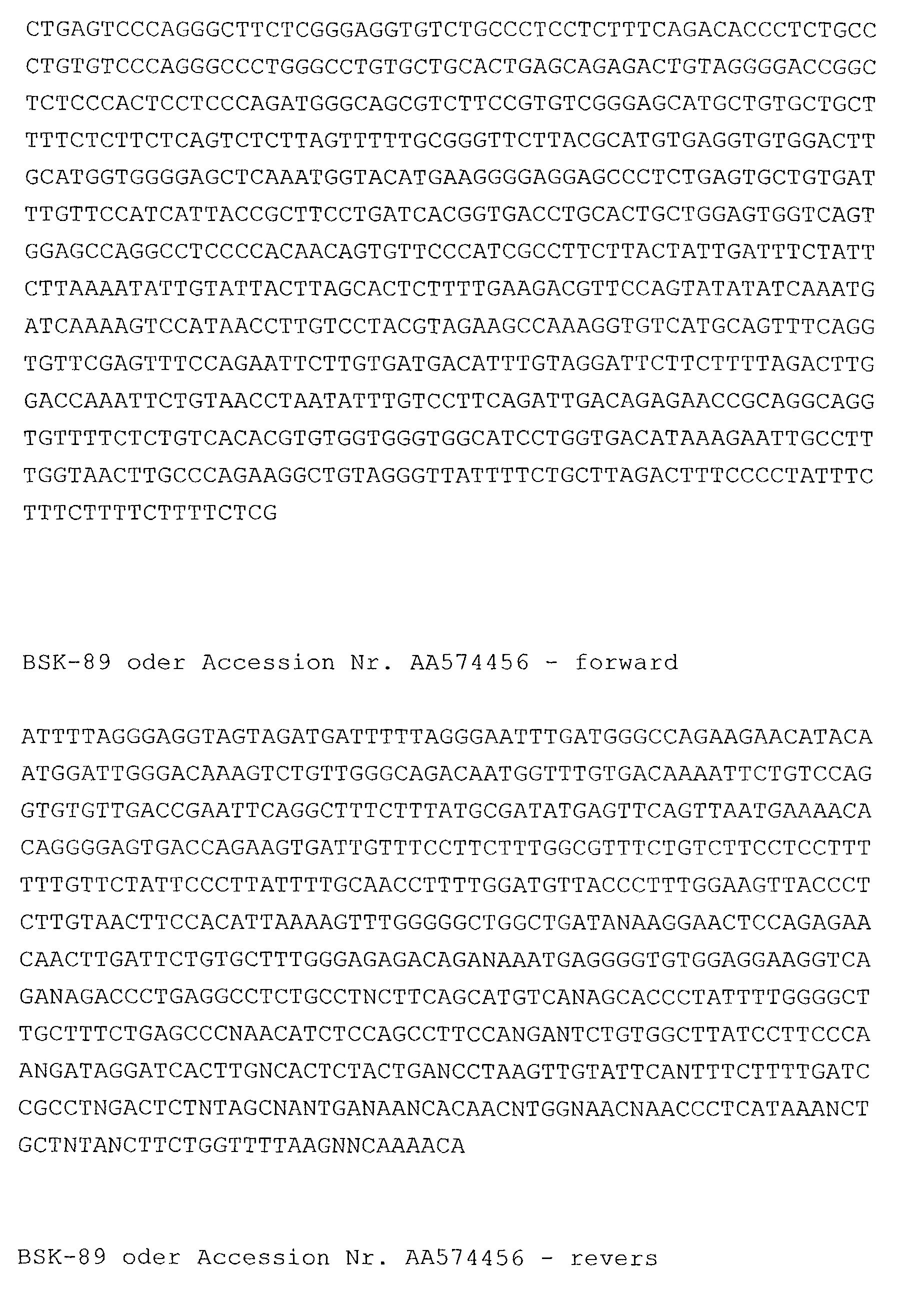

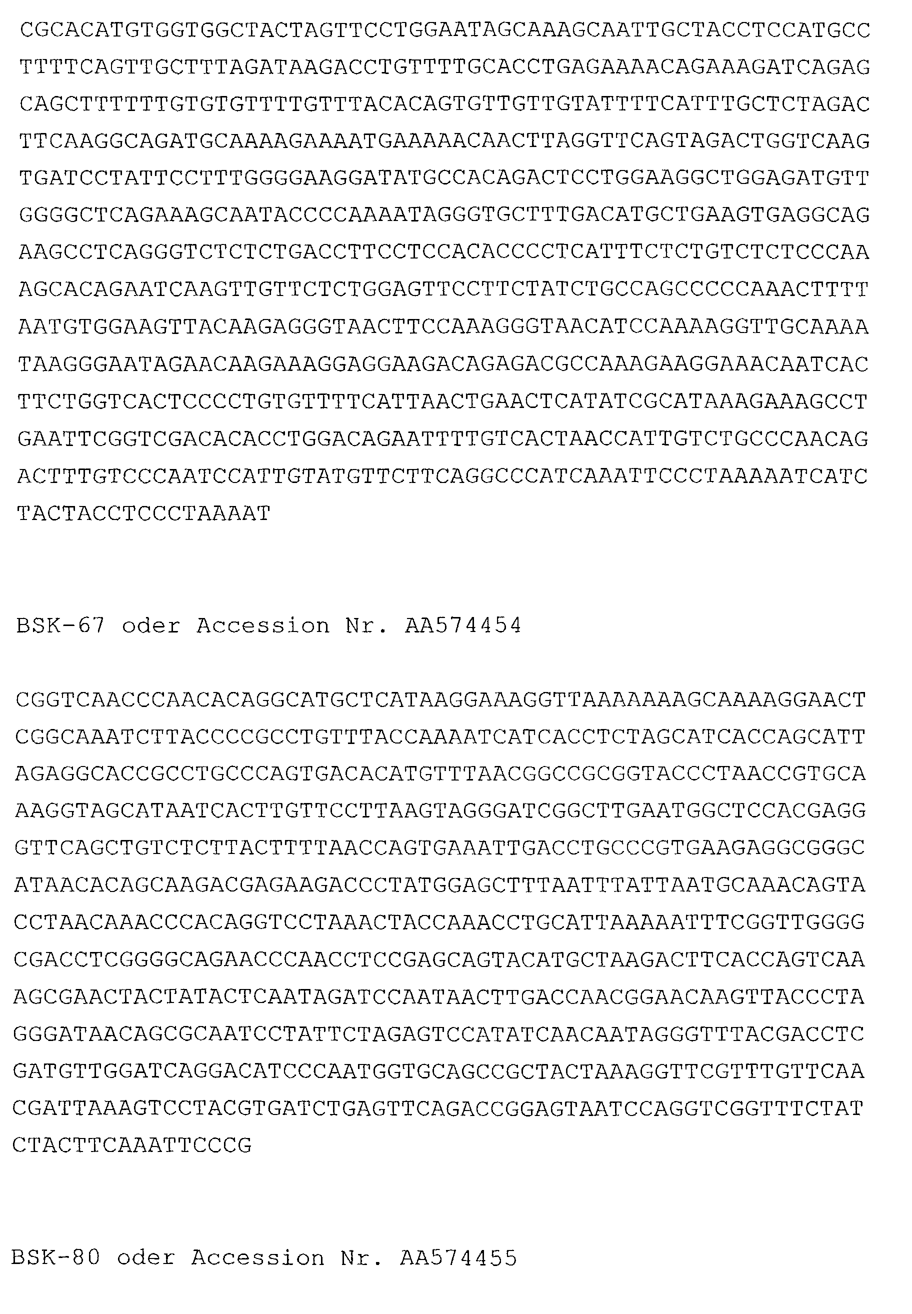

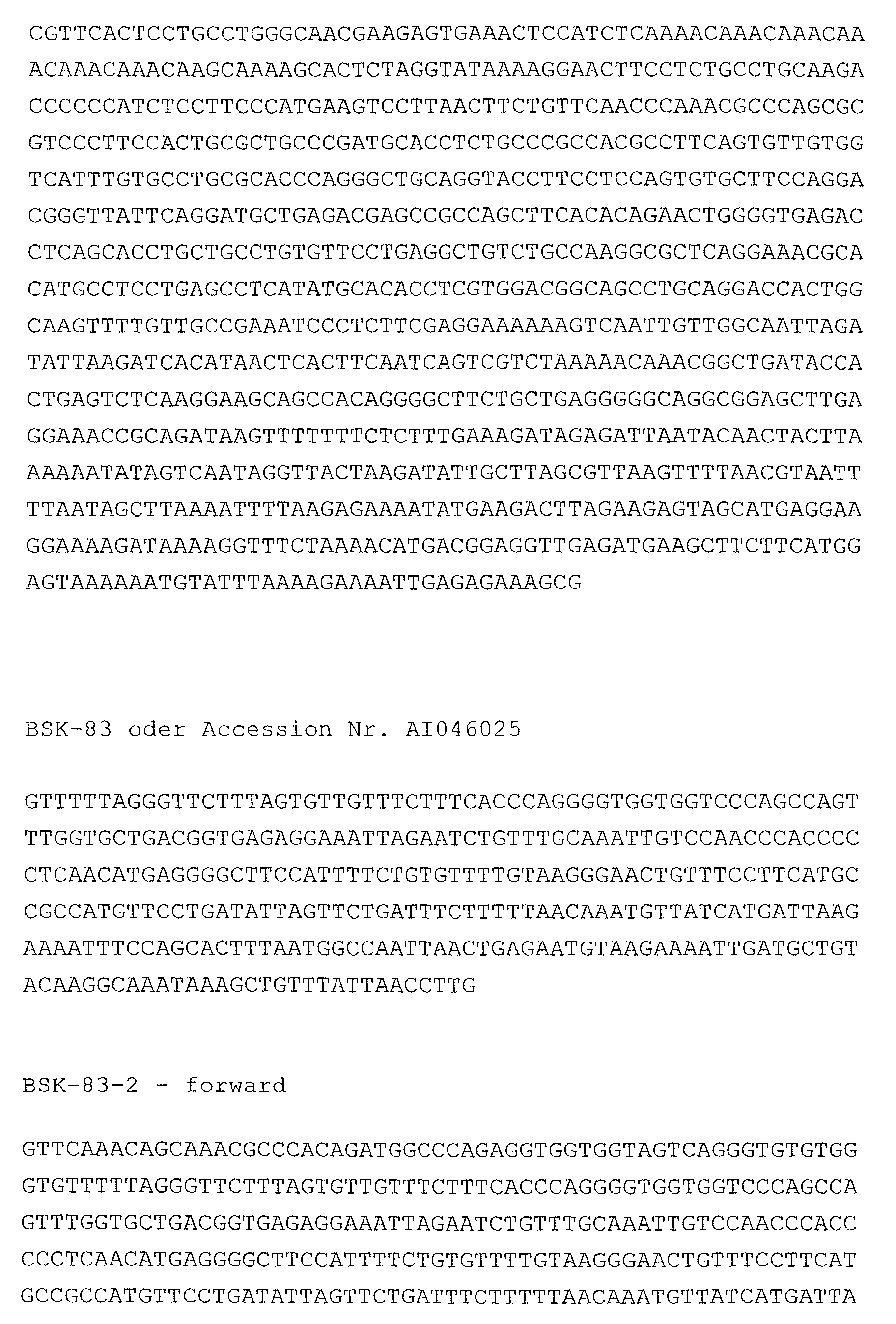

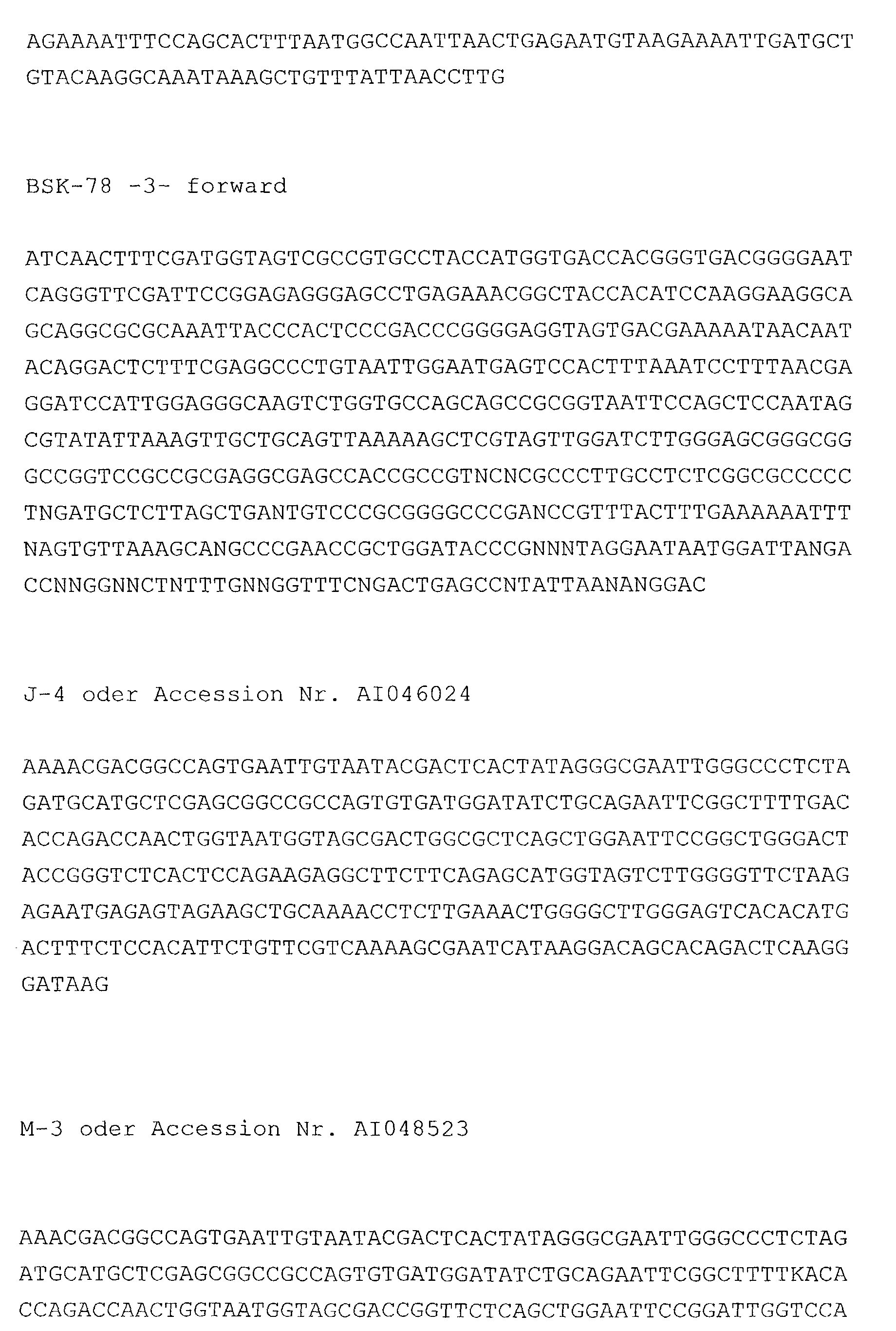

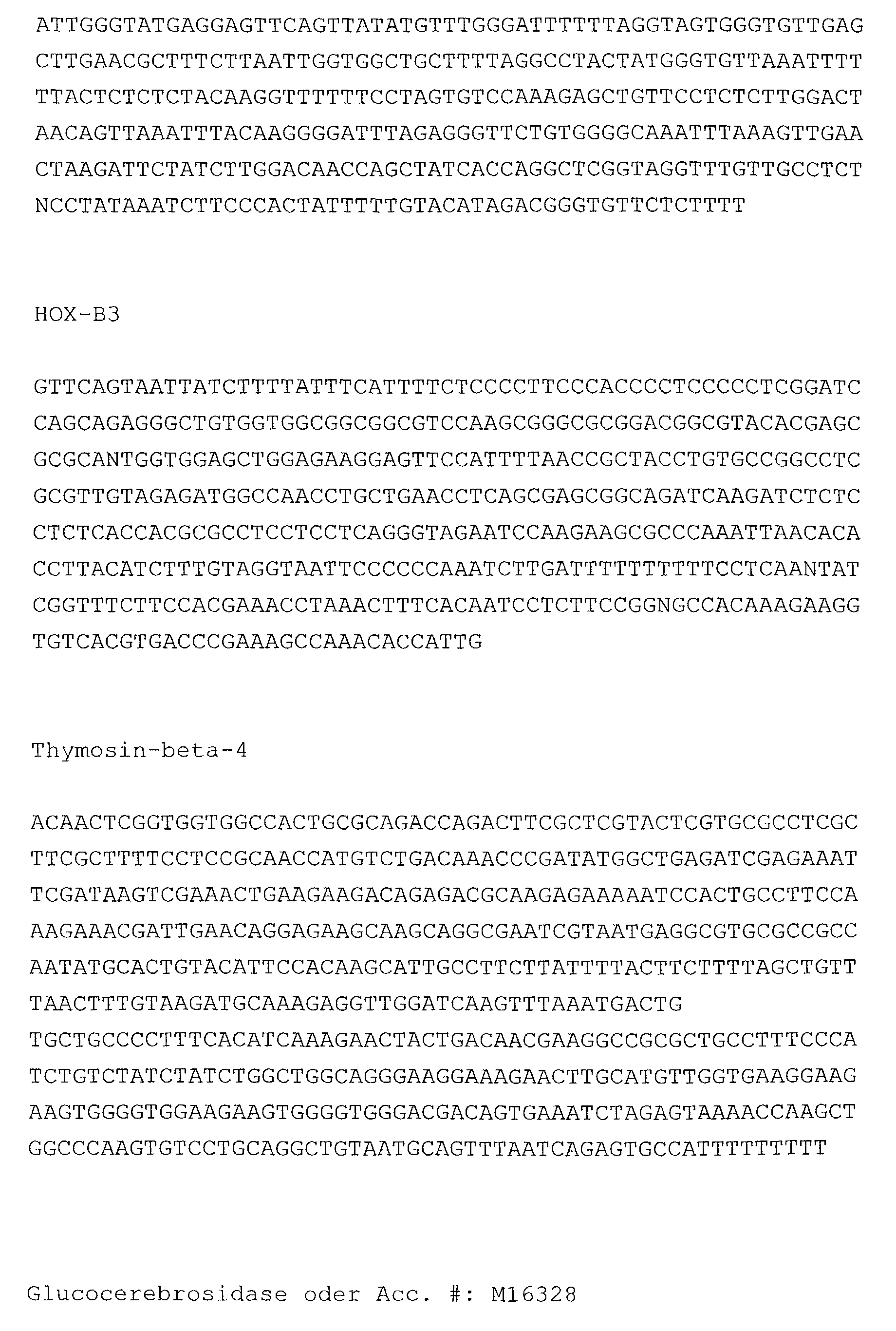

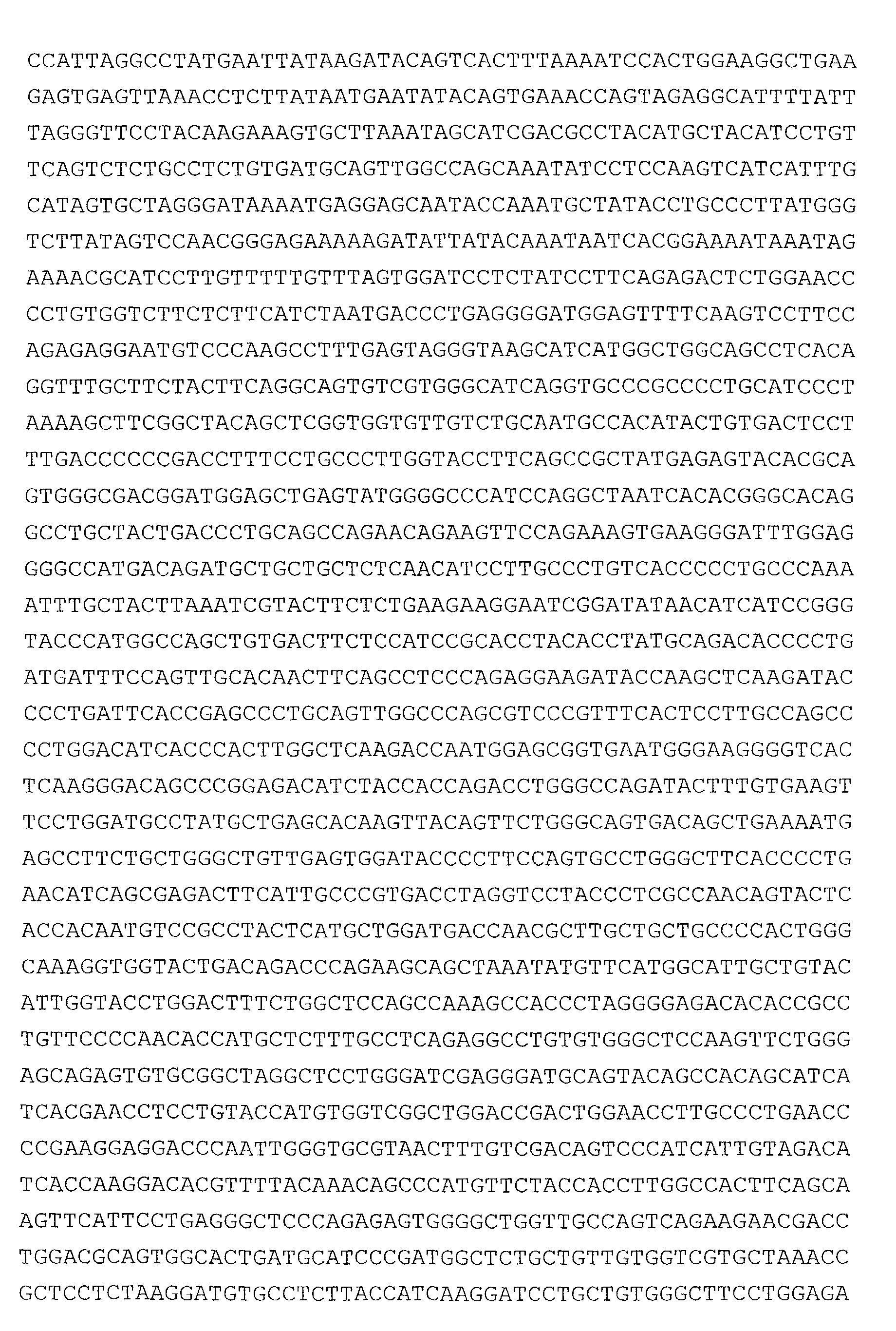

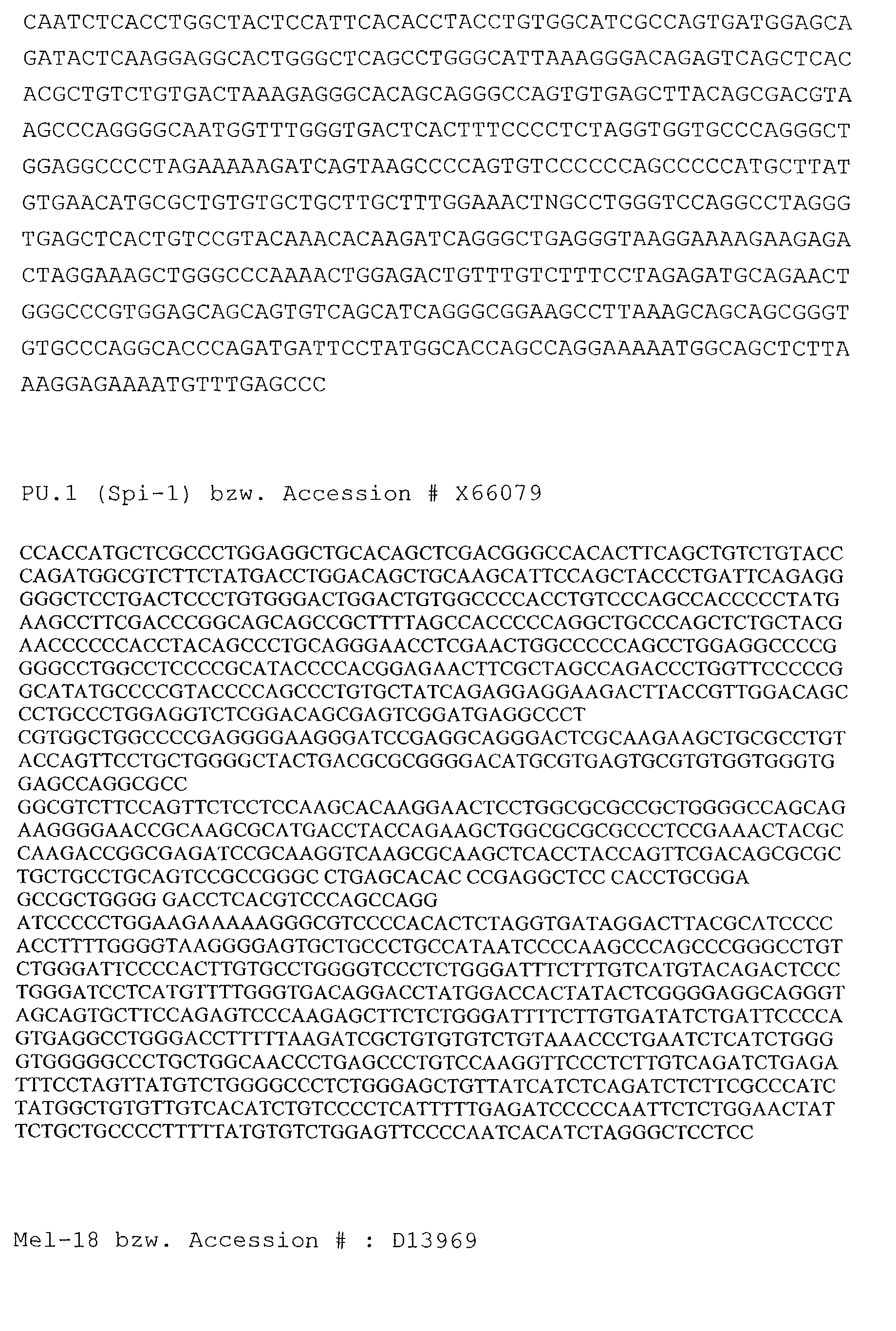

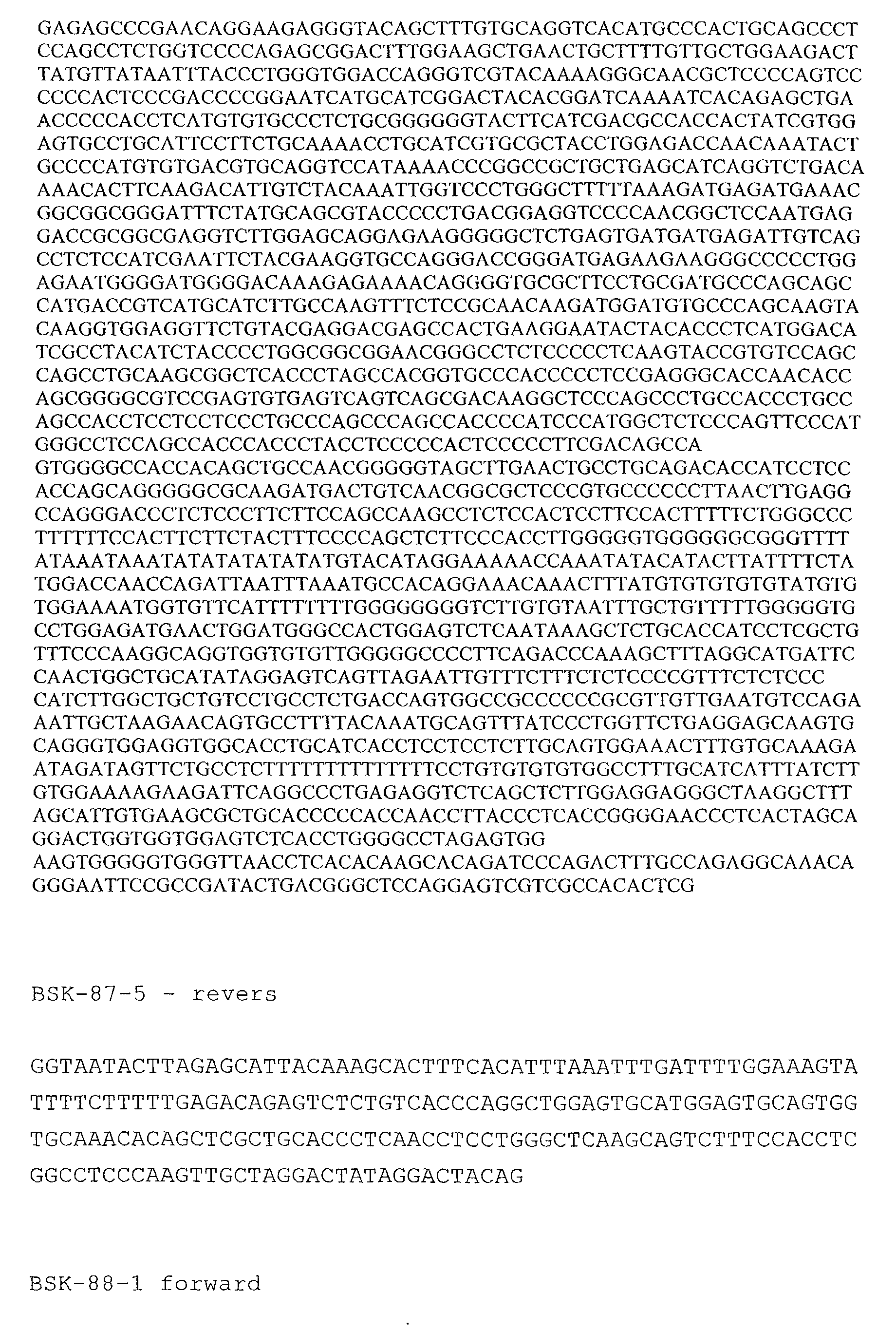

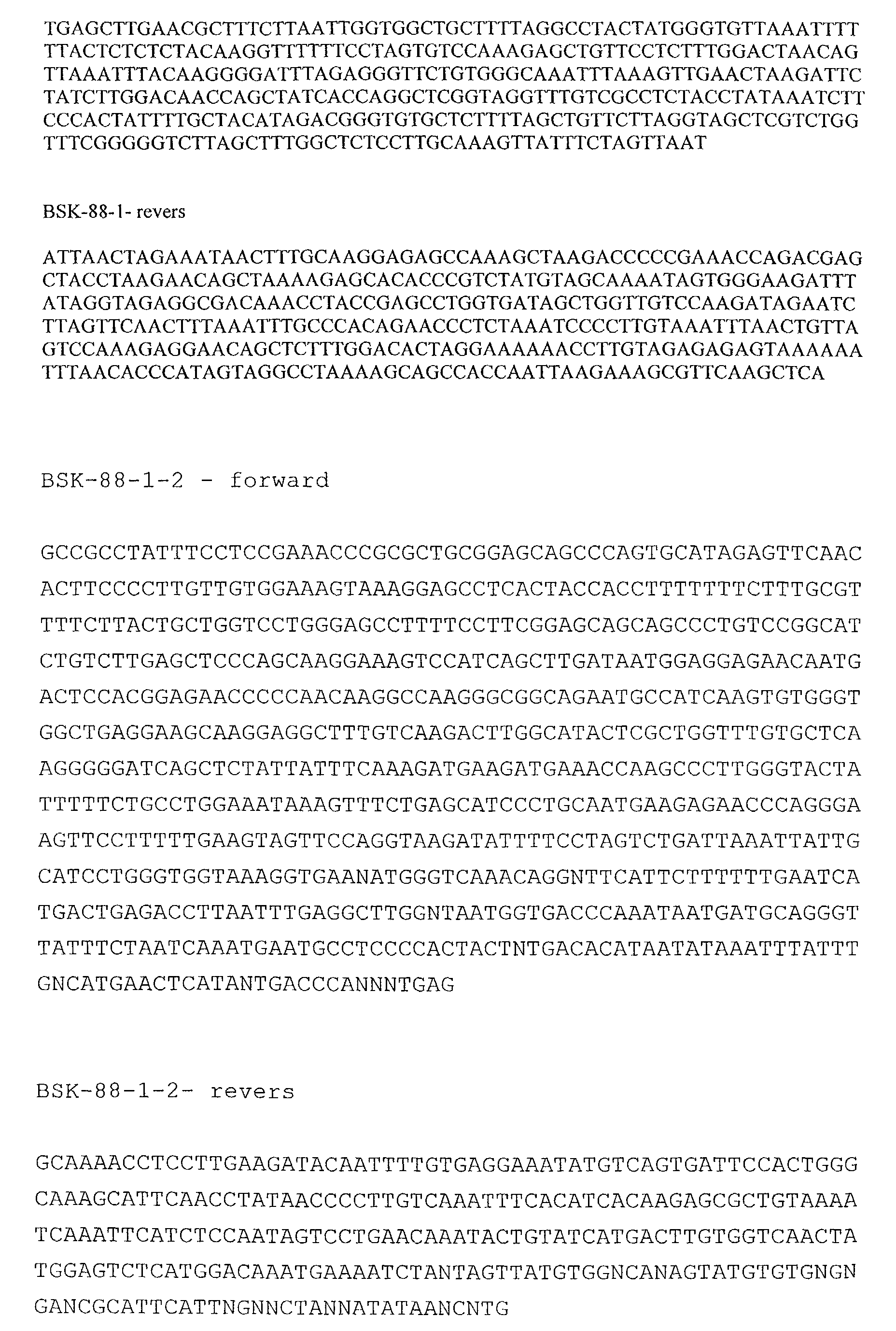

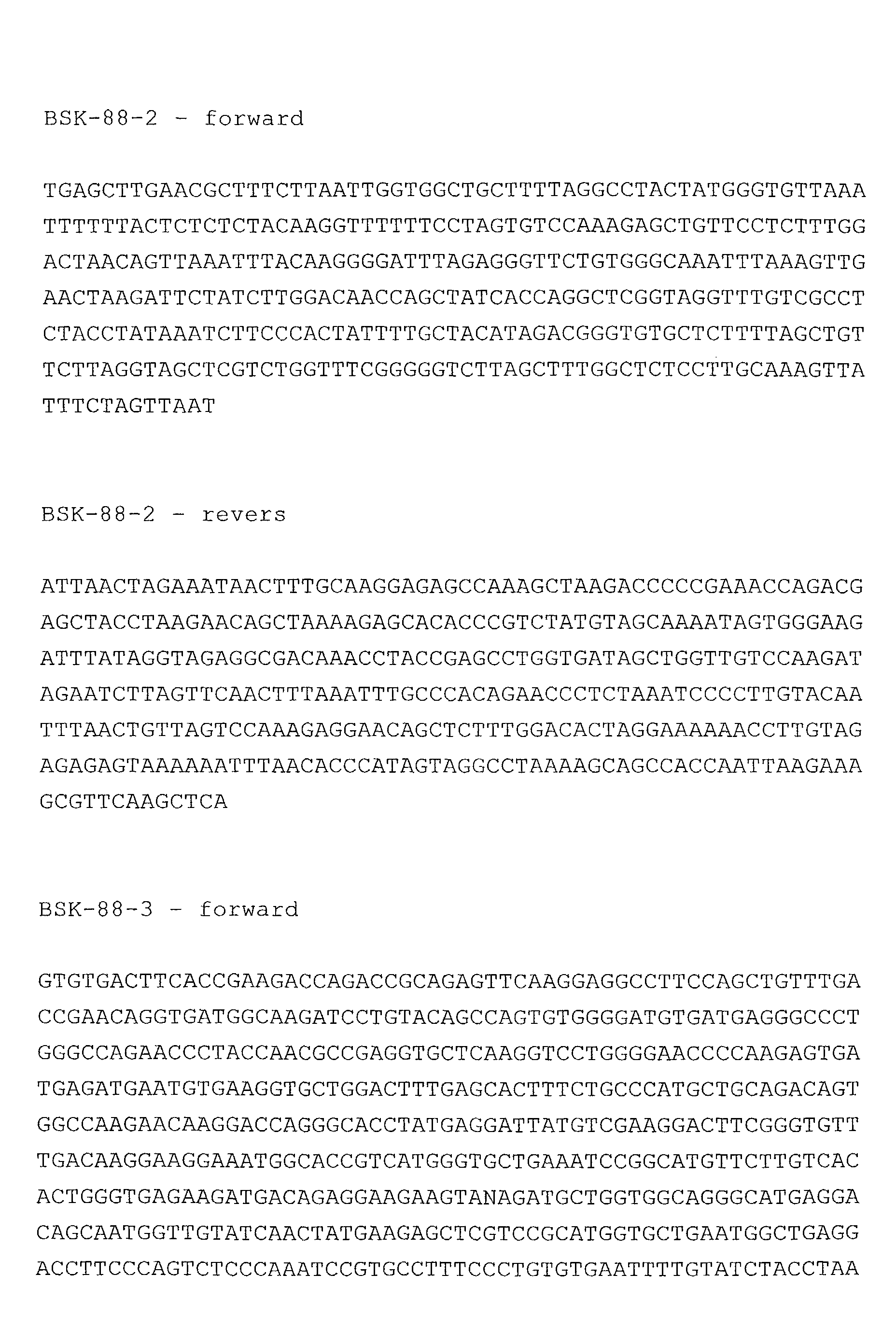

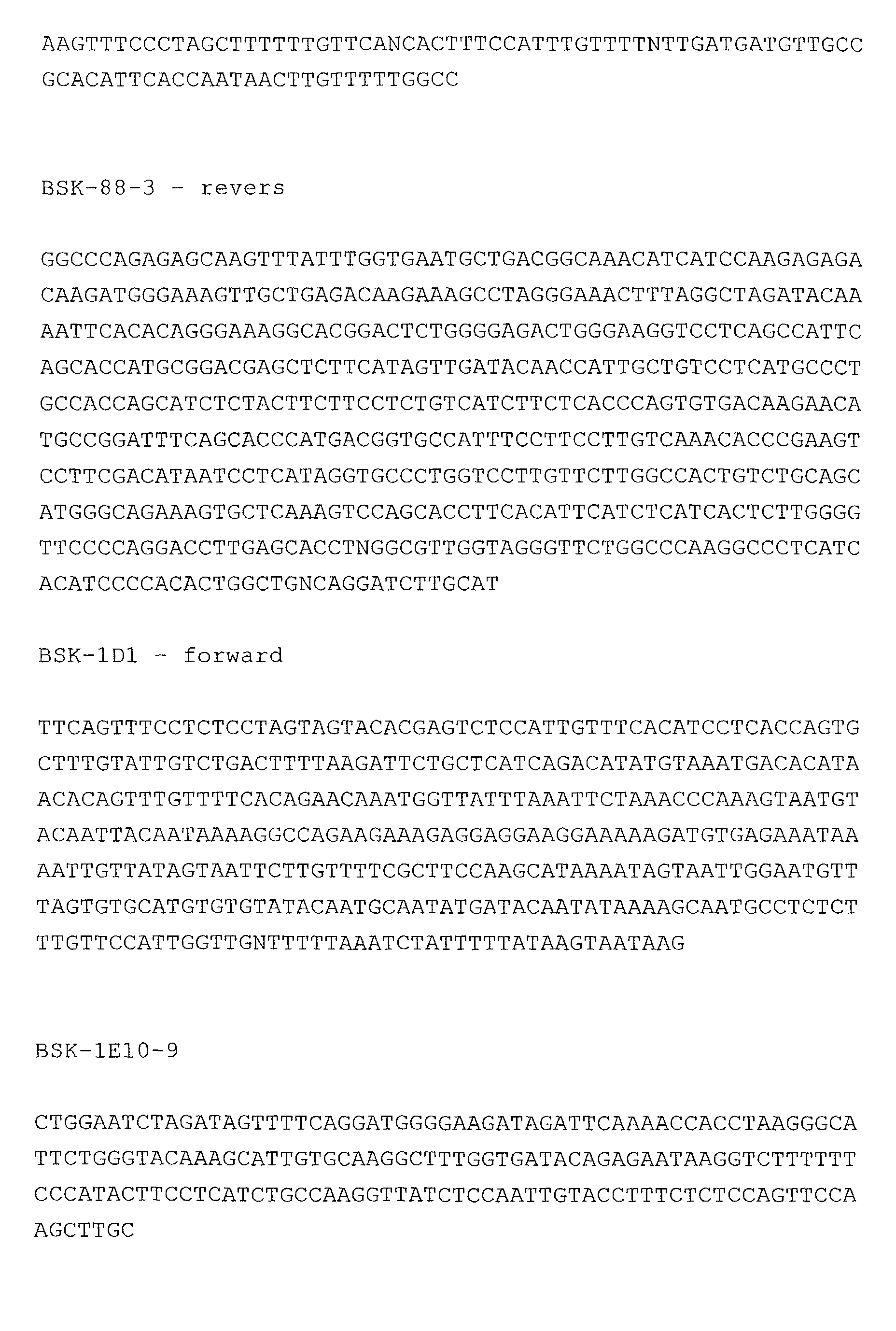

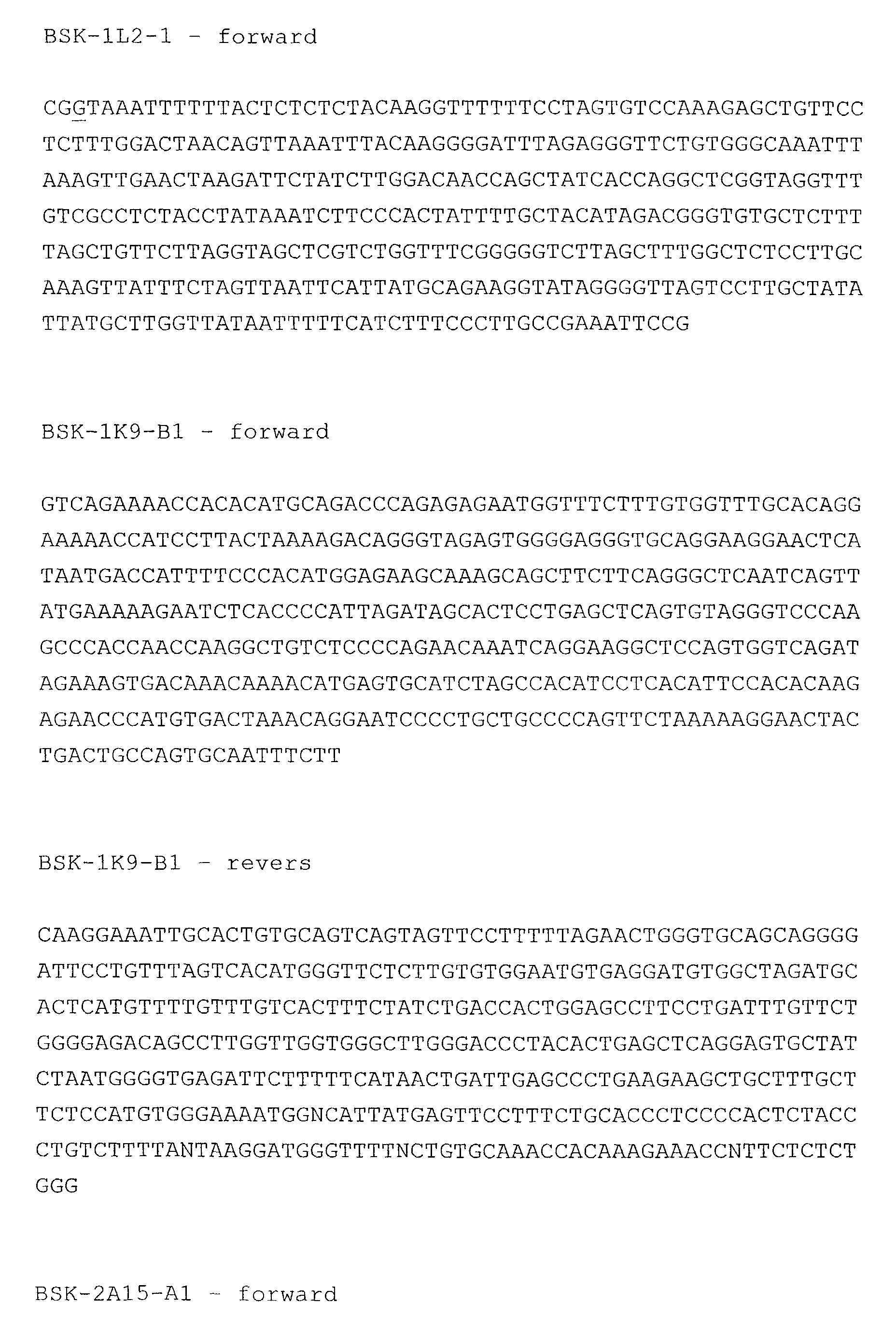

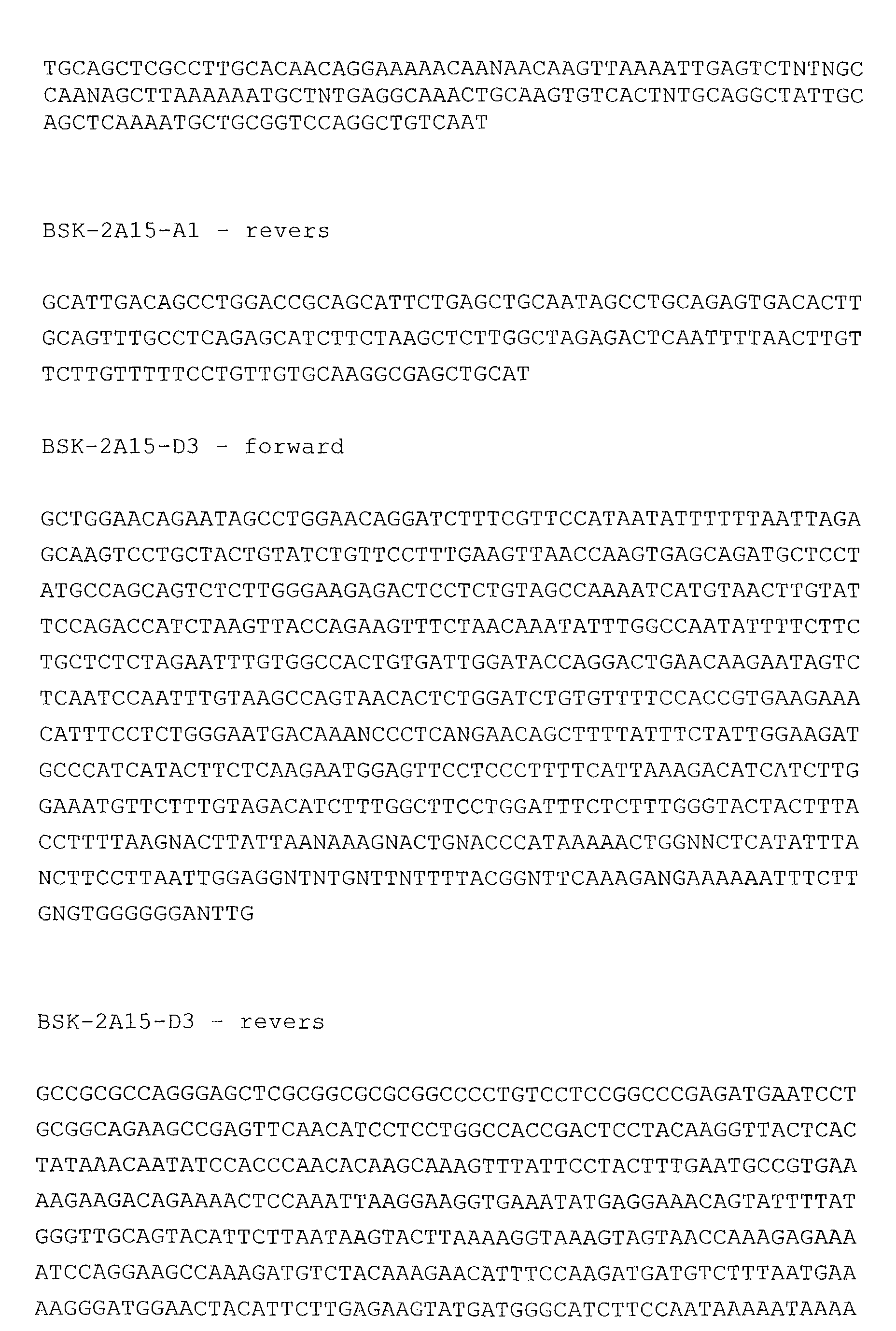

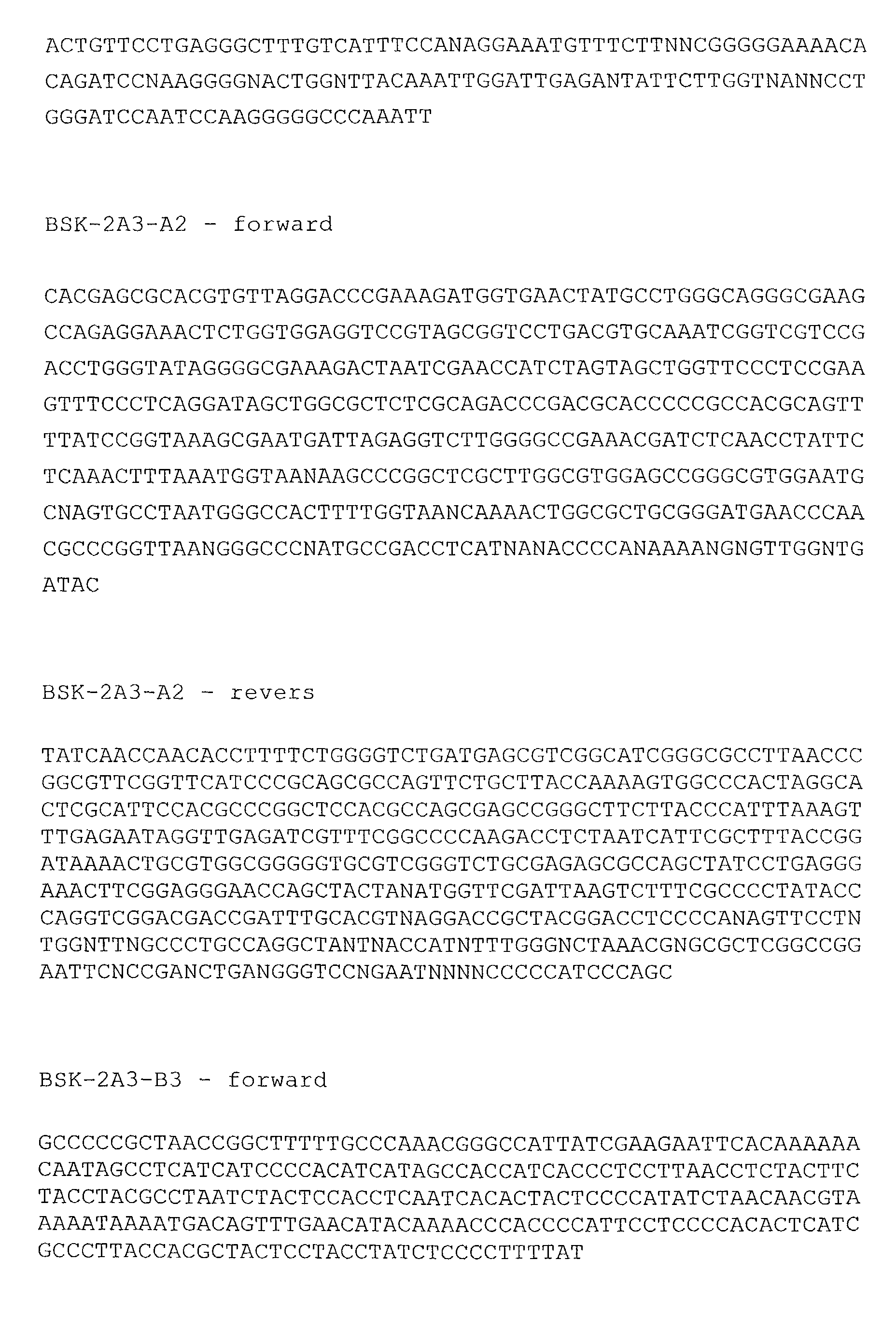

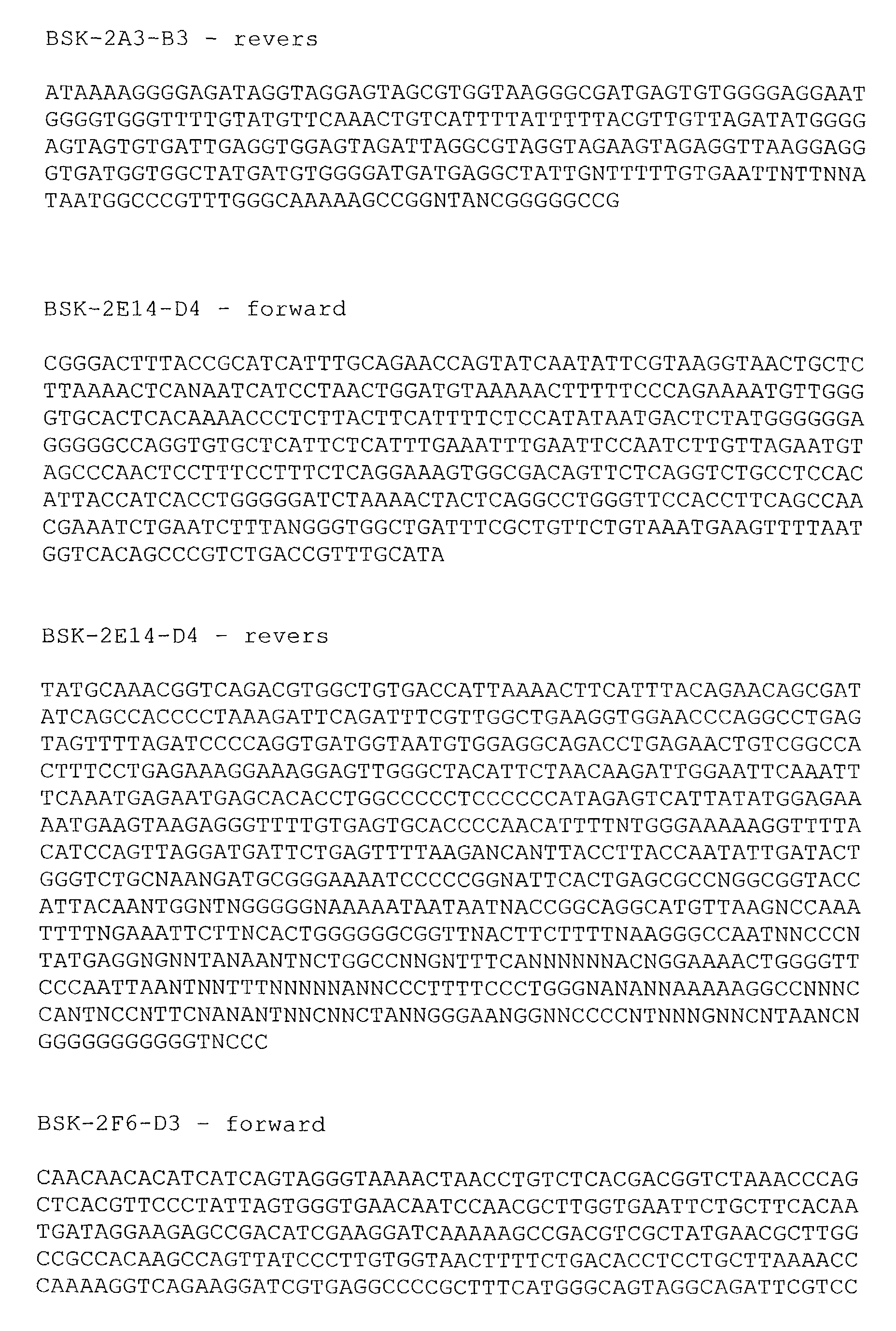

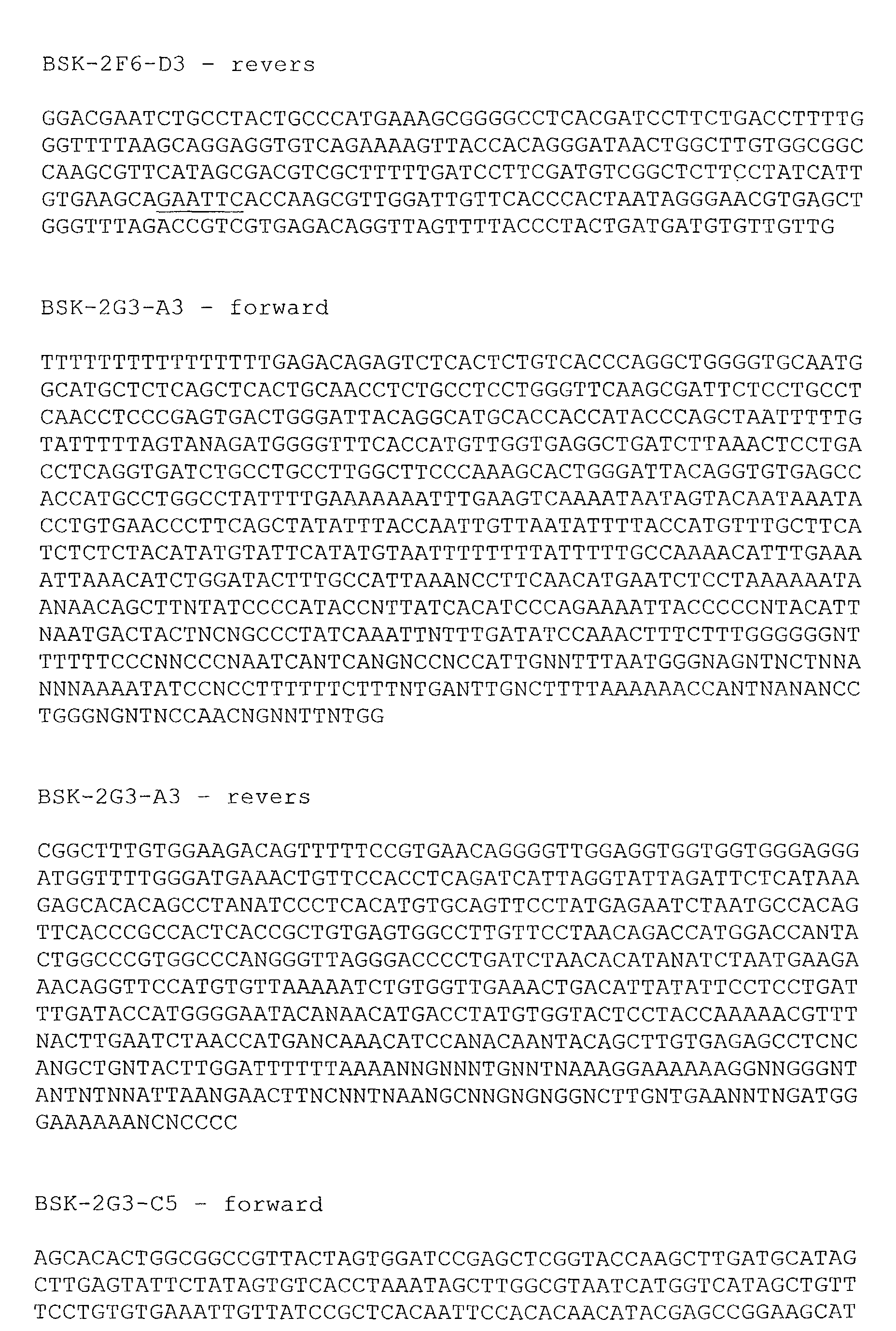

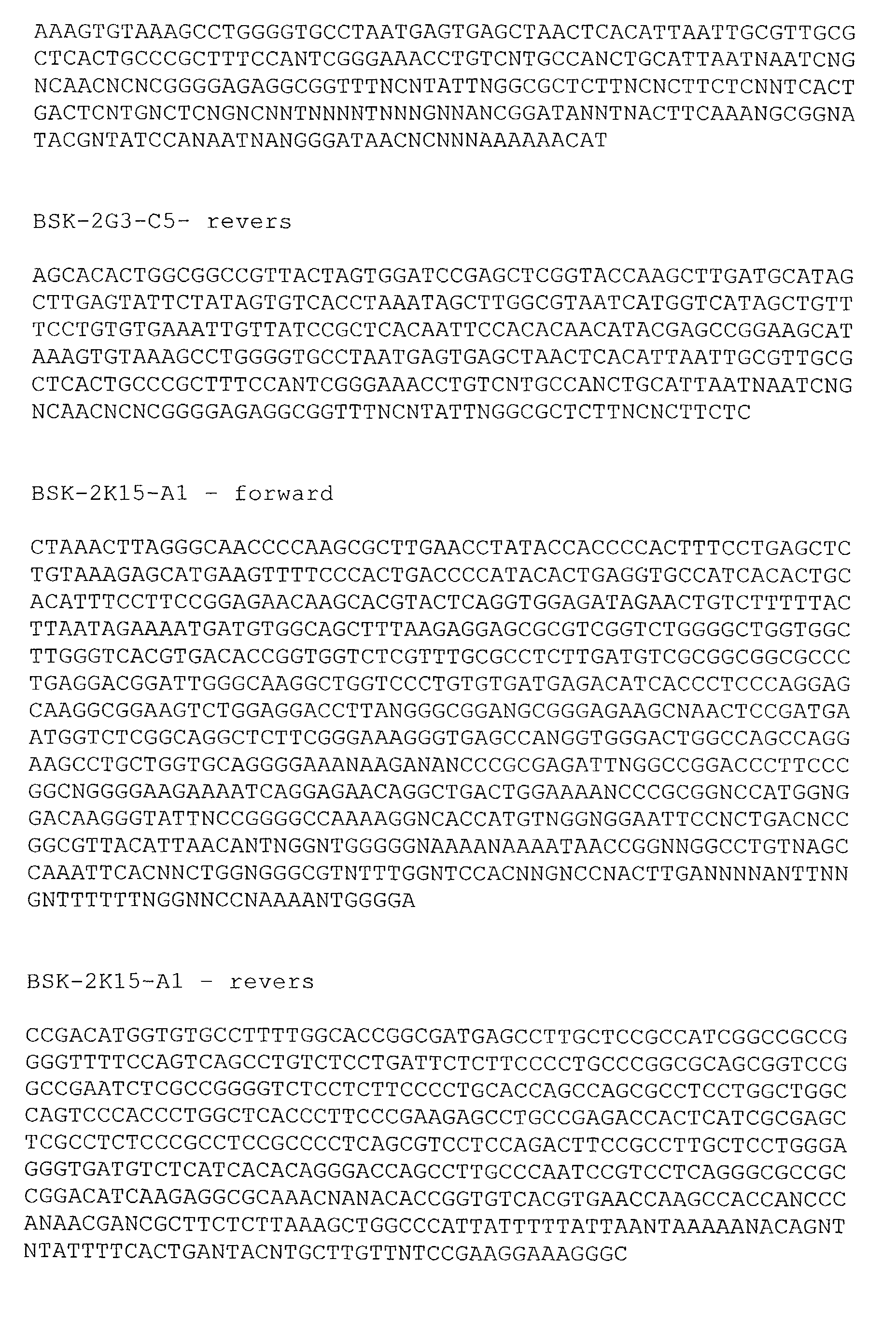

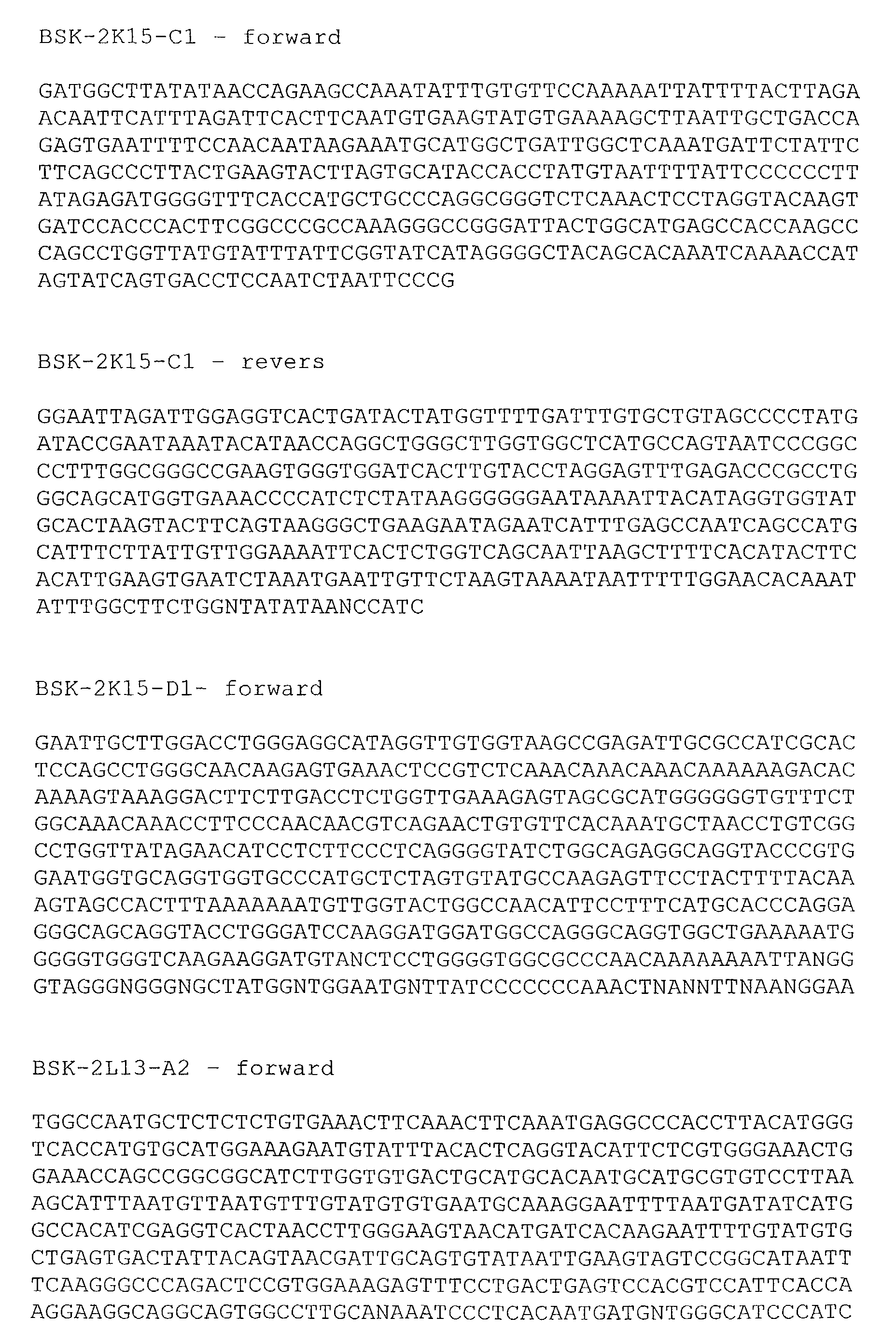

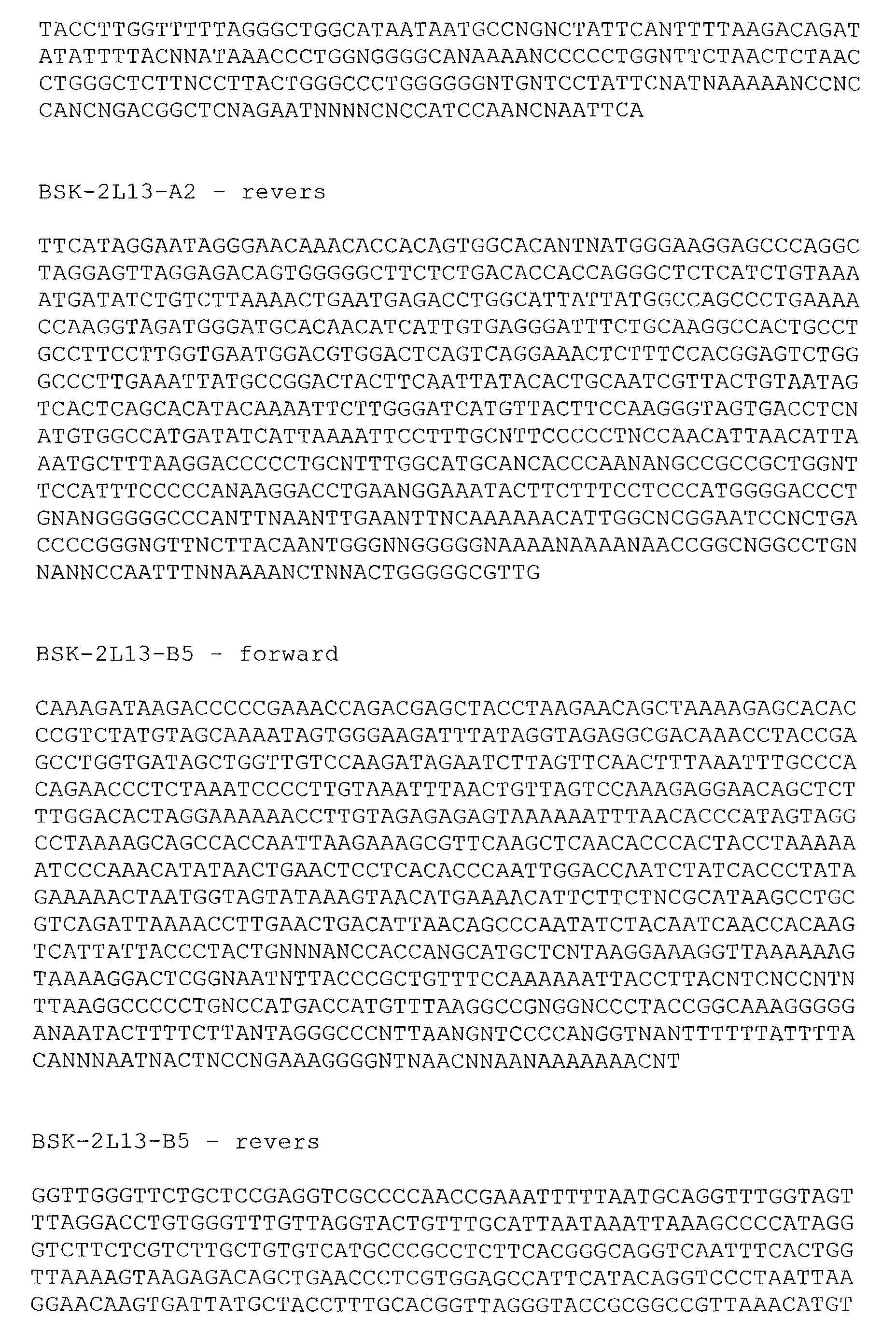

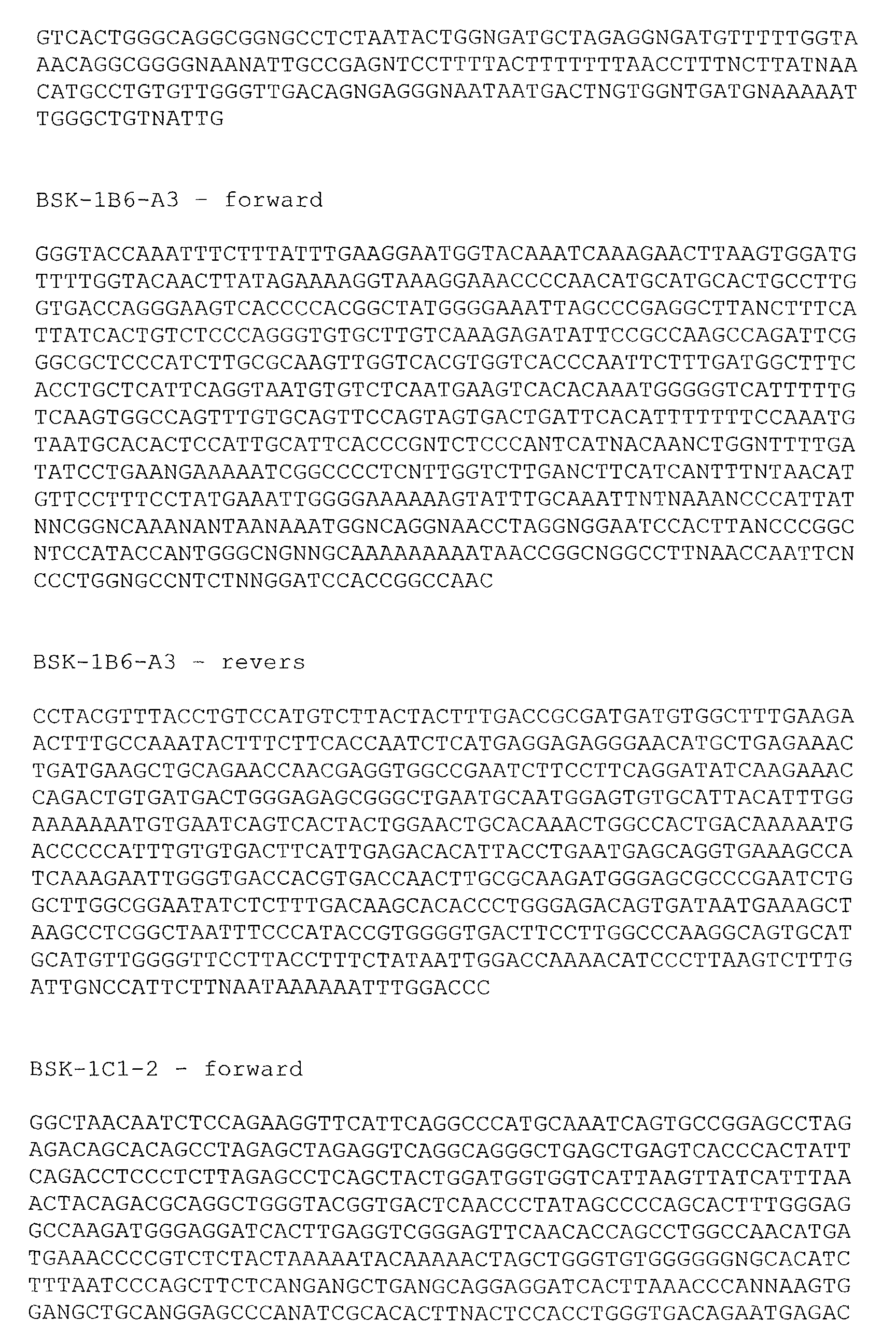

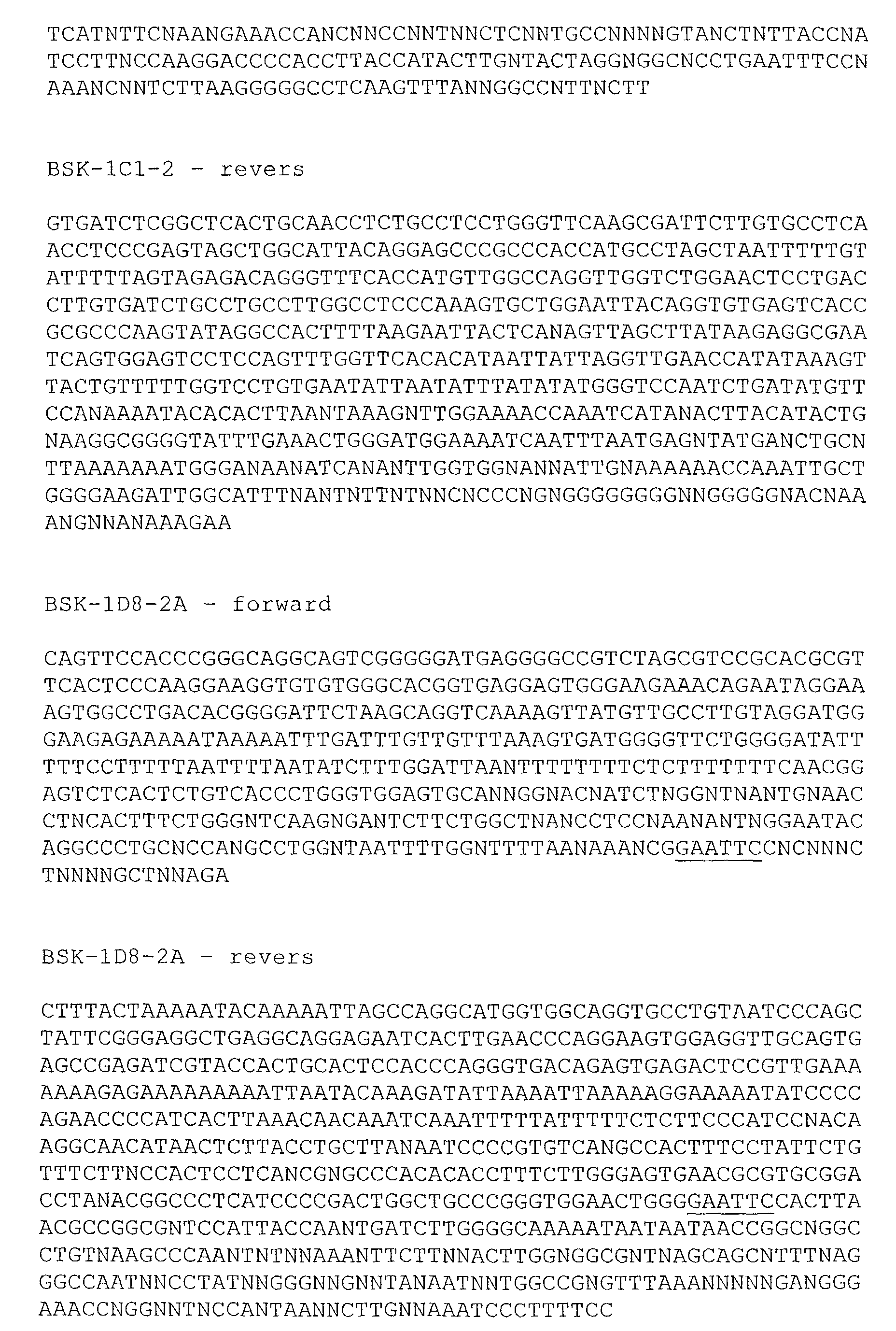

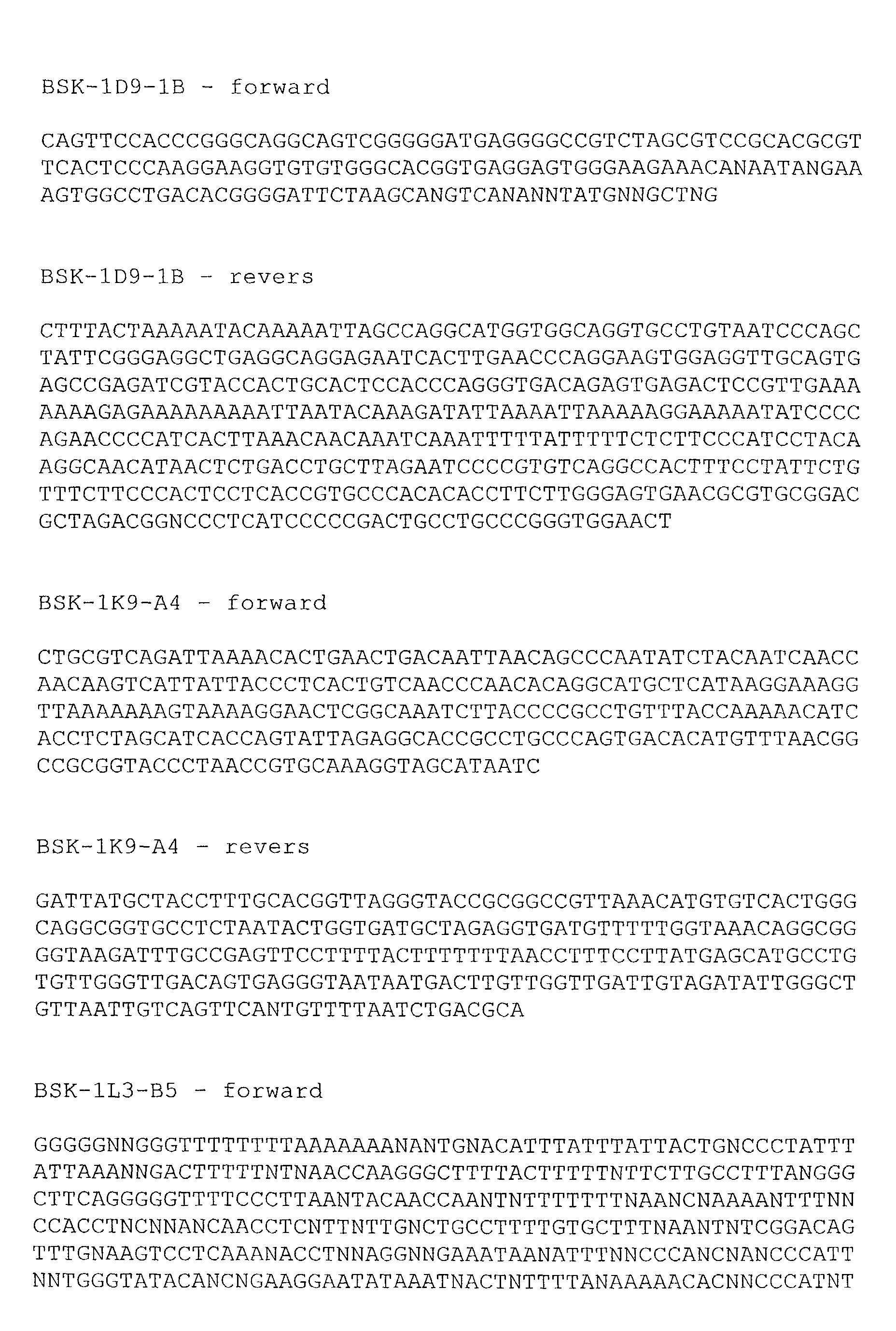

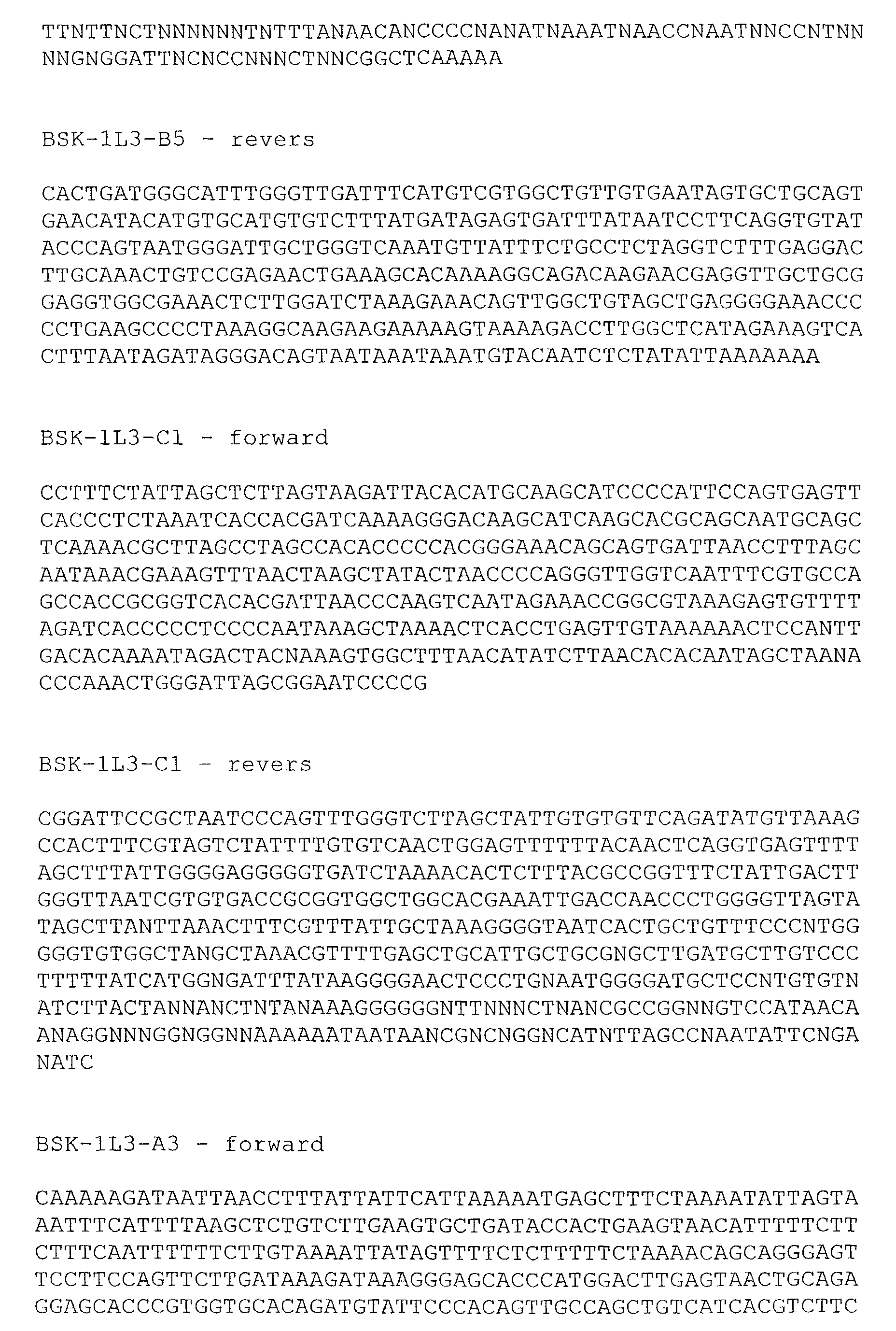

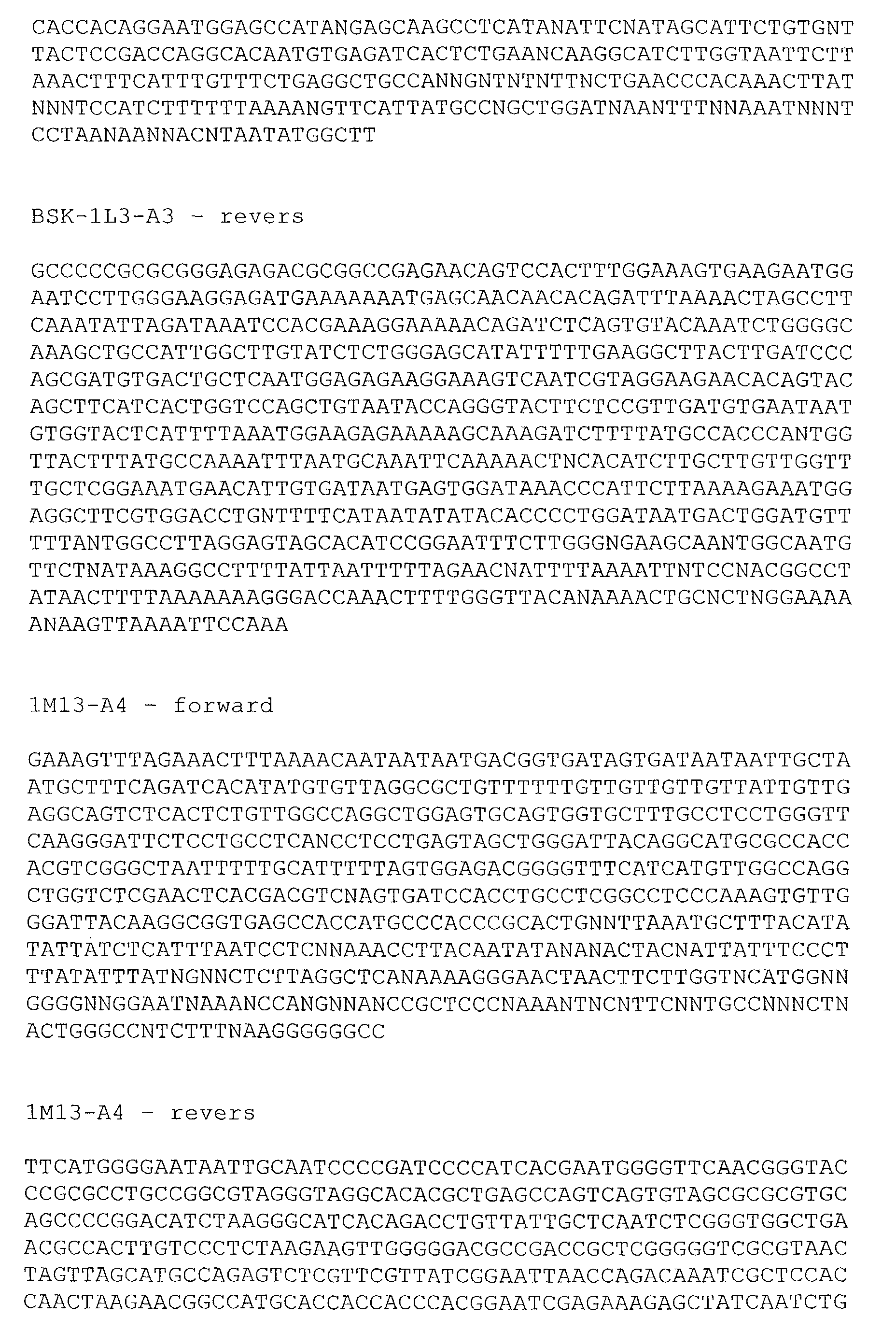

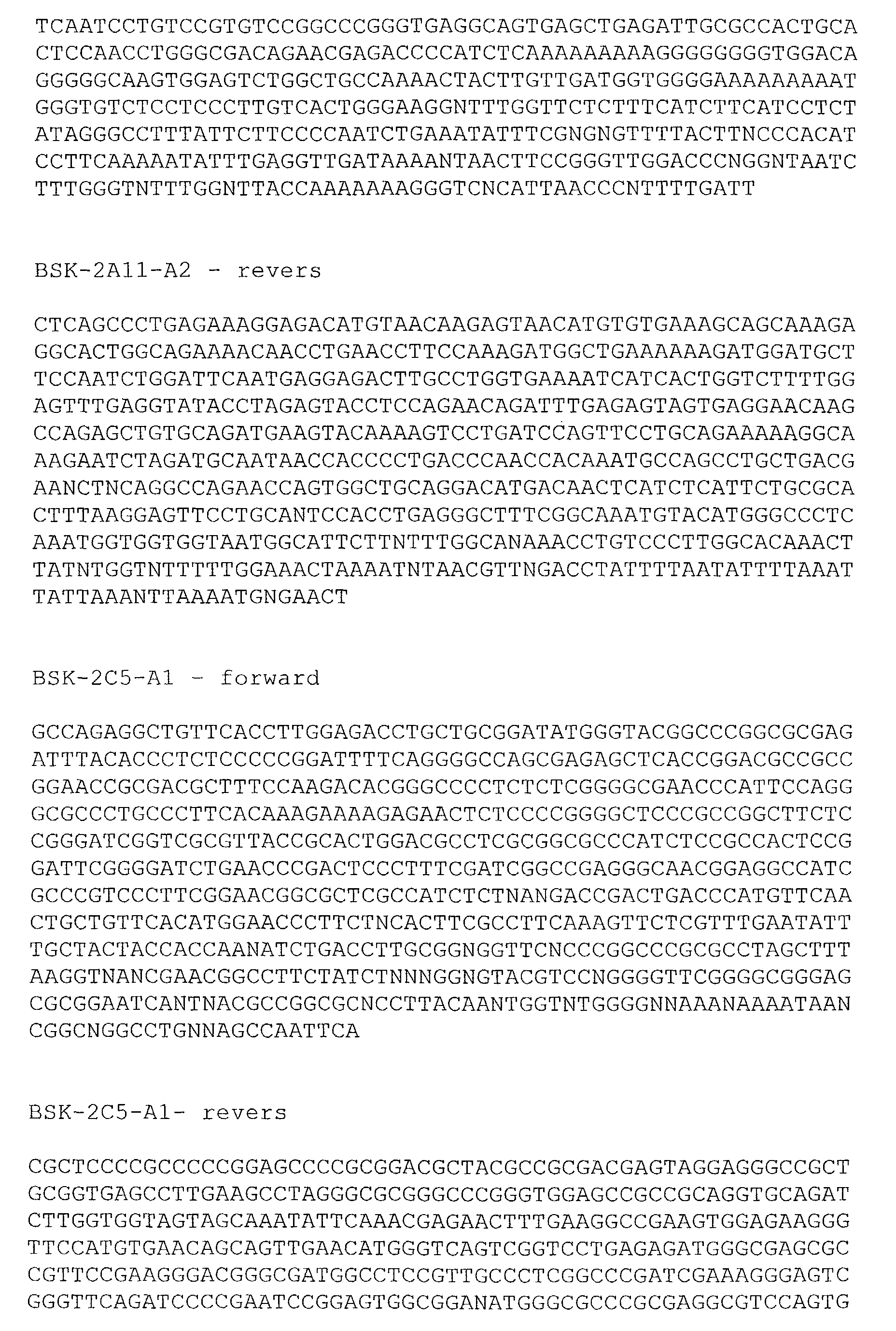

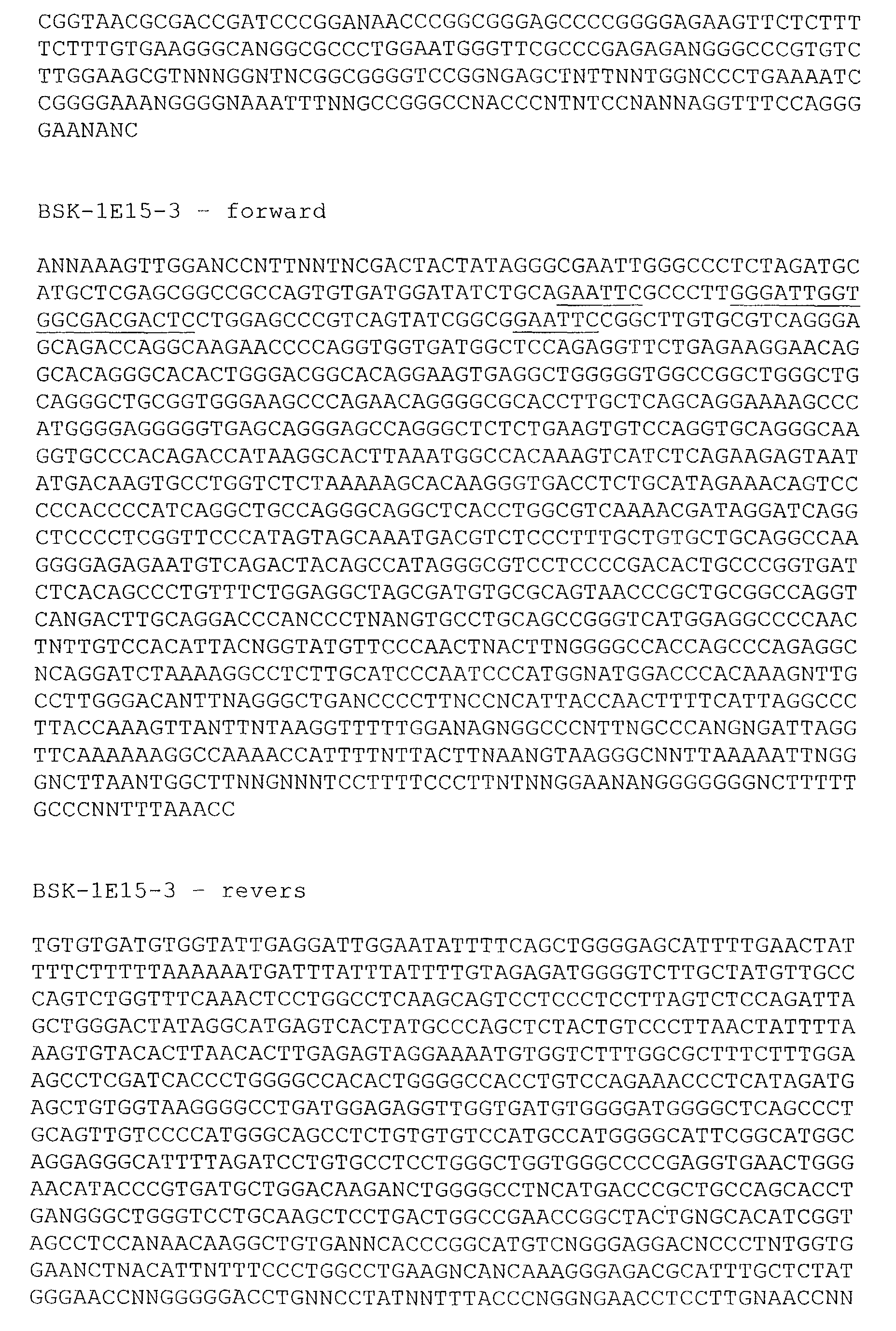

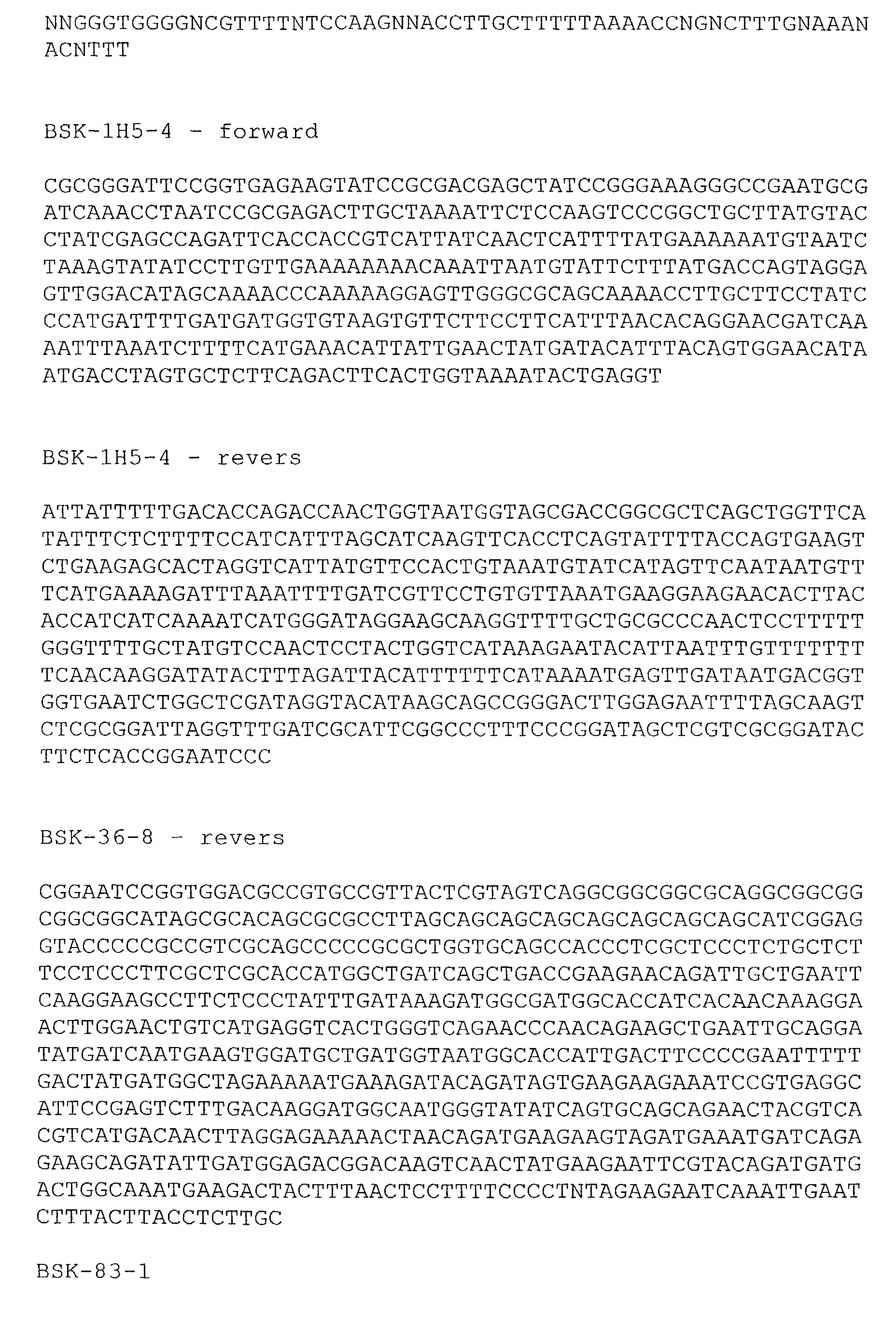

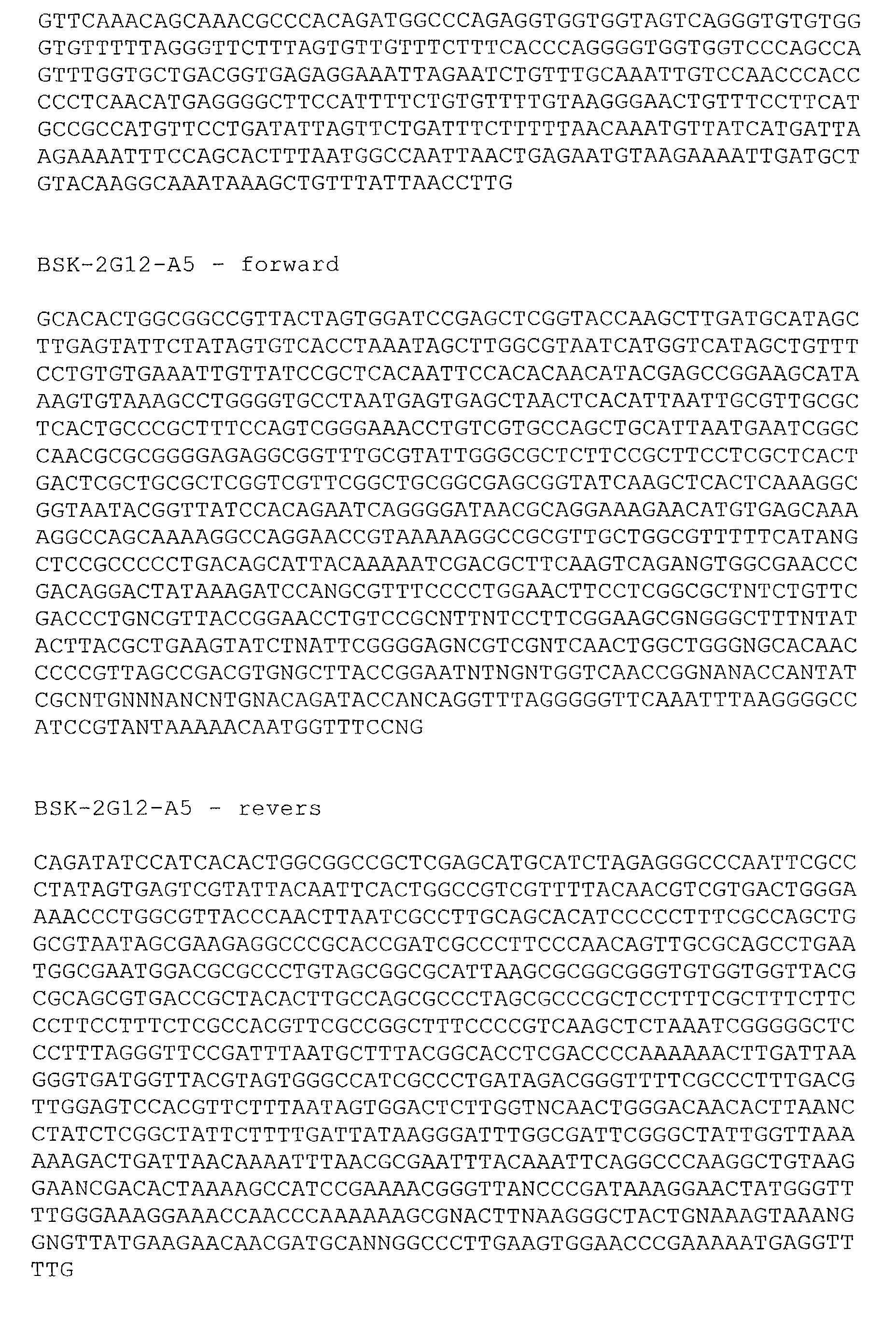

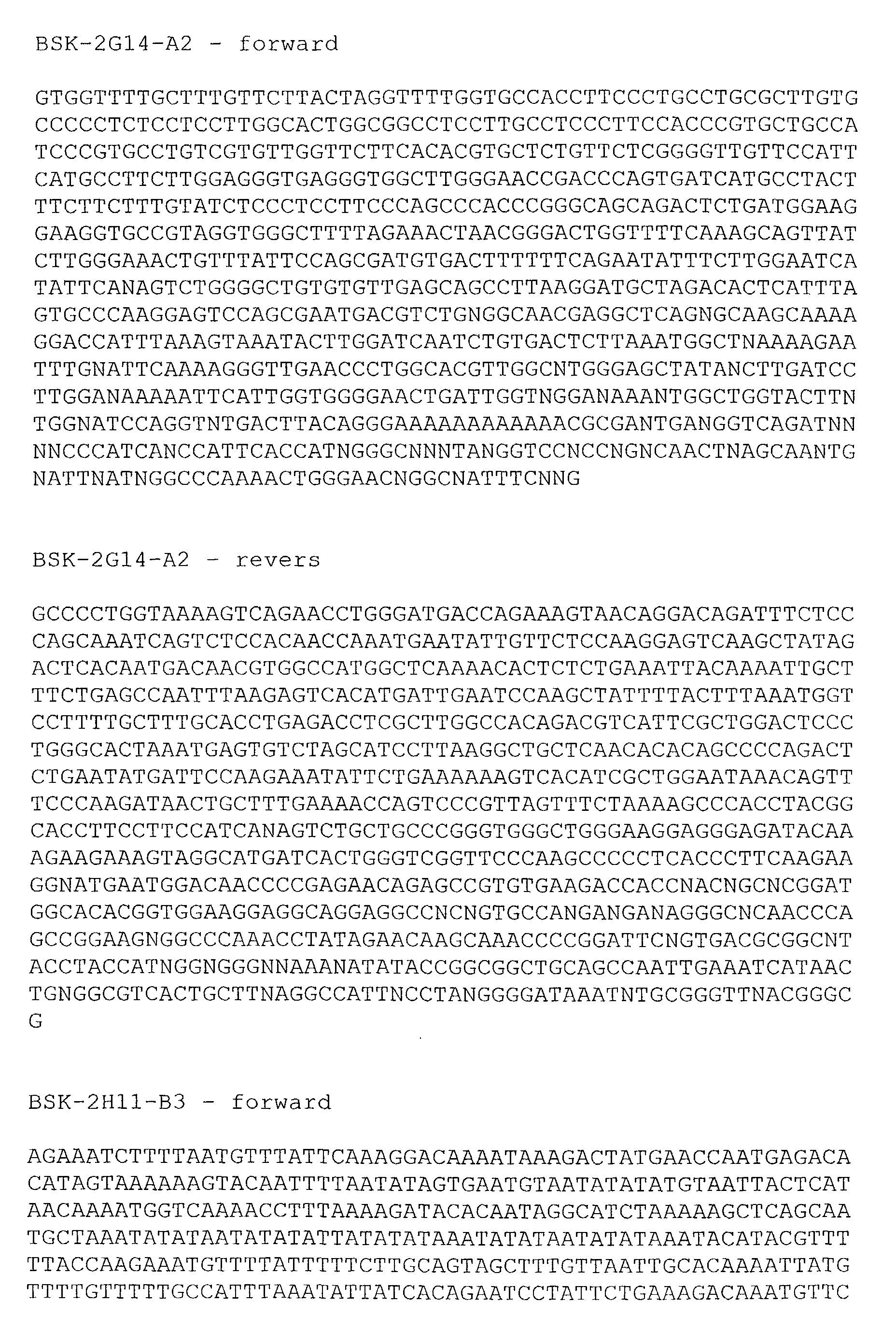

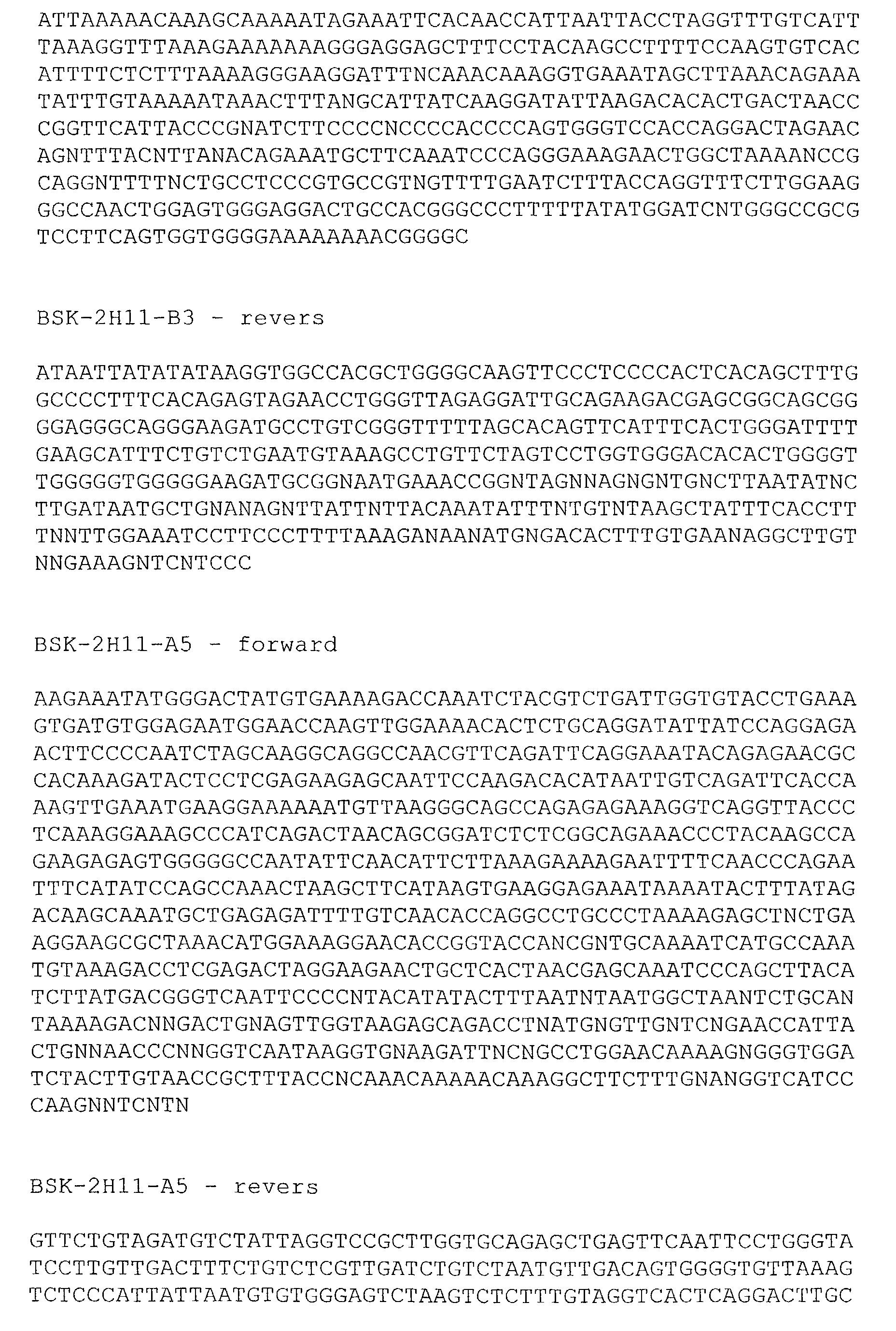

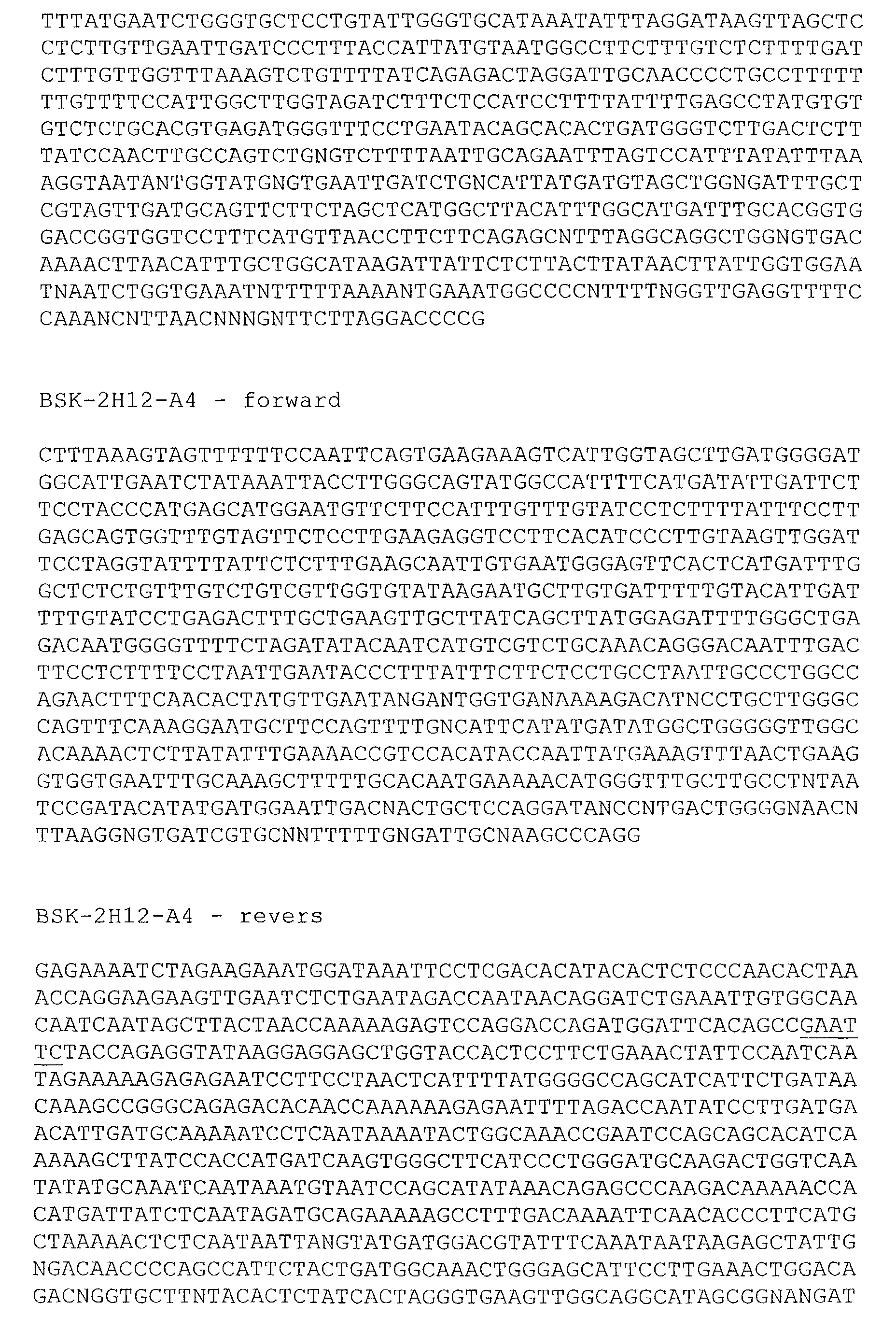

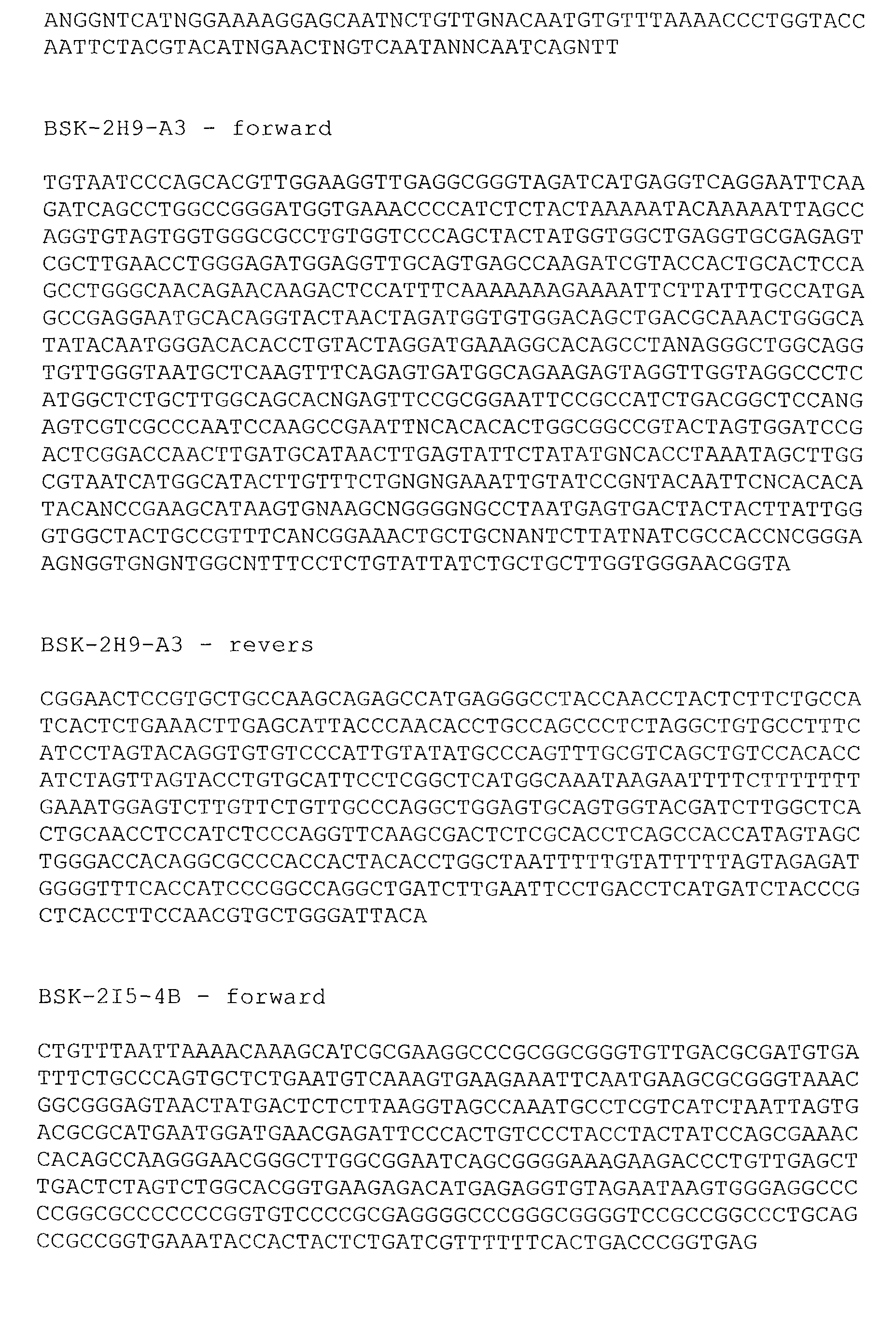

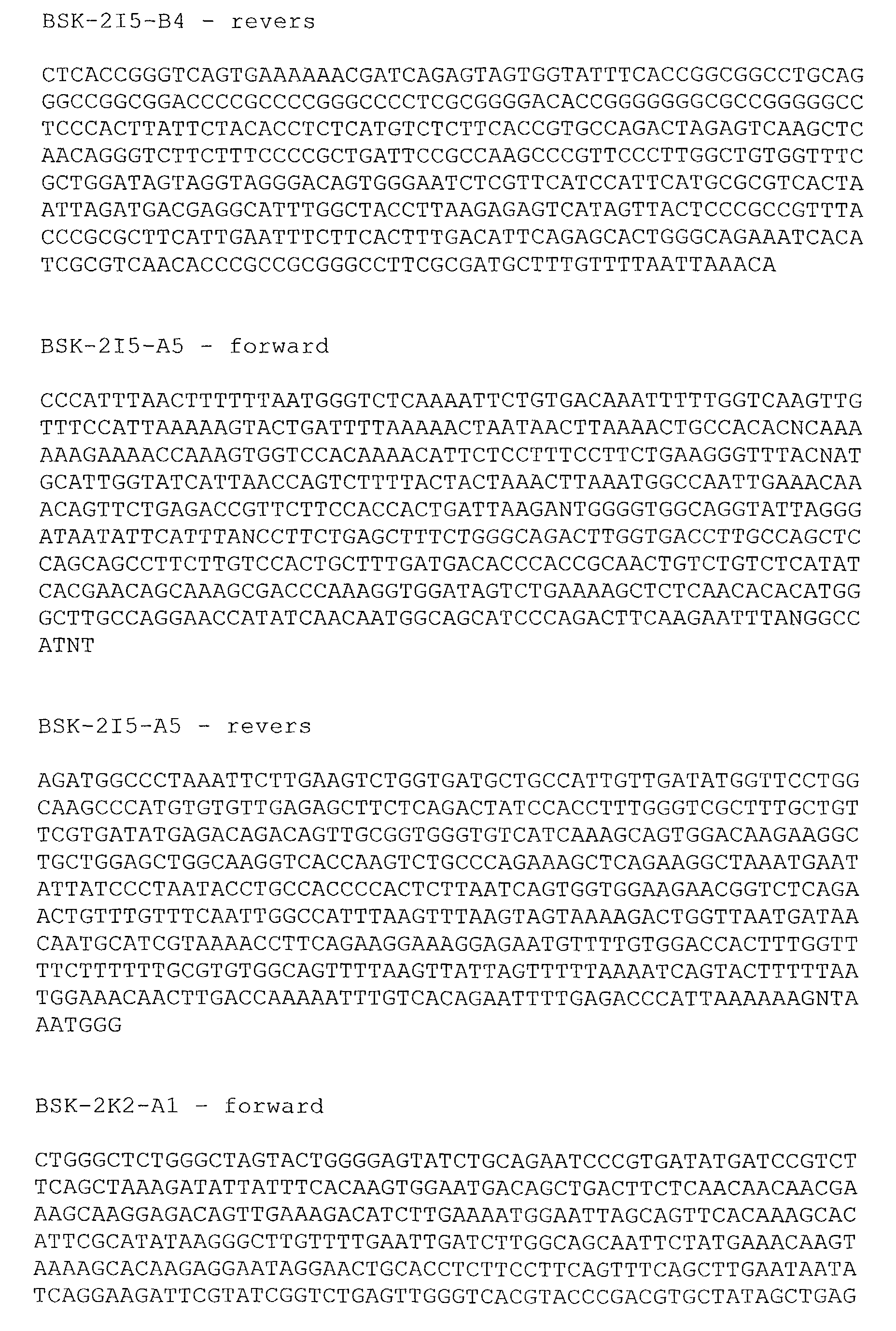

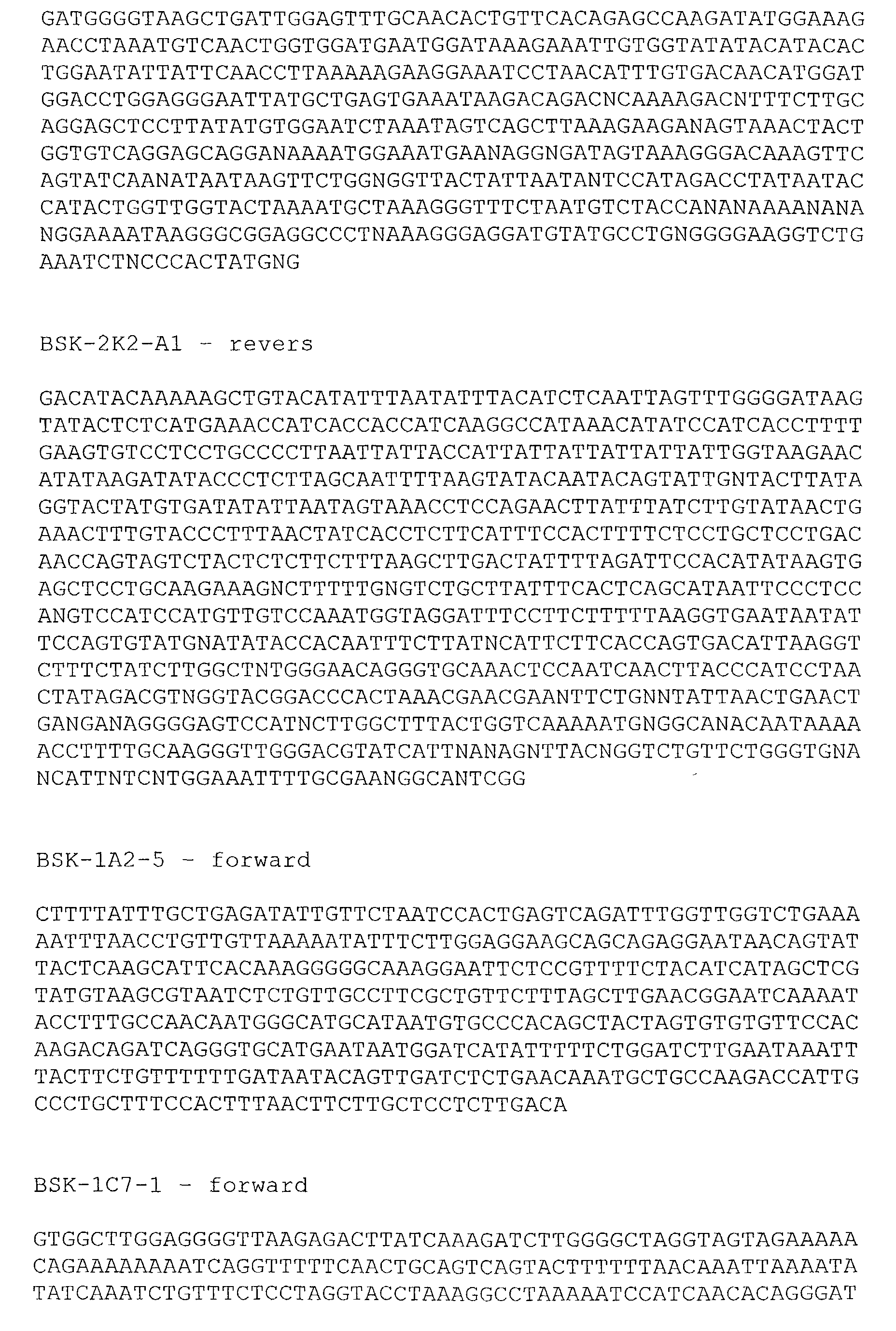

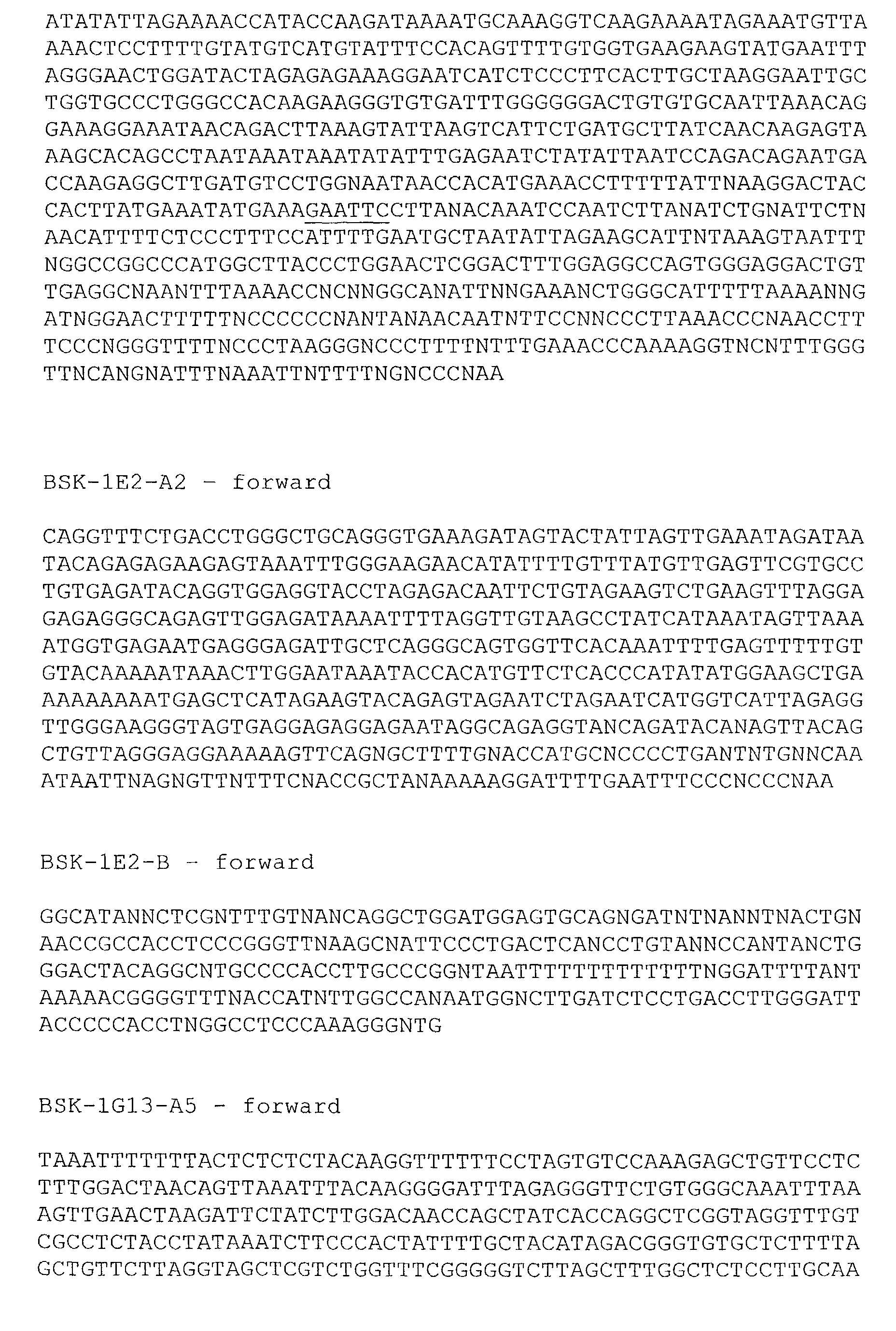

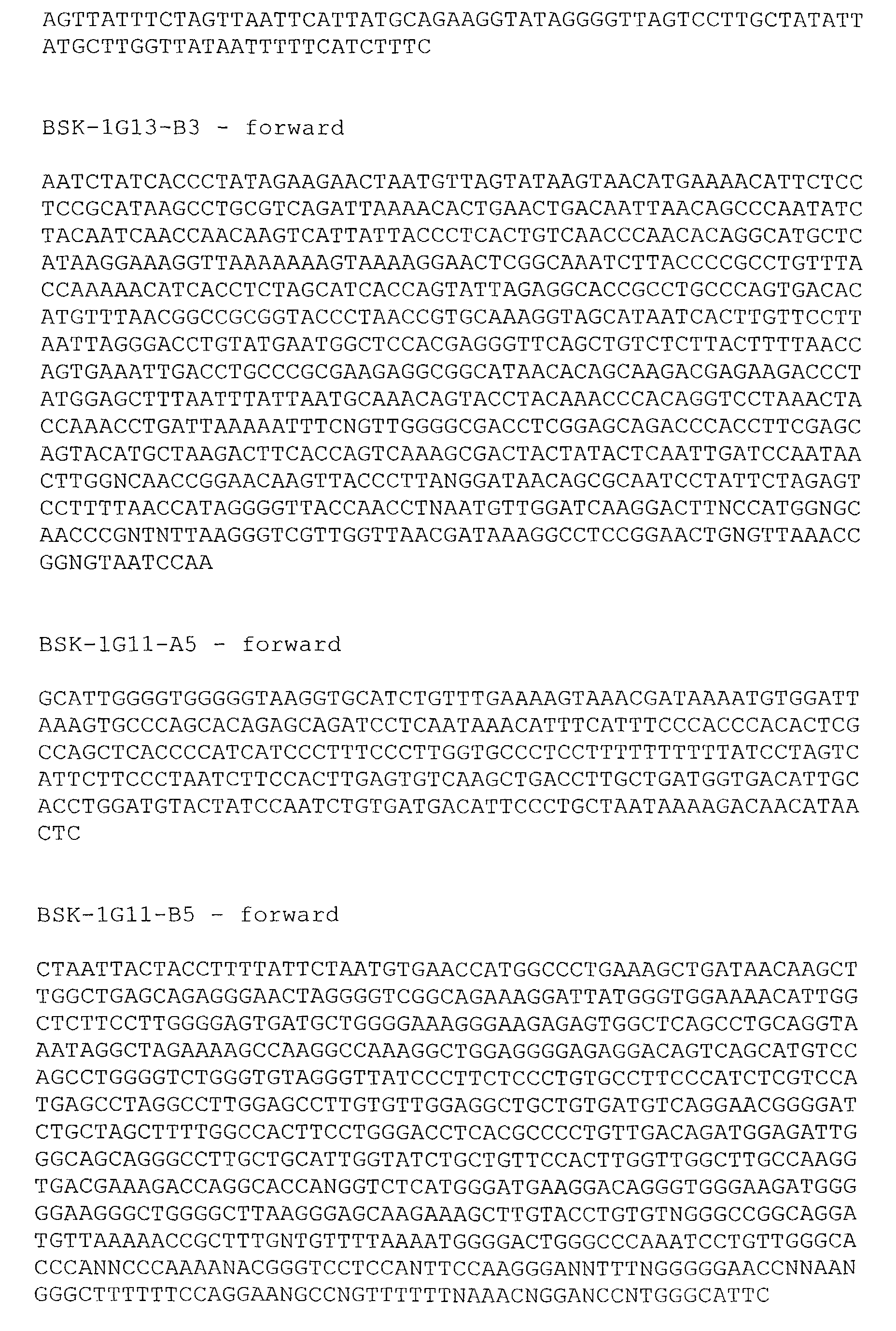

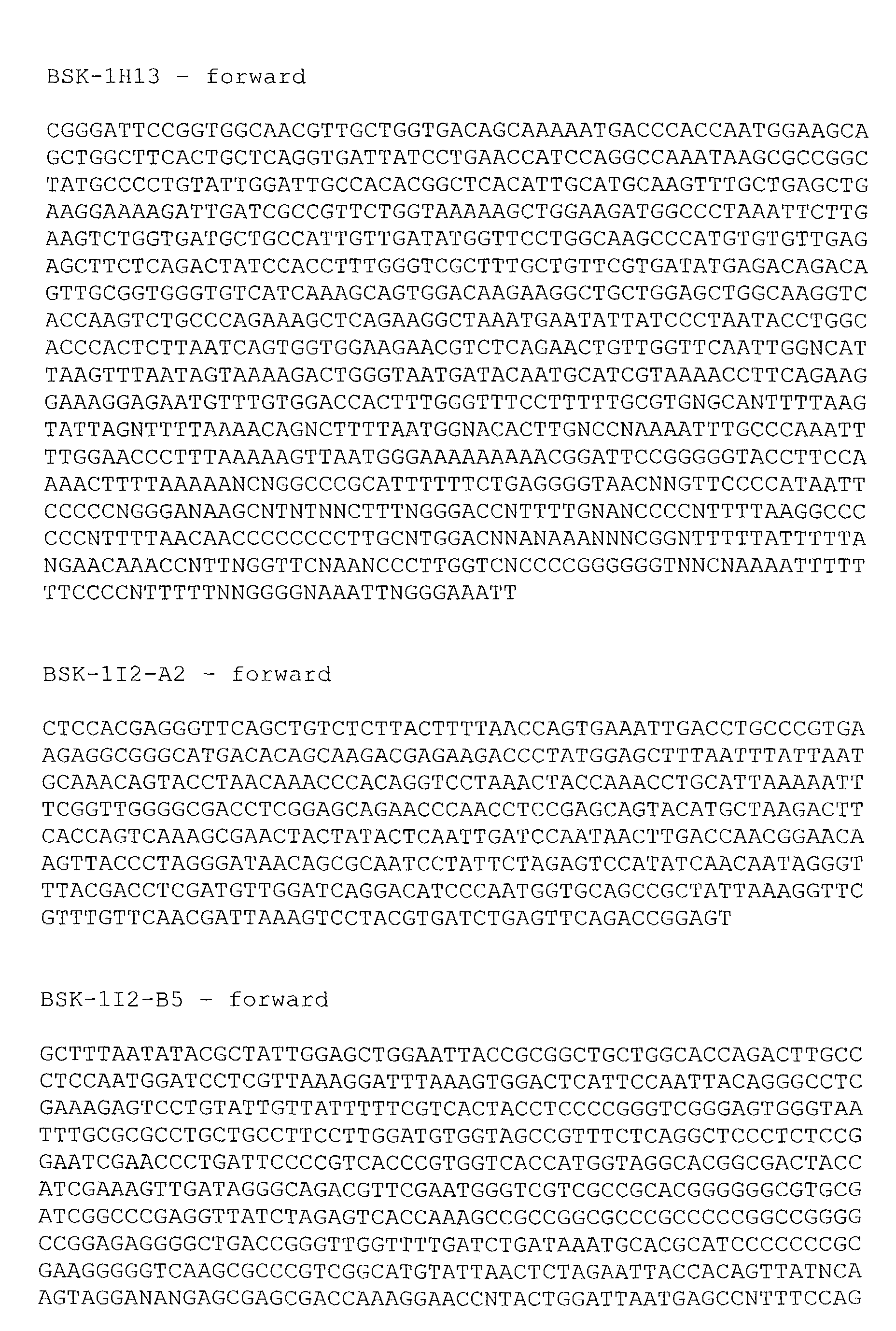

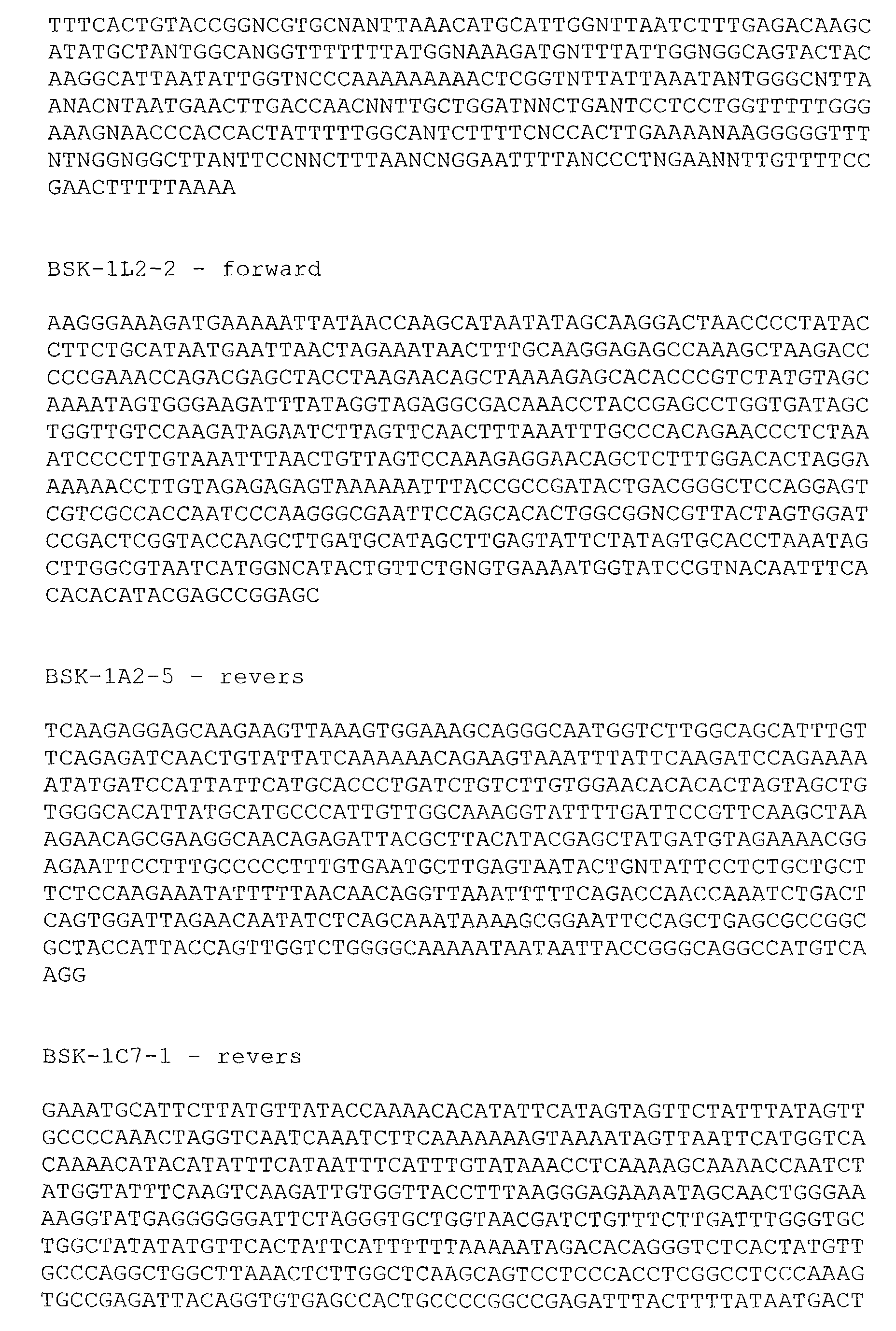

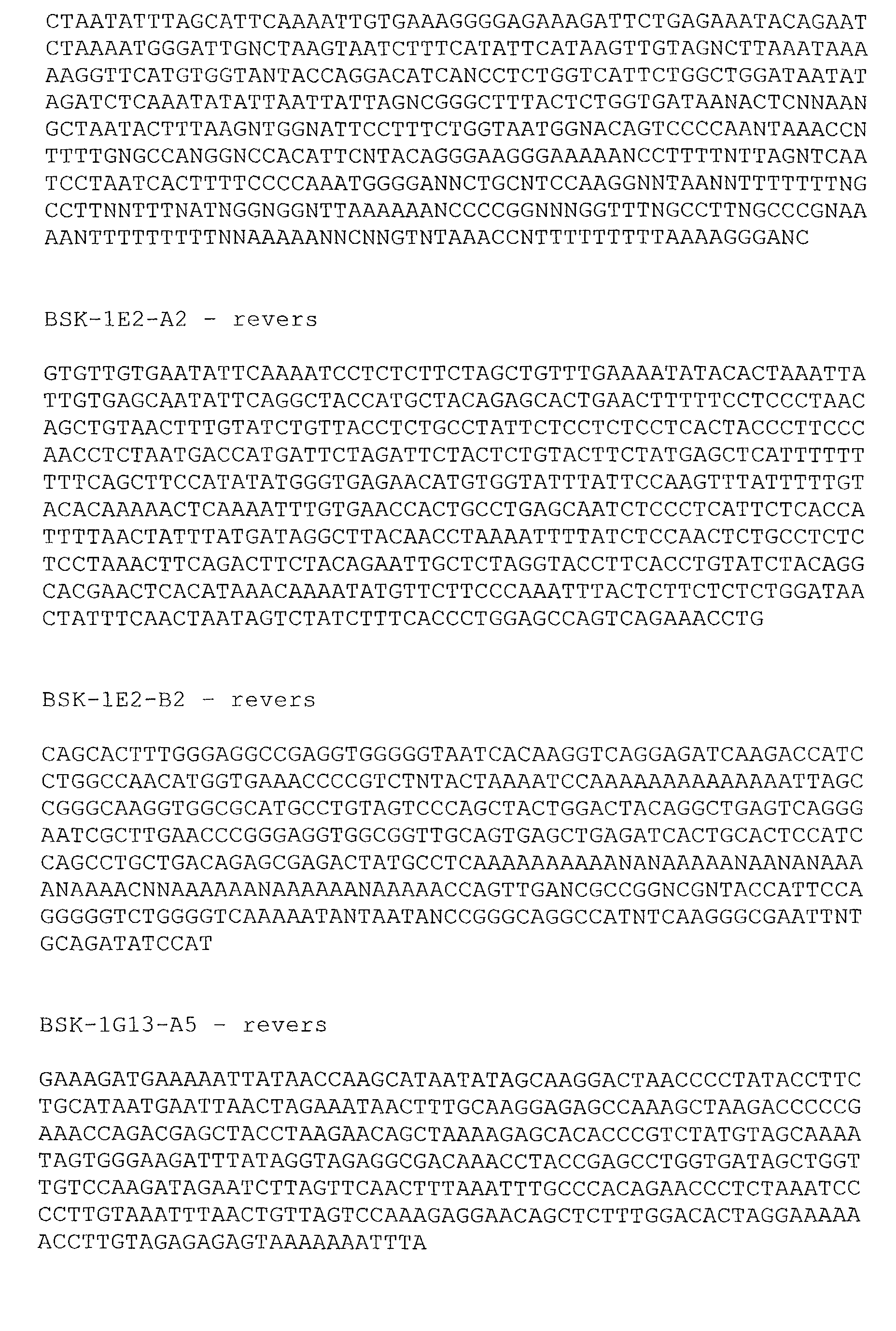

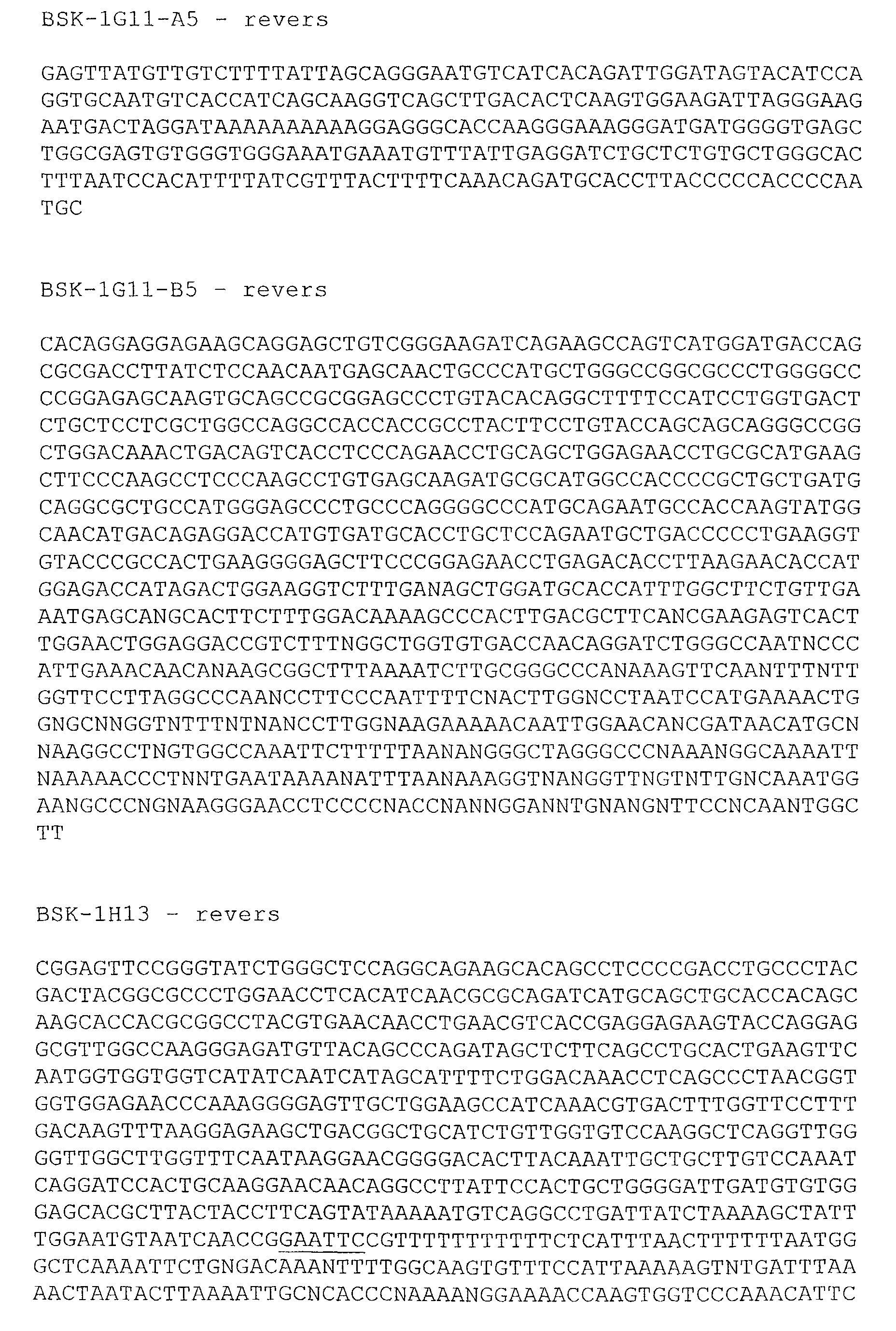

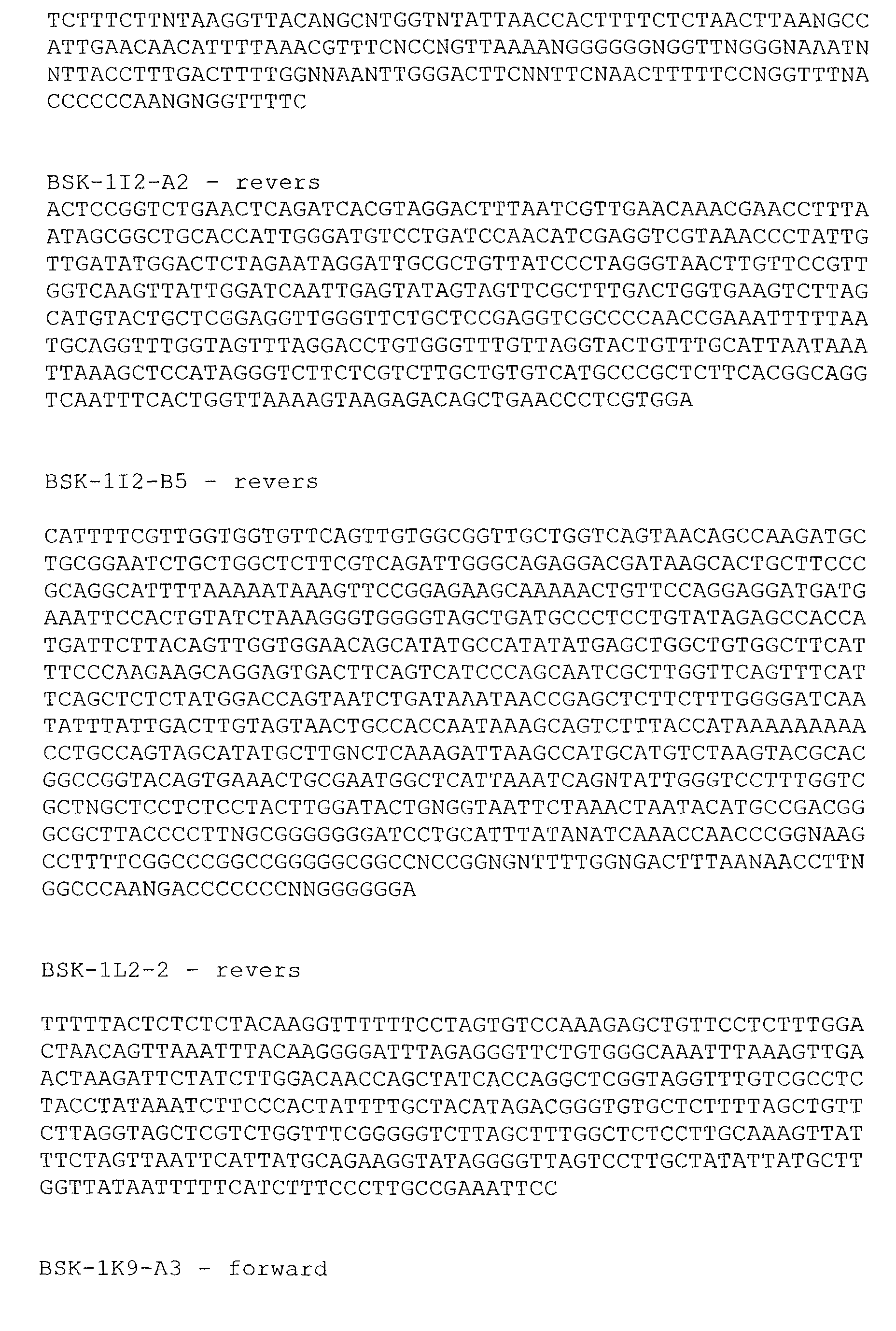

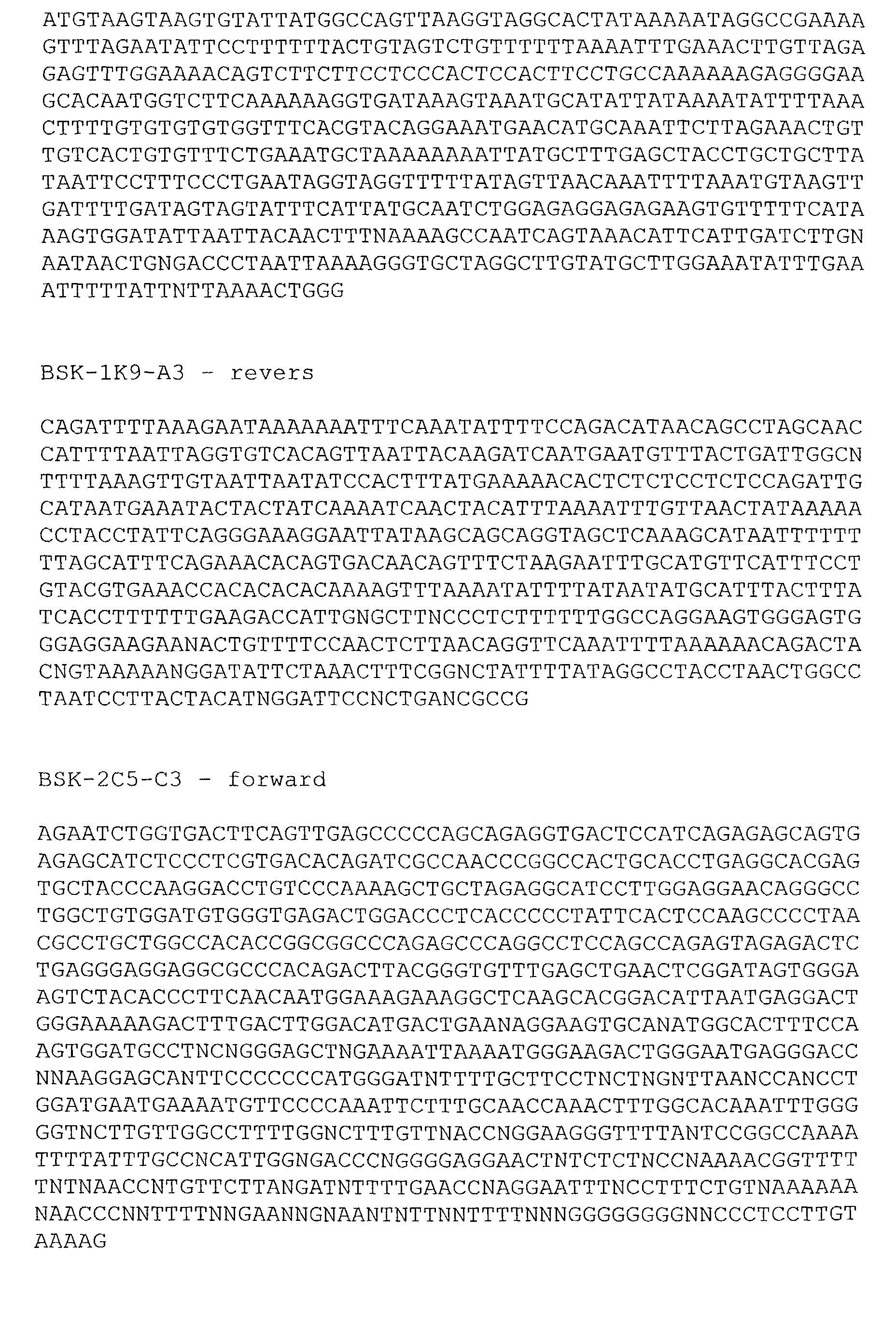

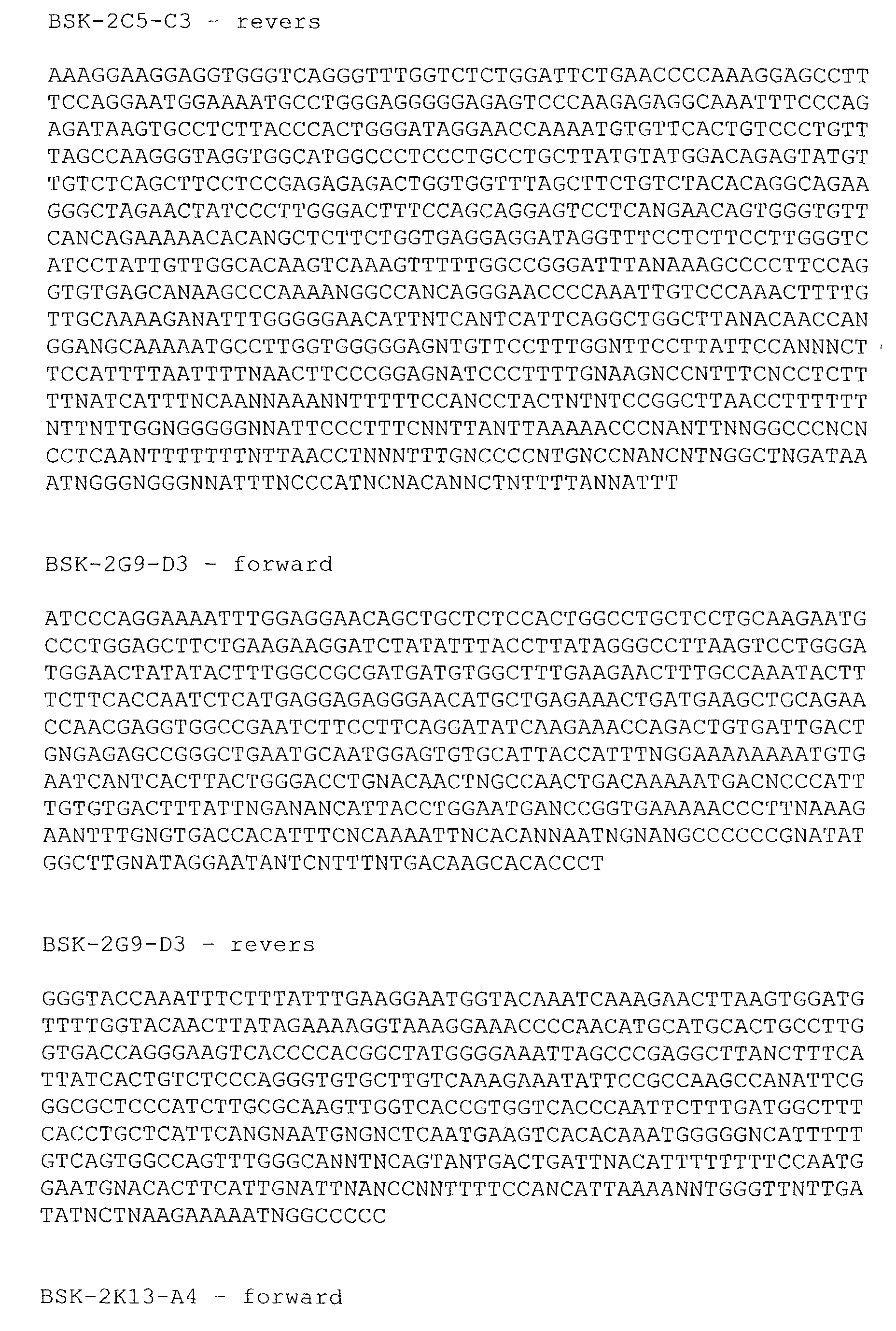

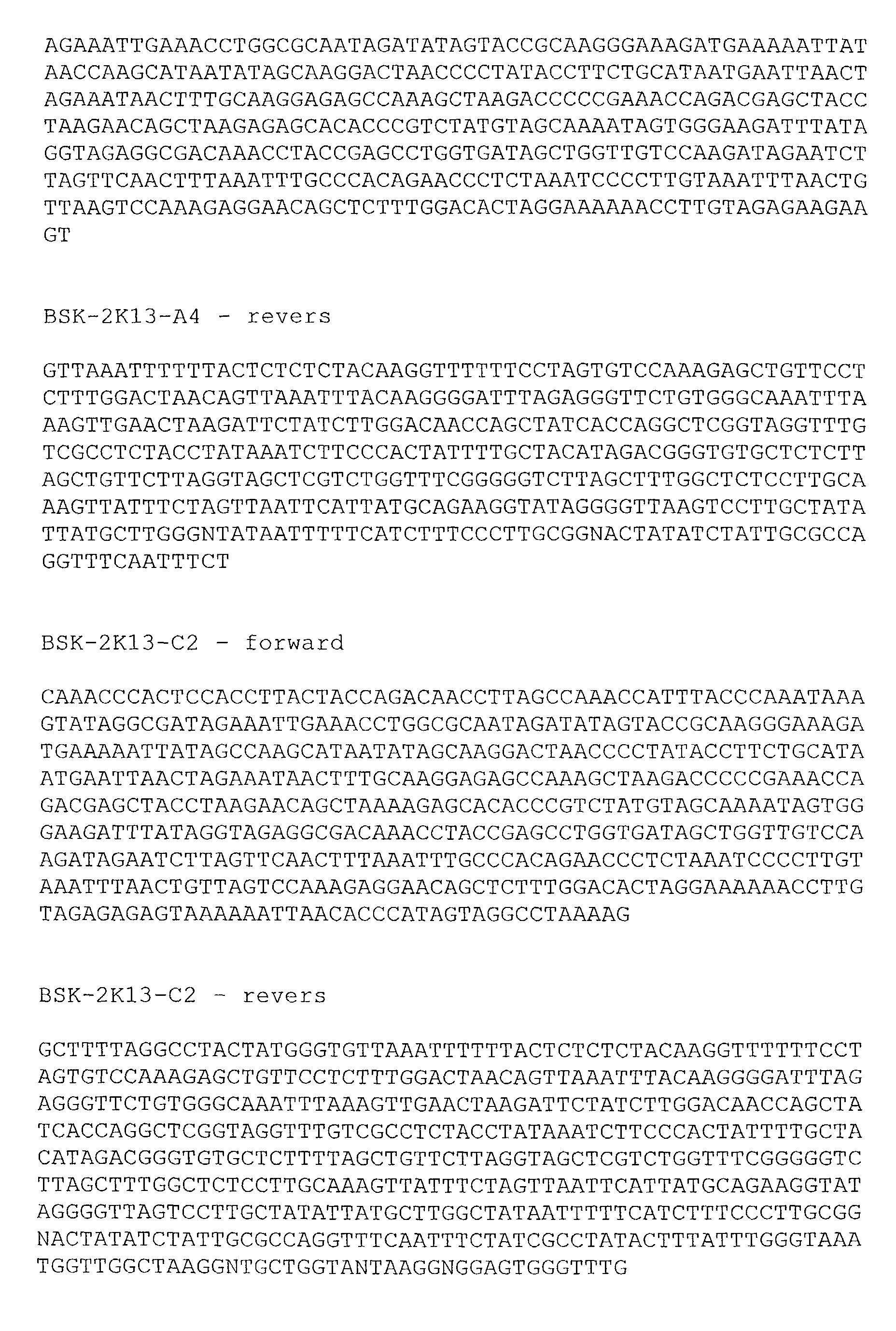

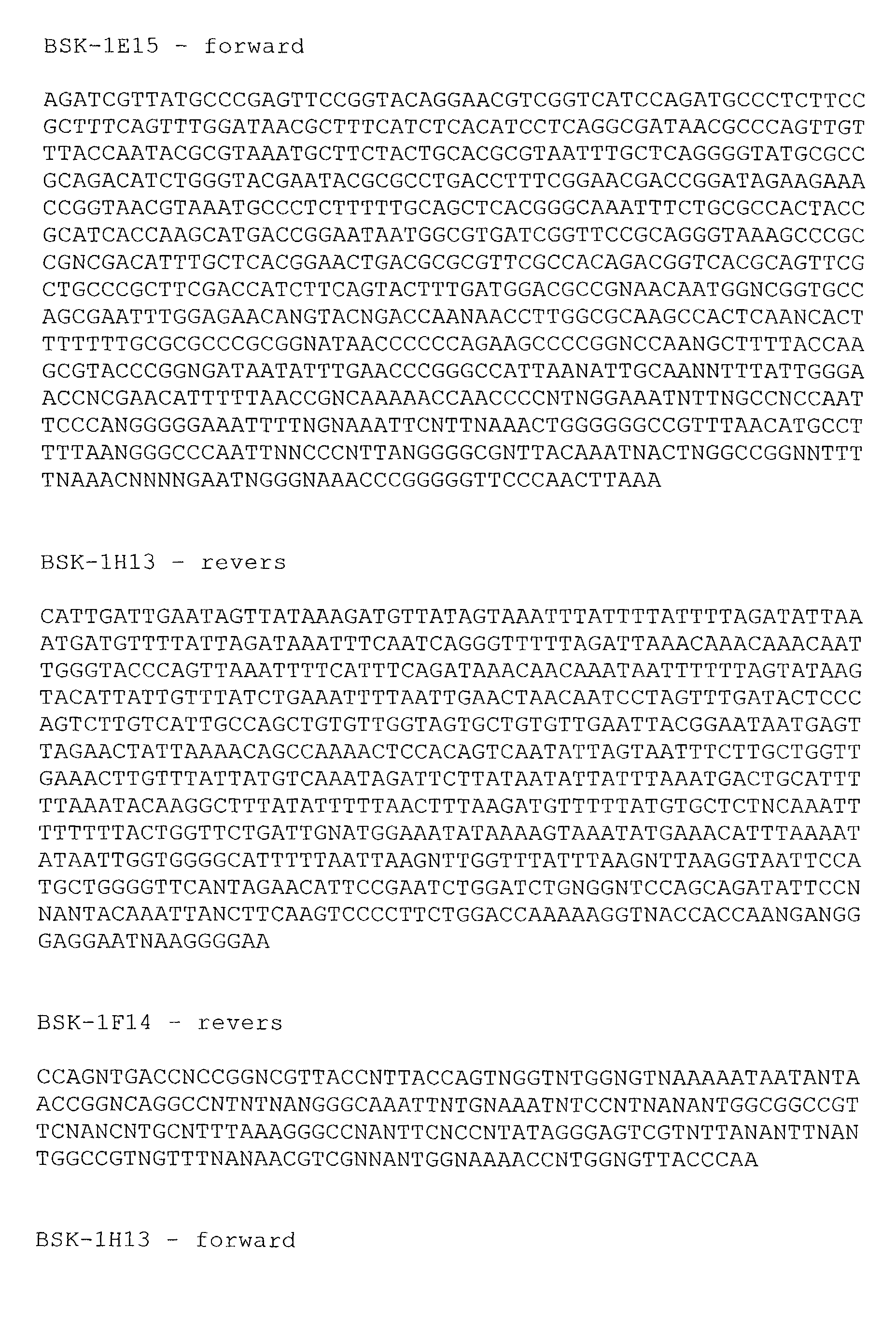

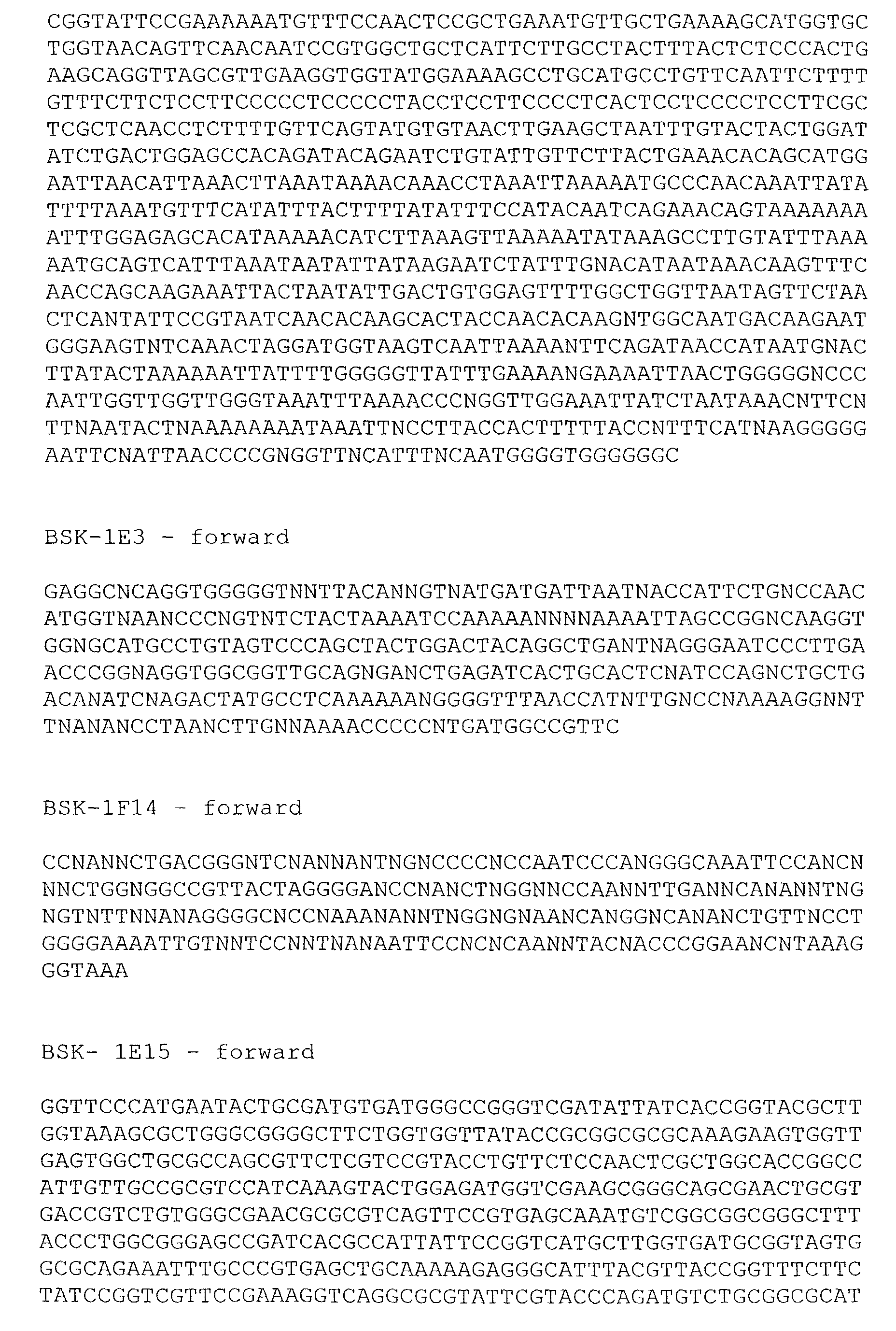

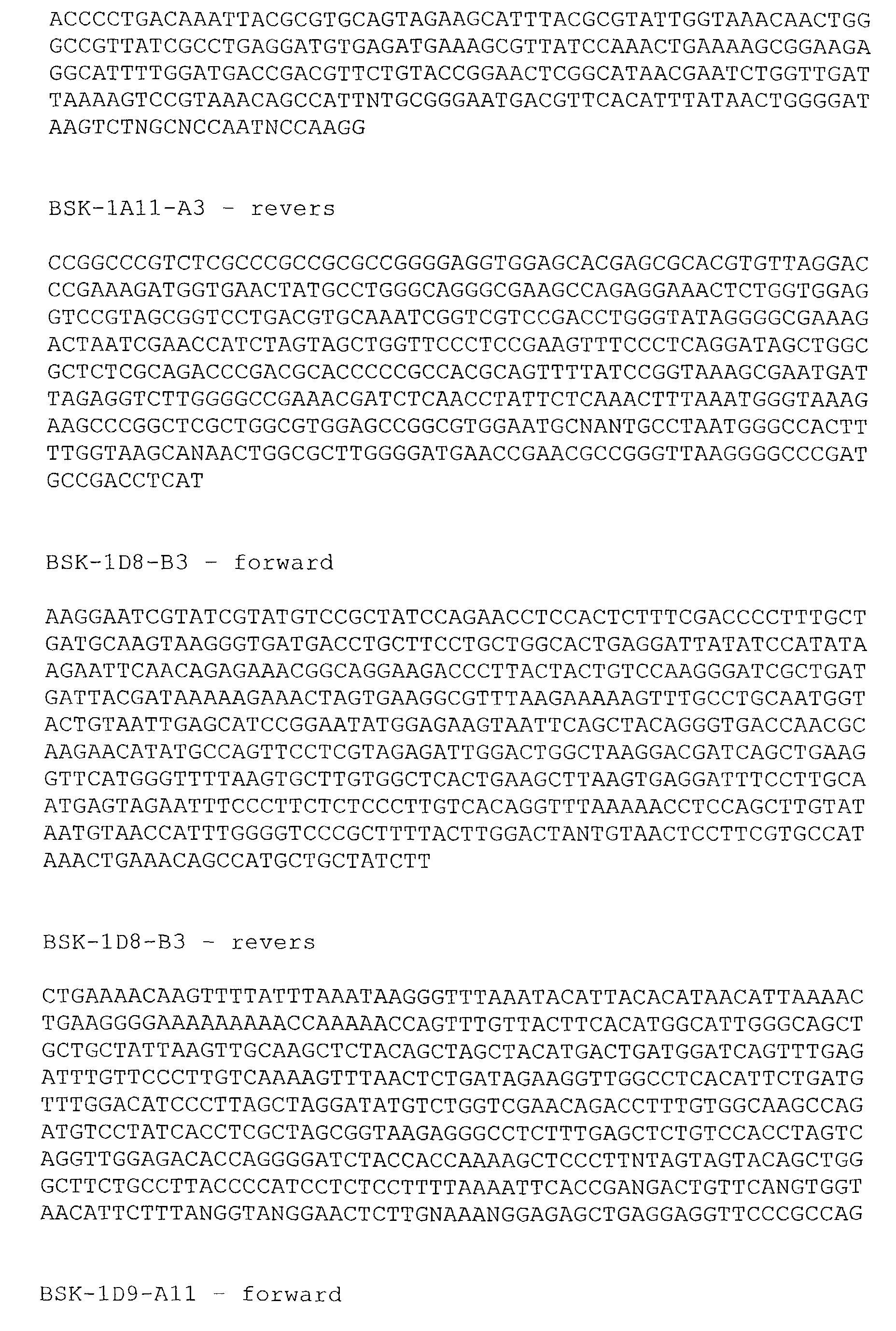

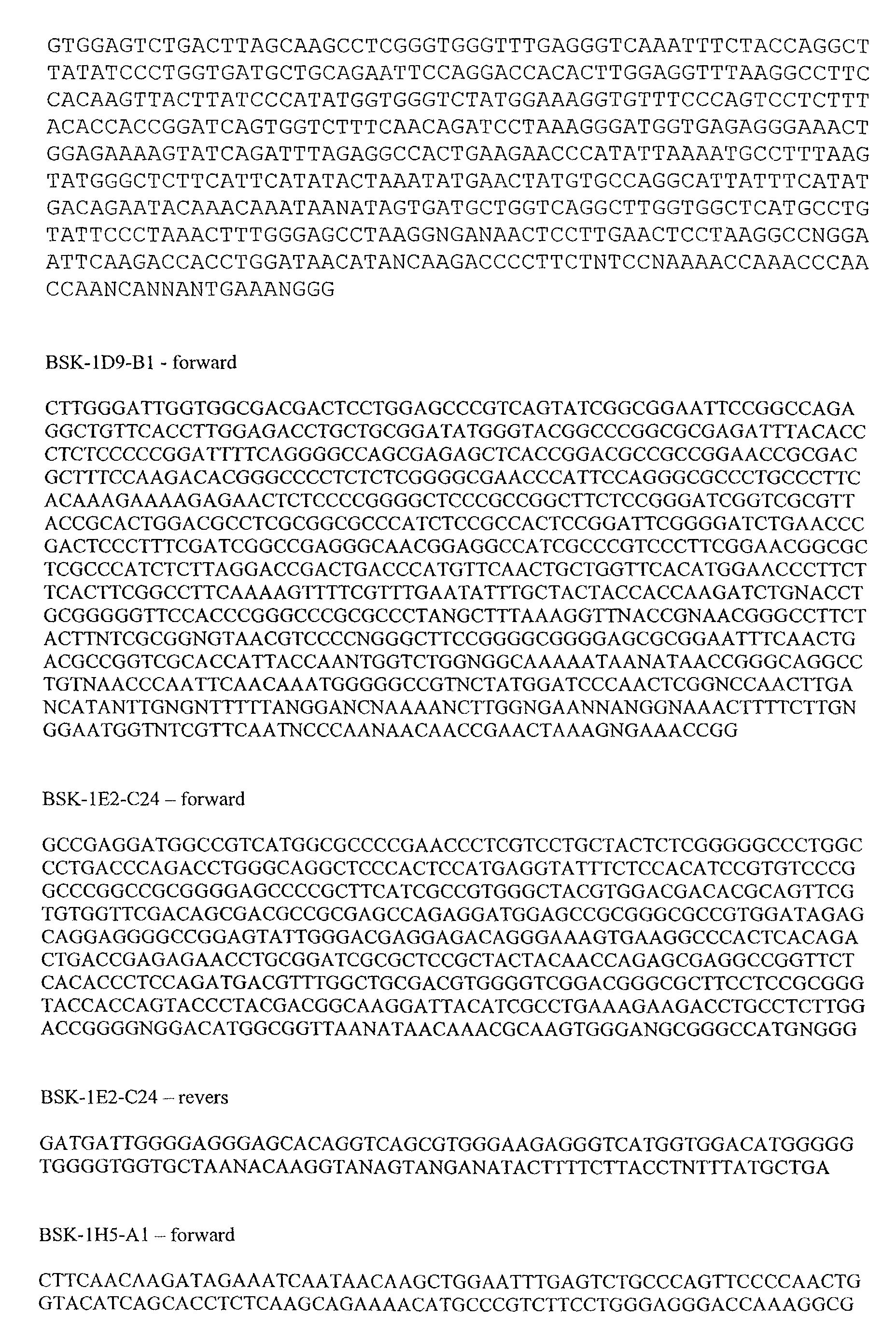

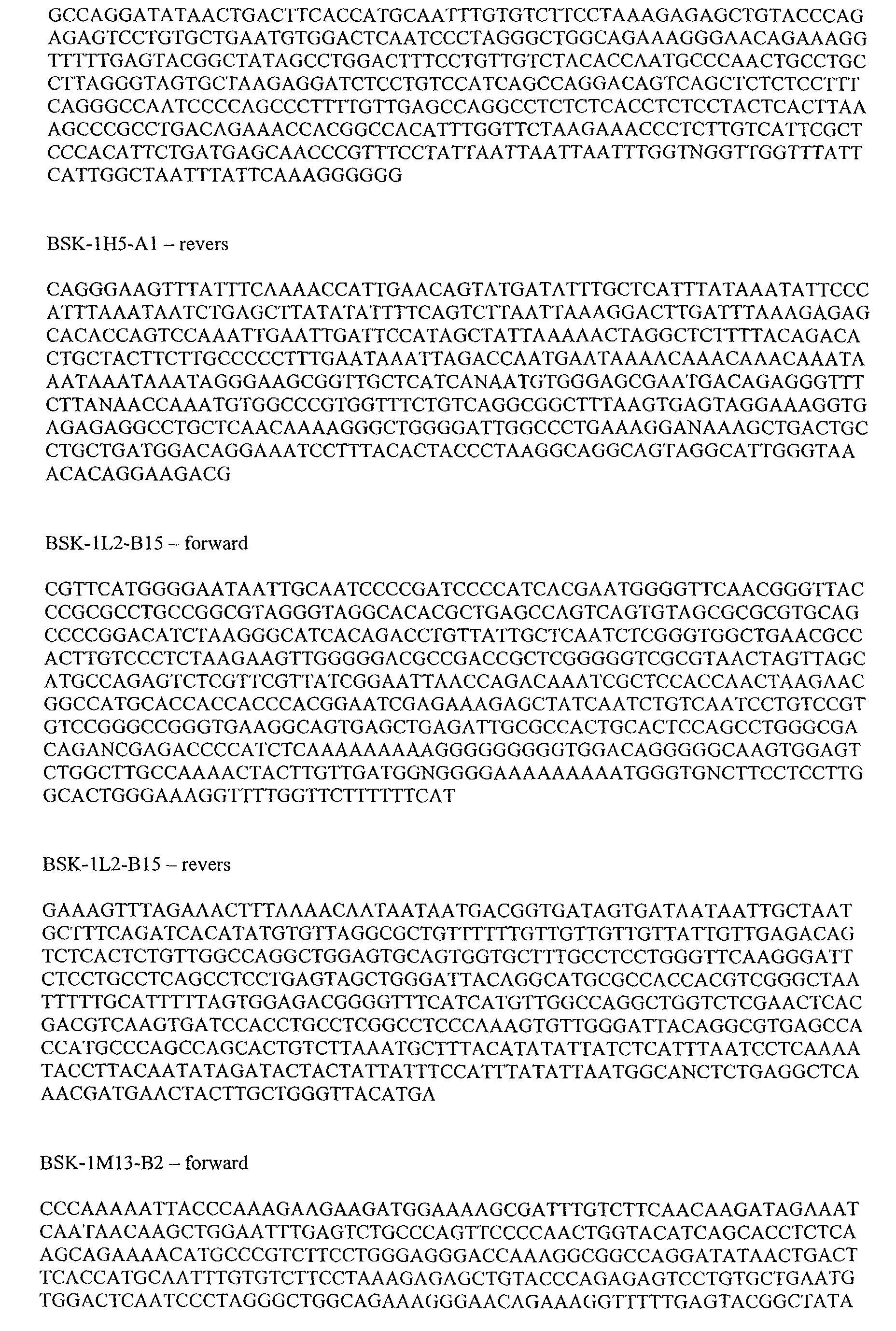

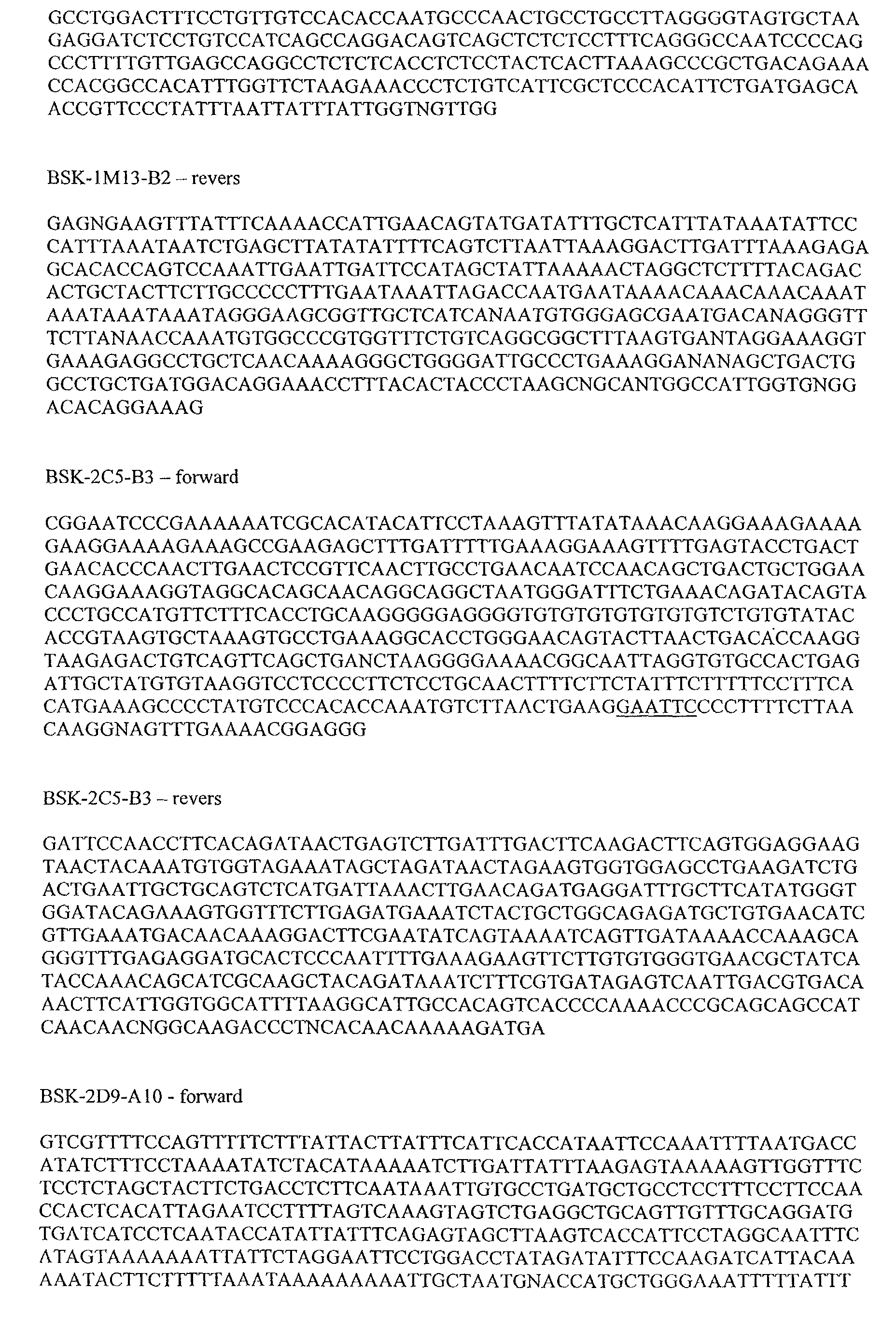

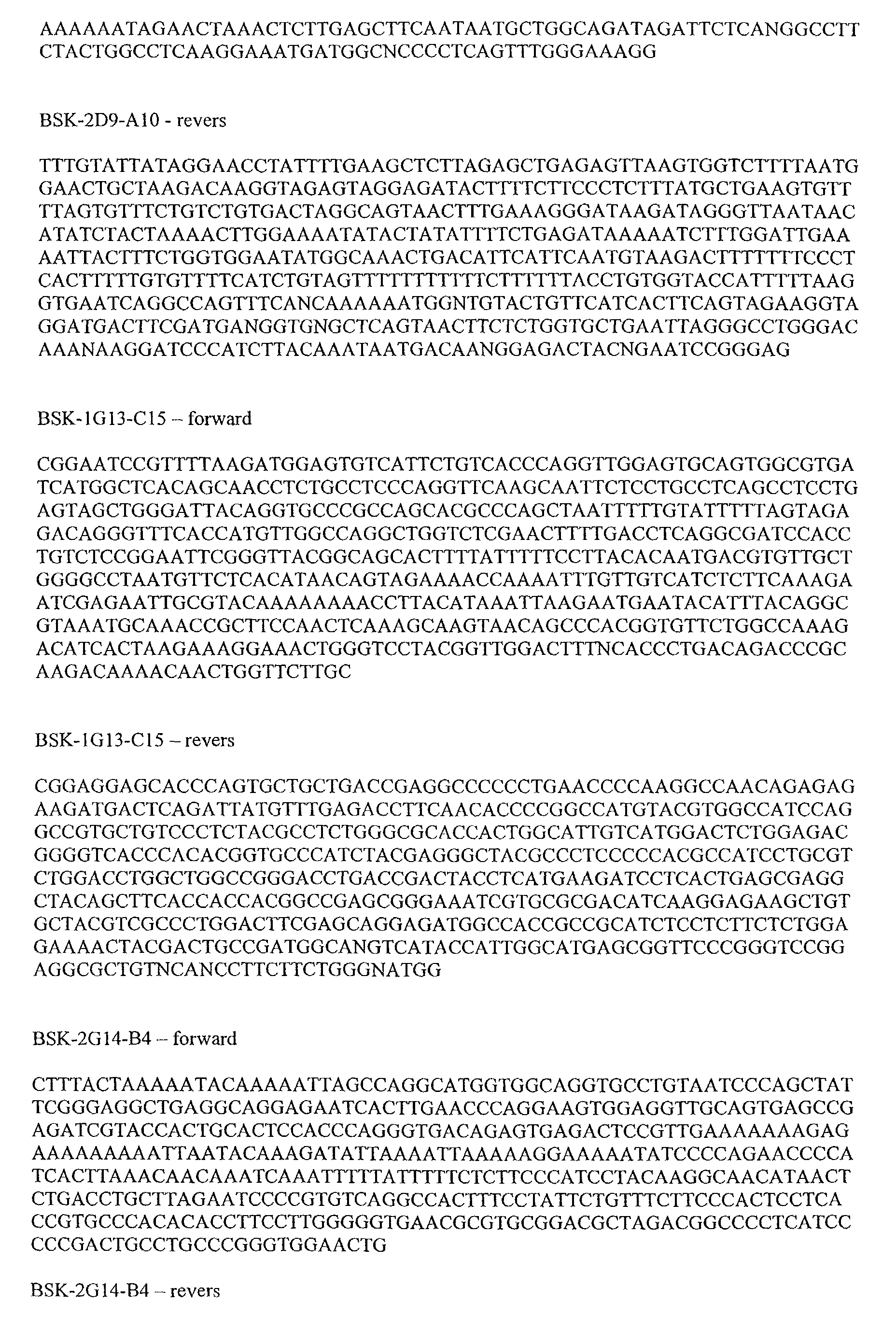

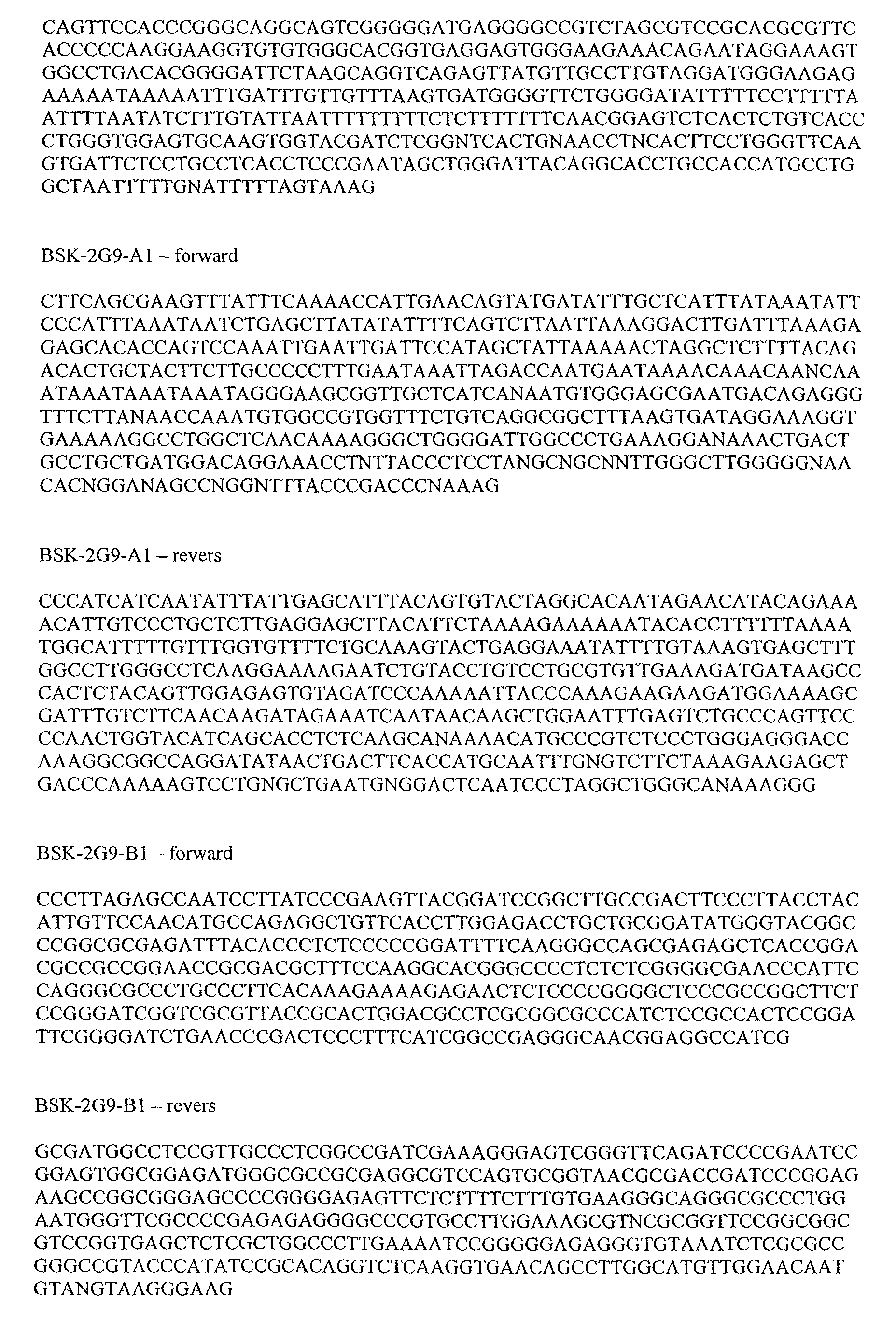

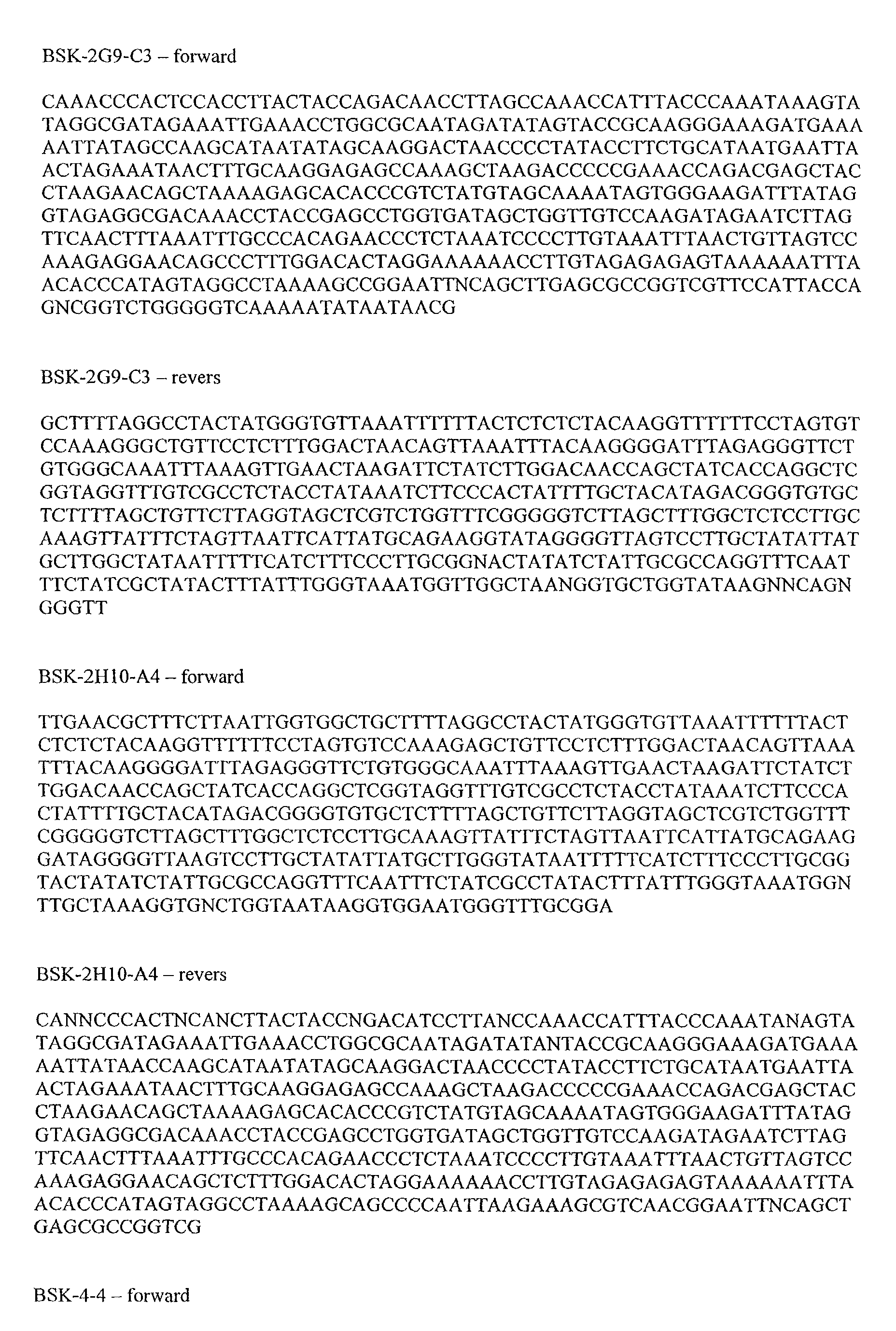

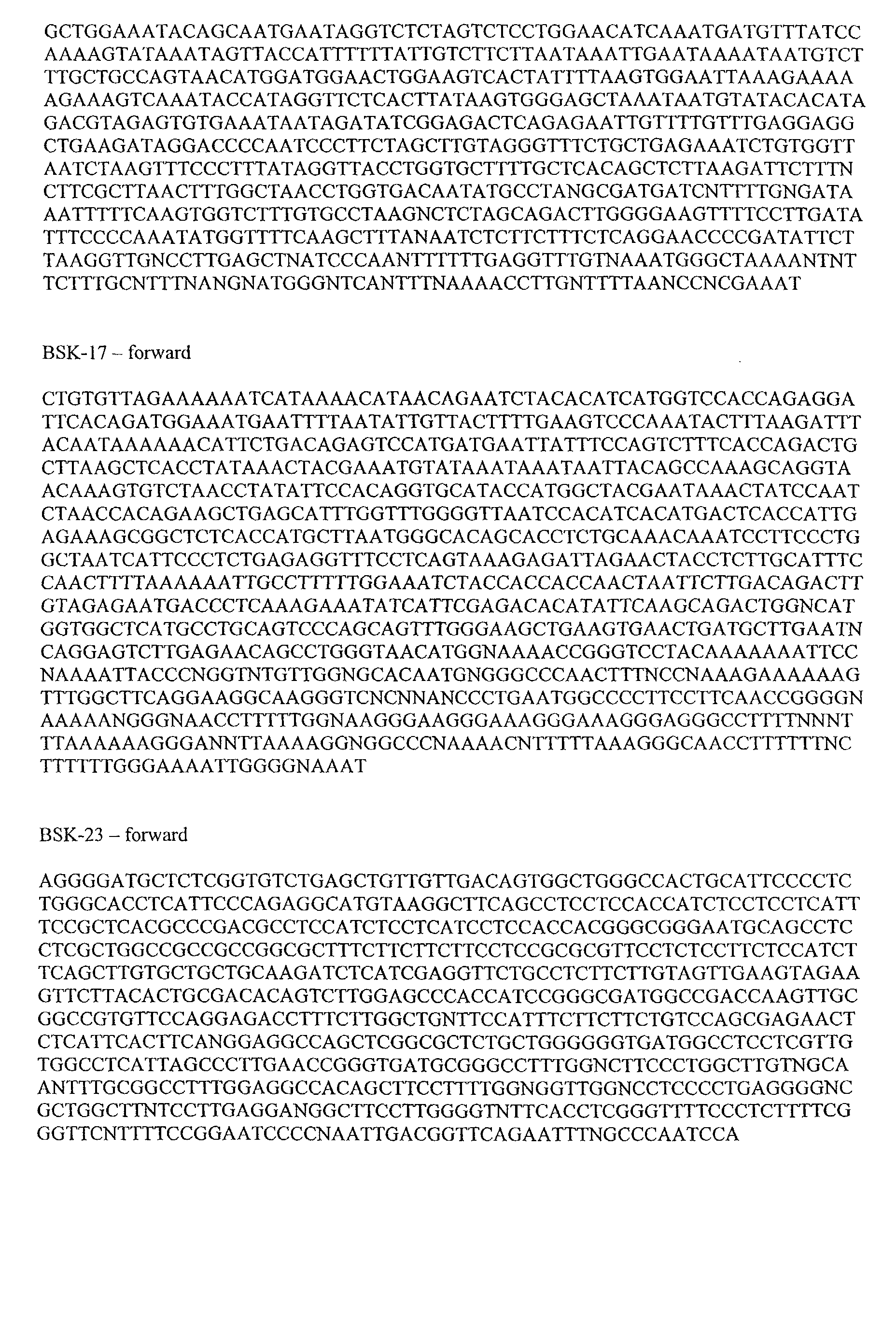

Diese Gene bzw. Werkzeuge, die von diesen Genen Gebrauch machen werden vorzugsweise zur Charakterisierung der molekularen Abläufe insbesondere im Monozyten/Makrophagen-System bei genannten Erkrankungen verwendet.

Die Gene in Tabelle 1 lassen sich, wie ersichtlich in verschiedene funktionelle Gruppen einteilen:
- Zytokine, lösliche Faktoren, Botenstoffe und Liganden, die einen steuernden Einfluß auf das Entzündungsgeschehen haben;
- Rezeptoren, Ionenkanäle und assoziierte Proteine, die eine Signalkette aktivieren, sobald sie selbst aktiviert werden;
- Kinasen, Proteinkinasen und deren Gegenspieler, die für die Aktivierung und Weiterleitung bzw. die Blokkierung der intrazellulären Signalübertragung Schaltstellen sind;
- Signaltransduktionsmoleküle, die Teil der intrazellulären Signalkette sind und zur Steuerung der Genexpression bzw. anderer Folgemechanismen beitragen (Aktivierung präformierter Moleküle);
- Transkriptions- und Translationsfaktoren sowie assoziierte Moleküle, die an der Regulation der spezifischen Genexpression und Proteinsynthese beteiligt sind;
- Ribosomale und ribonukleäre Regulatorproteine, die an der Proteinbiosynthese beteiligt sind;
- Enzyme und Enzym-assoziierte Proteine, die eine regulatorische Funktion für weitere Signalmoleküle besitzen;
- Proteinasen, Matrixmetalloproteinasen, Enzyme und deren Inhibitoren, die den Abbau der Gewebematrix beeinflussen und damit die Organschädigung;
- Onkogene, Protoonkogene, Differenzierungsfaktoren und Gene aus der embryonalen Entwicklung, die Einfluß auf die Zellentwicklung nehmen; (Proliferation, Differenzierung, Dedifferenzierung);
- Apoptosegene und Regulatoren des Zellzyklus und Zelltods;
- akute Phase Proteine; die Ausruck von Zellaktivierung und Entzündung sind;
- Oberflächenmembranmoleküle, die einen spezifischen Zelltyp bzw. eine Zellpopulation charakterisieren;
- andere Moleküle, die bei den genannten entzündlichen Erkrankung erhöht sind, aber keiner der oben genannten Gruppen zugeordnet werden können;
- bislang nicht näher charakterisierte Gensequenzen, die sich in den eigenen differentiellen Genexpressionsanalysen unterschiedlich exprimiert zeigen;
- Kontrollgene, die keine differentielle Genexpression zwischen chronischer Entzündung und Kontrollen zeigen und zur Standardisierung, Vergleichbarkeit und Quantifizierung einer Array-Analyse erforderlich sind.

Bei den Genen oder Gensequenzen kann es sich erfindungsgemäß auch um Allele, Derivate oder Splicingvarianten handeln.

Diese Gene wurden aus differentiellen Genexpressionsanalysen mittels Gensubstraktionsverfahren aus Blut und Gewebeproben chronisch entzündlicher Erkrankungen und geeigneter Kontrollen (Proben von Normalspendern oder Patienten mit degenerativen Erkrankungen) abgeleitet.

Durch diese Art der Selektionierung wurde vorteilhafterweise ein Genpool geschaffen, der sowohl eine Spezifität für die Entzündung, als auch eine Spezifität für Zellen des Monozyten/Makrophagen-Systems aufweist. Subtraktive Genverfahren erlauben es eine Vorauswahl an differentiell exprimierten Genen zu treffen. Dabei sind Methoden wie die Differentielle Hybridisierung oder aber Polymerasen-Ketten Reaktion basierte Verfahren wie Represential Differential Analysis (RDA) und Differential Display (DD) zu nennen. Subtraktive Methoden erlauben somit die Anzahl der auf einen Chip aufzubringenden Gene oder deren Teilsequenzen deutlich zu minimieren, zu spezifizieren, und haben für das Chipverfahren deshalb einen höheren Bedeutungswert mit Entzündungs- und Zellspezifitätscharakter.

Die Analyse der Genexpression in den entzündlichen Erkankungen soll auf die genannten Gene und benannten Gruppen konzentriert werden unter Verwendung der genetischen Information (cDNA, deren Teilsequenzen oder korrespondierenden Oligonukleotide, RNA) in einer Array- oder fluoreszenzzytometrischen Technologie oder durch Verwendung spezifischer Oligonucleotid-Paare in einer quantitativen PCR-Technologie. Es sollen dabei vorzugsweise ausschließlich die Gene in den genannten Gengruppen oder eine Teilgruppe davon für die quantitative DNA-/RNA-High-Throughput Genexpressionsanalyse angewandt werden. Alternativ kann eine umfangreicherer Array (z.B. Unigene Array) verwendet werden und erst im Schritt der bioinformatischen Analyse auf die genannten Gene fokussiert werden.

Insgesamt liefert das erfindungsgemäße molekulare Werkzeug die Möglichkeit, eine Krankheit molekular zu charakterisieren und daraus abzuleiten 1.) eine molekulare Klassifikation und Stadieneinteilung einer klinisch definierten entzündlichen Erkrankung, 2.) die Etablierung eines individuellen Prognoseprofils, 3.) Vorschläge für die molekulare Pathogenese, 4.) Therapieeffekte und Möglichkeiten der Therapieüberwachung und 5.) die Entwicklung neuer Therapiestrategien und pharmakologischer Konzepte zu erlauben.

So sollen DNA-/RNA-Mikroarrays, und das fluoreszenzcytometrische Verfahren, zur Diagnostik, Prognostik und Therapieüberwachung verwendet werden. DNA-/RNA-Mikroarrays sind Anordnungen von molekularen Spezies auf einem Träger, die dem Auffinden von wechselwirkenden Spezies (z.B. komplementäre Nukleinsäuren) dienen.

Bei den anzumeldenden Verfahren als Werkzeuge entzündlicher Erkrankungen aus Anspruch 1 werden deshalb nicht zufällige Gensequenzen, sondern zell-, gewebs- und krankheitsrelevante Genprodukte die einer bereits vorgegangenen Selektionierung unterzogen wurden verwendet.

Für die Array- und Mikroarray-Technologie werden geeignete Trägermaterialien (Glas, Kunststoff) verwendet und chemisch aktiviert oder modifiziert, mit Aminolinkern bindenden reaktiven Gruppen, Metallverbindungen oder Legierungen reaktiv beschichtet, um eine dauerhafte Bindung von der cDNA, cDNA Teilsequenzen, Oligonukleotide und RNA aus den selektiven Genabschnitten der zu untersuchenden Gene zu erreichen. Die individuellen cDNAs, RNAs oder Oligonukleotide werden durch geeignete Druckverfahren ortsspezifisch aufgetragen. Alternativ können auch lithographische Syntheseverfahren zum Einsatz kommen, um entsprechende Oligonukleotidsequenzen direkt und ortsspezifisch auf dem Träger zu synthetisieren.

Bei den DNA-Mikroarrays werden bevorzugt über Aminolinker gekoppelte cDNA's, wie auch DNA-Oligomere auf modifizierte und chemisch aktivierte Glasoberflächen der Biochips aufgebracht.

Dazu ist erfindungsgemäß ein DNA-Mikroarray zur diagnostischen, prognostischen und therapieüberwachenden Analyse von chronisch entzündlicher Erkrankungen, bakteriell induzierten Entzündungen, Tumorerkrankungen, Arteriosklerose und der Sepsis vorgesehen, auf dessen Oberfläche eine Vielzahl selektiver Monozyten/Makrophagen Gene, deren Genabschnitte oder Oligomersequenzen zum Nachweis gebunden sind.

Hierbei kann die Verwendung der selektiven Monozyten/Makrophagen Gene, deren Genabschnitte oder Oligomersequenzen in einem RNA-Mikroarray zum Ansatz kommen.

Bei den selektiven Genen oder deren Genabschnitten handelt es sich vorzugsweise um eine festphasengebundene Genbibliothek, die vorzugsweise als cDNA-Bibliothek kloniert in Phagen oder Plasmiden vorliegt.

Die cDNA-Bibliothek weist mindestens folgende genannte Gene oder Genabschnitte auf, die für die genannten Erkrankungen repräsentativ sind.

Bei den auf den Chip aufzubringenden cDNA Molekülen, deren Teilsequenzen oder aber deren beinhaltenden Oligomersequenzen handelt es sich einerseits um funktionell bekannte Monozyten/Makrophagen Gene zum anderen aber auch um funktionell unbekannte oder bis dato gänzlich unbekannte Gene.

Die benannten, funktionell bekannten Gene sind für die Analytik der Anmeldung notwendig und deshalb neben den in Anlage 1 funktionell unbekannten bzw. vollständig unbekannten Gene zusätzlich zu berücksichtigen.

Für die Array- und Mikroarray-Technologie werden geeignete Trägermaterialien (Glas, Kunststoff) verwendet und chemisch aktiviert oder modifiziert, mit Aminolinkern bindenden reaktiven Gruppen, Metallverbindungen oder Legierungen reaktiv beschichtet, um eine dauerhafte Bindung von der cDNA, RNA oder Oligonukleotide aus den selektiven Genabschnitten der zu untersuchenden Gene zu erreichen. Alternativ werden Trägermaterialien (Nyolonmembranen) zur Aufbringung verwendet. Die individuellen cDNAs, RNAs oder Oligonukleotide werden durch geeignete Druckverfahren (Piezo- oder Nadeltechnologie) ortsspezifisch aufgetragen. Alternativ können in situ Oligonukleotidsyntheseverfahren zum Einsatz kommen, um entsprechende Oligonukleotide direkt und ortsspezifisch auf dem Träger zu synthetisieren.

Für ein fluoreszenzzytometrisches Verfahren werden individuelle cDNAs, RNAs oder Oligonukleotide aus selektiven Genabschnitten der zu untersuchenden Gene gebunden an Träger-Perlen (Beads) aus Kunststoff oder Glas mit definierter, für jede spezifische Nukleotidsequenz individuell zuordenbarer Größe. Dabei können die Perlen (Beads) chemisch aktiviert oder modifiziert, mit Aminolinker bindenden reaktiven Gruppen, oder anderen reaktiven Gruppen in Form von Metallverbindungen oder Legierungen beschichtet sein, um eine dauerhafte Bindung der cDNAs, RNAs oder Oligonukleotide zu gewährleisten. Die Bindung der cDNAs, RNAs oder Oligonukleotide erfolgt durch Inkubation der jeweiligen Perlen (Beads) einer Größe in geeigneten Lösungen, die die jeweilige cDNA, RNA oder das Oligonukleotid enthalten und eine feste Bindung an die Perlen (Beads) vermitteln. Die verschiedenen Populationen an Nukleotidsequenz tragenden Perlen (Beads) werden, wobei jede individuelle Nukleotidsequenz einer definierten Perlen-Größe oder aber Fluoreszenzfarbstoffen zugeordnet ist, zu gleichen Perlenanteilen so gemischt, dass eine definierte Auswahl an Genen gleichzeitig in einem Ansatz untersucht werden kann.

Für die Array- und auch fluoreszenzzytometrische Technologie wird die zu untersuchende Probe markiert z.B. direkt mit einem Fluoreszenzfarbstoff oder mit einem Brükkenmolekül wie z.B. Biotin oder Digoxigenin für eine spätere Bindung des Fluoreszenzfarbstoffs mit oder ohne Signalverstärkung bevorzugt über Streptavidin oder Anti-Digoxigenin-Antikörper gekoppelt an Streptavidin. Bei der Fluoreszenzzytometrie kann entsprechend dazu der Nachweis auch über fluoreszenzspezifische Antikörper die auf der Oberfläche der Beads ein definiertes Zielantigen erkennen, mit oder ohne Verstärkungen mit dem Biotin-Streptavidin System durchgeführt werden. Alternativ dazu bieten sich in den Arrayverfahren und der Fluoreszenzzytometrie Verstärkersysteme über Metall-/Edelmetallkomplexe an. Weiterhin kann auch auf filtermembranbasierter Technologie mit Radioaktivität im Nachweisverfahren gearbeitet werden.

Die Hybridisierung der markierten Probe mit dem Array oder den Nukleotidsequenz tragenden Perlen (Beads) erfolgt quantitativ und kann nach abschließender Markierung mit einem Fluoreszenzfarbstoff mittels entsprechender Laserscanner auf dem Array oder in einem FACS-Analysegerät quantitativ ausgelesen werden.

Bei den Arrayverfahren und der Fluoreszenzzytometrie erfolt die Analyse der Rohdaten (Signalintensität) und biometrische Auswertung über verschiedene auf dem Markt erhältliche Software, oder mit den Programmen wie z.B Mikroarray Suite (Affymetrix) bei der Array-Technologie oder z.B. BD CBA Analysis Software (BD Biosciences).

Für die Interpretation hinsichtlich einer diagnostischen Zuordnung einer Krankheit zu einer molekular definierten Klassifizierung, einer Prognoseabschätzung, einer Therapieüberwachung oder -empfehlung, einer molekularen Pathogenese und der Entwicklung neuer Therapiekonzepte sind mehrere Vorgehensweisen möglich und erforderlich. Es kann eine rein quantitativ vergleichende Untersuchung der Genexpression individueller Gene zwischen den Proben einer erkrankten Person und einer Kontrollperson oder den Proben der erkrankten Person vor und während und zu verschiedenen Zeitpunkten einer Therapie oder des Krankheitsverlauf erfolgen z.B. für die Interpretation der molekularen Pathogenese und die Entwicklung neuer Therapiekonzepte. Für die diagnostische Interpretation (Gruppenund Stadieneinteilung, Prognoseabschätzung, Therapieüberwachung und -erfolgskontrolle) ist die Anwendung von Algorithmen z.B. durch die Verknüpfung der Expressionswerte zweier oder mehrerer Gene aussagekräftiger. Diese kann erfolgen durch Addition, Subtraktion, Multiplikation, Division, Exponential- oder Logarithmusfunktion als jeweils alleinige Rechenoperation des Algorithmus oder durch eine komplexe Kombination der verschiedenen Rechenoperationen in einen Algorithmus. Es können dabei 1.) die Expressionsdaten von allen oder einzelnen Genen einer Gruppe, als auch 2.) die Expressionsdaten von allen oder einzelnen Genen aus verschiedenen Gruppen durch einen Algorithmus miteinander verbunden werden.

Die erfindungsgemäßen Werkzeuge werden nach nachstehendem Verfahren hergestellt:

Die in den Verfahren genutzten selektiven molekularen zell- / gewebs- und entzündungsspezifischen Werkzeuge wurden durch Gensubstraktion erzeugt.

Die Herstellung von cDNA erfolgt durch Umschreibung der mRNA durch reverse Transkription. Die mRNA enstammt entzündlichem humanen Blutmonozyten oder dem entzündlichen Gewebsarealen mit hoher Makrophageninfiltration von Patienten mit entzündlichen Erkrankungen, die konventionell oder aber alternativ über Positiv- bzw. Negativselektion gereinigt werden.
Insbesondere die Negativselektion wird benutzt, um Aktivierungsartefakte, die während der Reinigung entstehen können, zu vermeiden.

Die revers transkribierten cDNA-Einzelstränge werden mit DNA-Polymerase in cDNA-Doppelstränge umgeschrieben.
Für die Differentielle Hybridisierung erfolgt die Klonierung in Plasmid- und/oder Phagen-Vektoren, uni- oder bidirektional gerichtet über Adapter- oder Linkermoleküle. Nach Transformation der Plasmide oder Verpackung der Phagenvektoren erfolgt die Differentielle Hybridisierung mit markierten cDNA Sonden(Radioaktivität, Digoxigenin, Biotin oder andere).

Ausgehend von der umgeschriebenen cDNA erfolgt alternativ die differentielle Expressionsanalyse über DD oder RDA oder über klassische Gensubtraktionsverfahren durch Hybridisierung der mRNA des einen mit der komplementären cDNA des zweiten Zellpools.
Für die Nutzung der selektiven differentiellen cDNAs werden entweder die klonierten Einheiten vorzugsweise mit NH₂-markierten Standardprimern für Lambda, T3 / T7 / SP6 / M13 und anderen vektorspezifischen Sequenzen über PCR amplifiziert, und dann über Säulenauschlußverfahren gereinigt. Danach erfolgt die Konzentrierung der NH2-markierten DNA durch Präzipitation mit anschließender Zentrifugation.

Nach der Kopplung der DNA auf der modifizierten kopplungsfähigen Oberfläche erfolgt die Sondenhybridisierung direkt mit Farbstoff markierter cRNA oder farbstoffmarkierten revers transkribierten cDNA Sonden, die dem Patientenmaterial (Blut oder Gewebe) entstammen.

Prinzipiell existieren neben dem o.g. Immobilisierungsverfahren mit NH2-cDNAs auch Verfahren Oligonukleotide zu spotten oder zu drucken (Nadel- / Piezotechnologie) oder aber auch *in situ* synthetisierte DNA Arrays herzustellen. So können qualitäts- und sequenzidentische DNA-Arrays hochparallel im Waferformat für diese Produktionstechniken hergestellt werden. Bei der *in situ* Synthese werden die einzelnen DNA-Arrays und Spots auf denselben dabei auf dem Wafer durch eine hochintegrierte, mikrosystemtechnisch hergestellte Druckmaske angesteuert. Die Anzahl der Druckporen pro DNA-Arrays und deren Geometrie zueinander ermöglichen dabei sehr hohe Integrationsdichten mit einer Einzelgröße von < 3 µm pro DNA-Chip. Bei diesem Mikrosiebdruckverfahren kommt es dabei in einem ersten Schritt zum Aufbringen der Maske auf den Substratwafer der mit Hilfe von Justiermarken positioniert wird. Im eigentlichen Druckprozeß werden die einzelnen Druckporen mikrofluidisch über einen Kanal angesteuert. Die entsprechende Proben-Substanz (z.B. Oligomernukleotide mit einer Länge von 20-50 Nukleotiden) kann danach mit der chemisch reaktiven und modifizierten Oberfläche des Wafers reagieren und geht eine kovalente Bindung mit der Array-Oberfläche ein. Nach dem Druckvorgang wird die Maske entfernt und gespült. Nach erneuter Positionierung (Versetzen um eine entsprechende Spot-Einheit) kann der Zyklus erneut durchlaufen werden. Im in situ Verfahren können so reproduzierend niedrig integrierte DNA-Arrays mit 400 Spots pro Einheit oder aber hochintegrative Arrays mit einer Größenordnung von bis zu 20.000 Spots hergestellt werden.

Der Erfindung liegt die Überlegung zugrunde, dass ein selektives differentielles Genspotmuster, dessen zugrundeliegenden Sequenzen durch ein stark selektionierendes Verfahren erzeugt wurden, auf einem Mikroarray genutzt werden kann, wobei gegenüber bisher verwendeten DNA-Mikoarrays der Vorteil in einer kostenreduzierten Herstellung und einer Aufwand- und kostenreduzierten Analytik besteht.

Auf den RNA-Mikroarrays werden Blut- oder aber gewebsspezifische RNA-Moleküle die aus Patientenmaterialien chronisch entzündlicher Erkrankungen, bakteriell induzierten entzündlichen Erkrankungen, Tumorerkrankungen, Artheriosklerose, der Organ- und Gewebstransplantationen und Sepsis gebunden. Der qualitative / quantitative Nachweis der Transkriptmenge relevanter Gene erfolgt dann mit den Spezies der im Abschnitt DNA-Mikroarray beschriebenen selektionierten Gene, Genabschnitte oder Oligomere. Die RNA-Proben werden auf Kopplungsträger gespottet und setzen sich aus Total-RNA oder messenger-RNA zusammen. Die RNA dient dabei als Target für die aus DNA-Mikroarray abgeleiteten hoch signifikant exprimierten Genen die als markierte Sonden zur Hybridisierung eingesetzt werden. Vorgeschlagen wird das Koppeln biotinylierter RNA oder messenger-RNA auf Streptavidin beschichteten Glasträgern (Slides). Nach Markierung der RNA mit Biotinderivaten, wird die RNA auf Poly-L-Lysin behandelten vorzugsweise aber auf mit Streptavidin beschichteten Glas- oder Plastikslides durch Spotting aufgebracht und getrocknet. Eine Degradation der RNA wird so verhindert. Alternativ bietet sich eine kovalente Kopplung der RNA durch Bindung an reaktive Trägermaterialien an die vorzugsweise durch UV-Bestrahlung katalysiert wird.

Zusätzlich ist eine multiple, gleichzeitige Markierung verschiedener Gene, Geneinheiten oder Oligomere mit verschiedenen Markierungs-Spezies, z.B. Radioaktivität, Fluoreszein, Digoxigenin und enzymatischen Markierungen.

Parallel unterschiedliche Markierungen der Sonden mit unterschiedlichen Fluoreszenzfarbstoffen sind möglich. Alternativ sind enzymatische oder aber radioaktive Sondenmarkierungen zu nennen.
Zur Quantifizierung und Qualitätskontrolle werden markierte Haushaltsgene (alpha-, beta, gamma-Aktin, GAPDH usw.) eingesetzt. Bevorzugt wird der Nachweis hier parallel und gleichzeitig mit maximal 50 Gensonden pro Ansatz gleichzeitig durchgeführt.
Neben der Vereinfachung der biometrischen Analyse durch Kopplung von RNA Spezies an Trägermaterialien erlaubt dieses System eine schnelle Diagnostik und bietet eine komplexe für den Patienten individuell schnelle Diagnostik, Prognostik und Therapiesteuerung. Insbesondere bei pharmakologischen Entwicklungsstrategien erlaubt das System eine schnelle Durchführung mit hohem Durchsatz.

Da in dieser Anmeldung phänotypische Zellzuordnung, hier, Bezug auf das Monozyten/Makrophagen System genommen wird, zum anderen auch eine bereits vorangegangene Selektionierung der Gene, Geneinheiten oder aber Oligomere vorherrrscht können so vielfältige Verfahrensweisen, die sich sowohl in gleichzeitigen oder aber getrennten Anwendungen von DNA-/RNA-Mikroarrays für die genannten Erkrankungen wieder finden. Kombinatorisch beinhalten dieses Verfahren sowohl einen kommerziellen Nutzen mit gleichzeitigem Nutzen für wissenschaftlichen Fragestellung.

Die Markerfunktionen der molekularen Werkzeuge in DNA-Mikroarray / DNA-fluoreszenzytometrischem Mikroarray, RNA-Mikroarray oder aber reverser Transkriptions-Polymerasen Kettenreaktion (RT-PCR) ermöglicht es, ein krankheitsspezifisches Genexpressionsmuster jedes einzelnen Entzündungspozesses zu generieren und die Relevanz bestimmter biologisch wirksamer Substanzen und deren Derivate abzuschätzen und neue Therapiemöglichkeiten über konventionelle chemische Pharmazeutika, naturstoffbezogene Pharmazeutika, antisense-RNA, Aptamere, Ribozyme, oder monoklonale Antikörper zu entwickeln. Zusätzlich können speziesspezifische Krankheitsverläufe sämtlicher genannter Erkrankungen entzündungsrelevant und krankheitsspezifisch genotypisch erfasst bzw. analysiert werden und die krankheitsspezifische Behandlung in Abhängigkeit von der Entzündungsaktivität entsprechend individuell abgestimmt werden.

## Patentansprüche

1. Werkzeuge zur diagnostischen, prognostischen und therapieüberwachenden Analyse sowie zur Durchführung von Screeningverfahren für pharmakologisch wirksame Substanzen und Substanzklassen der rheumatoiden Arthritis, chronisch entzündlicher Erkrankungen, bakteriell induzierter chronisch entzündlicher Erkrankungen, Arteriosklerose, Tumorerkrankungen, Organ- und Gewebstransplantationen, und der Sepsis, **dadurch gekennzeichnet,**
**dass** auf der Oberfläche des Werkzeuges Sequenzen einer Auswahl nachfolgend genannter selektiver Monozyten-Makrophagen Gene oder Sequenzen aller nachfolgend genannter Gene, auch unter Verwendung weiterer Gene, gebunden sind oder dass mit genannten Genen komplementäre RNA auf der Oberfläche des Werkzeuges gebunden ist.

2. Werkzeuge nach Anspruch 1, **dadurch gekennzeichnet, dass** es sich bei den Genen, deren Teilsequenzen und Oligomersequenzen um selektionierte / subtrahierte Gene handelt.

3. Werkzeuge nach Anspruch 1 oder 2 **dadurch gekennzeichnet, dass** auf der Oberfläche des Werkzeugs alternativ auch Allele, Derivate und/oder Splicingvarianten der Gen bzw. Genteilsequenzen und Oligomersequenzen vorliegen können.

4. Werkzeuge nach einem der Ansprüche 1 bis 3, **dadurch gekennzeichnet, dass** die Werkzeuge Gensequenzen einbeziehen, die mindestens eine Teil-Sequenzidentität in den Protein-kodierenden Abschnitten der mRNA besitzen.

5. Werkzeuge nach Anspruch 4, **dadurch gekennzeichnet, dass** die Werkzeuge Gensequenzen einbeziehen, die mindestens 80% Sequenzidentität in den Protein-kodierenden Abschnitten der korrespondierenden Nukleinsäure besitzen.

6. Werkzeuge nach den Ansprüchen 1 bis 5, **dadurch gekennzeichnet, dass** sie auf der Verwendung eines High-Throughput Verfahres der DNA-Array-Hybridisierung,
High-Throughput Verfahrens unter Verwendung der fluoreszenzzytometrischen Technologie (cytometric bead array)
High Throughput Verfahrens der RNA-Array-Hybri disierung, oder
High-Throughput Verfahrens mit Techniken der Polymerase-Ketten-Reaktion zur (Semi-) Quantifizierung beruhen.

7. Werkzeuge nach einem der Ansprüche 1 bis 6, **dadurch gekennzeichnet, dass** die Gene kovalent auf der Oberfläche gebunden sind.

8. Werkzeuge nach einem der Ansprüche 1 bis 7, **dadurch gekennzeichnet, dass** die Oberfläche aus Glas oder Kunststoff besteht, die chemisch aktiviert oder modifiziert ist.

9. Werkzeuge nach Anspruch 8, **dadurch gekennzeichnet, dass** die Oberfläche mit Aminolinkern bindenden reaktiven Gruppen, Metallverbindungen oder Legierungen reaktiv beschichtet ist.

10. Werkzeuge nach Anspruch 8 oder 9, **dadurch gekennzeichnet, dass** das Trägermaterial bzw. die Oberfläche aus einer Nylonmembran besteht.

11. Werkzeuge nach einem der Ansprüche 1 bis 10, **dadurch gekennzeichnet, dass** die cDNA durch reverse Transkription aus Total-RNA oder messenger-RNA menschlicher Zellen des Monozyten / Makrophagen Systems hergestellt ist

12. Werkzeuge nach einem der Ansprüche 1 bis 11, **dadurch gekennzeichnet, dass** die Gene durch Spottingverfahren von cDNA, Immobilisierungsverfahren und Syntheseverfahren von Oligomeren oder spiegelbildlich in Form von RNA aufgebracht sind.

13. Werkzeuge nach einem der Ansprüche 1 bis 12, **dadurch gekennzeichnet, dass** der Nachweis über cDNA-, deren Sequenzanteile oder über Oligosonden erfolgt.

14. Werkzeuge nach Anspruch 10, **dadurch gekennzeichnet, dass** die Sequenzen Abweichungen enthält.

15. Werkzeuge nach Anspruch 11, **dadurch gekennzeichnet, dass** die Abweichung von cDNA- und Oligosonden zum Nachweis bis zu 20% beträgt.

16. Werkzeuge nach einem der Ansprüche 1 bis 15, **dadurch gekennzeichnet, dass** die Sonden zum Nachweis fluoreszenzfarbstoff-, Enzym-, Protein- oder radioaktiv markiert sind und Verstärkung zulassen.

17. Werkzeuge nach Anspruch 1 bis 16, **dadurch gekennzeichnet, dass** Verstärkung über gekoppelte Biotin-, Digoxigenin-, (Edel-)Metallchelat- und Protein (Moleküle) erfolgt.

18. Werkzeuge nach Anspruch 17, **dadurch gekennzeichnet, dass** zur Verstärkung Streptavidin, (Edel-) Metallchellat oder Antikörper eingesetzt werden.

19. Werkzeuge nach Anspruch 18, **dadurch gekennzeichnet, dass** der Nachweis über Fluoreszenzfarbstoff, Radioaktivität oder enzymatisch erfolgt.

20. Werkzeuge nach einem der Ansprüche 1 bis 19, **dadurch gekennzeichnet, dass** die Werkzeuge durch ein DNA-Array ausgebildet sind.

21. Werkzeuge nach einem der Ansprüche 1 bis 19, **dadurch gekennzeichnet, dass** die Werkzeuge durch ein Zytofluoreszenz-DNA-Array (Beads) ausgebildet sind.

22. Werkzeuge nach einem der Ansprüche 1 bis 19, **dadurch gekennzeichnet, dass** die Werkzeuge durch einen DNA-Array spiegelbildlichen RNA-Array ausgebildet sind.

23. Werkzeuge nach Anspruch 1 bis 22, **dadurch gekennzeichnet, dass** die selektiven Gene, deren Teilsequenzen oder Oligomere zum Nachweis festphasengebundener Total-RNA oder messenger-RNA benutzt werden.

24. Werkzeuge nach Anspruch 1 bis 23, **dadurch gekennzeichnet, dass** die Sonden durch reverse Transkription aus messenger-RNA menschlicher Zellen des Monozyten / Makrophagen-Systems hergestellt wird

25. Werkzeuge nach einem der Ansprüche 1 bis 24, **dadurch gekennzeichnet, dass** RNA von bis zu 500 Gewebsund/oder Blutproben auf dem Array aufgebracht sind.

26. Werkzeug nach einem der Ansprüche 1 bis 25, **dadurch gekennzeichnet, dass** die Sondenmarkierung mit Fluoreszenzfarbstoffen, Biotin, Digoxigenin, Peroxidase, alkalischer Phosphatase und Radioaktivität durchgeführt ist.

27. Werkzeug nach einem der Ansprüche 1 bis 26, **dadurch gekennzeichnet, dass** Nachweisverfahren der genannten Sequenzen über reverse Transkriptions PCR (RT-PCR) durchzuführen sind.

28. Werkzeuge nach einem der Ansprüche 1 bis 27, **dadurch gekennzeichnet, daß** die Gene oder Gensequenzen mit einer Markierung oder einer Reporterfunktion ausgestattet sind, so dass diese für andere Nachweisverfahren nutzbar sind.

29. Verwendung der Werkzeuge nach einem der Ansprüche 1 bis 27, **dadurch gekennzeichnet, dass** die Werkzeuge zur Messung der Monozyten/Makrophagen Aktivierung resp. der Entzündungsaktivität im Blut oder aber im Zellgewebe verwendet werden.
**Tabelle 1**
| **Zytokine und Faktoren und Liganden:** | |
|---|---|
| Interleukin-1α | (Acc.# NM_000575) |
| Interleukin-1β | (Acc.# NM_000576) |
| Interleukin-6 | (Acc.# AF372214) |
| Interleukin-8 | (Acc.# L19591) |
| Interleukin-10 | (Acc.# XM_001409) |
| Interleukin-13 | (Acc.# HSU62858) |
| Interleukin-15 | (Acc.# XM_003529) |
| Interleukin-16 | (Acc.# AF053412) |
| Interleukin-18 | (Acc.# E17135) |
| Angiopoietin-like factor (CTD6) | (Acc.# XM_001529,XM_042319) |
| Inhibin β-B (INHBB) | (Acc.# NM_002193) |
| Tumor-Nekrosefaktor-α | (Acc.# NM_000595) |
| Tumor-Nekrosefaktor-β | (Acc.# D12614) |
| Transforming Growth Factor-β (TGF-β) | (Acc.# XM_008912,NM_00660) |
| Latent TGF-β binding prot. LTBP4 | (Acc.# NM_003573,XM_008868) |
| Melanoma stimulating activity (MGSA) | (Acc.# X54489) |
| Chemokine Gro-α/MGSA | (Acc.# X12510,XM_003504) |
| Chemokine (C-X-C motif) ligand 16 | (Acc.# NM_022059) |
| Chemokine alpha-3 (CKA3) | (Acc.# NM_002993) |
| CC-Chemokine (SLC) | (Acc.# AB002409) |
| EBI-1-Ligand Chemokine | (Acc.# AB000887) |
| Small inducible cytokine subfamily A(SCYA21) | (Acc.# XM_048450) |
| Small inducible cytokine(SCYA21) | (Acc.# NM_002989) |
| Megakaryocyte stimulating factor | (Acc.# U70136) |
| Monocyte colony stimulating factor (M-CSF) | (Acc.# NM_000757) |
| Granulo-/Monocyte colony stimu. factor (GM-CSF) | (Acc.#: E01817) |
| Macrophage inflammatory Protein (MIP-1) | (Acc.# HUMMIP1A) |
| Makrophage inflammatory Protein (MIP-2 | (Acc.# AF106911) |
| Monocyte migration inhibitory factor (MIF) | (Acc.# L19686) |
| Monocyte Tissue factor | (Acc.# M16553) |
| Monocyte Chemoattractant Protein-1 (MCP-1) | (Acc.# S71513) |
| Monocyte Chemoattractant Protein-2 (MCP-2) | (Acc.# NM_005623) |
| Monocyte Chemoattractant Protein-3 (MCP-3) | (Acc.# X72308; S57464) |
| Fraktalin small inducible cytokine | (Acc.# NM_002996) |
| Stromal derived factor-1 (SDF-1) | (Acc.# HSU16752) |
| Insulin-like growth factor-5 bind. Protein | (Acc.# NM_000599) |
| | |
| **Rezeptoren, Ionenkanäle und assoziierte Proteine:** | |
|---|---|
| Angiotensin Rezeptor-II Homolog (ATR-IIh) | (Acc.# L48211) |
| Toll-like Rezeptor-2 | (Acc.# XM_003304) |
| Toll-like Rezeptor-4 | (Acc.# XM_005336) |
| Opoid-Rezeptor Kappa | (Acc.# XM_011716) |
| Interleukin-1 receptor | (Acc.# XM_002686) |
| Interleukin-2 receptor α-Untereinheit | (Acc.# XM_043149) |
| Interleukin-2 Receptor β-Untereinheit | (Acc.# XM_009962,M26062) |
| Interleukin-2 Receptor γ-Untereinheit | (Acc.# XM_047675) |
| Interleukin-7 Receptor | (Acc.# AH007043, NM_008372) |
| Interleukin-8 receptor α (IL8RA) | (Acc.# XM_058007) |
| Interleukin 8 receptor β (IL8RB) | (Acc.# NM_001557 |
| Fc-Rezeptor-I | (Acc.# J03619, AF200220) |
| Fc-Rezeptor-II | (Acc.# M28696,M28697) |
| Fc-Rezeptor-III | (Acc.# Z46223,Z46223) |
| Tumor-Nekrosefaktor-α Rezeptor | (Acc.# S63368) |
| C-Chemokine (C-C motif) Rezeptor-5 (CCR5) | (Acc.# NM_000579,XM_030397) |
| C-Chemokine (C-C motif) Receptor-7 (CCR7) | (Acc.# XM_049959) |
| Chemokin-X-C-Rezeptor-4(CXCR-4) | (Acc.# NM_003467) |
| Progesterone Recept.-assoc. Immunophilin(FKBP54) | (Acc.# U42031) |
| Partial p58 gene for NK receptor | (Acc.# AJ000542) |
| Vascular endothial growth factor | (Acc.# AY047581) |
| Vascular endothial growth factor-β | (Acc.# BC008818) |
| Calcium activated potassium channel (KCNN3) | (Acc.# AF031815, AY049734) |
| G protein-coupled cytokine receptor EBI1 | (Acc.# L31581) |
| G protein-coupled cytokine receptor EBI3 | (Acc.# XM_012857,L08187) |
| EBI3-associated protein | (Acc.# U41806) |
| | |
| **Membranproteine und assoziierte Proteine:** | |
|---|---|
| CD14 | (Acc.# XM_003822) |
| CD68 | (Acc.# XM_008237) |
| CD69 | (Acc.# BC007037) |
| CD11b | (Acc.# J03925) |
| Adhesion receptor CD44 | (Acc.# M31165) |
| Actin binding coronin like protein (HCORO1) | (Acc.# U34690) |
| Integral membrane protein | (Acc.# L32185) |
| Epithelial membrane prot.-3 (EMP-3) / HMPMP-1 | (Acc.# X94771,U87947) |
| Mac-2 binding protein | (Acc.# L13210) |
| Integral membrane protein E16 | (Acc.# M80244) |
| HLA-D II beta chain | (Acc.# X03066) |
| Desmin | (Acc.# HSU59167, XM_002601) |
| Fibronectin precursor | (Acc.# X02761) |
| Adducin 1α | (Acc.# X58141, NM_014190) |
| HLA DRB1 | (Acc.# X88971) |
| Integrin-α 5 subunit | (Acc.# X06256) |
| Integrin cytopl. domain assoc. protein (Icap-1α) | (Acc.# AF012023) |
| Integrin cytopl. domain assoc. protein (Icap-1β) | (Acc.# AF012024) |
| Titin | (Acc.# X69490, NM_003319) |
| Thrombospondin-1 (TSP-1) | (Acc.# XM_007606) |
| Semaphorin-3 | (Acc.# AB000220) |
| Semaphorin-F Homolog | (Acc.# U52840) |
| TSP-2 | (Acc.# NM_003247) |
| TSP-1 / Semaphorin-5a Homolog | (Acc.# NM_003966) |
| VCAM-1 | (Acc.# x53051) |
| Periplakin (PPL) | (Acc.# XM_032727, NM_002705) |
| Envoplakin (EVPL) | (Acc.# XM_008135) |
| Peripheral myelin protein 22 (PMP-22) | (Acc.# XM_052499) |
| | |
| **(Proto)-Onoko-, Tumor-Suppressor-, Differenzierungsgene & assoz.Proteine:** | |
|---|---|
| H19 RNA | (Acc.# M32053) |
| Tumor suppressor Brush-1 | (Acc.# S69790) |
| Pim-2 Protoonkogen | (Acc.# U77735,XM_010208) |
| HOX-B3 | (Acc.# N70814) |
| MEL-18 | (Acc.# D13969) |
| c-fos | (Acc.# V01512) |
| c-jun | (Acc.# NM_002229) |
| c-myc | (Acc.# AH001511) |
| c-myc related oncogen (pHL-1) | (Acc.# X54629) |
| c-Ret tyrosine kinase receptor ligand 2 (RETL2) | (Acc.# U97145) |
| c-Ret tyrosine kinase receptor ligand 1 (RETL1) | (Acc.# U97144) |
| jun-B | (Acc.# XM_009064) |
| c-Jun activation domain binding protein | (Acc.# U65928) |
| Desmoyokin/AHNAK | (Acc.# X74818,M80899) |
| Rad mRNA | (Acc.# L24564) |
| PTEN | (Acc.# AH005966,XM_005867) |
| c-ras homolog gene family, member B (ARHB) | (Acc.# XM_002689,NM_004040) |
| Transforming activity oncogene (TRE-2) | (Acc.# X63596) |
| Transforming activity oncogene (TRE-17 | (Acc.# HSTRE213) |
| Kruppel-like fetal globin gene activator (FKLF) | (Acc.# AF272830) |
| c-fos related antigen (fra-1) | (Acc.# X16707) |
| c-fos related antigen (fra-2) | (Acc.# X16706) |
| | |
| **Akut Phase Protein**: | |
|---|---|
| Large-Ferritin Untereinheit | (Acc.# M11146) |
| Small-Ferritin Untereinheit | (Acc.# NM_000146) |
| | |
| **Enzyme, Enzym-assoziierte Proteine und Inhibitoren:** | |
|---|---|
| Activation-induced cytidine deaminase | (Acc.# AB040431,NM_020661) |
| Phospholipase-C | (Acc.# XM_041310) |
| Prostaglandin G/H Synthase | (Acc.# S36271) |
| Prostaglandin-Endoperoxide Synthase-1 | (Acc.# NM_000962) |
| Cyclooxygenase-1 | (Acc.# HSU63846) |
| Cyclooxygenase-2 | (Acc.# M90100) |
| Endothelin-1 (EDN1) | (Acc.# NM_001955) |
| Endothelin-1 (EDN2) | (Acc.# NM_001956) |
| Clustrin (complement lysis inhibitor, SP-40,40) | (Acc.# XM_027447, X14723) |
| Fettsäure Desaturase 1 (FADS1) | (Acc.# AF084558) |
| Cysteine dioxygenase 1 (CDO-1) | (Acc.# U80055) |
| Histidine biosynthesis protein | (Acc.# NM_007016) |
| Chitinase 1 | (Acc.# NM_003465) |
| Chitinase precursor | (Acc.# AF290004) |
| L-glycerol-3-phosphat: NAD oxidoreductase | (Acc.# L34041) |
| Alcohol dehydrogenase class I gamma subunit | (Acc.# M12272) |
| Procarboxypeptidase B1 | (Acc.# NM_001871) |
| Phosphoenolpyruvate carboxykinase (PCK1) | (Acc.# XM_009672, L05144) |
| Lysozym | (Acc.# BC004147) |
| Transaldolase | (Acc.# NM_006755) |
| Thymosin-β4 | (Acc.# M17733) |
| Metallothionein 1L (MT1L) | (Acc.# NM_002450) |
| Manganese-superoxide dismutase (Mn-SOD) | (Acc.# S77127) |
| Superoxide Dismutase 1 | (Acc.# K00065) |
| Superoxide Dismutase 2 | (Acc.# NM_000636) |
| Superoxide Dismutase 3 | (Acc.# NM_003102) |
| Copper/zinc-superoxide dismutase (Cu/Zn-SOD) | (Acc.# M13267) |
| Catalane | (Acc.# ) |
| Monoamine oxidase-A (MAOA) | (Acc.# M68840,XM_055485) |
| Fatty acid synthetase | (Acc.# U29344) |
| Glutathion peroxidase | (Acc.# X13710) |
| Glutathion peroxidase 3 | (Acc.# NM_002084) |
| Glucocerebrosidase | (Acc.# M16328) |
| Induzierbare Nitric oxide Synthase | (Acc.# AB022318) |
| Transglutaminase 1 (K polypeptide) | (Acc.# XM_007310) |
| Transglutaminase (TGase) | (Acc.# M55153, SEG_HUMETG)* |
| α-1-Antitrypsin | (Acc.# HSATPR1) |
| Protein Tyrosin-Phosphatase | (Acc.# U27193) |
| Carbonic anhydrase precursor(CA 12) | (Acc.# AF037335) |
| Metallothionein-IG gene (MT1G) | (Acc.# J03910) |
| Lymphocyte phosphatase assoc. Protein (LPAP) | (Acc.# X97267,AA011257) |
| Flap Endonuclease 1 DNA repair gene (FEN1) | (Acc.# AC004770) |
| Flap structure-specific endonuclease 1 (FEN1) | (Acc.# L37374, XM_043386) |
| | |
| **Kinasen, Protein Kinasen (PKN) und PKN-Inhibitoren**: | |
|---|---|
| Protein Kinase C-alpha Untereinheit | (Acc.# X52479) |
| Protein Kinase C-beta-1 Untereinheit | (Acc.# XM_047187) |
| Protein Kinase C-beta-2 Untereinheit | (Acc.# M13975) |
| Protein Kinase C-gamma Untereinheit | (Acc.# M34182) |
| Protein Kinase C-delta Untereinheit | (Acc.# D10495) |
| Protein Kinase-C Inhibitor | (Acc.# U51004 |
| Ik-Kinase-κ | (Acc.# AF029684 |
| PI3-Kinase | (Acc.# Y13892) |
| MAP Kinase-11 | (Acc.# XM_035889) |
| p38 MAP Kinase | (Acc.# AF031135) |
| p38 MAP Kinase interacting protein | (Acc.# XM_035930) |
| Serin/Threonin Kinase | (Acc.# AB015982) |
| Thyrosin Kinase-1 | (Acc.# XM_002037) |
| Thyrosin Kinase-2 | (Acc.# XM_005480) |
| Non-receptor protein tyrosine kinase tyk2 | (Acc.# X54637) |
| Mitogen- and stress-activated protein kinase-1 | (Acc.# AF074393) |
| Mitogen- and stress-activated Protein Kinase-2 | (Acc.# AF074715) |
| Casein Kinase 1, alpha 1 (CSNK1A1) | (Acc.# NM_001892, L37042) |
| Thyrosine kinase 1 (TIE-1) | (Acc.# XM_002037) |
| Thyrosine kinase 2 (TIE-2) | (Acc.# XM_005480) |
| | |
| **Differenzierungsgene:** | |
|---|---|
| WNT-6 | (Acc.# AY009401,AB059570) |
| WNT-13 | (Acc.# Z71621) |
| BMP-4 | (Acc.# M22490) |
| | |
| **Proteinasen, Matrixmetalloproteinasen (MMP) und MMP-Inhibitoren:** | |
|---|---|
| Cathepsin-B | (Acc.# XM_035662) |
| Cathepsin-G | (Acc.# M16117) |
| Cathepsin-K | (Acc.# NM000396) |
| Cathepsin-L | (Acc.# NM_001912) |
| Cathepsin-S | (Acc.# M86553) |
| Matricx metalloproteinase-1 (MMP-1) | (Acc.# NM_002421) |
| MMP-3 | (Acc.# X05232) |
| MMP-9 | (Acc.# XM_009491) |
| Disintrigin Protease | (Acc.# Y13323) |
| Tissue inhibtor of MMP type 1 (TIMP-1) | (Acc.# NM_003254) |
| TIMP-2 | (Acc.# NM_003255) |
| TIMP-3 | (Acc.# NM_000362) |
| TIMP-4 | (Acc.# NM_003256) |
| Serin Protease like mRNA | (Acc.# M17016) |
| | |
| **Apoptose- und Zellzyklus Regulatoren:** | |
|---|---|
| Annexin A-2II | (Acc.# BC001388) |
| Growth arrest DNA-damage-induc. prot. (GADD45) | (Acc.# M60974) |
| Growth arrest DNA-damage-induc. prot.α(GADD45A) | (Acc.# XM_056975, XM_040594) |
| Growth arrest DNA-damage-induc. prot.β(GADD45B) | (Acc.# NM_015675, AF087853) |
| Growth arrest DNA-damage-indcu. prot.g(GADD45G) | (Acc.# NM_006705) |
| Lymphocyte G0/G1 switch gene (GOS-3) | (Acc.# L49169) |
| | |
| **Signaltransduktions-Regulatoren:** | |
|---|---|
| STAT-1 | (Acc.# NM_007315) |
| STAT-4 | (Acc.# XM_002711) |
| Adenylate kinase 1 (AK1) | (Acc.# NM_000476) |
| Inositol 1,4,5-trisphosphate 3-kinase (ITPKC) | (Acc.# XM_047369, XM_047368) |
| Phosphatidylinositol-3'-kinase (PI3K) | (Acc.# Y11312) |
| | |
| **Transkriptionsfaktoren, Translationsfaktoren und assoziierte Proteine:** | |
|---|---|
| Transcription factor AREB6 | (Acc.# D15050) |
| Transcription factor 8 (TCF8) | (Acc.# XM_030006) |
| Nuklear factor kappa-B | (Acc.# M58603) |
| AP-1 | (Acc.# AB015319, AB015320) |
| PU.1 | (Acc.# X66079) |
| SPI-B | (Acc.# X66079) |
| v-maf musculoaponeurotic fibrosarcoma (MAFF) | (Acc.# XM_039249,XM_039250) |
| Zinc finger transcription factor (GKLF) | (Acc.# AF105036, AK026253) |
| Zinc finger Protein | (Acc.# M80583) |
| CCAATA enhancer binding Protein-beta | (Acc.# NM_005194) |
| RNA-polymerase II elongationsfactor | (Acc.# L47345) |
| Translation elongation factor-1 α-1 (EEF1A1) | (Acc.# BC009733) |
| Translation elongation factor-1 α-2 (EEF1A2) | (Acc.# XM_028863) |
| Translation elongation factor 2 (EEF2) | (Acc.# NM_001961) |
| L1-Element (L1.20) | (Acc.# U93569) |
| Leukemia Zink Finger PLZF | (Acc.# AF060568) |
| Activating transcription factor 3 (ATF3) | (Acc.# XM_016795, XM_034219) |
| Zinc finger transcriptional regulator (GOS-24) | (Acc.# M92843) |
| TGF-β-inducing early growth response 2 | (Acc.# AA427597) |
| SP1-like zinc finger transcript. factor (TIEG2) | (Acc.# AF028008) |
| snRNA activating protein complex | (Acc.# AF032387) |
| oct-binding factor-1 (OBF-1) | (Acc.# Z49194) |
| Early Growth Response protein 1 (EGR-1) | (Acc.# R75775) |
| | |
| **Ribosomale- / Ribonukeäre Regulatorgene und assoziierte Proteine:** | |
|---|---|
| hnRNP pseudogen(gp43) (Position: 97.026-98.073) | (Acc.# AL034397) |
| Ribosomal protein L19 | (Acc.# XM_002758) |
| Ribosomal protein S13 | (Acc.# XM_039215) |
| Histon-H1 (0) family mRNA | (Acc.# X03473) |
| H4-histone family, member H (H4FH), mRNA | (Acc.# NM_003543) |
| | |
| **Andere:** | |
|---|---|
| IER-3 | (Acc.# NM_003897) |
| Endoplasmatic glykoprotein Gp36 | (Acc.# U10362) |
| Natural resist.-assoc. Macroph.protein (Nrampl) | (Acc.# D50402) |
| Calgranulin - S100A12 protein | (Acc.# XM_001682, NM_005621) |
| 14-3-3 gamma Protein | (Acc.# AF142498) |
| Serum amyloid-A | (Acc.# M81349,M81451) |
| GDF-1 | (Acc.# NM_001492) |
| Solute carrier family 7 mRNA (SLC7A5) | (Acc.# NM_003486) |
| PLAB/MIC-1 | (Acc.# NM_004864) |
| EAP-(HBp15/L22) | (Acc.# NM_006755) |
| Small Proline-rich protein-1 | (Acc.# L05187) |
| NAG-1 | (Acc.# AF173860) |
| BST-1 | (Acc.# D21878) |
| II56KD | (Acc.# M24594) |
| Fibulin-1 D | (Acc.# NM_006486) |
| Nebulin | (Acc.# XM_040435) |
| VDUP1 upregulated by 1,25-dihydroxyvitamin D-3 | (Acc.# XM_002093, XP_002093) |
| Tumor nekrosis factor stimulated gene (TSG-6) | (Acc.# NM_007115) |
| Tumor nekrosis factor stimulated gene (TSG-37) | (Acc.# M31164) |
| Osteopontin | (Acc.# AF052124) |
| Tristetraproline (TTP) | (Acc.# M63625) |
| Nephropontin | (Acc.# M83248) |
| Tonsillar lymphocyte LD78 mRNA | (Acc.# X03754) |
| MB-1 gene (CD79a-B cell) | (Acc.# U05259) |
| Human Glykoprotein (gp39) | (Acc.# M80927,Y08374) |
| Glia derived nexin precursor | (Acc.# AI743134) |
| Heat shock protein 70B (HSP-70B) | (Acc.# X51757) |
| Apolipoprotein D | (Acc.# XM_049984,XM_003067) |
| Dead box, Y isoform (DBY), altern.transcr. 2 | (Acc.# AF000984) |
| Myocilin (GLC1A) | (Acc.# AH006047) |
| DR1-associated corepressor (DRAP1) | (Acc.# U41843) |
| DR1-associated protein 1 (neg. cofactor 2 α) | (Acc.# XM_055156) |
| FK506 bind.- 12-rapamycin assoc.prot.1 (FRAP1) | (Acc.# XM_001528, XM_042283) |
| Microfibril-associated glycoprotein-2 (MAGP-2) | (Acc.# AH007047, NM_003480) |
| Adrenomedullin (ADM) precursor | (Acc.# NM_001124,XM_051743) |
| DNA-damage-inducible transcript 3,clone MGC:4154 | (Acc.# BC003637) |
| Calretinin - calcium binding protein | (Acc.# X56667) |
| Breakpoint cluster region (BCR) mRNA | (Acc.# XM_017097) |
| Adipose most abundant gene transcript 1 (APM1) | (Acc.# NM_004797,XM_003191) |
| Novel adipose specific collagen-like factor | (Acc.# D45371) |
| | |
| **Funktionell unbekannte Gene und EST's:** | |
|---|---|
| IMAGE 745750 | (Acc.# AA420624) |
| KIAA0935 | (Acc.# AB023152) |
| KIAA0618 | (Acc.# AB014518) |
| Homolog zu FLJ23382 fis Klon HEP16349 | (Acc.# AK027035) |
| Hypothetical gene mRNA | (Acc.# XM_005331) |
| HDCMB07P/PCM-1 | (Acc.# AF068293) |
| cDNA clone DKFZp762M2311 | (Acc.# AL512760) |
| cDNA clone PP2684 | (Acc.# AF218004) |
| cDNA clone MGC:1811 (IMAGE:3506276) | (Acc.# BC015961) |
| cDNA clone IMAGE:979127 | (Acc.# AA522530) |
| cDNA clone IMAGE:4279495 5', mRNA sequence | (Acc.# Bf667722) |
| cDNA clone 137308 mRNA, partial cds | (Acc.# U60873) |
| cDNA clone IMAGE:159541 | (Acc.# H15814) |
| cDNA clone MAMMA1001272 | (Acc.# AU147646) |
| cDNA clone IMAGE:2419382 | (Acc.# AI826771) |
| cDNA clone IMAGE: 3941411 | (Acc.# BE797145 |
| cDNA clone IMAGE:3834583 | (Acc.# BE743390) |
| cDNA clone IMAGE:4565371 | (Acc.# BG397372) |
| cDNA clone MGC:2460 IMAGE:2964524 | (Acc.# BC009504) |
| cDNA clone RC3-HT0585-010400-013-all HT0585 | (Acc.# BE176664) |
| cDNA clone similar to CG8974 gene product | (Acc.# XM_018516) |
| cDNA clone BSK-65 | (Acc.# W99251) |
| cDNA clone IMAGE:3844696 | (Acc.# BE730665) |
| FLJ23382 fis, clone HEP16349 | (Acc.# AK027035) |
| FLJ20500 fis, clone KAT09159 | (Acc.# AK000507, BC015236) |
| GABBR1 Region von AL031983 | (Acc.# 12329558) |
| cDNA clone CS0DE006YI10 5' prime end | (Acc.# AL541302) |
| cDNA clone CS0DE006YI10 3' prime end | (Acc.# AL541301) |
| EST371586 IMAGE resequences | (Acc.# Aw959516) |
| MEN1 region clone epsilon/beta | (Acc.# Af001892) |
| | |
| **Kontrollen zum Quantifizierungsabgleich:** | |
|---|---|
| alpha-Aktin | (Acc.# M20543) |
| beta-Aktin | (Acc.# XM_037239) |
| gamma-Aktin | (Acc.# NM_001614) |
| Glyceraldehyd-3-phosphat-Dehydrogenase | (Acc.# XM_033258) |
| Glucose-6-phosphat-Dehydrogenase | (Acc.# XM_013149) |
| 28S rRNA | (Acc.# M27830) |
| 18S rRNA | (Acc.# M10098) |


<110>  Pathoarray GmbH
<112>  Jägerstr. 51
<113>  Berlin
<115>  Deutschland
<116>  10117

<120>  Nukleinsäure-Array

<130>  171

<151>  Diskette
<152>  IBM PC-kompatibel
<153>  Microsoft Windows 98
<154>  Patentin 3.1, Version 3.1.16

<160>  102 19 052.6

<210>  1
<211>  929
<212>  DNA
<261>  Homo Sapiens

<400>  1
cggttggggc tctggtcttg gatttgatgt gtggcgaagg ctgcaattgt ttaataaccc      60
ttcatgattc aacagctctt caagaacttt cctctgttct tgtgtggagc tcgtgacagc     120
cagtggtggt ggagctccag ccctctcttc ccacaggcac aagccgggtt cctgagtccc     180
agggcttctc gggaggtgtc tgccctcctc tttcagacac cctctgccct gtgtcccagg     240
gccctgggcc tgtgctgcac tgagcagaga ctgtagggga ccggctctcc cactcctccc     300
agatgggcag cgtcttccgt gtcgggagca tgctgtgctg cttttctctt ctcagtctct     360
tagtttttgc gggttcttac gcatgtgagg tgtggacttg catggtgggg agctcaaatg     420
gtacatgaag gggaggagcc ctctgagtgc tgtgatttgt tccatcatta ccgcttcctg     480
atcacggtga cctgcactgc tggagtggtc agtggagcca ggcctcccca caacagtgtt     540
cccatcgcct tcttactatt gatttctatt cttaaaatat tgtattactt agcactcttt     600
tgaagacgtt ccagtatata tcaaatgatc aaaagtccat aaccttgtcc tacgtagaag     660
ccaaaggtgt catgcagttt caggtgttcg agtttccaga attcttgtga tgacatttgt     720
aggattcttc ttttagactt ggaccaaatt ctgtaaccta atatttgtcc ttcagattga     780
cagagaaccg caggcaggtg ttttctctgt cacacgtgtg gtgggtggca tcctggtgac     840
ataaagaatt gcctttggta acttgcccag aaggctgtag ggttattttc tgcttagact     900
ttcccctatt tctttctttt cttttctcg                                       929


<210>  2
<211>  657
<212>  DNA
<213> Homo Sapiens

<400>  2
attttaggga ggtagtagat gatttttagg gaatttgatg ggccagaaga acatacaatg      60
gattgggaca aagtctgttg ggcagacaat ggtttgtgac aaaattctgt ccaggtgtgt     120
tgaccgaatt caggctttct ttatgcgata tgagttcagt taatgaaaac acaggggagt     180
gaccagaagt gattgtttcc ttctttggcg tttctgtctt cctccttttt tgttctattc     240
ccttattttg caaccttttg gatgttaccc tttggaagtt accctcttgt aacttccaca     300
ttaaaagttt gggggctggc tgatanaagg aactccagag aacaacttga ttctgtgctt     360
tgggagagac aganaaatga ggggtgtgga ggaaggtcag anagaccctg aggcctctgc     420
ctncttcagc atgtcanagc accctatttt ggggcttgct ttctgagccc naacatctcc     480
agccttccan gantctgtgg cttatccttc ccaangatag gatcacttgn cactctactg     540
ancctaagtt gtattcantt tcttttgatc cgcctngact ctntagcnan tganaancac     600
aacntggnaa cnaaccctca taaanctgct ntancttctg gttttaagnn caaaaca        657


<210>  3
<211>  657
<212>  DNA
<213> Homo Sapiens

<400>  3

attttaggga ggtagtagat gatttttagg gaatttgatg ggccagaaga acatacaatg      60
gattgggaca aagtctgttg ggcagacaat ggtttgtgac aaaattctgt ccaggtgtgt     120
tgaccgaatt caggctttct ttatgcgata tgagttcagt taatgaaaac acaggggagt     180
gaccagaagt gattgtttcc ttctttggcg tttctgtctt cctccttttt tgttctattc     240
ccttattttg caaccttttg gatgttaccc tttggaagtt accctcttgt aacttccaca     300
ttaaaagttt gggggctggc tgatanaagg aactccagag aacaacttga ttctgtgctt     360
tgggagagac aganaaatga ggggtgtgga ggaaggtcag anagaccctg aggcctctgc     420
ctncttcagc atgtcanagc accctatttt ggggcttgct ttctgagccc naacatctcc     480
agccttccan gantctgtgg cttatccttc ccaangatag gatcacttgn cactctactg     540
ancctaagtt gtattcantt tcttttgatc cgcctngact ctntagcnan tganaancac     600
aacntggnaa cnaaccctca taaanctgct ntancttctg gttttaagnn caaaaca        657

<210>  4
<211>  700
<212>  DNA
<213> Homo Sapiens

<400>  4
taatcacttg ttccttaagt agggatcggc ttgaatggct ccacgagggt tcagctgtct     240
cttactttta accagtgaaa ttgacctgcc cgtgaagagg cgggcataac acagcaagac     300
gagaagaccc tatggagctt taatttatta atgcaaacag tacctaacaa acccacaggt     360
cctaaactac caaacctgca ttaaaaattt cggttggggc gacctcgggg cagaacccaa     420
cctccgagca gtacatgcta agacttcacc agtcaaagcg aactactata ctcaatagat     480
ccaataactt gaccaacgga acaagttacc ctagggataa cagcgcaatc ctattctaga     540
gtccatatca acaatagggt ttacgacctc gatgttggat caggacatcc caatggtgca     600
gccgctacta aaggttcgtt tgttcaacga ttaaagtcct acgtgatctg agttcagacc     660
ggagtaatcc aggtcggttt ctatctactt caaattcccg                           700

<210>  5
<211>  893
<212>  DNA
<213> Homo Sapiens

<400>  5
cgttcactcc tgcctgggca acgaagagtg aaactccatc tcaaaacaaa caaacaaaca      60
aacaaacaag caaaagcact ctaggtataa aaggaacttc ctctgcctgc aagacccccc     120
atctccttcc catgaagtcc ttaacttctg ttcaacccaa acgcccagcg cgtcccttcc     180
actgcgctgc ccgatgcacc tctgcccgcc acgccttcag tgttgtggtc atttgtgcct     240
gcgcacccag ggctgcaggt accttcctcc agtgtgcttc caggacgggt tattcaggat     300
gctgagacga gccgccagct tcacacagaa ctggggtgag acctcagcac ctgctgcctg     360
tgttcctgag gctgtctgcc aaggcgctca ggaaacgcac atgcctcctg agcctcatat     420
gcacacctcg tggacggcag cctgcaggac cactggcaag ttttgttgcc gaaatccctc     480
ttcgaggaaa aaagtcaatt gttggcaatt agatattaag atcacataac tcacttcaat     540
cagtcgtcta aaaacaaacg gctgatacca ctgagtctca aggaagcagc cacaggggct     600
tctgctgagg gggcaggcgg agcttgagga aaccgcagat aagttttttt ctctttgaaa     660
gatagagatt aatacaacta cttaaaaaat atagtcaata ggttactaag atattgctta     720
gcgttaagtt ttaacgtaat tttaatagct taaaatttta agagaaaata tgaagactta     780
gaagagtagc atgaggaagg aaaagataaa aggtttctaa aacatgacgg aggttgagat     840
gaagcttctt catggagtaa aaaatgtatt taaaagaaaa ttgagagaaa gcg            893

<210>  6
<211>  316
<212>  DNA
<213> Homo Sapiens

<400>  6
gtttttaggg ttctttagtg ttgtttcttt cacccagggg tggtggtccc agccagtttg      60
gtgctgacgg tgagaggaaa ttagaatctg tttgcaaatt gtccaaccca ccccctcaac     120
atgaggggct tccattttct gtgttttgta agggaactgt ttccttcatg ccgccatgtt     180
cctgatatta gttctgattt ctttttaaca aatgttatca tgattaagaa aatttccagc     240
actttaatgg ccaattaact gagaatgtaa gaaaattgat gctgtacaag gcaaataaag     300
ctgtttatta accttg                                                     316

<210> 7
<211> 375
<212> DNA
<213> Homo Sapiens

<400> 7
gttcaaacag caaacgccca cagatggccc agaggtggtg gtagtcaggg tgtgtgggtg 60
tttttagggt tctttagtgt tgtttctttc acccaggggt ggtggtccca gccagtttgg 120
tgctgacggt gagaggaaat tagaatctgt ttgcaaattg tccaacccac cccctcaaca 180
tgaggggctt ccattttctg tgttttgtaa gggaactgtt tccttcatgc cgccatgttc 240
ctgatattag ttctgatttc tttttaacaa atgttatcat gattaagaaa atttccagca 300
ctttaatggc caattaactg agaatgtaag aaaattgatg ctgtacaagg caaataaagc 360
tgtttattaa ccttg 375

<210> 8
<211> 560
<212> DNA
<213> Homo Sapiens

<400> 8
atcaactttc gatggtagtc gccgtgccta ccatggtgac cacgggtgac ggggaatcag 60
ggttcgattc cggagaggga gcctgagaaa cggctaccac atccaaggaa ggcagcaggc 120
gcgcaaatta cccactcccg acccggggag gtagtgacga aaaataacaa tacaggactc 180
tttcgaggcc ctgtaattgg aatgagtcca ctttaaatcc tttaacgagg atccattgga 240
gggcaagtct ggtgccagca gccgcggtaa ttccagctcc aatagcgtat attaaagttg 300
ctgcagttaa aaagctcgta gttggatctt gggagcgggc gggccggtcc gccgcgaggc 360
gagccaccgc cgtncncgcc cttgcctctc ggcgccccct ngatgctctt agctgantgt 420
cccgcggggc ccganccgtt tactttgaaa aaatttnagt gttaaagcan gcccgaaccg 480
ctggataccc gnnntaggaa taatggatta ngaccnnggn nctntttgnn ggtttcngac 540
tgagccntat taananggac 560

<210> 9
<211> 348
<212> DNA
<213> Homo Sapiens

<400> 9
aaaacgacgg ccagtgaatt gtaatacgac tcactatagg gcgaattggg ccctctagat 60
gcatgctcga gcggccgcca gtgtgatgga tatctgcaga attcggcttt tgacaccaga 120
ccaactggta atggtagcga ctggcgctca gctggaattc cggctgggac taccgggtct 180
cactccagaa gaggcttctt cagagcatgg tagtcttggg gttctaagag aatgagagta 240
gaagctgcaa aacctcttga aactggggct tgggagtcac acatgacttt ctccacattc 300
tgttcgtcaa aagcgaatca taaggacagc acagactcaa gggataag 348

<210> 10
<211> 505
<212> DNA
<213> Homo Sapiens

<400> 10
aaacgacggc cagtgaattg taatacgact cactataggg cgaattgggc cctctagatg 60
catgctcgag cggccgccag tgtgatggat atctgcagaa ttcggctttt kacaccagac 120
caactggtaa tggtagcgac cggttctcag ctggaattcc ggattggtcc aattgggtat 180
gaggagttca gttatatgtt tgggattttt taggtagtgg gtgttgagct tgaacgcttt 240
cttaattggt ggctgctttt aggcctacta tgggtgttaa attttttact ctctctacaa 300
ggttttttcc tagtgtccaa agagctgttc ctctcttgga ctaacagtta aatttacaag 360
gggatttaga gggttctgtg gggcaaattt aaagttgaac taagattcta tcttggacaa 420
ccagctatca ccaggctcgg taggtttgtt gcctctncct ataaatcttc ccactatttt 480
tgtacataga cgggtgttct ctttt 505

<210> 11
<211> 430
<212> DNA
<213> Homo Sapiens

<400> 11
gttcagtaat tatcttttat ttcattttct ccccttccca cccctccccc tcggatccag 60
cagagggctg tggtggcggc ggcgtccaag cgggcgcgga cggcgtacac gagcgcgcan 120
tggtggagct ggagaaggag ttccatttta accgctacct gtgccggcct cgcgttgtag 180
agatggccaa cctgctgaac ctcagcgagc ggcagatcaa gatctctcct ctcaccacgc 240
gcctcctcct cagggtagaa tccaagaagc gcccaaatta acacacctta catctttgta 300
ggtaattccc cccaaatctt gatttttttt ttcctcaant atcggtttct tccacgaaac 360
ctaaactttc acaatcctct tccggngcca caaagaaggt gtcacgtgac ccgaaagcca 420
aacaccattg 430

<210> 12
<211> 556
<212> DNA
<213> Homo Sapiens

<400> 12
acaactcggt ggtggccact gcgcagacca gacttcgctc gtactcgtgc gcctcgcttc 60
gcttttcctc cgcaaccatg tctgacaaac ccgatatggc tgagatcgag aaattcgata 120
agtcgaaact gaagaagaca gagacgcaag agaaaaatcc actgccttcc aaagaaacga 180
ttgaacagga gaagcaagca ggcgaatcgt aatgaggcgt gcgccgccaa tatgcactgt 240
acattccaca agcattgcct tcttatttta cttcttttag ctgtttaact ttgtaagatg 300
caaagaggtt ggatcaagtt taaatgactg tgctgcccct ttcacatcaa agaactactg 360
acaacgaagg ccgcgctgcc tttcccatct gtctatctat ctggctggca gggaaggaaa 420
gaacttgcat gttggtgaag gaagaagtgg ggtggaagaa gtggggtggg acgacagtga 480
aatctagagt aaaaccaagc tggcccaagt gtcctgcagg ctgtaatgca gtttaatcag 540
agtgccattt tttttt 556

<210> 13
<211> 2586
<212> DNA
<213> Homo Sapiens

<400> 13
ccattaggcc tatgaattat aagatacagt cactttaaaa tccactggaa ggctgaagag 60
tgagttaaac ctcttataat gaatatacag tgaaaccagt agaggcattt tatttagggt 120
tcctacaaga aagtgcttaa atagcatcga cgcctacatg ctacatcctg ttcagtctct 180
gcctctgtga tgcagttggc cagcaaatat cctccaagtc atcatttgca tagtgctagg 240
gataaaatga ggagcaatac caaatgctat acctgccctt atgggtctta tagtccaacg 300
ggagaaaaag atattataca aataatcacg gaaaataaat agaaaacgca tccttgtttt 360
tgtttagtgg atcctctatc cttcagagac tctggaaccc ctgtggtctt ctcttcatct 420
aatgaccctg aggggatgga gttttcaagt ccttccagag aggaatgtcc caagcctttg 480
agtagggtaa gcatcatggc tggcagcctc acaggtttgc ttctacttca ggcagtgtcg 540
tgggcatcag gtgcccgccc ctgcatccct aaaagcttcg gctacagctc ggtggtgttg 600
tctgcaatgc cacatactgt gactcctttg accccccgac ctttcctgcc cttggtacct 660
tcagccgcta tgagagtaca cgcagtgggc gacggatgga gctgagtatg gggcccatcc 720
aggctaatca cacgggcaca ggcctgctac tgaccctgca gccagaacag aagttccaga 780
aagtgaaggg atttggaggg gccatgacag atgctgctgc tctcaacatc cttgccctgt 840
caccccctgc ccaaaatttg ctacttaaat cgtacttctc tgaagaagga atcggatata 900
acatcatccg ggtacccatg gccagctgtg acttctccat ccgcacctac acctatgcag 960
acacccctga tgatttccag ttgcacaact tcagcctccc agaggaagat accaagctca 1020
agatacccct gattcaccga gccctgcagt tggcccagcg tcccgtttca ctccttgcca 1080
gcccctggac atcacccact tggctcaaga ccaatggagc ggtgaatggg aaggggtcac 1140
tcaaggacaa gcccggagac atctaccacc agacctgggc cagatacttt gtgaagttcc 1200
tggatgccta tgctgagcac aagttacagt tctgggcagt gacagctgaa aatgagcctt 1260
ctgctgggct gttgagtgga taccccttcc agtgcctggg cttcacccct gaacatcagc 1320

gagacttcat tgcccgtgac ctaggtccta ccctcgccaa cagtactcac cacaatgtcc 1380
gcctactcat gctggatgac caacgcttgc tgctgcccca ctgggcaaag gtggtactga 1440
cagacccaga agcagctaaa tatgttcatg gcattgctgt acattggtac ctggactttc 1500
tggctccagc caaagccacc ctaggggaga cacaccgcct gttccccaac accatgctct 1560
ttgcctcaga ggcctgtgtg ggctccaagt tctgggagca gagtgtgcgg ctaggctcct 1620
gggatcgagg gatgcagtac agccacagca tcatcacgaa cctcctgtac catgtggtcg 1680
gctggaccga ctggaacctt gccctgaacc ccgaaggagg acccaattgg gtgcgtaact 1740
ttgtcgacag tcccatcatt gtagacatca ccaaggacac gttttacaaa cagcccatgt 1800
tctaccacct tggccacttc agcaagttca ttcctgaggg ctcccagaga gtggggctgg 1860
ttgccagtca gaagaacgac ctggacgcag tggcactgat gcatcccgat ggctctgctg 1920
ttgtggtcgt gctaaaccgc tcctctaagg atgtgcctct taccatcaag gatcctgctg 1980
tgggcttcct ggagacaatc tcacctggct actccattca cacctacctg tggcatcgcc 2040
agtgatggag cagatactca aggaggcact gggctcagcc tgggcattaa agggacagag 2100
tcagctcaca cgctgtctgt gactaaagag ggcacagcag ggccagtgtg agcttacagc 2160
gacgtaagcc caggggcaat ggtttgggtg actcactttc ccctctaggt ggtgcccagg 2220
gctggaggcc cctagaaaaa gatcagtaag ccccagtgtc cccccagccc ccatgcttat 2280
gtgaacatgc gctgtgtgct gcttgctttg gaaactngcc tgggtccagg cctagggtga 2340
gctcactgtc cgtacaaaca caagatcagg gctgagggta aggaaaagaa gagactagga 2400
aagctgggcc caaaactgga gactgtttgt ctttcctaga gatgcagaac tgggcccgtg 2460
gagcagcagt gtcagcatca gggcggaagc cttaaagcag cagcgggtgt gcccaggcac 2520
ccagatgatt cctatggcac cagccaggaa aaatggcagc tcttaaagga gaaaatgttt 2580
gagccc 2586

<210> 14
<211> 1448
<212> DNA
<213> Homo Sapiens

<400> 14
ccaccatgct cgccctggag gctgcacagc tcgacgggcc acacttcagc tgtctgtacc 60
cagatggcgt cttctatgac ctggacagct gcaagcattc cagctaccct gattcagagg 120
gggctcctga ctccctgtgg gactggactg tggccccacc tgtcccagcc accccctatg 180
aagccttcga cccggcagca gccgctttta gccaccccca ggctgcccag ctctgctacg 240
aaccccccac ctacagccct gcagggaacc tcgaactggc ccccagcctg gaggccccgg 300
ggcctggcct ccccgcatac cccacggaga acttcgctag ccagaccctg gttcccccgg 360
catatgcccc gtaccccagc cctgtgctat cagaggagga agacttaccg ttggacagcc 420
ctgccctgga ggtctcggac agcgagtcgg atgaggccct cgtggctggc cccgagggga 480
agggatccga ggcagggact cgcaagaagc tgcgcctgta ccagttcctg ctggggctac 540
tgacgcgcgg ggacatgcgt gagtgcgtgt ggtgggtgga gccaggcgcc ggcgtcttcc 600
agttctcctc caagcacaag gaactcctgg cgcgccgctg gggccagcag aaggggaacc 660
gcaagcgcat gacctaccag aagctggcgc gcgccctccg aaactacgcc aagaccggcg 720
agatccgcaa ggtcaagcgc aagctcacct accagttcga cagcgcgctg ctgcctgcag 780
tccgccgggc ctgagcacac ccgaggctcc cacctgcgga gccgctgggg gacctcacgt 840
cccagccagg atccccctgg aagaaaaagg gcgtccccac actctaggtg ataggactta 900
cgcatcccca ccttttgggg taaggggagt gctgccctgc cataatcccc aagcccagcc 960
cgggcctgtc tgggattccc cacttgtgcc tggggtccct ctgggatttc tttgtcatgt 1020
acagactccc tgggatcctc atgttttggg tgacaggacc tatggaccac tatactcggg 1080
gaggcagggt agcagtgctt ccagagtccc aagagcttct ctgggatttt cttgtgatat 1140
ctgattcccc agtgaggcct gggacctttt taagatcgct gtgtgtctgt aaaccctgaa 1200
tctcatctgg ggtgggggcc ctgctggcaa ccctgagccc tgtccaaggt tccctcttgt 1260
cagatctgag atttcctagt tatgtctggg gccctctggg agctgttatc atctcagatc 1320
tcttcgccca tctatggctg tgttgtcaca tctgtcccct catttttgag atcccccaat 1380
tctctggaac tattctgctg ccccttttta tgtgtctgga gttccccaat cacatctagg 1440
gctcctcc 1448

<210> 15
<211> 2227
<212> DNA
<213> Homo Sapiens

<400> 15
gagagcccga acaggaagag ggtacagctt tgtgcaggtc acatgcccac tgcagccctc 60
cagcctctgg tccccagagc ggactttgga agctgaactg cttttgttgc tggaagactt 120
atgttataat ttaccctggg tggaccaggg tcgtacaaaa gggcaacgct ccccagtccc 180
cccactcccg accccggaat catgcatcgg actacacgga tcaaaatcac agagctgaac 240

ccccacctca tgtgtgccct ctgcgggggg tacttcatcg acgccaccac tatcgtggag 300
tgcctgcatt ccttctgcaa aacctgcatc gtgcgctacc tggagaccaa caaatactgc 360
cccatgtgtg acgtgcaggt ccataaaacc cggccgctgc tgagcatcag gtctgacaaa 420
acacttcaag acattgtcta caaattggtc cctgggcttt ttaaagatga gatgaaacgg 480
cggcgggatt tctatgcagc gtaccccctg acggaggtcc ccaacggctc caatgaggac 540
cgcggcgagg tcttggagca ggagaagggg gctctgagtg atgatgagat tgtcagcctc 600
tccatcgaat tctacgaagg tgccagggac cgggatgaga agaagggccc cctggagaat 660
ggggatgggg acaaagagaa aacaggggtg cgcttcctgc gatgcccagc agccatgacc 720
gtcatgcatc ttgccaagtt tctccgcaac aagatggatg tgcccagcaa gtacaaggtg 780
gaggttctgt acgaggacga gccactgaag gaatactaca ccctcatgga catcgcctac 840
atctacccct ggcggcggaa cgggcctctc cccctcaagt accgtgtcca gccagcctgc 900
aagcggctca ccctagccac ggtgcccacc ccctccgagg gcaccaacac cagcggggcg 960
tccgagtgtg agtcagtcag cgacaaggct cccagccctg ccaccctgcc agccacctcc 1020
tcctccctgc ccagcccagc caccccatcc catggctctc ccagttccca tgggcctcca 1080
gccacccacc ctacctcccc cactccccct tcgacagcca gtggggccac cacagctgcc 1140
aacgggggta gcttgaactg cctgcagaca ccatcctcca ccagcagggg gcgcaagatg 1200
actgtcaacg gcgctcccgt gcccccctta acttgaggcc agggaccctc tcccttcttc 1260
cagccaagcc tctccactcc ttccactttt tctgggccct tttttccact tcttctactt 1320
tccccagctc ttcccacctt gggggtgggg ggcgggtttt ataaataaat atatatatat 1380
atgtacatag gaaaaaccaa atatacatac ttattttcta tggaccaacc agattaattt 1440
aaatgccaca ggaaacaaac tttatgtgtg tgtgtatgtg tggaaaatgg tgttcatttt 1500
ttttgggggg ggtcttgtgt aatttgctgt tttggggggt gcctggagat gaactggatg 1560
ggccactgga gtctcaataa agctctgcac catcctcgct gtttcccaag gcaggtggtg 1620
tgttgggggc cccttcagac ccaaagcttt aggcatgatt ccaactggct gcatatagga 1680
gtcagttaga attgtttctt tctctccccg tttctctccc catcttggct gctgtcctgc 1740
ctctgaccag tggccgcccc ccgcgttgtt gaatgtccag aaattgctaa gaacagtgcc 1800
ttttacaaat gcagtttatc cctggttctg aggagcaagt gcagggtgga ggtggcacct 1860
gcatcacctc ctcctcttgc agtggaaact ttgtgcaaag aatagatagt tctgcctctt 1920
tttttttttt ttcctgtgtg tgtggccttt gcatcattta tcttgtggaa aagaagattc 1980
aggccctgag aggtctcagc tcttggagga gggctaaggc tttagcattg tgaagcgctg 2040
cacccccacc aaccttaccc tcaccgggga accctcacta gcaggactgg tggtggagtc 2100
tcacctgggg cctagagtgg aagtgggggt gggttaacct cacacaagca cagatcccag 2160
actttgccag aggcaaacag ggaattccgc cgatactgac gggctccagg agtcgtcgcc 2220
acactcg 2227

<210> 16
<211> 205
<212> DNA
<213> Homo Sapiens

<400> 16
ggtaatactt agagcattac aaagcacttt cacatttaaa tttgattttg gaaagtattt 60
tctttttgag acagagtctc tgtcacccag gctggagtgc atggagtgca gtggtgcaaa 120
cacagctcgc tgcaccctca acctcctggg ctcaagcagt ctttccacct cggcctccca 180
agttgctagg actataggac tacag 205

<210> 17
<211> 354
<212> DNA
<213> Homo Sapiens

<400> 17
tgagcttgaa cgctttctta attggtggct gcttttaggc ctactatggg tgttaaattt 60
tttactctct ctacaaggtt ttttcctagt gtccaaagag ctgttcctct ttggactaac 120
agttaaattt acaaggggat ttagagggtt ctgtgggcaa atttaaagtt gaactaagat 180
tctatcttgg acaaccagct atcaccaggc tcggtaggtt tgtcgcctct acctataaat 240
cttcccacta ttttgctaca tagacgggtg tgctctttta gctgttctta ggtagctcgt 300
ctggtttcgg gggtcttagc tttggctctc cttgcaaagt tatttctagt taat 354

<210> 18
<211> 354
<212> DNA
<213> Homo Sapiens

<400> 18

attaactaga aataactttg caaggagagc caaagctaag acccccgaaa ccagacgagc 60
tacctaagaa cagctaaaag agcacacccg tctatgtagc aaaatagtgg gaagatttat 120
aggtagaggc gacaaaccta ccgagcctgg tgatagctgg ttgtccaaga tagaatctta 180
gttcaacttt aaatttgccc acagaaccct ctaaatcccc ttgtaaattt aactgttagt 240
ccaaagagga acagctcttt ggacactagg aaaaaacctt gtagagagag taaaaaattt 300
aacacccata gtaggcctaa aagcagccac caattaagaa agcgttcaag ctca 354


<210> 19
<211> 713
<212> DNA
<213> Homo Sapiens

<400> 19
gccgcctatt tcctccgaaa cccgcgctgc ggagcagccc agtgcataga gttcaacact 60
tccccttgtt gtggaaagta aaggagcctc actaccacct ttttttcttt gcgttttctt 120
actgctggtc ctgggagcct tttccttcgg agcagcagcc ctgtccggca tctgtcttga 180
gctcccagca aggaaagtcc atcagcttga taatggagga gaacaatgac tccacggaga 240
acccccaaca aggccaaggg cggcagaatg ccatcaagtg tgggtggctg aggaagcaag 300
gaggctttgt caagacttgg catactcgct ggtttgtgct caagggggat cagctctatt 360
atttcaaaga tgaagatgaa accaagccct tgggtactat ttttctgcct ggaaataaag 420
tttctgagca tccctgcaat gaagagaacc cagggaagtt cctttttgaa gtagttccag 480
gtaagatatt ttcctagtct gattaaatta ttgcatcctg ggtggtaaag gtgaanatgg 540
gtcaaacagg nttcattctt ttttgaatca tgactgagac cttaatttga ggcttggnta 600
atggtgaccc aaataatgat gcagggttat ttctaatcaa atgaatgcct ccccactact 660
ntgacacata atataaattt atttgncatg aactcatant gacccannnt gag 713



<210> 20
<211> 261
<212> DNA
<213> Homo Sapiens

<400> 20
gcaaaacctc cttgaagata caattttgtg aggaaatatg tcagtgattc cactgggcaa 60
agcattcaac ctataacccc ttgtcaaatt tcacatcaca agagcgctgt aaaatcaaat 120
tcatctccaa tagtcctgaa caaatactgt atcatgactt gtggtcaact atggagtctc 180
atggacaaat gaaaatctan tagttatgtg gncanagtat gtgtgngnga ncgcattcat 240
tngnnctann atataancnt g 261



<210> 21
<211> 354
<212> DNA
<213> Homo Sapiens

<400> 21
tgagcttgaa cgctttctta attggtggct gcttttaggc ctactatggg tgttaaattt 60
tttactctct ctacaaggtt ttttcctagt gtccaaagag ctgttcctct ttggactaac 120
agttaaattt acaaggggat ttagagggtt ctgtgggcaa atttaaagtt gaactaagat 180
tctatcttgg acaaccagct atcaccaggc tcggtaggtt tgtcgcctct acctataaat 240
cttcccacta ttttgctaca tagacgggtg tgctctttta gctgttctta ggtagctcgt 300
ctggtttcgg gggtcttagc tttggctctc cttgcaaagt tatttctagt taat 354


<210> 22
<211> 355
<212> DNA
<213> Homo Sapiens

<400> 22
attaactaga aataactttg caaggagagc caaagctaag acccccgaaa ccagacgagc 60
tacctaagaa cagctaaaag agcacacccg tctatgtagc aaaatagtgg gaagatttat 120
aggtagaggc gacaaaccta ccgagcctgg tgatagctgg ttgtccaaga tagaatctta 180
gttcaacttt aaatttgccc acagaaccct ctaaatcccc ttgtacaatt taactgttag 240
tccaaagagg aacagctctt tggacactag gaaaaaacct tgtagagaga gtaaaaaatt 300
taacacccat agtaggccta aaagcagcca ccaattaaga aagcgttcaa gctca 355

<210> 23
<211> 599
<212> DNA
<213> Homo Sapiens

<400> 23
gtgtgacttc accgaagacc agaccgcaga gttcaaggag gccttccagc tgtttgaccg 60
aacaggtgat ggcaagatcc tgtacagcca gtgtggggat gtgatgaggg ccctgggcca 120
gaaccctacc aacgccgagg tgctcaaggt cctggggaac cccaagagtg atgagatgaa 180
tgtgaaggtg ctggactttg agcactttct gcccatgctg cagacagtgg ccaagaacaa 240
ggaccagggc acctatgagg attatgtcga aggacttcgg gtgtttgaca aggaaggaaa 300
tggcaccgtc atgggtgctg aaatccggca tgttcttgtc acactgggtg agaagatgac 360
agaggaagaa gtanagatgc tggtggcagg gcatgaggac agcaatggtt gtatcaacta 420
tgaagagctc gtccgcatgg tgctgaatgg ctgaggacct tcccagtctc ccaaatccgt 480
gcctttccct gtgtgaattt tgtatctacc taaaagtttc cctagctttt ttgttcanca 540
ctttccattt gttttnttga tgatgttgcc gcacattcac caataacttg tttttggcc 599

<210> 24
<211> 545
<212> DNA
<213> Homo Sapiens

<400> 24
ggcccagaga gcaagtttat ttggtgaatg ctgacggcaa acatcatcca agagagacaa 60
gatgggaaag ttgctgagac aagaaagcct agggaaactt taggctagat acaaaattca 120
cacagggaaa ggcacggact ctggggagac tgggaaggtc ctcagccatt cagcaccatg 180
cggacgagct cttcatagtt gatacaacca ttgctgtcct catgccctgc caccagcatc 240
tctacttctt cctctgtcat cttctcaccc agtgtgacaa gaacatgccg gatttcagca 300
cccatgacgg tgccatttcc ttccttgtca aacacccgaa gtccttcgac ataatcctca 360
taggtgccct ggtccttgtt cttggccact gtctgcagca tgggcagaaa gtgctcaaag 420
tccagcacct tcacattcat ctcatcactc ttggggttcc ccaggacctt gagcacctng 480
gcgttggtag ggttctggcc caaggccctc atcacatccc cacactggct gncaggatct 540
tgcat 545

<210> 25
<211> 387
<212> DNA
<213> Homo Sapiens

<400> 25
ttcagtttcc tctcctagta gtacacgagt ctccattgtt tcacatcctc accagtgctt 60
tgtattgtct gacttttaag attctgctca tcagacatat gtaaatgaca cataacacag 120
tttgttttca cagaacaaat ggttatttaa attctaaacc caaagtaatg tacaattaca 180
ataaaaggcc agaagaaaga ggaggaagga aaaagatgtg agaaataaaa ttgttatagt 240
aattcttgtt ttcgcttcca agcataaaat agtaattgga atgtttagtg tgcatgtgtg 300
tatacaatgc aatatgatac aatataaaag caatgcctct ctttgttcca ttggttgntt 360
tttaaatcta tttttataag taataag 387

<210> 26
<211> 178
<212> DNA
<213> Homo Sapiens

<400> 26
ctggaatcta gatagttttc aggatgggga agatagattc aaaaccacct aagggcattc 60

tgggtacaaa gcattgtgca aggctttggt gatacagaga ataaggtctt ttttcccata 120
cttcctcatc tgccaaggtt atctccaatt gtacctttct ctccagttcc aagcttgc 178

<210> 27
<211> 387
<212> DNA
<213> Homo Sapiens

<400> 27
cggtaaattt tttactctct ctacaaggtt ttttcctagt gtccaaagag ctgttcctct 60
ttggactaac agttaaattt acaaggggat ttagagggtt ctgtgggcaa atttaaagtt 120
gaactaagat tctatcttgg acaaccagct atcaccaggc tcggtaggtt tgtcgcctct 180
acctataaat cttcccacta ttttgctaca tagacgggtg tgctctttta gctgttctta 240
ggtagctcgt ctggtttcgg gggtcttagc tttggctctc cttgcaaagt tatttctagt 300
taattcatta tgcagaaggt ataggggtta gtccttgcta tattatgctt ggttataatt 360
tttcatcttt cccttgccga aattccg 387

<210> 28
<211> 420
<212> DNA
<213> Homo Sapiens

<400> 28
gtcagaaaac cacacatgca gacccagaga gaatggtttc tttgtggttt gcacaggaaa 60
aaccatcctt actaaaagac agggtagagt ggggagggtg caggaaggaa ctcataatga 120
ccattttccc acatggagaa gcaaagcagc ttcttcaggg ctcaatcagt tatgaaaaag 180
aatctcaccc cattagatag cactcctgag ctcagtgtag ggtcccaagc ccaccaacca 240
aggctgtctc cccagaacaa atcaggaagg ctccagtggt cagatagaaa gtgacaaaca 300
aaacatgagt gcatctagcc acatcctcac attccacaca agagaaccca tgtgactaaa 360
caggaatccc ctgctgcccc agttctaaaa aggaactact gactgccagt gcaatttctt 420

<210> 29
<211> 402
<212> DNA
<213> Homo Sapiens

<400> 29

caaggaaatt gcactgtgca gtcagtagtt cctttttaga actgggtgca gcaggggatt 60
cctgtttagt cacatgggtt ctcttgtgtg gaatgtgagg atgtggctag atgcactcat 120
gttttgtttg tcactttcta tctgaccact ggagccttcc tgatttgttc tggggagaca 180
gccttggttg gtgggcttgg gaccctacac tgagctcagg agtgctatct aatggggtga 240
gattcttttt cataactgat tgagccctga agaagctgct ttgcttctcc atgtgggaaa 300
atggncatta tgagttcctt tctgcaccct ccccactcta ccctgtcttt tantaaggat 360
gggttttnct gtgcaaacca caaagaaacc nttctctctg gg 402

<210> 30
<211> 145
<212> DNA
<213> Homo Sapiens

<400> 30
tgcagctcgc cttgcacaac aggaaaaaca anaacaagtt aaaattgagt ctntngccaa 60
nagcttaaaa aatgctntga ggcaaactgc aagtgtcact ntgcaggcta ttgcagctca 120
aaatgctgcg gtccaggctg tcaat 145

<210> 31
<211>
<212> DNA
<213> Homo Sapiens

<400> 31
gcattgacag cctggaccgc agcattctga gctgcaatag cctgcagagt gacacttgca 60
gtttgcctca gagcatcttc taagctcttg gctagagact caattttaac ttgttcttgt 120
tttcctgtt gtgcaaggcg agctgcat 148

<210> 32
<211> 642
<212> DNA
<213> Homo Sapiens

<400> 32
gctggaacag aatagcctgg aacaggatct ttcgttccat aatatttttt aattagagca 60
agtcctgcta ctgtatctgt tcctttgaag ttaaccaagt gagcagatgc tcctatgcca 120
gcagtctctt gggaagagac tcctctgtag ccaaaatcat gtaacttgta ttccagacca 180
tctaagttac cagaagtttc taacaaatat ttggccaata ttttcttctg ctctctagaa 240
tttgtggcca ctgtgattgg ataccaggac tgaacaagaa tagtctcaat ccaatttgta 300
agccagtaac actctggatc tgtgttttcc accgtgaaga aacatttcct ctgggaatga 360
caaanccctc angaacagct tttatttcta ttggaagatg cccatcatac ttctcaagaa 420
tggagttcct cccttttcat taaagacatc atcttggaaa tgttctttgt agacatcttt 480
ggcttcctgg atttctcttt gggtactact ttacctttta agnacttatt aanaaagnac 540
tgnacccata aaaactggnn ctcatattta ncttccttaa ttggaggntn tgnttntttt 600
acggnttcaa agangaaaaa atttcttgng tggggggant tg 642

<210> 33
<211> 540
<212> DNA
<213> Homo Sapiens

<400> 33
gccgcgccag ggagctcgcg gcgcgcggcc cctgtcctcc ggcccgagat gaatcctgcg 60
gcagaagccg agttcaacat cctcctggcc accgactcct acaaggttac tcactataaa 120
caatatccac ccaacacaag caaagtttat tcctactttg aatgccgtga aaagaagaca 180
gaaaactcca aattaaggaa ggtgaaatat gaggaaacag tattttatgg gttgcagtac 240
attcttaata agtacttaaa aggtaaagta gtaaccaaag agaaaatcca ggaagccaaa 300
gatgtctaca aagaacattt ccaagatgat gtctttaatg aaaagggatg gaactacatt 360
cttgagaagt atgatgggca tcttccaata aaaataaaaa ctgttcctga gggctttgtc 420
atttccanag gaaatgtttc ttnncggggg aaaacacaga tccnaagggg nactggntta 480
caaattggat tgagantatt cttggtnann cctgggatcc aatccaaggg ggcccaaatt 540

<210> 34
<211> 460
<212> DNA
<213> Homo Sapiens

<400> 34
cacgagcgca cgtgttagga cccgaaagat ggtgaactat gcctgggcag ggcgaagcca 60
gaggaaactc tggtggaggt ccgtagcggt cctgacgtgc aaatcggtcg tccgacctgg 120
gtataggggc gaaagactaa tcgaaccatc tagtagctgg ttccctccga agtttccctc 180
aggatagctg gcgctctcgc agacccgacg cacccccgcc acgcagtttt atccggtaaa 240
gcgaatgatt agaggtcttg gggccgaaac gatctcaacc tattctcaaa ctttaaatgg 300
taanaagccc ggctcgcttg gcgtggagcc gggcgtggaa tgcnagtgcc taatggccca 360
cttttggtaa ncaaaactgg cgctgcggga tgaacccaac gcccggttaa ngggcccnat 420
gccgacctca tnanacccca naaaangngt tggntgatac 460

<210> 35
<211> 500
<212> DNA
<213> Homo Sapiens

<400> 35

tatcaaccaa caccttttct ggggtctgat gagcgtcggc atcgggcgcc ttaacccggc 60
gttcggttca tcccgcagcg ccagttctgc ttaccaaaag tggcccacta ggcactcgca 120
ttccacgccc ggctccacgc cagcgagccg ggcttcttac ccatttaaag tttgagaata 180
ggttgagatc gtttcggccc caagacctct aatcattcgc tttaccggat aaaactgcgt 240
ggcgggggtg cgtcgggtct gcgagagcgc cagctatcct gagggaaact tcggagggaa 300
ccagctacta natggttcga ttaagtcttt cgcccctata cccaggtcgg acgaccgatt 360
tgcacgtnag gaccgctacg gacctcccca nagttcctnt ggnttngccc tgccaggcta 420
ntnaccatnt ttgggnctaa acgngcgctc ggccggaatt cnccganctg angggtccng 480
aatnnnnccc ccatcccagc 500

<210> 36
<211>
<212> DNA
<213> Homo Sapiens

<400> 36
gcccccgcta accggctttt tgcccaaacg ggccattatc gaagaattca caaaaaacaa 60
tagcctcatc atccccacat catagccacc atcaccctcc ttaacctcta cttctaccta 120
cgcctaatct actccacctc aatcacacta ctccccatat ctaacaacgt aaaaataaaa 180
tgacagtttg aacatacaaa acccacccca ttcctcccca cactcatcgc ccttaccacg 240
ctactcctac ctatctcccc ttttat 266

<210> 37
<211> 268
<212> DNA
<213> Homo Sapiens

<400> 37
ataaaagggg agataggtag gagtagcgtg gtaagggcga tgagtgtggg gaggaatggg 60
gtgggttttg tatgttcaaa ctgtcatttt atttttacgt tgttagatat ggggagtagt 120
gtgattgagg tggagtagat taggcgtagg tagaagtaga ggttaaggag ggtgatggtg 180
gctatgatgt ggggatgatg aggctattgn tttttgtgaa ttnttnnata atggcccgtt 240
tgggcaaaaa gccggntanc gggggccg 268

<210> 38
<211> 427
<212> DNA
<213> Homo Sapiens

<400> 38
cgggacttta ccgcatcatt tgcagaacca gtatcaatat tcgtaaggta actgctctta 60
aaactcanaa tcatcctaac tggatgtaaa aactttttcc cagaaaatgt tggggtgcac 120
tcacaaaacc ctcttacttc attttctcca tataatgact ctatgggggg agggggccag 180
gtgtgctcat tctcatttga aatttgaatt ccaatcttgt tagaatgtag cccaactcct 240
ttcctttctc aggaaagtgg cgacagttct caggtctgcc tccacattac catcacctgg 300
gggatctaaa actactcagg cctgggttcc accttcagcc aacgaaatct gaatctttan 360
gggtggctga tttcgctgtt ctgtaaatga agttttaatg gtcacagccc gtctgaccgt 420
ttgcata 427

<210> 39
<211> 757
<212> DNA
<213> Homo Sapiens

<400> 39
   tatgcaaacg gtcagacgtg gctgtgacca ttaaaacttc atttacagaa cagcgatatc 60
   agccacccct aaagattcag atttcgttgg ctgaaggtgg aacccaggcc tgagtagttt 120
   tagatcccca ggtgatggta atgtggaggc agacctgaga actgtcggcc actttcctga 180
   gaaaggaaag gagttgggct acattctaac aagattggaa ttcaaatttc aaatgagaat 240
   gagcacacct ggccccctcc ccccatagag tcattatatg gagaaaatga agtaagaggg 300
   ttttgtgagt gcaccccaac attttntggg aaaaaggttt tacatccagt taggatgatt 360
   ctgagtttta agancantta ccttaccaat attgatactg ggtctgcnaa ngatgcggga 420
   aaatcccccg gnattcactg agcgccnggc ggtaccatta caantggntn gggggnaaaa 480
   ataataatna ccggcaggca tgttaagncc aaattttnga aattcttnca ctggggggcg 540
   gttnacttct tttnaagggc caatnncccn tatgaggngn ntanaantnc tggccnngnt 600
   ttcannnnnn acnggaaaac tggggttccc aattaantnn tttnnnnnan ncccttttcc 660
   ctgggnanan naaaaaggcc nnnccantnc cnttcnanan tnncnnctan ngggaanggn 720
   nccccntnnn gnncntaanc nggggggggg ggtnccc 757

<210> 40
<211> 285
<212> DNA
<213> Homo Sapiens

<400> 40
caacaacaca tcatcagtag ggtaaaacta acctgtctca cgacggtcta aacccagctc 60
acgttcccta ttagtgggtg aacaatccaa cgcttggtga attctgcttc acaatgatag 120
gaagagccga catcgaagga tcaaaaagcc gacgtcgcta tgaacgcttg gccgccacaa 180
gccagttatc ccttgtggta acttttctga cacctcctgc ttaaaaccca aaaggtcaga 240
aggatcgtga ggccccgctt tcatgggcag taggcagatt cgtcc 285

<210> 41
<211> 283
<212> DNA
<213> Homo Sapiens

<400> 42
ggacgaatct gcctactgcc catgaaagcg gggcctcacg atccttctga ccttttgggt 60
tttaagcagg aggtgtcaga aaagttacca cagggataac tggcttgtgg cggccaagcg 120
ttcatagcga cgtcgctttt tgatccttcg atgtcggctc ttcctatcat tgtgaagcag 180
aattcaccaa gcgttggatt gttcacccac taatagggaa cgtgagctgg gtttagaccg 240
tcgtgagaca ggttagtttt accctactga tgatgtgttg ttg 283

<210> 43
<211> 765
<212> DNA
<213> Homo Sapiens

<400> 43
   tttttttttt ttttttgaga cagagtctca ctctgtcacc caggctgggg tgcaatggca 60
   tgctctcagc tcactgcaac ctctgcctcc tgggttcaag cgattctcct gcctcaacct 120
   cccgagtgac tgggattaca ggcatgcacc accataccca gctaattttt gtatttttag 180
   tanagatggg gtttcaccat gttggtgagg ctgatcttaa actcctgacc tcaggtgatc 240
   tgcctgcctt ggcttcccaa agcactggga ttacaggtgt gagccaccat gcctggccta 300
   ttttgaaaaa aatttgaagt caaaataata gtacaataaa tacctgtgaa cccttcagct 360
   atatttacca attgttaata ttttaccatg tttgcttcat ctctctacat atgtattcat 420
   atgtaatttt ttttattttt gccaaaacat ttgaaaatta aacatctgga tactttgcca 480
   ttaaancctt caacatgaat ctcctaaaaa ataanaacag cttntatccc cataccntta 540
   tcacatccca gaaaattacc cccntacatt naatgactac tncngcccta tcaaattntt 600
   tgatatccaa actttctttg gggggntttt ttcccnnccc naatcantca ngnccnccat 660
   tgnntttaat gggnagntnc tnnannnaaa atatccncct tttttctttn tganttgnct 720
   tttaaaaaac cantnananc ctgggngntn ccaacngnnt tntgg 765

<210> 44
<211> 584
<212> DNA
<213> Homo Sapiens

<400> 44
cggctttgtg gaagacagtt tttccgtgaa caggggttgg aggtggtggt gggagggatg 60
gttttgggat gaaactgttc cacctcagat cattaggtat tagattctca taaagagcac 120
acagcctana tccctcacat gtgcagttcc tatgagaatc taatgccaca gttcacccgc 180
cactcaccgc tgtgagtggc cttgttccta acagaccatg gaccantact ggcccgtggc 240
ccangggtta gggacccctg atctaacaca tanatctaat gaagaaacag gttccatgtg 300
ttaaaaatct gtggttgaaa ctgacattat attcctcctg atttgatacc atggggaata 360

canaacatga cctatgtggt actcctacca aaaacgtttn acttgaatct aaccatganc 420
aaacatccan acaantacag cttgtgagag cctcncangc tgntacttgg attttttaaa 480
anngnnntgn ntnaaaggaa aaaaggnngg gntantntnn attaangaac ttncnntnaa 540
ngcnngngng gncttgntga anntngatgg gaaaaaancn cccc 584

<210> 45
<211> 439
<212> DNA
<213> Homo Sapiens

<400> 45
agcacactgg cggccgttac tagtggatcc gagctcggta ccaagcttga tgcatagctt 60
gagtattcta tagtgtcacc taaatagctt ggcgtaatca tggtcatagc tgtttcctgt 120
gtgaaattgt tatccgctca caattccaca caacatacga gccggaagca taaagtgtaa 180
agcctggggt gcctaatgag tgagctaact cacattaatt gcgttgcgct cactgcccgc 240
tttccantcg ggaaacctgt cntgccanct gcattaatna atcngncaac ncncggggag 300
aggcggtttn cntattnggc gctcttncnc ttctcnntca ctgactcntg nctcngncnn 360
tnnnntnnng nnancggata nntnacttca aangcggnat acgntatcca naatnanggg 420
ataacncnnn aaaaaacat 439

<210> 46
<211> 335
<212> DNA
<213> Homo Sapiens

<400> 46

agcacactgg cggccgttac tagtggatcc gagctcggta ccaagcttga tgcatagctt 60
gagtattcta tagtgtcacc taaatagctt ggcgtaatca tggtcatagc tgtttcctgt 120
gtgaaattgt tatccgctca caattccaca caacatacga gccggaagca taaagtgtaa 180
agcctggggt gcctaatgag tgagctaact cacattaatt gcgttgcgct cactgcccgc 240
tttccantcg ggaaacctgt cntgccanct gcattaatna atcngncaac ncncggggag 300
aggcggtttn cntattnggc gctcttncnc ttctc 335

<210> 47
<211> 768
<212> DNA
<213> Homo Sapiens

<400> 47
ctaaacttag ggcaacccca agcgcttgaa cctataccac cccactttcc tgagctctgt 60
aaagagcatg aagttttccc actgacccca tacactgagg tgccatcaca ctgcacattt 120
ccttccggag aacaagcacg tactcaggtg gagatagaac tgtcttttta cttaatagaa 180
aatgatgtgg cagctttaag aggagcgcgt cggtctgggg ctggtggctt gggtcacgtg 240
acaccggtgg tctcgtttgc gcctcttgat gtcgcggcgg cgccctgagg acggattggg 300
caaggctggt ccctgtgtga tgagacatca ccctcccagg agcaaggcgg aagtctggag 360
gaccttangg gcggangcgg gagaagcnaa ctccgatgaa tggtctcggc aggctcttcg 420
ggaaagggtg agccanggtg ggactggcca gccaggaagc ctgctggtgc aggggaaana 480
aganancccg cgagattngg ccggaccctt cccggcnggg gaagaaaatc aggagaacag 540
gctgactgga aaancccgcg gnccatggng gacaagggta ttnccggggc caaaaggnca 600
ccatgtnggn ggaattccnc tgacnccggc gttacattaa cantnggntg ggggnaaaan 660
aaaataaccg gnnggcctgt nagccaaatt cacnnctggn gggcgtnttt ggntccacnn 720
gnccnacttg annnnanttn ngnttttttn ggnnccnaaa antgggga 768

<210> 48
<211> 498
<212> DNA
<213> Homo Sapiens

<400> 48
ccgacatggt gtgccttttg gcaccggcga tgagccttgc tccgccatcg gccgccgggg 60
ttttccagtc agcctgtctc ctgattctct tcccctgccc ggcgcagcgg tccggccgaa 120
tctcgccggg gtctcctctt cccctgcacc agccagcgcc tcctggctgg ccagtcccac 180

cctggctcac ccttcccgaa gagcctgccg agaccactca tcgcgagctc gcctctcccg 240
cctccgcccc tcagcgtcct ccagacttcc gccttgctcc tgggagggtg atgtctcatc 300
acacagggac cagccttgcc caatccgtcc tcagggcgcc gccggacatc aagaggcgca 360
aacnanacac cggtgtcacg tgaaccaagc caccanccca naacgancgc ttctcttaaa 420
gctggcccat tatttttatt aantaaaaan acagntntat tttcactgan tacntgcttg 480
ttntccgaag gaaagggc 498

<210> 49
<211> 428
<212> DNA
<213> Homo Sapiens

<400> 49
gatggcttat ataaccagaa gccaaatatt tgtgttccaa aaattatttt acttagaaca 60
attcatttag attcacttca atgtgaagta tgtgaaaagc ttaattgctg accagagtga 120
attttccaac aataagaaat gcatggctga ttggctcaaa tgattctatt cttcagccct 180
tactgaagta cttagtgcat accacctatg taattttatt ccccccttat agagatgggg 240
tttcaccatg ctgcccaggc gggtctcaaa ctcctaggta caagtgatcc acccacttcg 300
gcccgccaaa gggccgggat tactggcatg agccaccaag cccagcctgg ttatgtattt 360
attcggtatc ataggggcta cagcacaaat caaaaccata gtatcagtga cctccaatct 420
aattcccg 428

<210> 50
<211> 426
<212> DNA
<213> Homo Sapiens

<400> 50
ggaattagat tggaggtcac tgatactatg gttttgattt gtgctgtagc ccctatgata 60
ccgaataaat acataaccag gctgggcttg gtggctcatg ccagtaatcc cggccctttg 120
gcgggccgaa gtgggtggat cacttgtacc taggagtttg agacccgcct gggcagcatg 180
gtgaaacccc atctctataa ggggggaata aaattacata ggtggtatgc actaagtact 240
tcagtaaggg ctgaagaata gaatcatttg agccaatcag ccatgcattt cttattgttg 300
gaaaattcac tctggtcagc aattaagctt ttcacatact tcacattgaa gtgaatctaa 360
atgaattgtt ctaagtaaaa taatttttgg aacacaaata tttggcttct ggntatataa 420
nccatc 426

<210> 51
<211> 570
<212> DNA
<213> Homo Sapiens

<400> 51
gaattgcttg gacctgggag gcataggttg tggtaagccg agattgcgcc atcgcactcc 60
agcctgggca acaagagtga aactccgtct caaacaaaca aacaaaaaag acacaaaagt 120
aaaggacttc ttgacctctg gttgaaagag tagcgcatgg ggggtgtttc tggcaaacaa 180
accttcccaa caacgtcaga actgtgttca caaatgctaa cctgtcggcc tggttataga 240
acatcctctt ccctcagggg tatctggcag aggcaggtac ccgtggaatg gtgcaggtgg 300
tgcccatgct ctagtgtatg ccaagagttc ctacttttac aaagtagcca ctttaaaaaa 360
atgttggtac tggccaacat tcctttcatg cacccaggag ggcagcaggt acctgggatc 420
caaggatgga tggccagggc aggtggctga aaaatggggg tgggtcaaga aggatgtanc 480
tcctggggtg gcgcccaaca aaaaaaatta ngggtagggn gggngctatg gntggaatgn 540
ttatcccccc caaactnann ttnaanggaa 570

<210> 52
<211>
<212> DNA
<213> Homo Sapiens

<400> 52
tggccaatgc tctctctgtg aaacttcaaa cttcaaatga ggcccacctt acatgggtca 60
ccatgtgcat ggaaagaatg tatttacact caggtacatt ctcgtgggaa actggaaacc 120
agccggcggc atcttggtgt gactgcatgc acaatgcatg cgtgtcctta aagcatttaa 180
tgttaatgtt tgtatgtgtg aatgcaaagg aattttaatg atatcatggc cacatcgagg 240

tcactaacct tgggaagtaa catgatcaca agaattttgt atgtgctgag tgactattac 300
agtaacgatt gcagtgtata attgaagtag tccggcataa tttcaagggc ccagactccg 360
tggaaagagt ttcctgactg agtccacgtc cattcaccaa ggaaggcagg cagtggcctt 420
gcanaaatcc ctcacaatga tgntgggcat cccatctacc ttggttttta gggctggcat 480
aataatgccn gnctattcan ttttaagaca gatatatttt acnnataaac cctggngggg 540
canaaaancc ccctggnttc taactctaac ctgggctctt nccttactgg gccctggggg 600
gntgntccta ttcnatnaaa aanccnccan cngacggctc nagaatnnnn cnccatccaa 660
ncnaattca 669

<210> 53
<211> 719
<212> DNA
<213> Homo Sapiens

<400> 53
ttcataggaa tagggaacaa acaccacagt ggcacantna tgggaaggag cccaggctag 60
gagttaggag acagtggggg cttctctgac accaccaggg ctctcatctg taaaatgata 120
tctgtcttaa aactgaatga gacctggcat tattatggcc agccctgaaa accaaggtag 180
atgggatgca caacatcatt gtgagggatt tctgcaaggc cactgcctgc cttccttggt 240
gaatggacgt ggactcagtc aggaaactct ttccacggag tctgggccct tgaaattatg 300
ccggactact tcaattatac actgcaatcg ttactgtaat agtcactcag cacatacaaa 360
attcttggga tcatgttact tccaagggta gtgacctcna tgtggccatg atatcattaa 420
aattcctttg cnttccccct nccaacatta acattaaatg ctttaaggac cccctgcntt 480
tggcatgcan cacccaanan gccgccgctg gnttccattt cccccanaag gacctgaang 540
gaaatacttc tttcctccca tggggaccct gnangggggc ccanttnaan ttgaanttnc 600
aaaaaacatt ggcncggaat ccnctgaccc cgggngttnc ttacaantgg gnngggggna 660
aaanaaaana accggcnggc ctgnnanncc aatttnnaaa anctnnactg ggggcgttg 719

<210> 54
<211> 786
<212> DNA
<213> Homo Sapiens

<400> 54
caaagataag acccccgaaa ccagacgagc tacctaagaa cagctaaaag agcacacccg 60
tctatgtagc aaaatagtgg gaagatttat aggtagaggc gacaaaccta ccgagcctgg 120
tgatagctgg ttgtccaaga tagaatctta gttcaacttt aaatttgccc acagaaccct 180
ctaaatcccc ttgtaaattt aactgttagt ccaaagagga acagctcttt ggacactagg 240
aaaaaacctt gtagagagag taaaaaattt aacacccata gtaggcctaa aagcagccac 300
caattaagaa agcgttcaag ctcaacaccc actacctaaa aaatcccaaa catataactg 360
aactcctcac acccaattgg accaatctat caccctatag aaaaactaat ggtagtataa 420
agtaacatga aaacattctt ctncgcataa gcctgcgtca gattaaaacc ttgaactgac 480
attaacagcc caatatctac aatcaaccac aagtcattat taccctactg nnnanccacc 540
angcatgctc ntaaggaaag gttaaaaaag taaaaggact cggnaatntt acccgctgtt 600
tccaaaaaat taccttacnt cnccntntta aggccccctg nccatgacca tgtttaaggc 660
cgnggnccct accggcaaag gggganaata cttttcttan tagggcccnt taangntccc 720
canggtnant tttttatttt acannnaatn actnccngaa aggggntnaa cnnaanaaaa 780
aaacnt 786

<210> 55
<211> 469
<212> DNA
<213> Homo Sapiens

<400> 55
ggttgggttc tgctccgagg tcgccccaac cgaaattttt aatgcaggtt tggtagttta 60
ggacctgtgg gtttgttagg tactgtttgc attaataaat taaagcccca tagggtcttc 120
tcgtcttgct gtgtcatgcc cgcctcttca cgggcaggtc aatttcactg gttaaaagta 180
agagacagct gaaccctcgt ggagccattc atacaggtcc ctaattaagg aacaagtgat 240
tatgctacct ttgcacggtt agggtaccgc ggccgttaaa catgtgtcac tgggcaggcg 300
gngcctctaa tactggngat gctagaggng atgtttttgg taaacaggcg gggnaanatt 360
gccgagntcc ttttactttt tttaaccttt ncttatnaac atgcctgtgt tgggttgaca 420
gngagggnaa taatgactng tggntgatgn aaaaattggg ctgtnattg 469

<210> 56
<211> 716
<212> DNA
<213> Homo Sapiens

<400> 56
gggtaccaaa tttctttatt tgaaggaatg gtacaaatca aagaacttaa gtggatgttt 60
tggtacaact tatagaaaag gtaaaggaaa ccccaacatg catgcactgc cttggtgacc 120
agggaagtca ccccacggct atggggaaat tagcccgagg cttanctttc attatcactg 180
tctcccaggg tgtgcttgtc aaagagatat tccgccaagc cagattcggg cgctcccatc 240
ttgcgcaagt tggtcacgtg gtcacccaat tctttgatgg ctttcacctg ctcattcagg 300
taatgtgtct caatgaagtc acacaaatgg gggtcatttt tgtcaagtgg ccagtttgtg 360
cagttccagt agtgactgat tcacattttt ttccaaatgt aatgcacact ccattgcatt 420
cacccgntct cccantcatn acaanctggn ttttgatatc ctgaangaaa aatcggcccc 480
tcnttggtct tgancttcat cantttntaa catgttcctt tcctatgaaa ttggggaaaa 540
aagtatttgc aaattntnaa ancccattat nncggncaaa nantaanaaa tggncaggna 600
acctaggngg aatccactta ncccggcntc cataccantg ggcngnngca aaaaaaaata 660
accggcnggc cttnaaccaa ttcnccctgg ngccntctnn ggatccaccg gccaac 716

<210> 57
<211> 602
<212> DNA
<213> Homo Sapiens

<400> 57
cctacgttta cctgtccatg tcttactact ttgaccgcga tgatgtggct ttgaagaact 60
ttgccaaata ctttcttcac caatctcatg aggagaggga acatgctgag aaactgatga 120
agctgcagaa ccaacgaggt ggccgaatct tccttcagga tatcaagaaa ccagactgtg 180
atgactggga gagcgggctg aatgcaatgg agtgtgcatt acatttggaa aaaaatgtga 240
atcagtcact actggaactg cacaaactgg ccactgacaa aaatgacccc catttgtgtg 300
acttcattga gacacattac ctgaatgagc aggtgaaagc catcaaagaa ttgggtgacc 360
acgtgaccaa cttgcgcaag atgggagcgc ccgaatctgg cttggcggaa tatctctttg 420
acaagcacac cctgggagac agtgataatg aaagctaagc ctcggctaat ttcccatacc 480
gtggggtgac ttccttggcc caaggcagtg catgcatgtt ggggttcctt acctttctat 540
aattggacca aaacatccct taagtctttg attgnccatt cttnaataaa aaatttggac 600
cc 602

<210> 58
<211> 612
<212> DNA
<213> Homo Sapiens

<400> 58
ggctaacaat ctccagaagg ttcattcagg cccatgcaaa tcagtgccgg agcctagaga 60
cagcacagcc tagagctaga ggtcaggcag ggctgagctg agtcacccac tattcagacc 120
tccctcttag agcctcagct actggatggt ggtcattaag ttatcattta aactacagac 180
gcaggctggg tacggtgact caaccctata gccccagcac tttgggaggc caagatggga 240
ggatcacttg aggtcgggag ttcaacacca gcctggccaa catgatgaaa ccccgtctct 300
actaaaaata caaaaactag ctgggtgtgg ggggngcaca tctttaatcc cagcttctca 360
ngangctgan gcaggaggat cacttaaacc cannaagtgg angctgcang gagcccanat 420
cgcacacttn actccacctg ggtgacagaa tgagactcat nttcnaanga aaccancnnc 480
cnntnnctcn ntgccnnnng tanctnttac cnatccttnc caaggacccc accttaccat 540
acttgntact aggnggcncc tgaatttccn aaancnntct taagggggcc tcaagtttan 600
nggccnttnc tt 612

<210> 59
<211> 640
<212> DNA
<213> Homo Sapiens

<400> 59

gtgatctcgg ctcactgcaa cctctgcctc ctgggttcaa gcgattcttg tgcctcaacc 60
tcccgagtag ctggcattac aggagcccgc ccaccatgcc tagctaattt ttgtattttt 120
agtagagaca gggtttcacc atgttggcca ggttggtctg gaactcctga ccttgtgatc 180
tgcctgcctt ggcctcccaa agtgctggaa ttacaggtgt gagtcaccgc gcccaagtat 240
aggccacttt taagaattac tcanagttag cttataagag gcgaatcagt ggagtcctcc 300
agtttggttc acacataatt attaggttga accatataaa gttactgttt ttggtcctgt 360
gaatattaat atttatatat gggtccaatc tgatatgttc canaaaatac acacttaant 420
aaagnttgga aaaccaaatc atanacttac atactgnaag gcggggtatt tgaaactggg 480
atggaaaatc aatttaatga gntatganct gcnttaaaaa aatggganaa natcanantt 540
ggtggnanna ttgnaaaaaa ccaaattgct ggggaagatt ggcatttnan tnttntnncn 600
cccngngggg ggggnngggg gnacnaaang nnanaaagaa 640

<210> 60
<211> 469
<212> DNA
<213> Homo Sapiens

<400> 60
cagttccacc cgggcaggca gtcgggggat gaggggccgt ctagcgtccg cacgcgttca 60
ctcccaagga aggtgtgtgg gcacggtgag gagtgggaag aaacagaata ggaaagtggc 120
ctgacacggg gattctaagc aggtcaaaag ttatgttgcc ttgtaggatg ggaagagaaa 180
aataaaaatt tgatttgttg tttaaagtga tggggttctg ggatattttt tcctttttaa 240
ttttaatatc tttggattaa nttttttttc tctttttttc aacggagtct cactctgtca 300
ccctgggtgg agtgcanngg nacnatctng gntnantgna acctncactt tctgggntca 360
agngantctt ctggctnanc ctccnaanan tnggaataca ggccctgcnc cangcctggn 420
taattttggn ttttaanaaa ncggaattcc ncnnnctnnn ngctnnaga 469

<210> 61
<211> 667
<212> DNA
<213> Homo Sapiens

<400> 61
ctttactaaa aatacaaaaa ttagccaggc atggtggcag gtgcctgtaa tcccagctat 60
tcgggaggct gaggcaggag aatcacttga acccaggaag tggaggttgc agtgagccga 120
gatcgtacca ctgcactcca cccagggtga cagagtgaga ctccgttgaa aaaaagagaa 180
aaaaaaatta atacaaagat attaaaatta aaaaggaaaa atatccccag aaccccatca 240
cttaaacaac aaatcaaatt tttatttttc tcttcccatc cnacaaggca acataactct 300
tacctgctta naatccccgt gtcangccac tttcctattc tgtttcttnc cactcctcan 360
cgngcccaca cacctttctt gggagtgaac gcgtgcggac ctanacggcc ctcatccccg 420
actggctgcc cgggtggaac tggggaattc cacttaacgc cggcgntcca ttaccaantg 480
atcttggggc aaaaataata ataaccggcn ggcctgtnaa gcccaantnt nnaaanttct 540
tnnacttggn ggcgntnagc agcntttnag ggccaatnnc ctatnngggn ngnntanaat 600
nntggccgng tttaaannnn ngangggaaa ccnggnntnc cantaannct tgnnaaatcc 660
cttttcc 667

<210> 62
<211> 161
<212> DNA
<213> Homo Sapiens

<400> 62
cagttccacc cgggcaggca gtcgggggat gaggggccgt ctagcgtccg cacgcgttca 60
ctcccaagga aggtgtgtgg gcacggtgag gagtgggaag aaacanaata ngaaagtggc 120
ctgacacggg gattctaagc angtcanann tatgnngctn g 161

<210> 63
<211> 443
<212> DNA
<213> Homo Sapiens

<400> 63
ctttactaaa aatacaaaaa ttagccaggc atggtggcag gtgcctgtaa tcccagctat 60

tcgggaggct gaggcaggag aatcacttga acccaggaag tggaggttgc agtgagccga 120
gatcgtacca ctgcactcca cccagggtga cagagtgaga ctccgttgaa aaaaagagaa 180
aaaaaaatta atacaaagat attaaaatta aaaaggaaaa atatccccag aaccccatca 240
cttaaacaac aaatcaaatt tttatttttc tcttcccatc ctacaaggca acataactct 300
gacctgctta gaatccccgt gtcaggccac tttcctattc tgtttcttcc cactcctcac 360
cgtgcccaca caccttcttg ggagtgaacg cgtgcggacg ctagacggnc cctcatcccc 420
cgactgcctg cccgggtgga act 443

<210> 64
<211> 263
<212> DNA
<213> Homo Sapiens

<400> 64
tgcgtcaga ttaaaacact gaactgacaa ttaacagccc aatatctaca atcaaccaac 60
aagtcattat taccctcact gtcaacccaa cacaggcatg ctcataagga aaggttaaaa 120
aaagtaaaag gaactcggca aatcttaccc cgcctgttta ccaaaaacat cacctctagc 180
atcaccagta ttagaggcac cgcctgccca gtgacacatg tttaacggcc gcggtaccct 240
aaccgtgcaa aggtagcata atc 263

<210> 65
<211> 262
<212> DNA
<213> Homo Sapiens

<400> 65
gattatgcta cctttgcacg gttagggtac cgcggccgtt aaacatgtgt cactgggcag 60
gcggtgcctc taatactggt gatgctagag gtgatgtttt tggtaaacag gcggggtaag 120
atttgccgag ttccttttac tttttttaac ctttccttat gagcatgcct gtgttgggtt 180
gacagtgagg gtaataatga cttgttggtt gattgtagat attgggctgt taattgtcag 240
ttcantgttt taatctgacg ca 262

<210> 66
<211> 431
<212> DNA
<213> Homo Sapiens

<400> 66
gggggnnggg ttttttttaa aaaaanantg nacatttatt tattactgnc cctatttatt 60
aaanngactt tttntnaacc aagggctttt actttttntt cttgccttta ngggcttcag 120
ggggttttcc cttaantaca accaantntt tttttnaanc naaaantttn nccacctncn 180
nancaacctc nttnttgnct gccttttgtg ctttnaantn tcggacagtt tgnaagtcct 240
caaanacctn naggnngaaa taanatttnn cccancnanc ccattnntgg gtatacancn 300
gaaggaatat aaatnactnt tttanaaaaa cacnncccat ntttnttnct nnnnnntntt 360
tanaacancc ccnanatnaa atnaaccnaa tnnccntnnn ngnggattnc nccnnnctnn 420
cggctcaaaa a 431

<210> 67
<211> 396
<212> DNA
<213> Homo Sapiens

<400> 67
cactgatggg catttgggtt gatttcatgt cgtggctgtt gtgaatagtg ctgcagtgaa 60
catacatgtg catgtgtctt tatgatagag tgatttataa tccttcaggt gtatacccag 120
taatgggatt gctgggtcaa atgttatttc tgcctctagg tctttgagga cttgcaaact 180
gtccgagaac tgaaagcaca aaaggcagac aagaacgagg ttgctgcgga ggtggcgaaa 240
ctcttggatc taaagaaaca gttggctgta gctgagggga acccccctga agcccctaaa 300
ggcaagaaga aaaagtaaaa gaccttggct catagaaagt cactttaata gatagggaca 360
gtaataaata aatgtacaat ctctatatta aaaaaa 396

<210> 68
<211> 426

<212>  DNA
<213> Homo Sapiens

<400>  68
cctttctatt agctcttagt aagattacac atgcaagcat ccccattcca gtgagttcac      60
cctctaaatc accacgatca aaagggacaa gcatcaagca cgcagcaatg cagctcaaaa     120
cgcttagcct agccacaccc ccacgggaaa cagcagtgat taacctttag caataaacga     180
aagtttaact aagctatact aaccccaggg ttggtcaatt tcgtgccagc caccgcggtc     240
acacgattaa cccaagtcaa tagaaaccgg cgtaaagagt gttttagatc accccctccc     300
caataaagct aaaactcacc tgagttgtaa aaaactccan ttgacacaaa atagactacn     360
aaagtggctt taacatatct taacacacaa tagctaanac ccaaactggg attagcggaa     420
tccccg                                                                426

<210>  69
<211>  517
<212>  DNA
<213> Homo Sapiens

<400>  69
cggattccgc taatcccagt ttgggtctta gctattgtgt gttcagatat gttaaagcca      60
ctttcgtagt ctattttgtg tcaactggag ttttttacaa ctcaggtgag ttttagcttt     120
attggggagg gggtgatcta aaacactctt tacgccggtt tctattgact tgggttaatc     180
gtgtgaccgc ggtggctggc acgaaattga ccaaccctgg ggttagtata gcttanttaa     240
actttcgttt attgctaaag gggtaatcac tgctgtttcc cntgggggtg tggctangct     300
aaacgttttg agctgcattg ctgcgngctt gatgcttgtc cctttttatc atggngattt     360
ataaggggaa ctccctgnaa tggggatgct ccntgtgtna tcttactann anctntanaa     420
aggggggntt nnnctnancg ccggnngtcc ataacaanag gnnnggnggn naaaaaataa     480
taancgncng gncatnttag ccnaatattc nganatc                              517

<210>  70
<211>  537
<212>  DNA
<213> Homo Sapiens

<400>  70
caaaaagata attaaccttt attattcatt aaaaatgagc tttctaaaat attagtaaat      60
ttcattttaa gctctgtctt gaagtgctga taccactgaa gtaacatttt tcttctttca     120
attttttctt gtaaaattat agttttctct ttttctaaaa cagcagggag ttccttccag     180
ttcttgataa agataaaggg agcacccatg gacttgagta actgcagagg agcacccgtg     240
gtgcacagat gtattcccac agttgccagc tgtcatcacg tcttccacca caggaatgga     300
gccatangag caagcctcat anattcnata gcattctgtg nttactccga ccaggcacaa     360
tgtgagatca ctctgaanca aggcatcttg gtaattctta aactttcatt tgtttctgag     420
gctgccanng ntntnttnct gaacccacaa acttatnnnt ccatcttttt taaaangttc     480
attatgccng ctggatnaan tttnnaaatn nntcctaana annacntaat atggctt        537

<210>  71
<211>  759
<212>  DNA
<213> Homo Sapiens

<400>  71
gcccccgcgc gggagagacg cggccgagaa cagtccactt tggaaagtga agaatggaat      60
ccttgggaag gagatgaaaa aaatgagcaa caacacagat ttaaaactag ccttcaaata     120
ttagataaat ccacgaaagg aaaaacagat ctcagtgtac aaatctgggg caaagctgcc     180
attggcttgt atctctggga gcatattttt gaaggcttac ttgatcccag cgatgtgact     240
gctcaatgga gagaaggaaa gtcaatcgta ggaagaacac agtacagctt catcactggt     300
ccagctgtaa taccagggta cttctccgtt gatgtgaata atgtggtact cattttaaat     360
ggaagagaaa aagcaaagat cttttatgcc acccantggt tactttatgc caaaatttaa     420
tgcaaattca aaaactncac atcttgcttg ttggtttgct cggaaatgaa cattgtgata     480
atgagtggat aaacccattc ttaaaagaaa tggaggcttc gtggacctgn ttttcataat     540
atatacaccc ctggataatg actggatgtt tttantggcc ttaggagtag cacatccgga     600
atttcttggg ngaagcaant ggcaatgttc tnataaaggc cttttattaa tttttagaac     660
nattttaaaa ttntccnacg gcctataact tttaaaaaaa gggaccaaac ttttgggtta     720
canaaaactg cnctnggaaa aanaagttaa aattccaaa                            759

<210> 72
<211> 595
<212> DNA
<213> Homo Sapiens

<400> 72
tctcactctg ttggccaggc tggagtgcag tggtgctttg cctcctgggt tcaagggatt 180
ctcctgcctc ancctcctga gtagctggga ttacaggcat gcgccaccac gtcgggctaa 240
tttttgcatt tttagtggag acggggtttc atcatgttgg ccaggctggt ctcgaactca 300
cgacgtcnag tgatccacct gcctcggcct cccaaagtgt tgggattaca aggcggtgag 360
ccaccatgcc cacccgcact gnnttaaatg ctttacatat attatctcat ttaatcctcn 420
naaaccttac aatatanana ctacnattat ttccctttat atttatngnn ctcttaggct 480
canaaaaggg aactaacttc ttggtncatg gnnggggnng gaatnaaanc cangnnancc 540
gctcccnaaa ntncnttcnn tgccnnnctn actgggccnt ctttnaaggg gggcc 595

<210> 73
<211> 734
<212> DNA
<213> Homo Sapiens

<400> 73
ttcatgggga ataattgcaa tccccgatcc ccatcacgaa tggggttcaa cgggtacccg 60
cgcctgccgg cgtagggtag gcacacgctg agccagtcag tgtagcgcgc gtgcagcccc 120
ggacatctaa gggcatcaca gacctgttat tgctcaatct cgggtggctg aacgccactt 180
gtccctctaa gaagttgggg gacgccgacc gctcgggggt cgcgtaacta gttagcatgc 240
cagagtctcg ttcgttatcg gaattaacca gacaaatcgc tccaccaact aagaacggcc 300
atgcaccacc acccacggaa tcgagaaaga gctatcaatc tgtcaatcct gtccgtgtcc 360
ggcccgggtg aggcagtgag ctgagattgc gccactgcac tccaacctgg gcgacagaac 420
gagaccccat ctcaaaaaaa aagggggggg tggacagggg gcaagtggag tctggctgcc 480
aaaactactt gttgatggtg gggaaaaaaa aatgggtgtc tcctcccttg tcactgggaa 540
ggntttggtt ctctttcatc ttcatcctct atagggcctt tattcttccc caatctgaaa 600
tatttcgngn gttttacttn cccacatcct tcaaaaatat ttgaggttga taaaantaac 660
ttccgggttg gacccnggnt aatctttggg tntttggntt accaaaaaaa gggtcncatt 720
aacccntttt gatt 734

<210> 74
<211> 592
<212> DNA
<213> Homo Sapiens

<400> 74
ctcagccctg agaaaggaga catgtaacaa gagtaacatg tgtgaaagca gcaaagaggc 60
actggcagaa aacaacctga accttccaaa gatggctgaa aaaagatgga tgcttccaat 120
ctggattcaa tgaggagact tgcctggtga aaatcatcac tggtcttttg gagtttgagg 180
tatacctaga gtacctccag aacagatttg agagtagtga ggaacaagcc agagctgtgc 240
agatgaagta caaaagtcct gatccagttc ctgcagaaaa aggcaaagaa tctagatgca 300
ataaccaccc ctgacccaac cacaaatgcc agcctgctga cgaanctnca ggccagaacc 360
agtggctgca ggacatgaca actcatctca ttctgcgcac tttaaggagt tcctgcantc 420
cacctgaggg ctttcggcaa atgtacatgg gccctcaaat ggtggtggta atggcattct 480
tntttggcan aaacctgtcc cttggcacaa acttatntgg tntttttgga aactaaaatn 540
taacgttnga cctattttaa tattttaaat tattaaantt aaaatgngaa ct 592

<210> 75
<211>
<212> DNA
<213> Homo Sapiens

<400> 75
gccagaggct gttcaccttg gagacctgct gcggatatgg gtacggcccg gcgcgagatt 60
tacaccctct cccccggatt ttcaggggcc agcgagagct caccggacgc cgccggaacc 120
gcgacgcttt ccaagacacg ggcccctctc tcggggcgaa cccattccag ggcgccctgc 180
ccttcacaaa gaaaagagaa ctctccccgg ggctcccgcc ggcttctccg ggatcggtcg 240

cgttaccgca ctggacgcct cgcggcgccc atctccgcca ctccggattc ggggatctga 300
acccgactcc ctttcgatcg gccgagggca acggaggcca tcgcccgtcc cttcggaacg 360
gcgctcgcca tctctnanga ccgactgacc catgttcaac tgctgttcac atggaaccct 420
tctncacttc gccttcaaag ttctcgtttg aatatttgct actaccacca anatctgacc 480
ttgcggnggt tcncccggcc cgcgcctagc tttaaggtna ncgaacggcc ttctatctnn 540
nggngtacgt ccnggggttc ggggcgggag cgcggaatca ntnacgccgg cgcnccttac 600
aantggtntg gggnnaaana aaataancgg cnggcctgnn agccaattca 650

<210> 76
<211> 577
<212> DNA
<213> Homo Sapiens

<400> 76
cgctccccgc ccccggagcc ccgcggacgc tacgccgcga cgagtaggag ggccgctgcg 60
gtgagccttg aagcctaggg cgcgggcccg ggtggagccg ccgcaggtgc agatcttggt 120
ggtagtagca aatattcaaa cgagaacttt gaaggccgaa gtggagaagg gttccatgtg 180
aacagcagtt gaacatgggt cagtcggtcc tgagagatgg gcgagcgccg ttccgaaggg 240
acgggcgatg gcctccgttg ccctcggccc gatcgaaagg gagtcgggtt cagatccccg 300
aatccggagt ggcgganatg ggcgcccgcg aggcgtccag tgcggtaacg cgaccgatcc 360
cgganaaccc ggcgggagcc ccggggagaa gttctctttt ctttgtgaag ggcanggcgc 420
cctggaatgg gttcgcccga gagangggcc cgtgtcttgg aagcgtnnng gntncggcgg 480
ggtccggnga gctnttnntg gnccctgaaa atccggggaa anggggnaaa tttnngccgg 540
gccnacccnt ntccnannag gtttccaggg gaananc 577

<210> 77
<211> 1154
<212> DNA
<213> Homo Sapiens

<400> 77
annaaagttg ganccnttnn tncgactact atagggcgaa ttgggccctc tagatgcatg 60
ctcgagcggc cgccagtgtg atggatatct gcagaattcg cccttgggat tggtggcgac 120
gactcctgga gcccgtcagt atcggcggaa ttccggcttg tgcgtcaggg agcagaccag 180
gcaagaaccc caggtggtga tggctccaga ggttctgaga aggaacaggc acagggcaca 240
ctgggacggc acaggaagtg aggctggggg tggccggctg ggctgcaggg ctgcggtggg 300
aagcccagaa caggggcgca ccttgctcag caggaaaagc ccatggggag ggggtgagca 360
gggagccagg gctctctgaa gtgtccaggt gcagggcaag gtgcccacag accataaggc 420
acttaaatgg ccacaaagtc atctcagaag agtaatatga caagtgcctg gtctctaaaa 480
agcacaaggg tgacctctgc atagaaacag tcccccaccc catcaggctg ccagggcagg 540
ctcacctggc gtcaaaacga taggatcagg ctcccctcgg ttcccatagt agcaaatgac 600
gtctcccttt gctgtgctgc aggccaaggg gagagaatgt cagactacag ccatagggcg 660
tcctccccga cactgcccgg tgatctcaca gccctgtttc tggaggctag cgatgtgcgc 720
agtaacccgc tgcggccagg tcangacttg caggacccan ccctnangtg cctgcagccg 780
ggtcatggag gccccaactn ttgtccacat tacnggtatg ttcccaactn acttnggggc 840
caccagccca gaggcncagg atctaaaagg cctcttgcat cccaatccca tggnatggac 900
ccacaaagnt tgccttggga canttnaggg ctgancccct tnccncatta ccaacttttc 960
attaggccct taccaaagtt anttntaagg tttttggana gnggcccntt ngcccangng 1020
attaggttca aaaaaggcca aaaccatttt nttacttnaa ngtaagggcn nttaaaaatt 1080
ngggncttaa ntggcttnng nnntcctttt cccttntnng gaanangggg gggncttttt 1140
gcccnnttta aacc 1154

<210> 78
<211> 861
<212> DNA
<213> Homo Sapiens

<400> 78
tgtgtgatgt ggtattgagg attggaatat tttcagctgg ggagcatttt gaactatttt 60
ctttttaaaa aatgatttat ttattttgta gagatggggt cttgctatgt tgcccagtct 120
ggtttcaaac tcctggcctc aagcagtcct ccctccttag tctccagatt agctgggact 180
ataggcatga gtcactatgc ccagctctac tgtcccttaa ctattttaaa gtgtacactt 240
aacacttgag agtaggaaaa tgtggtcttt ggcgctttct ttggaagcct cgatcaccct 300

ggggccacac tggggccacc tgtccagaaa ccctcataga tgagctgtgg taaggggcct 360
gatggagagg ttggtgatgt ggggatgggg ctcagccctg cagttgtccc catgggcagc 420
ctctgtgtgt ccatgccatg gggcattcgg catggcagga gggcatttta gatcctgtgc 480
ctcctgggct ggtgggcccc gaggtgaact gggaacatac ccgtgatgct ggacaaganc 540
tggggcctnc atgacccgct gccagcacct gangggctgg gtcctgcaag ctcctgactg 600
gccgaaccgg ctactgngca catcggtagc ctccanaaca aggctgtgan ncacccggca 660
tgtcngggag gacnccctnt ggtggaanct nacattnttt ccctggcctg aagncancaa 720
agggagacgc atttgctcta tgggaaccnn gggggacctg nncctatnnt ttacccnggn 780
gaacctcctt gnaaccnnnn gggtggggnc gttttntcca agnnaccttg ctttttaaaa 840
ccngnctttg naaanacntt t 861

<210> 79
<211> 442
<212> DNA
<213> Homo Sapiens

<400> 79
cgcgggattc cggtgagaag tatccgcgac gagctatccg ggaaagggcc gaatgcgatc 60
aaacctaatc cgcgagactt gctaaaattc tccaagtccc ggctgcttat gtacctatcg 120
agccagattc accaccgtca ttatcaactc attttatgaa aaaatgtaat ctaaagtata 180
tccttgttga aaaaaaacaa attaatgtat tctttatgac cagtaggagt tggacatagc 240
aaaacccaaa aaggagttgg gcgcagcaaa accttgcttc ctatcccatg attttgatga 300
tggtgtaagt gttcttcctt catttaacac aggaacgatc aaaatttaaa tcttttcatg 360
aaacattatt gaactatgat acatttacag tggaacataa tgacctagtg ctcttcagac 420
ttcactggta aaatactgag gt 442

<210> 80
<211> 529
<212> DNA
<213> Homo Sapiens

<400> 80
attatttttg acaccagacc aactggtaat ggtagcgacc ggcgctcagc tggttcatat 60
ttctcttttc catcatttag catcaagttc acctcagtat tttaccagtg aagtctgaag 120
agcactaggt cattatgttc cactgtaaat gtatcatagt tcaataatgt ttcatgaaaa 180
gatttaaatt ttgatcgttc ctgtgttaaa tgaaggaaga acacttacac catcatcaaa 240
atcatgggat aggaagcaag gttttgctgc gcccaactcc tttttgggtt ttgctatgtc 300
caactcctac tggtcataaa gaatacatta atttgttttt tttcaacaag gatatacttt 360
agattacatt ttttcataaa atgagttgat aatgacggtg gtgaatctgg ctcgataggt 420
acataagcag ccgggacttg gagaatttta gcaagtctcg cggattaggt ttgatcgcat 480
tcggcccttt cccggatagc tcgtcgcgga tacttctcac cggaatccc 529

<210> 81
<211> 701
<212> DNA
<213> Homo Sapiens

<400> 81
cggaatccgg tggacgccgt gccgttactc gtagtcaggc ggcggcgcag gcggcggcgg 60
cggcatagcg cacagcgcgc cttagcagca gcagcagcag cagcagcatc ggaggtaccc 120
ccgccgtcgc agcccccgcg ctggtgcagc caccctcgct ccctctgctc ttcctccctt 180
cgctcgcacc atggctgatc agctgaccga agaacagatt gctgaattca aggaagcctt 240
ctccctattt gataaagatg gcgatggcac catcacaaca aaggaacttg gaactgtcat 300
gaggtcactg ggtcagaacc caacagaagc tgaattgcag gatatgatca atgaagtgga 360
tgctgatggt aatggcacca ttgacttccc cgaatttttg actatgatgg ctagaaaaat 420
gaaagataca gatagtgaag aagaaatccg tgaggcattc cgagtctttg acaaggatgg 480
caatgggtat atcagtgcag cagaactacg tcacgtcatg acaacttagg agaaaaacta 540
acagatgaag aagtagatga aatgatcaga gaagcagata ttgatggaga cggacaagtc 600
aactatgaag aattcgtaca gatgatgact ggcaaatgaa gactacttta actccttttc 660
ccctntagaa gaatcaaatt gaatctttac ttacctcttg c 701

<210> 82
<211> 375
<212> DNA
<213> Homo Sapiens

<400> 82
gttcaaacag caaacgccca cagatggccc agaggtggtg gtagtcaggg tgtgtgggtg 60
tttttagggt tctttagtgt tgtttctttc acccaggggt ggtggtccca gccagtttgg 120
tgctgacggt gagaggaaat tagaatctgt ttgcaaattg tccaacccac cccctcaaca 180
tgaggggctt ccattttctg tgttttgtaa gggaactgtt tccttcatgc cgccatgttc 240
ctgatattag ttctgatttc tttttaacaa atgttatcat gattaagaaa atttccagca 300
ctttaatggc caattaactg agaatgtaag aaaattgatg ctgtacaagg caaataaagc 360
tgtttattaa ccttg 375

<210> 83
<211> 882
<212> DNA
<213> Homo Sapiens

<400> 83
gcacactggc ggccgttact agtggatccg agctcggtac caagcttgat gcatagcttg 60
agtattctat agtgtcacct aaatagcttg gcgtaatcat ggtcatagct gtttcctgtg 120
tgaaattgtt atccgctcac aattccacac aacatacgag ccggaagcat aaagtgtaaa 180
gcctggggtg cctaatgagt gagctaactc acattaattg cgttgcgctc actgcccgct 240
ttccagtcgg gaaacctgtc gtgccagctg cattaatgaa tcggccaacg cgcggggaga 300
ggcggtttgc gtattgggcg ctcttccgct tcctcgctca ctgactcgct gcgctcggtc 360
gttcggctgc ggcgagcggt atcaagctca ctcaaaggcg gtaatacggt tatccacaga 420
atcaggggat aacgcaggaa agaacatgtg agcaaaaggc cagcaaaagg ccaggaaccg 480
taaaaaggcc gcgttgctgg cgtttttcat angctccgcc ccctgacagc attacaaaaa 540
tcgacgcttc aagtcagang tggcgaaccc gacaggacta taaagatcca ngcgtttccc 600
ctggaacttc ctcggcgctn tctgttcgac cctgncgtta ccggaacctg tccgcnttnt 660
ccttcggaag cgngggcttt ntatacttac gctgaagtat ctnattcggg gagncgtcgn 720
tcaactggct gggngcacaa cccccgttag ccgacgtgng cttaccggaa tntngntggt 780
caaccggnan accantatcg cntgnnnanc ntgnacagat accancaggt ttagggggtt 840
caaatttaag gggccatccg tantaaaaac aatggtttcc ng 882

<210> 84
<211> 858
<212> DNA
<213> Homo Sapiens

<400> 84
cagatatcca tcacactggc ggccgctcga gcatgcatct agagggccca attcgcccta 60
tagtgagtcg tattacaatt cactggccgt cgttttacaa cgtcgtgact gggaaaaccc 120
tggcgttacc caacttaatc gccttgcagc acatccccct ttcgccagct ggcgtaatag 180
cgaagaggcc cgcaccgatc gcccttccca acagttgcgc agcctgaatg gcgaatggac 240
gcgccctgta gcggcgcatt aagcgcggcg ggtgtggtgg ttacgcgcag cgtgaccgct 300
acacttgcca gcgccctagc gcccgctcct ttcgctttct tcccttcctt tctcgccacg 360
ttcgccggct ttccccgtca agctctaaat cgggggctcc ctttagggtt ccgatttaat 420
gctttacggc acctcgaccc caaaaaactt gattaagggt gatggttacg tagtgggcca 480
tcgccctgat agacgggttt tcgccctttg acgttggagt ccacgttctt taatagtgga 540
ctcttggtnc aactgggaca acacttaanc ctatctcggc tattcttttg attataaggg 600
atttggcgat tcgggctatt ggttaaaaaa gactgattaa caaaatttaa cgcgaattta 660
caaattcagg cccaaggctg taaggaancg acactaaaag ccatccgaaa acgggttanc 720
ccgataaagg aactatgggt tttgggaaag gaaaccaacc caaaaaagcg nacttnaagg 780
gctactgnaa agtaaanggn gttatgaaga acaacgatgc annggccctt gaagtggaac 840
ccgaaaaatg aggttttg 858

<210> 85
<211> 836
<212> DNA
<213> Homo Sapiens

<400> 85
gtggttttgc tttgttctta ctaggttttg gtgccacctt ccctgcctgc gcttgtgccc 60
cctctcctcc ttggcactgg cggcctcctt gcctcccttc cacccgtgct gccatcccgt 120
gcctgtcgtg ttggttcttc acacgtgctc tgttctcggg gttgttccat tcatgccttc 180

ttggagggtg agggtggctt gggaaccgac ccagtgatca tgcctacttt cttctttgta 240
tctccctcct tcccagccca cccgggcagc agactctgat ggaaggaagg tgccgtaggt 300
gggcttttag aaactaacgg gactggtttt caaagcagtt atcttgggaa actgtttatt 360
ccagcgatgt gacttttttc agaatatttc ttggaatcat attcanagtc tggggctgtg 420
tgttgagcag ccttaaggat gctagacact catttagtgc ccaaggagtc cagcgaatga 480
cgtctgnggc aacgaggctc agngcaagca aaaggaccat ttaaagtaaa tacttggatc 540
aatctgtgac tcttaaatgg ctnaaaagaa tttgnattca aaagggttga accctggcac 600
gttggcntgg gagctatanc ttgatccttg ganaaaaatt cattggtggg gaactgattg 660
gtngganaaa ntggctggta cttntggnat ccaggtntga cttacaggga aaaaaaaaaa 720
acgcgantga nggtcagatn nnncccatca nccattcacc atngggcnnn tanggtccnc 780
cngncaactn agcaantgna ttnatnggcc caaaactggg aacnggcnat ttcnng 836

<210> 86
<211> 856
<212> DNA
<213> Homo Sapiens

<400> 86
gcccctggta aaagtcagaa cctgggatga ccagaaagta acaggacaga tttctcccag 60
caaatcagtc tccacaacca aatgaatatt gttctccaag gagtcaagct atagactcac 120
aatgacaacg tggccatggc tcaaaacact ctctgaaatt acaaaattgc tttctgagcc 180
aatttaagag tcacatgatt gaatccaagc tattttactt taaatggtcc ttttgctttg 240
cacctgagac ctcgcttggc cacagacgtc attcgctgga ctccctgggc actaaatgag 300
tgtctagcat ccttaaggct gctcaacaca cagccccaga ctctgaatat gattccaaga 360
aatattctga aaaaagtcac atcgctggaa taaacagttt cccaagataa ctgctttgaa 420
aaccagtccc gttagtttct aaaagcccac ctacggcacc ttccttccat canagtctgc 480
tgcccgggtg ggctgggaag gagggagata caaagaagaa agtaggcatg atcactgggt 540
cggttcccaa gccccctcac ccttcaagaa ggnatgaatg gacaaccccg agaacagagc 600
cgtgtgaaga ccaccnacng cncggatggc acacggtgga aggaggcagg aggccncngt 660
gccanganga nagggcncaa cccagccgga agnggcccaa acctatagaa caagcaaacc 720
ccggattcng tgacgcggcn tacctaccat nggngggnna aanatatacc ggcggctgca 780
gccaattgaa atcataactg nggcgtcact gcttnaggcc attncctang gggataaatn 840
tgcgggttna cgggcg 856

<210> 87
<211> 828
<212> DNA
<213> Homo Sapiens

<400> 87
agaaatcttt taatgtttat tcaaaggaca aaataaagac tatgaaccaa tgagacacat 60
agtaaaaaag tacaatttta atatagtgaa tgtaatatat atgtaattac tcataacaaa 120
atggtcaaaa cctttaaaag atacacaata ggcatctaaa aagctcagca atgctaaata 180
tataatatat attatatata aatatataat atataaatac atacgttttt accaagaaat 240
gttttatttt tcttgcagta gctttgttaa ttgcacaaaa ttatgttttg tttttgccat 300
ttaaatatta tcacagaatc ctattctgaa agacaaatgt tcattaaaaa caaagcaaaa 360
atagaaattc acaaccatta attacctagg tttgtcattt aaaggtttaa agaaaaaaag 420
ggaggagctt tcctacaagc cttttccaag tgtcacattt tctctttaaa agggaaggat 480
ttncaaacaa aggtgaaata gcttaaacag aaatatttgt aaaaataaac tttangcatt 540
atcaaggata ttaagacaca ctgactaacc cggttcatta cccgnatctt ccccncccca 600
ccccagtggg tccaccagga ctagaacagn tttacnttan acagaaatgc ttcaaatccc 660
agggaaagaa ctggctaaaa nccgcaggnt tttnctgcct cccgtgccgt ngttttgaat 720
ctttaccagg tttcttggaa gggccaactg gagtgggagg actgccacgg gcccttttta 780
tatggatcnt gggccgcgtc cttcagtggt ggggaaaaaa aacggggc 828

<210> 88
<211> 424
<212> DNA
<213> Homo Sapiens

<400> 88
ataattatat ataaggtggc cacgctgggg caagttccct ccccactcac agctttggcc 60
cctttcacag agtagaacct gggttagagg attgcagaag acgagcggca gcggggaggg 120
cagggaagat gcctgtcggg tttttagcac agttcatttc actggatttt tgaagcattt 180

ctgtctgaat gtaaagcctg ttctagtcct ggtgggacac actggggttg ggggtggggg 240
aagatgcggn aatgaaaccg gntagnnagn gntgncttaa tatncttgat aatgctgnan 300
agnttattnt tacaaatatt tntgtntaag ctatttcacc tttnnttgga aatccttccc 360
ttttaaagan aanatgngac actttgtgaa naggcttgtn ngaaagntcn tccc 414

<210> 89
<211> 866
<212> DNA
<213> Homo Sapiens

<400> 89
aagaaatatg ggactatgtg aaaagaccaa atctacgtct gattggtgta cctgaaagtg 60
atgtggagaa tggaaccaag ttggaaaaca ctctgcagga tattatccag gagaacttcc 120
ccaatctagc aaggcaggcc aacgttcaga ttcaggaaat acagagaacg ccacaaagat 180
actcctcgag aagagcaatt ccaagacaca taattgtcag attcaccaaa gttgaaatga 240
aggaaaaaat gttaagggca gccagagaga aaggtcaggt taccctcaaa ggaaagccca 300
tcagactaac agcggatctc tcggcagaaa ccctacaagc cagaagagag tgggggccaa 360
tattcaacat tcttaaagaa aagaattttc aacccagaat ttcatatcca gccaaactaa 420
gcttcataag tgaaggagaa ataaaatact ttatagacaa gcaaatgctg agagattttg 480
tcaacaccag gcctgcccta aaagagctnc tgaaggaagc gctaaacatg gaaaggaaca 540
ccggtaccan cgntgcaaaa tcatgccaaa tgtaaagacc tcgagactag gaagaactgc 600
tcactaacga gcaaatccca gcttacatct tatgacgggt caattccccn tacatatact 660
ttaatntaat ggctaantct gcantaaaag acnngactgn agttggtaag agcagacctn 720
atgngttgnt cngaaccatt actgnnaacc cnnggtcaat aaggtgnaag attncngcct 780
ggaacaaaag ngggtggatc tacttgtaac cgctttaccn caaacaaaaa caaaggcttc 840
tttgnanggt catcccaagn ntcntn 866

<210> 90
<211> 829
<212> DNA
<213> Homo Sapiens

<400> 90
gttctgtaga tgtctattag gtccgcttgg tgcagagctg agttcaattc ctgggtatcc 60
ttgttgactt tctgtctcgt tgatctgtct aatgttgaca gtggggtgtt aaagtctccc 120
attattaatg tgtgggagtc taagtctctt tgtaggtcac tcaggacttg ctttatgaat 180
ctgggtgctc ctgtattggg tgcataaata tttaggataa gttagctcct cttgttgaat 240
tgatcccttt accattatgt aatggccttc tttgtctctt ttgatctttg ttggtttaaa 300
gtctgtttta tcagagacta ggattgcaac ccctgccttt ttttgttttc cattggcttg 360
gtagatcttt ctccatcctt ttattttgag cctatgtgtg tctctgcacg tgagatgggt 420
ttcctgaata cagcacactg atgggtcttg actctttatc caacttgcca gtctgngtct 480
tttaattgca gaatttagtc catttatatt taaaggtaat antggtatgn gtgaattgat 540
ctgncattat gatgtagctg gngatttgct cgtagttgat gcagttcttc tagctcatgg 600
cttacatttg gcatgatttg cacggtggac cggtggtcct ttcatgttaa ccttcttcag 660
agcntttagg caggctggng tgacaaaact taacatttgc tggcataaga ttattctctt 720
acttataact tattggtgga atnaatctgg tgaaatnttt ttaaaantga aatggccccn 780
ttttnggttg aggttttcca aancnttaac nnngnttctt aggaccccg 829

<210> 91
<211> 840
<212> DNA
<213> Homo Sapiens

<400> 91
ctttaaagta gttttttcca attcagtgaa gaaagtcatt ggtagcttga tggggatggc 60
attgaatcta taaattacct tgggcagtat ggccattttc atgatattga ttcttcctac 120
ccatgagcat ggaatgttct tccatttgtt tgtatcctct tttatttcct tgagcagtgg 180
tttgtagttc tccttgaaga ggtccttcac atcccttgta agttggattc ctaggtattt 240
tattctcttt gaagcaattg tgaatgggag ttcactcatg atttggctct ctgtttgtct 300
gtcgttggtg tataagaatg cttgtgattt ttgtacattg attttgtatc ctgagacttt 360
gctgaagttg cttatcagct tatggagatt ttgggctgag acaatggggt tttctagata 420
tacaatcatg tcgtctgcaa acagggacaa tttgacttcc tcttttccta attgaatacc 480
ctttatttct tctcctgcct aattgccctg gccagaactt tcaacactat gttgaatang 540

antggtgana aaagacatnc ctgcttgggc cagtttcaaa ggaatgcttc cagttttgnc 600
attcatatga tatggctggg ggttggcaca aaactcttat atttgaaaac cgtccacata 660
ccaattatga aagtttaact gaaggtggtg aatttgcaaa gctttttgca caatgaaaaa 720
catgggtttg cttgcctnta atccgataca tatgatggaa ttgacnactg ctccaggata 780
nccntgactg gggnaacntt aaggngtgat cgtgcnnttt ttgngattgc naagcccagg 840

<210> 92
<211> 838
<212> DNA
<213> Homo Sapiens

<400> 92
gagaaaatct agaagaaatg gataaattcc tcgacacata cactctccca acactaaacc 60
aggaagaagt tgaatctctg aatagaccaa taacaggatc tgaaattgtg gcaacaatca 120
atagcttact aaccaaaaag agtccaggac cagatggatt cacagccgaa ttctaccaga 180
ggtataagga ggagctggta ccactccttc tgaaactatt ccaatcaata gaaaaagaga 240
gaatccttcc taactcattt tatggggcca gcatcattct gataacaaag ccgggcagag 300
acacaaccaa aaaagagaat tttagaccaa tatccttgat gaacattgat gcaaaaatcc 360
tcaataaaat actggcaaac cgaatccagc agcacatcaa aaagcttatc caccatgatc 420
aagtgggctt catccctggg atgcaagact ggtcaatata tgcaaatcaa taaatgtaat 480
ccagcatata aacagagccc aagacaaaaa ccacatgatt atctcaatag atgcagaaaa 540
agcctttgac aaaattcaac acccttcatg ctaaaaactc tcaataatta ngtatgatgg 600
acgtatttca aataataaga gctattgnga caaccccagc cattctactg atggcaaact 660
gggagcattc cttgaaactg gacagacngg tgcttntaca ctctatcact agggtgaagt 720
tggcaggcat agcggnanga tanggntcat nggaaaagga gcaatnctgt tgnacaatgt 780
gtttaaaacc ctggtaccaa ttctacgtac atngaactng tcaatannca atcagntt 838

<210> 93
<211> 850
<212> DNA
<213> Homo Sapiens

<400> 93
tgtaatccca gcacgttgga aggttgaggc gggtagatca tgaggtcagg aattcaagat 60
cagcctggcc gggatggtga aaccccatct ctactaaaaa tacaaaaatt agccaggtgt 120
agtggtgggc gcctgtggtc ccagctacta tggtggctga ggtgcgagag tcgcttgaac 180
ctgggagatg gaggttgcag tgagccaaga tcgtaccact gcactccagc ctgggcaaca 240
gaacaagact ccatttcaaa aaaagaaaat tcttatttgc catgagccga ggaatgcaca 300
ggtactaact agatggtgtg gacagctgac gcaaactggg catatacaat gggacacacc 360
tgtactagga tgaaaggcac agcctanagg gctggcaggt gttgggtaat gctcaagttt 420
cagagtgatg gcagaagagt aggttggtag gccctcatgg ctctgcttgg cagcacngag 480
ttccgcggaa ttccgccatc tgacggctcc angagtcgtc gcccaatcca agccgaattn 540
cacacactgg cggccgtact agtggatccg actcggacca acttgatgca taacttgagt 600
attctatatg ncacctaaat agcttggcgt aatcatggca tacttgtttc tgngngaaat 660
tgtatccgnt acaattcnca cacatacanc cgaagcataa gtgnaagcng gggngcctaa 720
tgagtgacta ctacttattg ggtggctact gccgtttcan cggaaactgc tgcnantctt 780
atnatcgcca ccncgggaag nggtgngntg gcntttcctc tgtattatct gctgcttggt 840
gggaacggta 850

<210> 94
<211> 483
<212> DNA
<213> Homo Sapiens

<400> 94
cggaactccg tgctgccaag cagagccatg agggcctacc aacctactct tctgccatca 60
ctctgaaact tgagcattac ccaacacctg ccagccctct aggctgtgcc tttcatccta 120
gtacaggtgt gtcccattgt atatgcccag tttgcgtcag ctgtccacac catctagtta 180
gtacctgtgc attcctcggc tcatggcaaa taagaatttt ctttttttga aatggagtct 240
tgttctgttg cccaggctgg agtgcagtgg tacgatcttg gctcactgca acctccatct 300
cccaggttca agcgactctc gcacctcagc caccatagta gctgggacca caggcgccca 360
ccactacacc tggctaattt ttgtattttt agtagagatg gggtttcacc atcccggcca 420

ggctgatctt gaattcctga cctcatgatc tacccgctca ccttccaacg tgctgggatt 480
aca 483

<210> 95
<211> 449
<212> DNA
<213> Homo Sapiens

<400> 95
ctgtttaatt aaaacaaagc atcgcgaagg cccgcggcgg gtgttgacgc gatgtgattt 60
ctgcccagtg ctctgaatgt caaagtgaag aaattcaatg aagcgcgggt aaacggcggg 120
agtaactatg actctcttaa ggtagccaaa tgcctcgtca tctaattagt gacgcgcatg 180
aatggatgaa cgagattccc actgtcccta cctactatcc agcgaaacca cagccaaggg 240
aacgggcttg gcggaatcag cggggaaaga agaccctgtt gagcttgact ctagtctggc 300
acggtgaaga gacatgagag gtgtagaata agtgggaggc ccccggcgcc cccccggtgt 360
ccccgcgagg ggcccgggcg gggtccgccg gccctgcagc cgccggtgaa ataccactac 420
tctgatcgtt ttttcactga cccggtgag 449

<210> 96
<211> 450
<212> DNA
<213> Homo Sapiens

<400> 96
ctcaccgggt cagtgaaaaa acgatcagag tagtggtatt tcaccggcgg cctgcagggc 60
cggcggaccc cgccccgggc ccctcgcggg gacaccgggg gggcgccggg ggcctcccac 120
ttattctaca cctctcatgt ctcttcaccg tgccagacta gagtcaagct caacagggtc 180
ttctttcccc gctgattccg ccaagcccgt tcccttggct gtggtttcgc tggatagtag 240
gtagggacag tgggaatctc gttcatccat tcatgcgcgt cactaattag atgacgaggc 300
atttggctac cttaagagag tcatagttac tcccgccgtt tacccgcgct tcattgaatt 360
tcttcacttt gacattcaga gcactgggca gaaatcacat cgcgtcaaca cccgccgcgg 420
gccttcgcga tgctttgttt taattaaaca 450

<210> 97
<211> 517
<212> DNA
<213> Homo Sapiens

<400> 97
cccatttaac ttttttaatg ggtctcaaaa ttctgtgaca aatttttggt caagttgttt 60
ccattaaaaa gtactgattt taaaaactaa taacttaaaa ctgccacacn caaaaaagaa 120
aaccaaagtg gtccacaaaa cattctcctt tccttctgaa gggtttacna tgcattggta 180
tcattaacca gtcttttact actaaactta aatggccaat tgaaacaaac agttctgaga 240
ccgttcttcc accactgatt aagantgggg tggcaggtat tagggataat attcatttan 300
ccttctgagc tttctgggca gacttggtga ccttgccagc tccagcagcc ttcttgtcca 360
ctgctttgat gacacccacc gcaactgtct gtctcatatc acgaacagca aagcgaccca 420
aaggtggata gtctgaaaag ctctcaacac acatgggctt gccaggaacc atatcaacaa 480
tggcagcatc ccagacttca agaatttang gccatnt 517

<210> 98
<211> 519
<212> DNA
<213> Homo Sapiens

<400> 98
agatggccct aaattcttga agtctggtga tgctgccatt gttgatatgg ttcctggcaa 60
gcccatgtgt gttgagagct tctcagacta tccacctttg ggtcgctttg ctgttcgtga 120
tatgagacag acagttgcgg tgggtgtcat caaagcagtg gacaagaagg ctgctggagc 180
tggcaaggtc accaagtctg cccagaaagc tcagaaggct aaatgaatat tatccctaat 240
acctgccacc ccactcttaa tcagtggtgg aagaacggtc tcagaactgt ttgtttcaat 300
tggccattta agtttaagta gtaaaagact ggttaatgat aacaatgcat cgtaaaacct 360
tcagaaggaa aggagaatgt tttgtggacc actttggttt tcttttttgc gtgtggcagt 420
tttaagttat tagtttttaa aatcagtact ttttaatgga aacaacttga ccaaaaattt 480
gtcacagaat tttgagaccc attaaaaaag ntaaatggg 519

<210> 99
<211> 873
<212> DNA
<213> Homo Sapiens

<400> 100
ctgggctctg ggctagtact ggggagtatc tgcagaatcc cgtgatatga tccgtcttca 60
gctaaagata ttatttcaca agtggaatga cagctgactt ctcaacaaca acgaaagcaa 120
ggagacagtt gaaagacatc ttgaaaatgg aattagcagt tcacaaagca cattcgcata 180
taagggcttg ttttgaattg atcttggcag caattctatg aaacaagtaa aagcacaaga 240
ggaataggaa ctgcacctct tccttcagtt tcagcttgaa taatatcagg aagattcgta 300
tcggtctgag ttgggtcacg tacccgacgt gctatagctg aggatggggt aagctgattg 360
gagtttgcaa cactgttcac agagccaaga tatggaaaga acctaaatgt caactggtgg 420
atgaatggat aaagaaattg tggtatatac atacactgga atattattca accttaaaaa 480
gaaggaaatc ctaacatttg tgacaacatg gatggacctg gagggaatta tgctgagtga 540
aataagacag acncaaaaga cntttcttgc aggagctcct tatatgtgga atctaaatag 600
tcagcttaaa gaaganagta aactactggt gtcaggagca gganaaaatg gaaatgaana 660
ggngatagta aagggacaaa gttcagtatc aanataataa gttctggngg ttactattaa 720
tantccatag acctataata ccatactggt tggtactaaa atgctaaagg gtttctaatg 780
tctaccanan aaaananang gaaaataagg gcggaggccc tnaaagggag gatgtatgcc 840
tgnggggaag gtctgaaatc tncccactat gng 873

<210> 101
<211> 832
<212> DNA
<213> Homo Sapiens

<400> 101
gacatacaaa aagctgtaca tatttaatat ttacatctca attagtttgg ggataagtat 60
actctcatga aaccatcacc accatcaagg ccataaacat atccatcacc ttttgaagtg 120
tcctcctgcc ccttaattat taccattatt attattatta ttggtaagaa catataagat 180
ataccctctt agcaatttta agtatacaat acagtattgn tacttatagg tactatgtga 240
tatattaata gtaaacctcc agaacttatt tatcttgtat aactgaaact ttgtaccctt 300
taactatcac ctcttcattt ccacttttct cctgctcctg acaaccagta gtctactctc 360
ttctttaagc ttgactattt tagattccac atataagtga gctcctgcaa gaaagncttt 420
ttgngtctgc ttatttcact cagcataatt ccctccangt ccatccatgt tgtccaaatg 480
gtaggatttc cttcttttta aggtgaataa tattccagtg tatgnatata ccacaatttc 540
ttatncattc ttcaccagtg acattaaggt ctttctatct tggctntggg aacagggtgc 600
aaactccaat caacttaccc atcctaacta tagacgtngg tacggaccca ctaaacgaac 660
gaanttctgn ntattaactg aactgangan aggggagtcc atncttggct ttactggtca 720
aaaatgnggc anacaataaa aaccttttgc aagggttggg acgtatcatt nanagnttac 780
nggtctgttc tgggtgnanc attntcntgg aaattttgcg aanggcantc gg 832

<210> 102
<211> 436
<212> DNA
<213> Homo Sapiens

<400> 102
cttttatttg ctgagatatt gttctaatcc actgagtcag atttggttgg tctgaaaaat 60
ttaacctgtt gttaaaaata tttcttggag gaagcagcag aggaataaca gtattactca 120
agcattcaca aagggggcaa aggaattctc cgttttctac atcatagctc gtatgtaagc 180
gtaatctctg ttgccttcgc tgttctttag cttgaacgga atcaaaatac ctttgccaac 240
aatgggcatg cataatgtgc ccacagctac tagtgtgtgt tccacaagac agatcagggt 300
gcatgaataa tggatcatat ttttctggat cttgaataaa tttacttctg ttttttgata 360
atacagttga tctctgaaca aatgctgcca agaccattgc cctgctttcc actttaactt 420
cttgctcctc ttgaca 436

<210> 103
<211> 944

<212>  DNA
<213> Homo Sapiens

<400>  103
gtggcttgga ggggttaaga gacttatcaa agatcttggg gctaggtagt agaaaaacag      60
aaaaaaaatc aggtttttca actgcagtca gtactttttt aacaaattaa aatatatcaa     120
atctgtttct cctaggtacc taaaggccta aaaatccatc aacacaggga tatatattag     180
aaaaccatac caagataaaa tgcaaaggtc aagaaaatag aaatgttaaa actccttttg     240
tatgtcatgt atttccacag ttttgtggtg aagaagtatg aatttaggga actggatact     300
agagagaaag gaatcatctc ccttcacttg ctaaggaatt gctggtgccc tgggccacaa     360
gaagggtgtg atttgggggg actgtgtgca attaaacagg aaaggaaata acagacttaa     420
agtattaagt cattctgatg cttatcaaca agagtaaagc acagcctaat aaataaatat     480
atttgagaat ctatattaat ccagacagaa tgaccaagag gcttgatgtc ctggnaataa     540
ccacatgaaa cctttttatt naaggactac cacttatgaa atatgaaaga attccttana     600
caaatccaat cttanatctg nattctnaac attttctccc tttccatttt gaatgctaat     660
attagaagca ttntaaagta atttnggccg gcccatggct taccctggaa ctcggacttt     720
ggaggccagt gggaggactg ttgaggcnaa ntttaaaacc ncnnggcana ttnngaaanc     780
tgggcatttt taaaanngat nggaactttt tnccccccna ntanaacaat nttccnnccc     840
ttaaacccna acctttcccn gggttttncc ctaagggncc cttttntttg aaacccaaaa     900
ggtncntttg ggttncangn atttnaaatt nttttngncc cnaa                      944

<210> 104
<211>  568
<212>  DNA
<213> Homo Sapiens

<400>  104
caggtttctg acctgggctg cagggtgaaa gatagtacta ttagttgaaa tagataatac      60
agagagaaga gtaaatttgg gaagaacata ttttgtttat gttgagttcg tgcctgtgag     120
atacaggtgg aggtacctag agacaattct gtagaagtct gaagtttagg agagagggca     180
gagttggaga taaaatttta ggttgtaagc ctatcataaa tagttaaaat ggtgagaatg     240
agggagattg ctcagggcag tggttcacaa attttgagtt tttgtgtaca aaaataaact     300
tggaataaat accacatgtt ctcacccata tatggaagct gaaaaaaaaa tgagctcata     360
gaagtacaga gtagaatcta gaatcatggt cattagaggt tgggaagggt agtgaggaga     420
ggagaatagg cagaggtanc agatacanag ttacagctgt tagggaggaa aaagttcagn     480
gcttttgnac catgcncccc tgantntgnn caaataattn agngttnttt cnaccgctan     540
aaaaaggatt ttgaatttcc cncccnaa                                        568

<210>  105
<211>  256
<212>  DNA
<213> Homo Sapiens

<400>  105
ggcatannct cgntttgtna ncaggctgga tggagtgcag ngatntnann tnactgnaac      60
cgccacctcc cgggttnaag cnattccctg actcancctg tannccanta nctgggacta     120
caggcntgcc ccaccttgcc cggntaattt tttttttttt nggattttan taaaaacggg     180
gtttnaccat nttggccana atggncttga tctcctgacc ttgggattac ccccacctng     240
gcctcccaaa gggntg                                                     256

<210>  106
<211>
<212>  DNA
<213> Homo Sapiens

<400>  106

taaatttttt tactctctct acaaggtttt ttcctagtgt ccaaagagct gttcctcttt      60
ggactaacag ttaaatttac aaggggattt agagggttct gtgggcaaat ttaaagttga     120
actaagattc tatcttggac aaccagctat caccaggctc ggtaggtttg tcgcctctac     180
ctataaatct tcccactatt ttgctacata gacgggtgtg ctcttttagc tgttcttagg     240
tagctcgtct ggtttcgggg gtcttagctt tggctctcct tgcaaagtta tttctagtta     300
attcattatg cagaaggtat aggggttagt ccttgctata ttatgcttgg ttataatttt     360

tcatctttc                                                          369

<210>  107
<211>
<212>  DNA
<213> Homo Sapiens

<400>  107
aatctatcac cctatagaag aactaatgtt agtataagta acatgaaaac attctcctcc      60
gcataagcct gcgtcagatt aaaacactga actgacaatt aacagcccaa tatctacaat     120
caaccaacaa gtcattatta ccctcactgt caacccaaca caggcatgct cataaggaaa     180
ggttaaaaaa agtaaaagga actcggcaaa tcttaccccg cctgtttacc aaaaacatca     240
cctctagcat caccagtatt agaggcaccg cctgcccagt gacacatgtt taacggccgc     300
ggtaccctaa ccgtgcaaag gtagcataat cacttgttcc ttaattaggg acctgtatga     360
atggctccac gagggttcag ctgtctctta cttttaacca gtgaaattga cctgcccgcg     420
aagaggcggc ataacacagc aagacgagaa gaccctatgg agctttaatt tattaatgca     480
aacagtacct acaaacccac aggtcctaaa ctaccaaacc tgattaaaaa tttcngttgg     540
ggcgacctcg gagcagaccc accttcgagc agtacatgct aagacttcac cagtcaaagc     600
gactactata ctcaattgat ccaataactt ggncaaccgg aacaagttac ccttanggat     660
aacagcgcaa tcctattcta gagtcctttt aaccataggg gttaccaacc tnaatgttgg     720
atcaaggact tnccatggng caacccgntn ttaagggtcg ttggttaacg ataaaggcct     780
ccggaactgn gttaaaccgg ngtaatccaa                                      810

<210>  108
<211>
<212>  DNA
<213> Homo Sapiens

<400>  108
gcattggggt gggggtaagg tgcatctgtt tgaaaagtaa acgataaaat gtggattaaa      60
gtgcccagca cagagcagat cctcaataaa catttcattt cccacccaca ctcgccagct     120
caccccatca tccctttccc ttggtgccct cctttttttt ttatcctagt cattcttccc     180
taatcttcca cttgagtgtc aagctgacct tgctgatggt gacattgcac ctggatgtac     240
tatccaatct gtgatgacat tccctgctaa taaaagacaa cataactc                  288

<210>  109
<211>  735
<212>  DNA
<213> Homo Sapiens

<400>  109
ctaattacta ccttttattc taatgtgaac catggccctg aaagctgata acaagcttgg      60
ctgagcagag ggaactaggg gtcggcagaa aggattatgg gtggaaaaca ttggctcttc     120
cttggggagt gatgctgggg aaagggaaga gagtggctca gcctgcaggt aaataggcta     180
gaaaagccaa ggccaaaggc tggaggggag aggacagtca gcatgtccag cctggggtct     240
gggtgtaggg ttatcccttc tccctgtgcc ttcccatctc gtccatgagc ctaggccttg     300
gagccttgtg ttggaggctg ctgtgatgtc aggaacgggg atctgctagc ttttggccac     360
ttcctgggac ctcacgcccc tgttgacaga tggagattgg gcagcagggc cttgctgcat     420
tggtatctgc tgttccactt ggttggcttg ccaaggtgac gaaagaccag gcaccanggt     480
ctcatgggat gaaggacagg gtgggaagat gggggaaggg ctggggctta agggagcaag     540
aaagcttgta cctgtgtngg gccggcagga tgttaaaaac cgctttgntg ttttaaaatg     600
gggactgggc ccaaatcctg ttgggcaccc anncccaaaa nacgggtcct ccanttccaa     660
ggganntttn gggggaaccn naangggctt ttttccagga angccngttt tttnaaacng     720
ganccntggg cattc                                                      735

<210>  110
<211>  1002
<212>  DNA
<213> Homo Sapiens

<400>  110
cgggattccg gtggcaacgt tgctggtgac agcaaaaatg acccaccaat ggaagcagct      60
ggcttcactg ctcaggtgat tatcctgaac catccaggcc aaataagcgc cggctatgcc     120
cctgtattgg attgccacac ggctcacatt gcatgcaagt ttgctgagct gaaggaaaag     180

attgatcgcc gttctggtaa aaagctggaa gatggcccta aattcttgaa gtctggtgat 240
gctgccattg ttgatatggt tcctggcaag cccatgtgtg ttgagagctt ctcagactat 300
ccacctttgg gtcgctttgc tgttcgtgat atgagacaga cagttgcggt gggtgtcatc 360
aaagcagtgg acaagaaggc tgctggagct ggcaaggtca ccaagtctgc ccagaaagct 420
cagaaggcta aatgaatatt atccctaata cctggcaccc actcttaatc agtggtggaa 480
gaacgtctca gaactgttgg ttcaattggn cattaagttt aatagtaaaa gactgggtaa 540
tgatacaatg catcgtaaaa ccttcagaag gaaaggagaa tgtttgtgga ccactttggg 600
tttccttttt gcgtgngcan ttttaagtat tagnttttaa aacagncttt taatggnaca 660
cttgnccnaa aatttgccca aattttggaa ccctttaaaa agttaatggg aaaaaaaaac 720
ggattccggg ggtaccttcc aaaactttta aaaancnggc ccgcattttt tctgaggggt 780
aacnngttcc ccataattcc cccngggana agcntntnnc tttngggacc nttttgnanc 840
cccnttttaa ggccccccnt tttaacaacc ccccccttgc ntggacnnan aaannncggn 900
tttttatttt tangaacaaa ccnttnggtt cnaanccctt ggtcnccccg gggggtnncn 960
aaaatttttt tccccntttt tnnggggnaa attngggaaa tt 1002

<210> 111
<211> 1002
<212> DNA
<213> Homo Sapiens

<400> 111
cgggattccg gtggcaacgt tgctggtgac agcaaaaatg acccaccaat ggaagcagct 60
ggcttcactg ctcaggtgat tatcctgaac catccaggcc aaataagcgc cggctatgcc 120
cctgtattgg attgccacac ggctcacatt gcatgcaagt ttgctgagct gaaggaaaag 180
attgatcgcc gttctggtaa aaagctggaa gatggcccta aattcttgaa gtctggtgat 240
gctgccattg ttgatatggt tcctggcaag cccatgtgtg ttgagagctt ctcagactat 300
ccacctttgg gtcgctttgc tgttcgtgat atgagacaga cagttgcggt gggtgtcatc 360
aaagcagtgg acaagaaggc tgctggagct ggcaaggtca ccaagtctgc ccagaaagct 420
cagaaggcta aatgaatatt atccctaata cctggcaccc actcttaatc agtggtggaa 480
gaacgtctca gaactgttgg ttcaattggn cattaagttt aatagtaaaa gactgggtaa 540
tgatacaatg catcgtaaaa ccttcagaag gaaaggagaa tgtttgtgga ccactttggg 600
tttccttttt gcgtgngcan ttttaagtat tagnttttaa aacagncttt taatggnaca 660
cttgnccnaa aatttgccca aattttggaa ccctttaaaa agttaatggg aaaaaaaaac 720
ggattccggg ggtaccttcc aaaactttta aaaancnggc ccgcattttt tctgaggggt 780
aacnngttcc ccataattcc cccngggana agcntntnnc tttngggacc nttttgnanc 840
cccnttttaa ggccccccnt tttaacaacc ccccccttgc ntggacnnan aaannncggn 900
tttttatttt tangaacaaa ccnttnggtt cnaanccctt ggtcnccccg gggggtnncn 960
aaaatttttt tccccntttt tnnggggnaa attngggaaa tt 1002

<210> 112
<211> 925
<212> DNA
<213> Homo Sapiens

<400> 112
gctttaatat acgctattgg agctggaatt accgcggctg ctggcaccag acttgccctc 60
caatggatcc tcgttaaagg atttaaagtg gactcattcc aattacaggg cctcgaaaga 120
gtcctgtatt gttatttttc gtcactacct ccccgggtcg ggagtgggta atttgcgcgc 180
ctgctgcctt ccttggatgt ggtagccgtt tctcaggctc cctctccgga atcgaaccct 240
gattccccgt cacccgtggt caccatggta ggcacggcga ctaccatcga aagttgatag 300
ggcagacgtt cgaatgggtc gtcgccgcac ggggggcgtg cgatcggccc gaggttatct 360
agagtcacca aagccgccgg cgcccgcccc cggccggggc cggagagggg ctgaccgggt 420
tggttttgat ctgataaatg cacgcatccc ccccgcgaag ggggtcaagc gcccgtcggc 480
atgtattaac tctagaatta ccacagttat ncaagtagga nangagcgag cgaccaaagg 540
aaccntactg gattaatgag ccntttccag tttcactgta ccggncgtgc nanttaaaca 600
tgcattggnt taatctttga gacaagcata tgctantggc anggtttttt tatggnaaag 660
atgntttatt ggnggcagta ctacaaggca ttaatattgg tncccaaaaa aaaactcggt 720
nttattaaat antgggcntt aanacntaat gaacttgacc aacnnttgct ggatnnctga 780
ntcctcctgg tttttgggaa agnaacccac cactattttt ggcantcttt tcnccacttg 840
aaaanaaggg ggtttntngg nggcttantt ccnnctttaa ncnggaattt tanccctnga 900
annttgtttt ccgaactttt taaaa 925

<210> 113
<211> 589
<212> DNA

<213> Homo Sapiens

<400> 4
aagggaaaga tgaaaaatta taaccaagca taatatagca aggactaacc cctatacctt 60
ctgcataatg aattaactag aaataacttt gcaaggagag ccaaagctaa gacccccgaa 120
accagacgag ctacctaaga acagctaaaa gagcacaccc gtctatgtag caaaatagtg 180
ggaagattta taggtagagg cgacaaacct accgagcctg gtgatagctg gttgtccaag 240
atagaatctt agttcaactt taaatttgcc cacagaaccc tctaaatccc cttgtaaatt 300
taactgttag tccaaagagg aacagctctt tggacactag gaaaaaacct tgtagagaga 360
gtaaaaaatt taccgccgat actgacgggc tccaggagtc gtcgccacca atcccaaggg 420
cgaattccag cacactggcg gncgttacta gtggatccga ctcggtacca agcttgatgc 480
atagcttgag tattctatag tgcacctaaa tagcttggcg taatcatggn catactgttc 540
tgngtgaaaa tggtatccgt nacaatttca cacacatacg agccggagc 589

<210> 114
<211> 516
<212> DNA
<213> Homo Sapiens

<400> 114
tcaagaggag caagaagtta aagtggaaag cagggcaatg gtcttggcag catttgttca 60
gagatcaact gtattatcaa aaaacagaag taaatttatt caagatccag aaaaatatga 120
tccattattc atgcaccctg atctgtcttg tggaacacac actagtagct gtgggcacat 180
tatgcatgcc cattgttggc aaaggtattt tgattccgtt caagctaaag aacagcgaag 240
gcaacagaga ttacgcttac atacgagcta tgatgtagaa aacggagaat tcctttgccc 300
cctttgtgaa tgcttgagta atactgntat tcctctgctg cttctccaag aaatattttt 360
aacaacaggt taaatttttc agaccaacca aatctgactc agtggattag aacaatatct 420
cagcaaataa aagcggaatt ccagctgagc gccggcgcta ccattaccag ttggtctggg 480
gcaaaaataa taattaccgg gcaggccatg tcaagg 516

<210> 115
<211> 965
<212> DNA
<213> Homo Sapiens

<400> 115
gaaatgcatt cttatgttat accaaaacac atattcatag tagttctatt tatagttgcc 60
ccaaactagg tcaatcaaat cttcaaaaaa agtaaaatag ttaattcatg gtcacaaaac 120
atacatattt cataatttca tttgtataaa cctcaaaagc aaaaccaatc tatggtattt 180
caagtcaaga ttgtggttac ctttaaggga gaaaatagca actgggaaaa ggtatgaggg 240
gggattctag ggtgctggta acgatctgtt tcttgatttg ggtgctggct atatatgttc 300
actattcatt ttttaaaaat agacacaggg tctcactatg ttgcccaggc tggcttaaac 360
tcttggctca agcagtcctc ccacctcggc ctcccaaagt gccgagatta caggtgtgag 420
ccactgcccc ggccgagatt tacttttata atgactctaa tatttagcat tcaaaattgt 480
gaaaggggag aaagattctg agaaatacag aatctaaaat gggattgnct aagtaatctt 540
tcatattcat aagttgtagn cttaaataaa aaggttcatg tggtantacc aggacatcan 600
cctctggtca ttctggctgg ataatataga tctcaaatat attaattatt agncgggctt 660
tactctggtg ataanactcn naangctaat actttaagnt ggnattcctt tctggtaatg 720
gnacagtccc caantaaacc nttttgngcc anggnccaca ttcntacagg gaagggaaaa 780
anccttttnt tagntcaatc ctaatcactt ttccccaaat ggggannctg cntccaaggn 840
ntaanntttt tttngccttn ntttnatngg nggnttaaaa aanccccggn nnggtttngc 900
cttngcccgn aaaanttttt ttttnnaaaa anncnngtnt aaaccntttt tttttaaaag 960
gganc 965

<210> 116
<211> 974
<212> DNA
<213> Homo Sapiens

<400> 116
gtgttgtgaa tattcaaaat cctctcttct agctgtttga aaatatacac taaattattg 60
tgagcaatat tcaggctacc atgctacaga gcactgaact ttttcctccc taacagctgt 120
aactttgtat ctgttacctc tgcctattct cctctcctca ctacccttcc caacctctaa 180
tgaccatgat tctagattct actctgtact tctatgagct catttttttt tcagcttcca 240

tatatgggtg agaacatgtg gtatttattc caagtttatt tttgtacaca aaaactcaaa 300
atttgtgaac cactgcctga gcaatctccc tcattctcac cattttaact atttatgata 360
ggcttacaac ctaaaatttt atctccaact ctgcctctct cctaaacttc agacttctac 420
agaattgctc taggtacctt cacctgtatc tacaggcacg aactcacata aacaaaatat 480
gttcttccca aatttactct tctctctgga taactatttc aactaatagt ctatctttca 540
ccctggagcc agtcagaaac ctgcagcact ttgggaggcc gaggtggggg taatcacaag 600
gtcaggagat caagaccatc ctggccaaca tggtgaaacc ccgtctntac taaaatccaa 660
aaaaaaaaaa aattagccgg gcaaggtggc gcatgcctgt agtcccagct actggactac 720
aggctgagtc agggaatcgc ttgaacccgg gaggtggcgg ttgcagtgag ctgagatcac 780
tgcactccat ccagcctgct gacagagcga gactatgcct caaaaaaaaa ananaaaaan 840
aananaaaan aaaacnnaaa aaanaaaaaa naaaaaccag ttgancgccg gncgntacca 900
ttccaggggg tctggggtca aaaatantaa tanccgggca ggccatntca agggcgaatt 960
ntgcagatat ccat 974

<210> 117
<211> 411
<212> DNA
<213> Homo Sapiens

<400> 117
cagcactttg ggaggccgag gtgggggtaa tcacaaggtc aggagatcaa gaccatcctg 60
gccaacatgg tgaaaccccg tctntactaa aatccaaaaa aaaaaaaaat tagccgggca 120
aggtggcgca tgcctgtagt cccagctact ggactacagg ctgagtcagg gaatcgcttg 180
aacccgggag gtggcggttg cagtgagctg agatcactgc actccatcca gcctgctgac 240
agagcgagac tatgcctcaa aaaaaaaana naaaaanaan anaaaanaaa acnnaaaaaa 300
naaaaaanaa aaaccagttg ancgccggnc gntaccattc cagggggtct ggggtcaaaa 360
atantaatan ccgggcaggc catntcaagg gcgaattntg cagatatcca t 411

<210> 118
<211>
<212> DNA
<213> Homo Sapiens

<400> 118
gaaagatgaa aaattataac caagcataat atagcaagga ctaaccccta taccttctgc 60
ataatgaatt aactagaaat aactttgcaa ggagagccaa agctaagacc cccgaaacca 120
gacgagctac ctaagaacag ctaaaagagc acacccgtct atgtagcaaa atagtgggaa 180
gatttatagg tagaggcgac aaacctaccg agcctggtga tagctggttg tccaagatag 240
aatcttagtt caactttaaa tttgcccaca gaaccctcta aatccccttg taaatttaac 300
tgttagtcca aagaggaaca gctctttgga cactaggaaa aaaccttgta gagagagtaa 360
aaaaatttta 369

<210> 119
<211> 288
<212> DNA
<213> Homo Sapiens

<400> 119
gagttatgtt gtcttttatt agcagggaat gtcatcacag attggatagt acatccaggt 60
gcaatgtcac catcagcaag gtcagcttga cactcaagtg gaagattagg gaagaatgac 120
taggataaaa aaaaaaggag ggcaccaagg gaaagggatg atggggtgag ctggcgagtg 180
tgggtgggaa atgaaatgtt tattgaggat ctgctctgtg ctgggcactt taatccacat 240
tttatcgttt acttttcaaa cagatgcacc ttacccccac cccaatgc 288

<210> 120
<211> 1028
<212> DNA
<213> Homo Sapiens

<400> 120
cacaggagga gaagcaggag ctgtcgggaa gatcagaagc cagtcatgga tgaccagcgc 60
gaccttatct ccaacaatga gcaactgccc atgctgggcc ggcgccctgg ggccccggag 120
agcaagtgca gccgcggagc cctgtacaca ggcttttcca tcctggtgac tctgctcctc 180

gctggccagg ccaccaccgc ctacttcctg taccagcagc agggccggct ggacaaactg 240
acagtcacct cccagaacct gcagctggag aacctgcgca tgaagcttcc caagcctccc 300
aagcctgtga gcaagatgcg catggccacc ccgctgctga tgcaggcgct gccatgggag 360
ccctgcccag gggcccatgc agaatgccac caagtatggc aacatgacag aggaccatgt 420
gatgcacctg ctccagaatg ctgaccccct gaaggtgtac ccgccactga aggggagctt 480
cccggagaac ctgagacacc ttaagaacac catggagacc atagactgga aggtctttga 540
nagctggatg caccatttgg cttctgttga aatgagcang cacttctttg gacaaaagcc 600
cacttgacgc ttcancgaag agtcacttgg aactggagga ccgtctttng gctggtgtga 660
ccaacaggat ctgggccaat ncccattgaa acaacanaag cggctttaaa atcttgcggg 720
cccanaaagt tcaantttnt tggttcctta ggcccaancc ttcccaattt tcnacttggn 780
cctaatccat gaaaactggn gcnnggtntt tntnancctt ggnaagaaaa acaattggaa 840
cancgataac atgcnnaagg cctngtggcc aaattctttt taanangggc tagggcccna 900
aanggcaaaa ttnaaaaacc ctnntgaata aaanatttaa naaaggtnan ggttngtntt 960
gncaaatgga angcccngna agggaacctc cccnaccnan ngganntgna ngnttccnca 1020
antggctt 1028

<210> 121
<211> 930
<212> DNA
<213> Homo Sapiens

<400> 121
cggagttccg ggtatctggg ctccaggcag aagcacagcc tccccgacct gccctacgac 60
tacggcgccc tggaacctca catcaacgcg cagatcatgc agctgcacca cagcaagcac 120
cacgcggcct acgtgaacaa cctgaacgtc accgaggaga agtaccagga ggcgttggcc 180
aagggagatg ttacagccca gatagctctt cagcctgcac tgaagttcaa tggtggtggt 240
catatcaatc atagcatttt ctggacaaac ctcagcccta acggtggtgg agaacccaaa 300
ggggagttgc tggaagccat caaacgtgac tttggttcct ttgacaagtt taaggagaag 360
ctgacggctg catctgttgg tgtccaaggc tcaggttggg gttggcttgg tttcaataag 420
gaacggggac acttacaaat tgctgcttgt ccaaatcagg atccactgca aggaacaaca 480
ggccttattc cactgctggg gattgatgtg tgggagcacg cttactacct tcagtataaa 540
aatgtcaggc ctgattatct aaaagctatt tggaatgtaa tcaaccggaa ttccgttttt 600
ttttttctca tttaactttt ttaatgggct caaaattctg ngacaaantt ttggcaagtg 660
tttccattaa aaagtntgat ttaaaactaa tacttaaaat tgcncacccn aaaanggaaa 720
accaagtggt cccaaacatt ctctttcttn taaggttaca ngcntggtnt attaaccact 780
tttctctaac ttaangccat tgaacaacat tttaaacgtt tcnccngtta aaangggggg 840
nggttngggn aaatnnttac ctttgacttt tggnnaantt gggacttcnn ttcnaacttt 900
ttccnggttt naccccccaa ngnggttttc 930

<210> 122
<211> 444
<212> DNA
<213> Homo Sapiens

<400> 122
actccggtct gaactcagat cacgtaggac tttaatcgtt gaacaaacga acctttaata 60
gcggctgcac cattgggatg tcctgatcca acatcgaggt cgtaaaccct attgttgata 120
tggactctag aataggattg cgctgttatc cctagggtaa cttgttccgt tggtcaagtt 180
attggatcaa ttgagtatag tagttcgctt tgactggtga agtcttagca tgtactgctc 240
ggaggttggg ttctgctccg aggtcgcccc aaccgaaatt tttaatgcag gtttggtagt 300
ttaggacctg tgggtttgtt aggtactgtt tgcattaata aattaaagct ccatagggtc 360
ttctcgtctt gctgtgtcat gcccgctctt cacggcaggt caatttcact ggttaaaagt 420
aagagacagc tgaaccctcg tgga 444

<210> 123
<211> 767
<212> DNA
<213> Homo Sapiens

<400> 123
cattttcgtt ggtggtgttc agttgtggcg gttgctggtc agtaacagcc aagatgctgc 60
ggaatctgct ggctcttcgt cagattgggc agaggacgat aagcactgct tcccgcaggc 120
attttaaaaa taaagttccg gagaagcaaa aactgttcca ggaggatgat gaaattccac 180
tgtatctaaa gggtggggta gctgatgccc tcctgtatag agccaccatg attcttacag 240

ttggtggaac agcatatgcc atatatgagc tggctgtggc ttcatttccc aagaagcagg 300
agtgacttca gtcatcccag caatcgcttg gttcagtttc attcagctct ctatggacca 360
gtaatctgat aaataaccga gctcttcttt ggggatcaat atttattgac ttgtagtaac 420
tgccaccaat aaagcagtct ttaccataaa aaaaaacctg ccagtagcat atgcttgnct 480
caaagattaa gccatgcatg tctaagtacg cacggccggt acagtgaaac tgcgaatggc 540
tcattaaatc agntattggg tcctttggtc gctngctcct ctcctacttg gatactgngg 600
taattctaaa ctaatacatg ccgacgggcg cttacccctt ngcggggggg atcctgcatt 660
tatanatcaa accaacccgg naagcctttt cggcccggcc gggggcggcc nccggngntt 720
ttggngactt taanaacctt nggcccaang acccccccnn gggggga 767

<210> 124
<211> 378
<212> DNA
<213> Homo Sapiens

<400> 124
tttttactct ctctacaagg ttttttccta gtgtccaaag agctgttcct ctttggacta 60
acagttaaat ttacaagggg atttagaggg ttctgtgggc aaatttaaag ttgaactaag 120
attctatctt ggacaaccag ctatcaccag gctcggtagg tttgtcgcct ctacctataa 180
atcttcccac tattttgcta catagacggg tgtgctcttt tagctgttct taggtagctc 240
gtctggtttc gggggtctta gctttggctc tccttgcaaa gttatttcta gttaattcat 300
tatgcagaag gtataggggt tagtccttgc tatattatgc ttggttataa tttttcatct 360
ttcccttgcc gaaattcc 378

<210> 125
<211> 604
<212> DNA
<213> Homo Sapiens

<400> 125
atgtaagtaa gtgtattatg gccagttaag gtaggcacta taaaaatagg ccgaaaagtt 60
tagaatattc cttttttact gtagtctgtt ttttaaaatt tgaaacttgt tagagagttt 120
ggaaaacagt cttcttcctc ccactccact tcctgccaaa aaagagggga agcacaatgg 180
tcttcaaaaa aggtgataaa gtaaatgcat attataaaat attttaaact tttgtgtgtg 240
tggtttcacg tacaggaaat gaacatgcaa attcttagaa actgttgtca ctgtgtttct 300
gaaatgctaa aaaaaattat gctttgagct acctgctgct tataattcct ttccctgaat 360
aggtaggttt ttatagttaa caaattttaa atgtaagttg attttgatag tagtatttca 420
ttatgcaatc tggagaggag agaagtgttt ttcataaagt ggatattaat tacaactttn 480
aaaagccaat cagtaaacat tcattgatct tgnaataact gngaccctaa ttaaaagggt 540
gctaggcttg tatgcttgga aatatttgaa atttttattn ttaaaactgg g 591

<210> 126
<211>
<212> DNA
<213> Homo Sapiens

<400> 126
cagattttaa agaataaaaa aatttcaaat attttccaga cataacagcc tagcaaccat 60
tttaattagg tgtcacagtt aattacaaga tcaatgaatg tttactgatt ggcnttttaa 120
agttgtaatt aatatccact ttatgaaaaa cactctctcc tctccagatt gcataatgaa 180
atactactat caaaatcaac tacatttaaa atttgttaac tataaaaacc tacctattca 240
gggaaaggaa ttataagcag caggtagctc aaagcataat tttttttagc atttcagaaa 300
cacagtgaca acagtttcta agaatttgca tgttcatttc ctgtacgtga aaccacacac 360
acaaaagttt aaaatatttt ataatatgca tttactttat cacctttttt gaagaccatt 420
gngcttnccc tcttttttgg ccaggaagtg ggagtgggag gaagaanact gttttccaac 480
tcttaacagg ttcaaatttt aaaaaacaga ctacngtaaa aanggatatt ctaaactttc 540
ggnctatttt ataggcctac ctaactggcc taatccttac tacatnggat tccnctganc 600
gccg 604

<210> 127
<211> 860
<212> DNA

<213> Homo Sapiens

<400> 127
agaatctggt gacttcagtt gagcccccag cagaggtgac tccatcagag agcagtgaga 60
gcatctccct cgtgacacag atcgccaacc cggccactgc acctgaggca cgagtgctac 120
ccaaggacct gtcccaaaag ctgctagagg catccttgga ggaacagggc ctggctgtgg 180
atgtgggtga gactggaccc tcacccccta ttcactccaa gcccctaacg cctgctggcc 240
acaccggcgg cccagagccc aggcctccag ccagagtaga gactctgagg gaggaggcgc 300
ccacagactt acgggtgttt gagctgaact cggatagtgg gaagtctaca cccttcaaca 360
atggaaagaa aggctcaagc acggacatta atgaggactg ggaaaaagac tttgacttgg 420
acatgactga anaggaagtg canatggcac tttccaagtg gatgcctncn gggagctnga 480
aaattaaaat gggaagactg ggaatgaggg accnnaagga gcanttcccc cccatgggat 540
nttttgcttc ctnctngntt aanccancct ggatgaatga aaatgttccc caaattcttt 600
gcaaccaaac tttggcacaa atttgggggt ncttgttggc cttttggnct ttgttnaccn 660
ggaagggttt tantccggcc aaaattttat ttgccncatt ggngacccng gggaggaact 720
ntctctnccn aaaacggttt ttntnaaccn tgttcttang atnttttgaa ccnaggaatt 780
tncctttctg tnaaaaaana acccnntttt nngaanngna antnttnntt ttnnnggggg 840
gggnnccctc cttgtaaaag 860

<210> 127
<211> 899
<212> DNA
<213> Homo Sapiens

<400> 127
aaaggaagga ggtgggtcag ggtttggtct ctggattctg aaccccaaag gagcctttcc 60
aggaatggaa aatgcctggg agggggagag tcccaagaga ggcaaatttc ccagagataa 120
gtgcctctta cccactggga taggaaccaa aatgtgttca ctgtccctgt ttagccaagg 180
gtaggtggca tggccctccc tgcctgctta tgtatggaca gagtatgttg tctcagcttc 240
ctccgagaga gactggtggt ttagcttctg tctacacagg cagaagggct agaactatcc 300
cttgggactt tccagcagga gtcctcanga acagtgggtg ttcancagaa aaacacangc 360
tcttctggtg aggaggatag gtttcctctt ccttgggtca tcctattgtt ggcacaagtc 420
aaagtttttg gccgggattt anaaagcccc ttccaggtgt gagcanaagc ccaaaanggc 480
cancagggaa ccccaaattg tcccaaactt ttgttgcaaa aganatttgg gggaacattn 540
tcantcattc aggctggctt anacaaccan ggangcaaaa atgccttggt gggggagntg 600
ttcctttggn ttccttattc cannncttcc attttaattt tnaacttccc ggagnatccc 660
ttttgnaagn ccntttcncc tcttttnatc atttncaann aaannttttt ccancctact 720
ntntccggct taaccttttt tnttnttggn gggggnnatt ccctttcnnt tanttaaaaa 780
cccnanttnn ggcccncncc tcaanttttt ttnttaacct nnntttgncc ccntgnccna 840
ncntnggctn gataaatngg gngggnnatt tncccatncn acannctntt ttannattt 899

<210> 128
<211> 552
<212> DNA
<213> Homo Sapiens

<400> 128
atcccaggaa aatttggagg aacagctgct ctccactggc ctgctcctgc aagaatgccc 60
tggagcttct gaagaaggat ctatatttac cttatagggc cttaagtcct gggatggaac 120
tatatacttt ggccgcgatg atgtggcttt gaagaacttt gccaaatact ttcttcacca 180
atctcatgag gagagggaac atgctgagaa actgatgaag ctgcagaacc aacgaggtgg 240
ccgaatcttc cttcaggata tcaagaaacc agactgtgat tgactgngag agccgggctg 300
aatgcaatgg agtgtgcatt accatttngg aaaaaaaatg tgaatcantc acttactggg 360
acctgnacaa ctngccaact gacaaaaatg acncccattt gtgtgacttt attnganan c 420
attacctgga atganccggt gaaaaaccct tnaaagaant ttgngtgacc acatttcnca 480
aaattncaca nnaatngnan gccccccgna tatggcttgn ataggaatan tcntttntga 540
caagcacacc ct 552

<210> 129
<211> 401
<212> DNA
<213> Homo Sapiens

<400> 129

agaaattgaa acctggcgca atagatatag taccgcaagg gaaagatgaa aaattataac 60
caagcataat atagcaagga ctaaccccta taccttctgc ataatgaatt aactagaaat 120
aactttgcaa ggagagccaa agctaagacc cccgaaacca gacgagctac ctaagaacag 180
ctaagagagc acacccgtct atgtagcaaa atagtgggaa gatttatagg tagaggcgac 240
aaacctaccg agcctggtga tagctggttg tccaagatag aatcttagtt caactttaaa 300
tttgcccaca gaaccctcta aatccccttg taaatttaac tgttaagtcc aaagaggaac 360
agctctttgg acactaggaa aaaaccttgt agagaagaag t 401

<210> 130
<211> 412
<212> DNA
<213> Homo Sapiens

<400> 130
gttaaatttt ttactctctc tacaaggttt tttcctagtg tccaaagagc tgttcctctt 60
tggactaaca gttaaattta caaggggatt tagagggttc tgtgggcaaa tttaaagttg 120
aactaagatt ctatcttgga caaccagcta tcaccaggct cggtaggttt gtcgcctcta 180
cctataaatc ttcccactat tttgctacat agacgggtgt gctctcttag ctgttcttag 240
gtagctcgtc tggtttcggg ggtcttagct ttggctctcc ttgcaaagtt atttctagtt 300
aattcattat gcagaaggta taggggttag gtccttgcta tattatgctt gggntataat 360
ttttcatctt tcccttgcgg nactatatct attgcgccag gtttcaattt ct 412

<210> 131
<211> 497
<212> DNA
<213> Homo Sapiens

<400> 131
caaacccact ccaccttact accagacaac cttagccaaa ccatttaccc aaataaagta 60
taggcgatag aaattgaaac ctggcgcaat agatatagta ccgcaaggga aagatgaaaa 120
attatagcca agcataatat agcaaggact aacccctata ccttctgcat aatgaattaa 180
ctagaaataa ctttgcaagg agagccaaag ctaagacccc cgaaaccaga cgagctacct 240
aagaacagct aaaagagcac acccgtctat gtagcaaaat agtgggaaga tttataggta 300
gaggcgacaa acctaccgag cctggtgata gctggttgtc caagatagaa tcttagttca 360
actttaaatt tgcccacaga accctctaaa tccccttgta aatttaactg ttagtccaaa 420
gaggaacagc tctttggaca ctaggaaaaa accttgtaga gagagtaaaa aattaacacc 480
catagtaggc ctaaaag 497

<210> 132
<211> 841
<212> DNA
<213> Homo Sapiens

<400> 132
agatcgttat gcccgagttc cggtacagga acgtcggtca tccagatgcc ctcttccgct 60
ttcagtttgg ataacgcttt catctcacat cctcaggcga taacgcccag ttgtttacca 120
atacgcgtaa atgcttctac tgcacgcgta atttgctcag gggtatgcgc cgcagacatc 180
tgggtacgaa tacgcgcctg acctttcgga acgaccggat agaagaaacc ggtaacgtaa 240
atgccctctt tttgcagctc acgggcaaat ttctgcgcca ctaccgcatc accaagcatg 300
accggaataa tggcgtgatc ggttccgcag ggtaaagccc gccgncgaca tttgctcacg 360
gaactgacgc gcgttcgcca cagacggtca cgcagttcgc tgcccgcttc gaccatcttc 420
agtactttga tggacgccgn aacaatggnc ggtgccagcg aatttggaga acangtacng 480
accaanaacc ttggcgcaag ccactcaanc actttttttg cgcgcccgcg gnataacccc 540
ccagaagccc cggnccaang cttttaccaa gcgtacccgg ngataatatt tgaacccggg 600
ccattaanat tgcaannttt attgggaacc ncgaacattt ttaaccgnca aaaaccaacc 660
ccntnggaaa tnttngccnc caattcccan gggggaaatt ttngnaaatt cnttnaaact 720
ggggggccgt ttaacatgcc ttttaanggg cccaattnnc ccnttanggg gcgnttacaa 780
atnactnggc cggnnttttn aaacnnnnga atngggnaaa cccgggggtt cccaacttaa 840
a 841

<210> 133
<211> 700
<212> DNA
<213> Homo Sapiens

<400> 133
cattgattga atagttataa agatgttata gtaaatttat tttattttag atattaaatg 60
atgttttatt agataaattt caatcagggt ttttagatta aacaaacaaa caattgggta 120
cccagttaaa ttttcatttc agataaacaa caaataattt tttagtataa gtacattatt 180
gtttatctga aattttaatt gaactaacaa tcctagtttg atactcccag tcttgtcatt 240
gccagctgtg ttggtagtgc tgtgttgaat tacggaataa tgagttagaa ctattaaaac 300
agccaaaact ccacagtcaa tattagtaat ttcttgctgg ttgaaacttg tttattatgt 360
caaatagatt cttataatat tatttaaatg actgcatttt taaatacaag gctttatatt 420
tttaacttta agatgttttt atgtgctctn caaatttttt ttactggttc tgattgnatg 480
gaaatataaa agtaaatatg aaacatttaa aatataattg gtggggcatt tttaattaag 540
nttggtttat ttaagnttaa ggtaattcca tgctggggtt cantagaaca ttccgaatct 600
ggatctgngg ntccagcaga tattccnnan tacaaattan cttcaagtcc ccttctggac 660
caaaaaggtn accaccaang angggaggaa tnaaggggaa 700

<210> 134
<211> 221
<212> DNA
<213> Homo Sapiens

<400> 134
ccagntgacc nccggncgtt accnttacca gtnggtntgg ngtnaaaaat aatantaacc 60
ggncaggccn tntnangggc aaattntgna aatntccntn anantggcgg ccgttcnanc 120
ntgcntttaa agggccnant tcnccntata gggagtcgtn ttananttna ntggccgtng 180
tttnanaacg tcgnnantgg naaaaccntg gngttaccca a 221

<210> 135
<211> 956
<212> DNA
<213> Homo Sapiens

<400> 135
cggtattccg aaaaaatgtt tccaactccg ctgaaatgtt gctgaaaagc atggtgctgg 60
taacagttca acaatccgtg gctgctcatt cttgcctact ttactctccc actgaagcag 120
gttagcgttg aaggtggtat ggaaaagcct gcatgcctgt tcaattcttt tgtttcttct 180
ccttccccct ccccctacct ccttcccctc actcctcccc tccttcgctc gctcaacctc 240
ttttgttcag tatgtgtaac ttgaagctaa tttgtactac tggatatctg actggagcca 300
cagatacaga atctgtattg ttcttactga aacacagcat ggaattaaca ttaaacttaa 360
ataaaacaaa cctaaattaa aaatgcccaa caaattatat tttaaatgtt tcatatttac 420
ttttatattt ccatacaatc agaaacagta aaaaaaattt ggagagcaca taaaaacatc 480
ttaaagttaa aaatataaag ccttgtattt aaaaatgcag tcatttaaat aatattataa 540
gaatctattt gnacataata aacaagtttc aaccagcaag aaattactaa tattgactgt 600
ggagttttgg ctggttaata gttctaactc antattccgt aatcaacaca agcactacca 660
acacaagntg gcaatgacaa gaatgggaag tntcaaacta ggatggtaag tcaattaaaa 720
nttcagataa ccataatgna cttatactaa aaaattattt tgggggttat ttgaaaanga 780
aaattaactg ggggncccaa ttggttggtt gggtaaattt aaaacccngg ttggaaatta 840
tctaataaac nttcnttnaa tactnaaaaa aaataaattn ccttaccact ttttaccntt 900
tcatnaaggg ggaattcnat taaccccgng gttncatttn caatggggtg gggggc 956

<210> 136
<211> 325
<212> DNA
<213> Homo Sapiens

<400> 136
gaggcncagg tgggggtnnt tacanngtna tgatgattaa tnaccattct gnccaacatg 60
gtnaancccn gtntctacta aaatccaaaa annnnaaaat tagccggnca aggtggngca 120
tgcctgtagt cccagctact ggactacagg ctgantnagg gaatcccttg aacccggnag 180
gtggcggttg cagnganctg agatcactgc actcnatcca gnctgctgac anatcnagac 240
tatgcctcaa aaaanggggt ttaaccatnt tgnccnaaaa ggnnttnana ncctaanctt 300
gnnaaaaccc ccntgatggc cgttc 325

<210> 137
<211> 234

<212> DNA
<213> Homo Sapiens

<400> 137
ccnannctga cgggntcnan nantngnccc cnccaatccc angggcaaat tccancnnnc 60
tggnggccgt tactagggga nccnanctng gnnccaannt tganncanan ntngngtntt 120
nnanaggggc nccnaaanan ntnggngnaa ncanggncan anctgttncc tggggaaaat 180
tgtnntccnn tnanaattcc ncncaannta cnacccggaa ncntaaaggg taaa 234

<210> 138
<211>
<212> DNA
<213> Homo Sapiens

<400> 138
ggttcccatg aatactgcga tgtgatgggc cgggtcgata ttatcaccgg tacgcttggt 60
aaagcgctgg gcggggcttc tggtggttat accgcggcgc gcaaagaagt ggttgagtgg 120
ctgcgccagc gttctcgtcc gtacctgttc tccaactcgc tggcaccggc cattgttgcc 180
gcgtccatca aagtactgga gatggtcgaa gcgggcagcg aactgcgtga ccgtctgtgg 240
gcgaacgcgc gtcagttccg tgagcaaatg tcggcggcgg gctttaccct ggcgggagcc 300
gatcacgcca ttattccggt catgcttggt gatgcggtag tggcgcagaa atttgcccgt 360
gagctgcaaa aagagggcat ttacgttacc ggtttcttct atccggtcgt tccgaaaggt 420
caggcgcgta ttcgtaccca gatgtctgcg gcgcataccc ctgacaaatt acgcgtgcag 480
tagaagcatt tacgcgtatt ggtaaacaac tgggccgtta tcgcctgagg atgtgagatg 540
aaagcgttat ccaaactgaa aagcggaaga ggcattttgg atgaccgacg ttctgtaccg 600
gaactcggca taacgaatct ggttgattaa aagtccgtaa acagccattn tgcgggaatg 660
acgttcacat ttataactgg ggataagtct ngcnccaatn ccaagg 706

<210> 139
<211>
<212> DNA
<213> Homo Sapiens

<400> 139
ccggcccgtc tcgcccgccg cgccggggag gtggagcacg agcgcacgtg ttaggacccg 60
aaagatggtg aactatgcct gggcagggcg aagccagagg aaactctggt ggaggtccgt 120
agcggtcctg acgtgcaaat cggtcgtccg acctgggtat aggggcgaaa gactaatcga 180
accatctagt agctggttcc ctccgaagtt tccctcagga tagctggcgc tctcgcagac 240
ccgacgcacc cccgccacgc agttttatcc ggtaaagcga atgattagag gtcttggggc 300
cgaaacgatc tcaacctatt ctcaaacttt aaatgggtaa agaagcccgg ctcgctggcg 360
tggagccggc gtggaatgcn antgcctaat gggccacttt tggtaagcan aactggcgct 420
tggggatgaa ccgaacgccg ggttaagggg cccgatgccg acctcat 467

<210> 140
<211> 540
<212> DNA
<213> Homo Sapiens

<400> 140
aaggaatcgt atcgtatgtc cgctatccag aacctccact ctttcgaccc ctttgctgat 60
gcaagtaagg gtgatgacct gcttcctgct ggcactgagg attatatcca tataagaatt 120
caacagagaa acggcaggaa gacccttact actgtccaag ggatcgctga tgattacgat 180
aaaaagaaac tagtgaaggc gtttaagaaa aagtttgcct gcaatggtac tgtaattgag 240
catccggaat atggagaagt aattcagcta cagggtgacc aacgcaagaa catatgccag 300
ttcctcgtag agattggact ggctaaggac gatcagctga aggttcatgg gttttaagtg 360
cttgtggctc actgaagctt aagtgaggat ttccttgcaa tgagtagaat ttcccttctc 420
tcccttgtca caggtttaaa aacctccagc ttgtataatg taaccatttg gggtcccgct 480
tttacttgga ctantgtaac tccttcgtgc cataaactga aacagccatg ctgctatctt 540

<210> 141
<211> 513
<212> DNA
<213> Homo Sapiens

<400> 141
ctgaaaacaa gttttattta aataagggtt taaatacatt acacataaca ttaaaactga 60
aggggaaaaa aaaaccaaaa accagtttgt tacttcacat ggcattgggc agctgctgct 120
attaagttgc aagctctaca gctagctaca tgactgatgg atcagtttga gatttgttcc 180
cttgtcaaaa gtttaactct gatagaaggt tggcctcaca ttctgatgtt tggacatccc 240
ttagctagga tatgtctggt cgaacagacc tttgtggcaa gccagatgtc ctatcacctc 300
gctagcggta agagggcctc tttgagctct gtccacctag tcaggttgga gacaccaggg 360
gatctaccac caaaagctcc cttntagtag tacagctggg cttctgcctt accccatcct 420
ctccttttaa aattcaccga ngactgttca ngtggtaaca ttctttangg tanggaactc 480
ttgnaaangg agagctgagg aggttcccgc cag 513

<210> 142
<211> 533
<212> DNA
<213> Homo Sapiens

<400> 142
gtggagtctg acttagcaag cctcgggtgg gtttgagggt caaatttcta ccaggcttat 60
atccctggtg atgctgcaga attccaggac cacacttgga ggtttaaggc cttccacaag 120
ttacttatcc catatggtgg gtctatggaa aggtgtttcc cagtcctctt tacaccaccg 180
gatcagtggt ctttcaacag atcctaaagg gatggtgaga gggaaactgg agaaaagtat 240
cagatttaga ggccactgaa gaacccatat taaaatgcct ttaagtatgg gctcttcatt 300
catatactaa atatgaacta tgtgccaggc attatttcat atgacagaat acaaacaaat 360
aanatagtga tgctggtcag gcttggtggc tcatgcctgt attccctaaa ctttgggagc 420
ctaaggngan aactccttga actcctaagg ccnggaattc aagaccacct ggataacata 480
ncaagacccc ttctntccna aaaccaaacc caaccaanca nnantgaaan ggg 533

<210> 143
<211> 885
<212> DNA
<213> Homo Sapiens

<400> 143
cttgggattg gtggcgacga ctcctggagc ccgtcagtat cggcggaatt ccggccagag 60
gctgttcacc ttggagacct gctgcggata tgggtacggc ccggcgcgag atttacaccc 120
tctcccccgg attttcaggg gccagcgaga gctcaccgga cgccgccgga accgcgacgc 180
tttccaagac acgggcccct ctctcggggc gaacccattc cagggcgccc tgcccttcac 240
aaagaaaaga gaactctccc cggggctccc gccggcttct ccgggatcgg tcgcgttacc 300
gcactggacg cctcgcggcg cccatctccg ccactccgga ttcggggatc tgaacccgac 360
tccctttcga tcggccgagg gcaacggagg ccatcgcccg tcccttcgga acggcgctcg 420
cccatctctt aggaccgact gacccatgtt caactgctgg ttcacatgga acccttcttc 480
acttcggcct tcaaaagttt tcgtttgaat atttgctact accaccaaga tctgnacctg 540
cgggggttcc acccgggccc gcgccctang ctttaaaggt tnaccgnaac gggccttcta 600
cttntcgcgg ngtaacgtcc ccngggcttc cggggcgggg agcgcggaat ttcaactgac 660
gccggtcgca ccattaccaa ntggtctggn ggcaaaaata anataaccgg gcaggcctgt 720
naacccaatt caacaaatgg gggccgtnct atggatccca actcggncca acttgancat 780
anttgngntt tttangganc naaaancttg gngaannang gnaaactttt cttgnggaat 840
ggtntcgttc aatncccaan aacaaccgaa ctaaagngaa accgg 885

<210> 144
<211>
<212> DNA
<213> Homo Sapiens

<400> 144
gccgaggatg gccgtcatgg cgccccgaac cctcgtcctg ctactctcgg gggccctggc 60
cctgacccag acctgggcag gctcccactc catgaggtat ttctccacat ccgtgtcccg 120
gcccggccgc ggggagcccc gcttcatcgc cgtgggctac gtggacgaca cgcagttcgt 180
gtggttcgac agcgacgccg cgagccagag gatggagccg cgggcgccgt ggatagagca 240
ggaggggccg gagtattggg acgaggagac agggaaagtg aaggcccact cacagactga 300
ccgagagaac ctgcggatcg cgctccgcta ctacaaccag agcgaggccg gttctcacac 360
cctccagatg acgtttggct gcgacgtggg gtcggacggg cgcttcctcc gcgggtacca 420
ccagtaccct acgacggcaa ggattacatc gcctgaaaga agacctgcct cttggaccgg 480

ggnggacatg gcggttaana taacaaacgc aagtgggang cgggccatgn ggg 533

<210> 145
<211> 116
<212> DNA
<213> Homo Sapiens

<400> 145
gatgattggg gagggagcac aggtcagcgt gggaagaggg tcatggtgga catgggggtg 60
gggtggtgct aanacaaggt anagtangan atacttttct tacctnttta tgctga 116

<210> 146
<211> 567
<212> DNA
<213> Homo Sapiens

<400> 146
cttcaacaag atagaaatca ataacaagct ggaatttgag tctgcccagt tccccaactg 60
gtacatcagc acctctcaag cagaaaacat gcccgtcttc ctgggaggga ccaaaggcgg 120
ccaggatata actgacttca ccatgcaatt tgtgtcttcc taaagagagc tgtacccaga 180
gagtcctgtg ctgaatgtgg actcaatccc tagggctggc agaaagggaa cagaaaggtt 240
tttgagtacg gctatagcct ggactttcct gttgtctaca ccaatgccca actgcctgcc 300
ttagggtagt gctaagagga tctcctgtcc atcagccagg acagtcagct ctctcctttc 360
agggccaatc cccagccctt ttgttgagcc aggcctctct cacctctcct actcacttaa 420
agcccgcctg acagaaacca cggccacatt tggttctaag aaaccctctt gtcattcgct 480
cccacattct gatgagcaac ccgtttccta ttaattaatt aatttggtng gttggtttat 540
tcattggcta atttattcaa agggggg 567

<210> 147
<211>
<212> DNA
<213> Homo Sapiens

<400> 147
cagggaagtt tatttcaaaa ccattgaaca gtatgatatt tgctcattta taaatattcc 60
catttaaata atctgagctt atatattttc agtcttaatt aaaggacttg atttaaagag 120
agcacaccag tccaaattga attgattcca tagctattaa aaactaggct cttttacaga 180
cactgctact tcttgccccc tttgaataaa ttagaccaat gaataaaaca aacaaacaaa 240
taaataaata aatagggaag cggttgctca tcanaatgtg ggagcgaatg acagagggtt 300
tcttanaacc aaatgtggcc cgtggtttct gtcaggcggc tttaagtgag taggaaaggt 360
gagagaggcc tgctcaacaa aagggctggg gattggccct gaaagganaa agctgactgc 420
ctgctgatgg acaggaaatc ctttacacta ccctaaggca ggcagtaggc attgggtaaa 480
cacaggaaga cg 492

<210> 148
<211> 567
<212> DNA
<213> Homo Sapiens

<400> 148
cgttcatggg gaataattgc aatccccgat ccccatcacg aatggggttc aacgggttac 60
ccgcgcctgc cggcgtaggg taggcacacg ctgagccagt cagtgtagcg cgcgtgcagc 120
cccggacatc taagggcatc acagacctgt tattgctcaa tctcgggtgg ctgaacgcca 180
cttgtccctc taagaagttg ggggacgccg accgctcggg ggtcgcgtaa ctagttagca 240
tgccagagtc tcgttcgtta tcggaattaa ccagacaaat cgctccacca actaagaacg 300
gccatgcacc accacccacg gaatcgagaa agagctatca atctgtcaat cctgtccgtg 360
tccgggccgg gtgaaggcag tgagctgaga ttgcgccact gcactccagc ctgggcgaca 420
gancgagacc ccatctcaaa aaaaaagggg gggggtggac agggggcaag tggagtctgg 480
cttgccaaaa ctacttgttg atggngggga aaaaaaaatg ggtgncttcc tccttggcac 540
tgggaaaggt tttggttctt ttttcat 567

<210> 149
<211> 512

<212>  DNA
<213> Homo Sapiens

<400>  149
gaaagtttag aaactttaaa acaataataa tgacggtgat agtgataata attgctaatg      60
ctttcagatc acatatgtgt taggcgctgt tttttgttgt tgttgttatt gttgagacag     120
tctcactctg ttggccaggc tggagtgcag tggtgctttg cctcctgggt tcaagggatt     180
ctcctgcctc agcctcctga gtagctggga ttacaggcat gcgccaccac gtcgggctaa     240
tttttgcatt tttagtggag acggggtttc atcatgttgg ccaggctggt ctcgaactca     300
cgacgtcaag tgatccacct gcctcggcct cccaaagtgt tgggattaca ggcgtgagcc     360
accatgccca gccagcactg tcttaaatgc tttacatata ttatctcatt taatcctcaa     420
aataccttac aatatagata ctactattat ttccatttat attaatggca nctctgaggc     480
tcaaacgatg aactacttgc tgggttacat ga                                   512

<210>  150
<211>  572
<212>  DNA
<213> Homo Sapiens

<400>  150
cccaaaaatt acccaaagaa gaagatggaa aagcgatttg tcttcaacaa gatagaaatc      60
aataacaagc tggaatttga gtctgcccag ttccccaact ggtacatcag cacctctcaa     120
gcagaaaaca tgcccgtctt cctgggaggg accaaaggcg gccaggatat aactgacttc     180
accatgcaat ttgtgtcttc ctaaagagag ctgtacccag agagtcctgt gctgaatgtg     240
gactcaatcc ctagggctgg cagaaaggga acagaaaggt ttttgagtac ggctatagcc      300
tggactttcc tgttgtccac accaatgccc aactgcctgc cttaggggta gtgctaagag     360
gatctcctgt ccatcagcca ggacagtcag ctctctcctt tcagggccaa tccccagccc     420
ttttgttgag ccaggcctct ctcacctctc ctactcactt aaagcccgct gacagaaacc     480
acggccacat ttggttctaa gaaaccctct gtcattcgct cccacattct gatgagcaac     540
cgttccctat ttaattattt attggtngtt gg                                   572

<210>  151
<211>
<212>  DNA
<213> Homo Sapiens

<400>  151
gagngaagtt tatttcaaaa ccattgaaca gtatgatatt tgctcattta taaatattcc      60
catttaaata atctgagctt atatattttc agtcttaatt aaaggacttg atttaaagag     120
agcacaccag tccaaattga attgattcca tagctattaa aaactaggct cttttacaga     180
cactgctact tcttgccccc tttgaataaa ttagaccaat gaataaaaca aacaaacaaa     240
taaataaata aatagggaag cggttgctca tcanaatgtg ggagcgaatg acanagggtt     300
tcttanaacc aaatgtggcc cgtggtttct gtcaggcggc tttaagtgan taggaaaggt     360
gaaagaggcc tgctcaacaa aagggctggg gattgccctg aaagganana gctgactggc     420
ctgctgatgg acaggaaacc tttacactac cctaagcngc antggccatt ggtgnggaca     480
caggaaag                                                              488

<210>  152
<211>  488
<212>  DNA
<213> Homo Sapiens

<400>  152
gagngaagtt tatttcaaaa ccattgaaca gtatgatatt tgctcattta taaatattcc      60
catttaaata atctgagctt atatattttc agtcttaatt aaaggacttg atttaaagag     120
agcacaccag tccaaattga attgattcca tagctattaa aaactaggct cttttacaga     180
cactgctact tcttgccccc tttgaataaa ttagaccaat gaataaaaca aacaaacaaa     240
taaataaata aatagggaag cggttgctca tcanaatgtg ggagcgaatg acanagggtt     300
tcttanaacc aaatgtggcc cgtggtttct gtcaggcggc tttaagtgan taggaaaggt     360
gaaagaggcc tgctcaacaa aagggctggg gattgccctg aaagganana gctgactggc     420
ctgctgatgg acaggaaacc tttacactac cctaagcngc antggccatt ggtgnggaca     480
caggaaag                                                              488

<210>  153
<211>

<212>  DNA
<213> Homo Sapiens

<400>  153
gattccaacc ttcacagata actgagtctt gatttgactt caagacttca gtggaggaag      60
taactacaaa tgtggtagaa atagctagat aactagaagt ggtggagcct gaagatctga     120
ctgaattgct gcagtctcat gattaaactt gaacagatga ggatttgctt catatgggtg     180
gatacagaaa gtggtttctt gagatgaaat ctactgctgg cagagatgct gtgaacatcg     240
ttgaaatgac aacaaaggac ttcgaatatc agtaaaatca gttgataaaa ccaaagcagg     300
gtttgagagg atgcactccc aattttgaaa gaagttcttg tgtgggtgaa cgctatcata     360
ccaaacagca tcgcaagcta cagataaatc tttcgtgata gagtcaattg acgtgacaaa     420
cttcattggt ggcattttaa ggcattgcca cagtcacccc aaaacccgca gcagccatca     480
acaacnggca agaccctnca caacaaaaag atga                                 514

<210>  154
<211>  531
<212>  DNA
<213> Homo Sapiens

<400>  154
gtcgttttcc agtttttctt tattacttat ttcattcacc ataattccaa attttaatga      60
ccatatcttt cctaaaatat ctacataaaa atcttgatta tttaagagta aaaagttggt     120
ttctcctcta gctacttctg acctcttcaa taaattgtgc ctgatgctgc ctcctttcct     180
tccaaccact cacattagaa tccttttagt caaagtagtc tgaggctgca gttgtttgca     240
ggatgtgatc atcctcaata ccatattatt tcagagtagc ttaagtcacc attcctaggc     300
aatttcatag taaaaaaatt attctaggaa ttcctggacc tatagatatt tccaagatca     360
ttacaaaaat acttcttttt aaataaaaaa aaattgctaa tgnaccatgc tgggaaattt     420
ttatttaaaa aatagaacta aactcttgag cttcaataat gctggcagat agattctcan     480
ggccttctac tggcctcaag gaaatgatgg cncccctcag tttgggaaag g              531

<210>  155
<211>  539
<212>  DNA
<213> Homo Sapiens

<400>  155
tttgtattat aggaacctat tttgaagctc ttagagctga gagttaagtg gtcttttaat      60
ggaactgcta agacaaggta gagtaggaga tacttttctt ccctctttat gctgaagtgt     120
tttagtgttt ctgtctgtga ctaggcagta actttgaaag ggataagata gggttaataa     180
catatctact aaaacttgga aaatatacta tattttctga gataaaaatc tttggattga     240
aaattacttt ctggtggaat atggcaaact gacattcatt caatgtaaga ctttttttcc     300
ctcacttttt gtgttttcat ctgtagtttt tttttttctt ttttacctgt ggtaccattt     360
ttaaggtgaa tcaggccagt ttcancaaaa aatggntgta ctgttcatca cttcagtaga     420
aggtaggatg acttcgatga nggtgngctc agtaacttct ctggtgctga attagggcct     480
gggacaaana aggatcccat cttacaaata atgacaangg agactacnga atccgggag      539

<210>  156
<211>  562
<212>  DNA
<213> Homo Sapiens

<400>  156
cggaatccgt tttaagatgg agtgtcattc tgtcacccag gttggagtgc agtggcgtga      60
tcatggctca cagcaacctc tgcctcccag gttcaagcaa ttctcctgcc tcagcctcct     120
gagtagctgg gattacaggt gcccgccagc acgcccagct aatttttgta tttttagtag     180
agacagggtt tcaccatgtt ggccaggctg gtctcgaact tttgacctca ggcgatccac     240
ctgtctccgg aattcgggtt acggcagcac ttttattttt ccttacacaa tgacgtgttg     300
ctggggccta atgttctcac ataacagtag aaaaccaaaa tttgttgtca tctcttcaaa     360
gaatcgagaa ttgcgtacaa aaaaaacctt acataaatta agaatgaata catttacagg     420
cgtaaatgca aaccgcttcc aactcaaagc aagtaacagc ccacggtgtt ctggccaaag     480
acatcactaa gaaaggaaac tgggtcctac ggttggactt tncaccctga cagacccgca     540
agacaaaaca actggttctt gc                                              562

<210> 157
<211> 506
<212> DNA
<213> Homo Sapiens

<400> 158
cggaggagca cccagtgctg ctgaccgagg cccccctgaa ccccaaggcc aacagagaga 60
agatgactca gattatgttt gagaccttca acaccccggc catgtacgtg gccatccagg 120
ccgtgctgtc cctctacgcc tctgggcgca ccactggcat tgtcatggac tctggagacg 180
gggtcaccca cacggtgccc atctacgagg gctacgccct cccccacgcc atcctgcgtc 240
tggacctggc tggccgggac ctgaccgact acctcatgaa gatcctcact gagcgaggct 300
acagcttcac caccacggcc gagcgggaaa tcgtgcgcga catcaaggag aagctgtgct 360
acgtcgccct ggacttcgag caggagatgg ccaccgccgc atctcctctt ctctggagaa 420
aactacgact gccgatggca ngtcatacca ttggcatgag cggttcccgg gtccggaggc 480
gctgtncanc cttcttctgg gnatgg 506

<210> 159
<211> 445
<212> DNA
<213> Homo Sapiens

<400> 159
ctttactaaa aatacaaaaa ttagccaggc atggtggcag gtgcctgtaa tcccagctat 60
tcgggaggct gaggcaggag aatcacttga acccaggaag tggaggttgc agtgagccga 120
gatcgtacca ctgcactcca cccagggtga cagagtgaga ctccgttgaa aaaaagagaa 180
aaaaaaatta atacaaagat attaaaatta aaaaggaaaa atatccccag aaccccatca 240
cttaaacaac aaatcaaatt tttatttttc tcttcccatc ctacaaggca acataactct 300
gacctgctta gaatccccgt gtcaggccac tttcctattc tgtttcttcc cactcctcac 360
cgtgcccaca caccttcctt gggggtgaac gcgtgcggac gctagacggc ccctcatccc 420
ccgactgcct gcccgggtgg aactg 445

<210> 160
<211> 445
<212> DNA
<213> Homo Sapiens

<400> 160
cagttccacc cgggcaggca gtcgggggat gaggggccgt ctagcgtccg cacgcgttca 60
cccccaagga aggtgtgtgg gcacggtgag gagtgggaag aaacagaata ggaaagtggc 120
ctgacacggg gattctaagc aggtcagagt tatgttgcct tgtaggatgg gaagagaaaa 180
ataaaaattt gatttgttgt ttaagtgatg gggttctggg gatatttttc ctttttaatt 240
ttaatatctt tgtattaatt tttttttctc tttttttcaa cggagtctca ctctgtcacc 300
ctgggtggag tgcaagtggt acgatctcgg ntcactgnaa cctncacttc ctgggttcaa 360
gtgattctcc tgcctcacct cccgaatagc tgggattaca ggcacctgcc accatgcctg 420
gctaattttt gnatttttag taaag 445

<210> 161
<211> 511
<212> DNA
<213> Homo Sapiens

<400> 161
cttcagcgaa gtttatttca aaaccattga acagtatgat atttgctcat ttataaatat 60
tcccatttaa ataatctgag cttatatatt ttcagtctta attaaaggac ttgatttaaa 120
gagagcacac cagtccaaat tgaattgatt ccatagctat taaaaactag gctcttttac 180
agacactgct acttcttgcc ccctttgaat aaattagacc aatgaataaa acaaacaanc 240
aaataaataa ataaataggg aagcggttgc tcatcanaat gtgggagcga atgacagagg 300
gtttcttana accaaatgtg gccgtggttt ctgtcaggcg gctttaagtg ataggaaagg 360
tgaaaaaggc ctggctcaac aaaagggctg gggattggcc ctgaaaggan aaactgactg 420
cctgctgatg gacaggaaac ctnttaccct cctangcngc nnttgggctt gggggnaaca 480
cngganagcc nggntttacc cgacccnaaa g 511

<210> 162
<211> 534

<212> DNA
<213> Homo Sapiens

<400> 162
cccatcatca atatttattg agcatttaca gtgtactagg cacaatagaa catacagaaa 60
acattgtccc tgctcttgag gagcttacat tctaaaagaa aaaatacacc ttttttaaaa 120
tggcattttt gtttggtgtt ttctgcaaag tactgaggaa atattttgta aagtgagctt 180
tggccttggg cctcaaggaa aagaatctgt acctgtcctg cgtgttgaaa gatgataagc 240
ccactctaca gttggagagt gtagatccca aaaattaccc aaagaagaag atggaaaagc 300
gatttgtctt caacaagata gaaatcaata acaagctgga atttgagtct gcccagttcc 360
ccaactggta catcagcacc tctcaagcan aaaacatgcc cgtctccctg ggagggacca 420
aaggcggcca ggatataact gacttcacca tgcaatttgn gtcttctaaa gaagagctga 480
cccaaaaagt cctgngctga atgnggactc aatccctagg ctgggcanaa aggg 534

<210> 163
<211> 416
<212> DNA
<213> Homo Sapiens

<400> 163
cccttagagc caatccttat cccgaagtta cggatccggc ttgccgactt cccttaccta 60
cattgttcca acatgccaga ggctgttcac cttggagacc tgctgcggat atgggtacgg 120
cccggcgcga gatttacacc ctctcccccg gattttcaag ggccagcgag agctcaccgg 180
acgccgccgg aaccgcgacg ctttccaagg cacgggcccc tctctcgggg cgaacccatt 240
ccagggcgcc ctgcccttca caaagaaaag agaactctcc ccggggctcc cgccggcttc 300
tccgggatcg gtcgcgttac cgcactggac gcctcgcggc gcccatctcc gccactccgg 360
attcggggat ctgaacccga ctccctttca tcggccgagg gcaacggagg ccatcg 416

<210> 164
<211> 369
<212> DNA
<213> Homo Sapiens

<400> 164
gcgatggcct ccgttgccct cggccgatcg aaagggagtc gggttcagat ccccgaatcc 60
ggagtggcgg agatgggcgc cgcgaggcgt ccagtgcggt aacgcgaccg atcccggaga 120
agccggcggg agccccgggg agagttctct tttctttgtg aagggcaggg cgccctggaa 180
tgggttcgcc ccgagagagg ggcccgtgcc ttggaaagcg tncgcggttc cggcggcgtc 240
cggtgagctc tcgctggccc ttgaaatccg gggggagagg gtgtaaatct cgcgccgggc 300
cgtacccata tccgcacagg tctcaaggtg aacagccttg gcatgttgga acaatgtang 360
taagggaag 369

<210> 165
<211> 566
<212> DNA
<213> Homo Sapiens

<400> 165
caaacccact ccaccttact accagacaac cttagccaaa ccatttaccc aaataaagta 60
taggcgatag aaattgaaac ctggcgcaat agatatagta ccgcaaggga aagatgaaaa 120
attatagcca agcataatat agcaaggact aacccctata ccttctgcat aatgaattaa 180
ctagaaataa ctttgcaagg agagccaaag ctaagacccc cgaaaccaga cgagctacct 240
aagaacagct aaaagagcac acccgtctat gtagcaaaat agtgggaaga tttataggta 300
gaggcgacaa acctaccgag cctggtgata gctggttgtc caagatagaa tcttagttca 360
actttaaatt tgcccacaga accctctaaa tccccttgta aatttaactg ttagtccaaa 420
gaggaacagc cctttggaca ctaggaaaaa accttgtaga gagagtaaaa aatttaacac 480
ccatagtagg cctaaaagcc ggaattncag cttgagcgcc ggtcgttcca ttaccagncg 540
gtctgggggt caaaaatata ataacg 566

<210> 166
<211> 492
<212> DNA

<213> Homo Sapiens

<400> 166
gcttttaggc ctactatggg tgttaaattt tttactctct ctacaaggtt ttttcctagt 60
gtccaaaggg ctgttcctct ttggactaac agttaaattt acaaggggat ttagagggtt 120
ctgtgggcaa atttaaagtt gaactaagat tctatcttgg acaaccagct atcaccaggc 180
tcggtaggtt tgtcgcctct acctataaat cttcccacta ttttgctaca tagacgggtg 240
tgctctttta gctgttctta ggtagctcgt ctggtttcgg gggtcttagc tttggctctc 300
cttgcaaagt tatttctagt taattcatta tgcagaaggt ataggggtta gtccttgcta 360
tattatgctt ggctataatt tttcatcttt cccttgcggn actatatcta ttgcgccagg 420
tttcaatttc tatcgctata ctttatttgg gtaaatggtt ggctaanggt gctggtataa 480
gnncagnggg tt 492

<210> 167
<211> 528
<212> DNA
<213> Homo Sapiens

<400> 167
ttgaacgctt tcttaattgg tggctgcttt taggcctact atgggtgtta aattttttac 60
tctctctaca aggttttttc ctagtgtcca aagagctgtt cctctttgga ctaacagtta 120
aatttacaag gggatttaga gggttctgtg ggcaaattta aagttgaact aagattctat 180
cttggacaac cagctatcac caggctcggt aggtttgtcg cctctaccta taaatcttcc 240
cactattttg ctacatagac ggggtgtgct cttttagctg ttcttaggta gctcgtctgg 300
tttcgggggt cttagctttg gctctccttg caaagttatt tctagttaat tcattatgca 360
gaaggatagg ggttaagtcc ttgctatatt atgcttgggt ataatttttc atctttccct 420
tgcggtacta tatctattgc gccaggtttc aatttctatc gcctatactt tatttgggta 480
aatggnttgc taaaggtgnc tggtaataag gtggaatggg tttgcgga 528

<210> 168
<211> 547
<212> DNA
<213> Homo Sapiens

<400> 168
canncccact ncancttact accngacatc cttanccaaa ccatttaccc aaatanagta 60
taggcgatag aaattgaaac ctggcgcaat agatatanta ccgcaaggga aagatgaaaa 120
attataacca agcataatat agcaaggact aacccctata ccttctgcat aatgaattaa 180
ctagaaataa ctttgcaagg agagccaaag ctaagacccc cgaaaccaga cgagctacct 240
aagaacagct aaaagagcac acccgtctat gtagcaaaat agtgggaaga tttataggta 300
gaggcgacaa acctaccgag cctggtgata gctggttgtc caagatagaa tcttagttca 360
actttaaatt tgcccacaga accctctaaa tccccttgta aatttaactg ttagtccaaa 420
gaggaacagc tctttggaca ctaggaaaaa accttgtaga gagagtaaaa aatttaacac 480
ccatagtagg cctaaaagca gccccaatta agaaagcgtc aacggaattn cagctgagcg 540
ccggtcg 547

<210> 169
<211> 718
<212> DNA
<213> Homo Sapiens

<400> 169
gctggaaata cagcaatgaa taggtctcta gtctcctgga acatcaaatg atgtttatcc 60
aaaagtataa atagttacca ttttttattg tcttcttaat aaattgaata aaataatgtc 120
tttgctgcca gtaacatgga tggaactgga agtcactatt ttaagtggaa ttaaagaaaa 180
agaaagtcaa ataccatagg ttctcactta taagtgggag ctaaataatg tatacacata 240
gacgtagagt gtgaaataat agatatcgga gactcagaga attgttttgt ttgaggaggc 300
tgaagatagg accccaatcc cttctagctt gtagggtttc tgctgagaaa tctgtggtta 360
atctaagttt ccctttatag gttacctggt gcttttgctc acagctctta agattctttn 420
cttcgcttaa ctttggctaa cctggtgaca atatgcctan gcgatgatcn ttttgngata 480
aatttttcaa gtggtctttg tgcctaagnc tctagcagac ttggggaagt tttccttgat 540
atttccccaa atatggtttt caagctttan aatctcttct ttctcaggaa ccccgatatt 600
cttaaggttg nccttgagct natcccaant tttttgaggt ttgtnaaatg ggctaaaant 660
nttctttgcn tttnangnat gggntcantt tnaaaacctt gnttttaanc cncgaaat 718

<210> 170
<211> 979
<212> DNA
<213> Homo Sapiens

<400> 170
ctgtgttaga aaaaatcata aaacataaca gaatctacac atcatggtcc accagaggat 60
tcacagatgg aaatgaattt taatattgtt acttttgaag tcccaaatac tttaagattt 120
acaataaaaa acattctgac agagtccatg atgaattatt tccagtcttt caccagactg 180
cttaagctca cctataaact acgaaatgta taaataaata attacagcca aagcaggtaa 240
caaagtgtct aacctatatt ccacaggtgc ataccatggc tacgaataaa ctatccaatc 300
taaccacaga agctgagcat ttggtttggg gttaatccac atcacatgac tcaccattga 360
gaaagcggct ctcaccatgc ttaatgggca cagcacctct gcaaacaaat ccttccctgg 420
ctaatcattc cctctgagag gtttcctcag taaagagatt agaactacct cttgcatttc 480
caacttttaa aaaattgcct ttttggaaat ctaccaccac caactaattc ttgacagact 540
tgtagagaat gaccctcaaa gaaatatcat tcgagacaca tattcaagca gactggncat 600
ggtggctcat gcctgcagtc ccagcagttt gggaagctga agtgaactga tgcttgaatn 660
caggagtctt gagaacagcc tgggtaacat ggnaaaaccg ggtcctacaa aaaaattccn 720
aaaattaccc nggtntgttg gngcacaatg ngggcccaac tttnccnaaa gaaaaaagtt 780
tggcttcagg aaggcaaggg tcncnnancc ctgaatggcc ccttccttca accggggnaa 840
aaangggnaa cctttttggn aagggaaggg aaagggaaag ggagggcctt ttnnntttaa 900
aaaagggann ttaaaaggng gcccnaaaac ntttttaaag ggcaaccttt tttncttttt 960
tgggaaaatt ggggnaaat 979

<210> 171
<211> 718
<212> DNA
<213> Homo Sapiens

<400> 171
aggggatgct ctcggtgtct gagctgttgt tgacagtggc tgggccactg cattcccctc 60
tgggcacctc attcccagag gcatgtaagg cttcagcctc ctccaccatc tcctcctcat 120
ttccgctcac gcccgacgcc tccatctcct catcctccac cacgggcggg aatgcagcct 180
cctcgctggc cgccgccggc gctttcttct tcttcctccg cgcgttcctc tccttctcca 240
tcttcagctt gtgctgctgc aagatctcat cgaggttctg cctcttcttg tagttgaagt 300
agaagttctt acactgcgac acagtcttgg agcccaccat ccgggcgatg gccgaccaag 360
ttgcggccgt gttccaggag acctttcttg gctgnttcca tttcttcttc tgtccagcga 420
gaactctcat tcacttcang gaggccagct cggcgctctg ctggggggtg atggcctcct 480
cgttgtggcc tcattagccc ttgaaccggg tgatgcgggc ctttggnctt ccctggcttg 540
tngcaanttt gcggcctttg gaggccacag cttccttttg gnggttggnc ctcccctgag 600
gggncgctgg cttntccttg agganggctt ccttggggtn ttcacctcgg gttttccctc 660
ttttcgggtt cnttttccgg aatccccnaa ttgacggttc agaatttngc ccaatcca 718